# EUROPÄISCHE PATENTSCHRIFT

(11) **EP 4 122 028 B1**
(45) Veröffentlichungstag und Bekanntmachung des Hinweises auf die Patenterteilung: **16.10.2024**
(21) Anmeldenummer: 21711872.8
(22) Anmeldetag: 16.03.2021
(51) Int. Cl.: C07D 209/86, C07D 487/22, C07F 5/02, H10K 71/12, H10K 85/10, H10K 85/60, H10K 50/11

(54) **HETEROCYCLISCHE VERBINDUNGEN FÜR ORGANISCHE ELEKTROLUMINESZENZVORRICHTUNGEN**
HETEROCYCLIC COMPOUNDS FOR ORGANIC ELECTROLUMINESCENT DEVICES
COMPOSÉS HÉTÉROCYCLIQUES POUR DISPOSITIFS ÉLECTROLUMINESCENTS ORGANIQUES

(30) Priorität: 17.03.2020 EP 20163669
(43) Veröffentlichungstag der Anmeldung: 25.01.2023
(73) Patentinhaber: Merck Patent GmbH, 64293 Darmstadt (DE)
(72) Erfinder: STOESSEL, Philipp, 64293 Darmstadt (DE)
(74) Vertreter: Merck Patent Association
(86) Internationale Anmeldenummer: PCT/EP2021/056675
(87) Internationale Veröffentlichungsnummer: WO 2021/185829

(56) Entgegenhaltungen:
- WO-A1-2019/128633
- US-A1- 2020 066 997

## Beschreibung

Die vorliegende Erfindung betrifft heterocyclische Verbindungen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese heterocyclischen Verbindungen.

In organischen Elektrolumineszenzvorrichtungen werden als emittierende Materialien häufig phosphoreszierende metallorganische Komplexe oder fluoreszierende Verbindungen eingesetzt. Generell gibt es bei Elektrolumineszenzvorrichtungen immer noch Verbesserungsbedarf.

Aus WO 2010/104047 A1, US 2020/066997 A1, und WO 2019/132506 A1 sind polycyclische Verbindungen bekannt, die in organischen Elektrolumineszenzvorrichtungen eingesetzt werden können. Verbindungen gemäß der vorliegenden Erfindung sind nicht offenbart. Weiterhin werden antiaromatische Eigenschaften von Verbindungen von Wang et al., in Nature Communications | 8: 1948 (2017) untersucht. Allerdings wird die Verwendung dieser Verbindungen in organischen Elektrolumineszenzvorrichtungen nicht von Wang et al. beschrieben.

Generell besteht bei diesen heterocyclischen Verbindungen, beispielsweise für die Verwendung als Emitter, insbesondere als fluoreszierender Emitter, noch Verbesserungsbedarf, insbesondere in Bezug auf die Lebensdauer, die Farbreinheit, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen.

Weiterhin sollten die Verbindungen eine ausgezeichnete Verarbeitbarkeit aufweisen, wobei die Verbindungen insbesondere eine gute Löslichkeit zeigen sollten.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Emitter. Insbesondere ist es eine Aufgabe der vorliegenden Erfindung, Emitter bereitzustellen, welche sich für rote, grüne oder blaue Elektrolumineszenzvorrichtungen eignen.

Weiterhin sollten die Verbindungen, insbesondere bei ihrem Einsatz als Emitter in organischen Elektrolumineszenzvorrichtungen, zu Vorrichtungen führen, die eine ausgezeichnete Farbreinheit aufweisen.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot, gelb und blau phosphoreszierende Elektrolumineszenzvorrichtungen eignen.

Weiterhin sollten die Verbindungen, insbesondere bei ihrem Einsatz als Matrixmaterialien, als Lochtransportmaterialien oder als Elektronentransportmaterialien in organischen Elektrolumineszenzvorrichtung zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich sehr gut für die Verwendung in Elektrolumineszenzvorrichtungen eignen und zu organischen Elektrolumineszenzvorrichtungen führen, die insbesondere in Bezug auf die Lebensdauer, der Farbreinheit, der Effizienz und der Betriebsspannung sehr gute Eigenschaften vorweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur der Formel (IIa), (IIb), (IIc), (IId), (IIe), 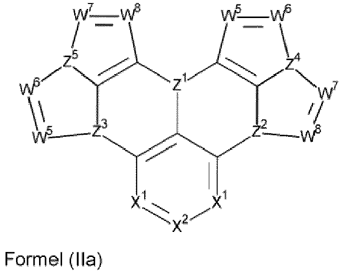 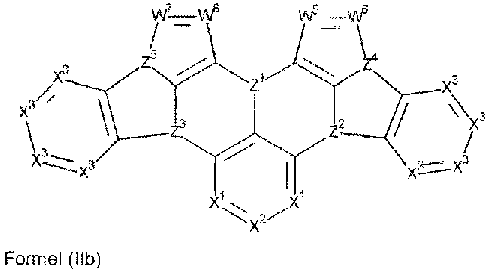 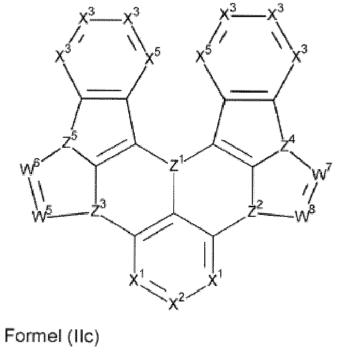 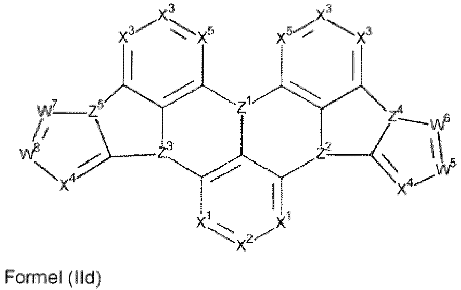 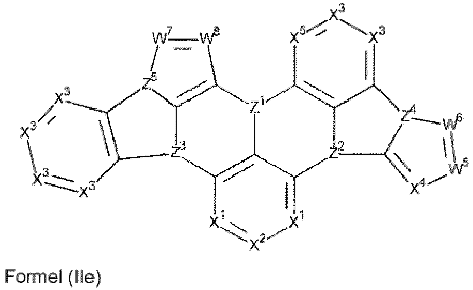 wobei für die verwendeten Symbole und Indizes gilt:
Z¹ ist B;
Z² , Z³ , Z⁴ , Z⁵ ist bei jedem Auftreten gleich oder verschieden N oder B; wobei zwei der Gruppen Z² , Z³ , Z⁴ , Z⁵ für N stehen,
W⁵ , W⁶ , W⁷ , W⁸ steht bei jedem Auftreten gleich oder verschieden für C(Ar) oder X⁶ ;
Ar ist bei jedem Auftreten gleich oder verschieden ein aromatisches oder heteroaromatisches Ringsystem mit 5 bis 60 aromatischen Ringatomen, das mit einem oder mehreren Resten R substituiert sein kann, hierbei kann die Gruppe Ar mit mindestens einer Gruppe Ar, X¹ , R oder einer weiteren Gruppe ein Ringsystem bilden;
X¹ steht bei jedem Auftreten gleich oder verschieden für N, CR^{a} oder C, falls durch eine Bindung mit einem der Ringe Ar^{c}, Ar^{d} oder einer weiteren Gruppe ein Ringsystem gebildet wird, mit der Maßgabe, dass nicht mehr als zwei der Gruppen X¹ , X² in einem Cyclus für N stehen;
X2 steht bei jedem Auftreten gleich oder verschieden für N oder CR^{b} , mit der Maßgabe, dass nicht mehr als zwei der Gruppen X¹ , X² in einem Cyclus für N stehen;
X3 steht bei jedem Auftreten gleich oder verschieden für N oder CR^{c} mit der Maßgabe, dass nicht mehr als zwei der Gruppen X³ , X⁵ in einem Cyclus für N stehen;
X⁴ steht bei jedem Auftreten gleich oder verschieden für N oder CR^{d} mit der Maßgabe, dass nicht mehr als zwei der Gruppen X⁴ , X⁶ in einem Cyclus für N stehen;
X⁵ steht bei jedem Auftreten gleich oder verschieden für N, CR^{e} oder C, falls durch eine Bindung mit einer Gruppe Ar oder einer Gruppe X⁵ oder einer Gruppe X⁶ ein Ringsystem gebildet wird, mit der Maßgabe, dass nicht mehr als zwei der Gruppen X³ , X⁵ in einem Cyclus für N stehen;
X⁶ steht bei jedem Auftreten gleich oder verschieden für N, CR^{f} oder C, falls durch eine Bindung mit einer Gruppe Ar, einer Gruppe X⁵ oder einer Gruppe X⁶ ein Ringsystem gebildet wird, mit der Maßgabe, dass nicht mehr als zwei der Gruppen X⁴ , X⁶ in einem Cyclus für N stehen;
R, R^{a} , R^{b} ist bei jedem Auftreten gleich oder verschieden H, D, OH, F, Cl, Br, I, CN, NO₂ , N(Ar')² , N(R¹)₂ , C(=O)OAr', C(=O)OR¹ , C(=O)N(Ar')₂ , C(=O)N(R¹)₂ , C(Ar')₃ , C(R1)₃ , Si(Ar')₃ , Si(R1)₃ , B(Ar')₂ , B(R¹)₂ , C(=O)Ar', C(=O)R¹ , P(=O)(Ar')₂ , P(=O) (R¹)₂ , P(Ar')₂ , P(R¹)₂ , S(=O)Ar', S(=O)R¹ , S(=O)₂ Ar', S(=O)₂R', OSO₂ Ar', OSO₂ R', eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH2 -Gruppen durch R¹ C=CR¹ , C=C, Si(R¹)₂ , C=O, C=S, C=Se, C=NR¹ , -C(=O)O-, -C(=O)NR¹ -, NR¹ , P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R' substituiert sein kann, oder eine Aryloxy- oder Heteroaryloxygruppe mit 5 bis 60 aromatischen Ringatomen, die durch einen oder mehrere Reste R' substituiert sein kann; dabei können zwei Reste R,
R^{a} , R^{b} auch miteinander oder einer weiteren Gruppe ein Ringsystem bilden;
R^{c}, R^{d} , R^{e} , R^{f} ist bei jedem Auftreten gleich oder verschieden H, D, OH, F, Cl, Br, I, CN, NO2 , N(Ar')2 , N(R')2 , C(=O)OAr', C(=O)OR¹ , C(=O)N(Ar')2 , C(=O)N(R¹)₂ , C(Ar')₃ , C(R¹)₃ , Si(Ar')₃ , Si(R¹)₃ , B(Ar')₂ , B(R¹)₂ , C(=O)Ar', C(=O)R¹ , P(=O)(Ar')2 , P(=O)(R¹)₂ , P(Ar')2 , P(R¹)₂ , S(=O)Ar', S(=O)R¹ , S(=O)2 Ar', S(=O)2 R', OSO2 Ar', OSO₂ R', eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂ -Gruppen durch R¹ C=CR¹ , C=C, Si(R¹)2 , C=O, C=S, C=Se, C=NR¹ , -C(=O)O-,-C(=O)NR¹ -, NR¹ , P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{c} , R^{d} , R^{e} , R^{f} auch miteinander oder einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂ , Si(R¹)₂ , C=O, C=NR¹ , C=C(R¹)2 , O, S, S=O, SO₂, N(R¹), P(R¹) und P(=O)R¹ , miteinander verbrückt sein;
R¹ ist bei jedem Auftreten gleich oder verschieden H, D, F, Cl, Br, I, CN, NO2 , N(Ar")2 , N(R²)₂ , C(=O)OAr", C(=O)OR² , C(=O)Ar", C(=O)R² , P(=O)(Ar")2 , P(Ar")2 , B(Ar")₂ , B(R²)₂ , C(Ar")₃ , C(R²)₃ , Si(Ar")₃ , Si(R²)₃ , eine geradkettige Alkyl-, Alkoxy-oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂ -Gruppen durch -R² C=CR² -, -C=C-, Si(R²)₂ , C=O, C=S, C=Se, C=NR² , -C(=O)O-,-C(=O)NR² -, NR² , P(=O)(R²), -O-, -S-, SO oder SO2 ersetzt sein können und wobei ein oder mehrere H-Atome durch D, F, Cl, Br, I, CN oder NO2 ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere Reste R¹ miteinander ein Ringsystem bilden, dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)2 , Si(R²)2 , C=O, C=NR² , C=C(R²)2 , O, S, S=O, SO₂ , N(R2), P(R2) und P(=O)R² , miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere Substituenten R² miteinander ein Ringsystem bilden.

Eine Arylgruppe im Sinne dieser Erfindung enthält 6 bis 40 C-Atome; eine Heteroarylaruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cvclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsrina mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9`-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

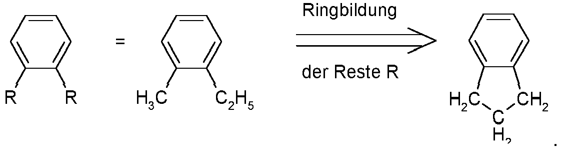

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

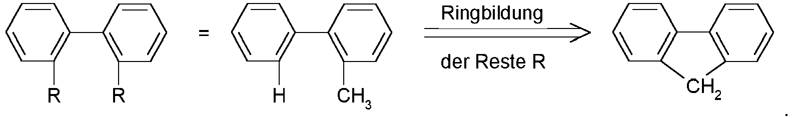

In einer weiterhin bevorzugten Ausgestaltung kann vorgesehen sein, dass die erfindungsgemäßen Verbindungen eine Struktur der Formel (III-1) bis (III-25) umfassen, wobei die erfindungsgemäßen Verbindungen besonders bevorzugt ausgewählt sein können aus den Verbindungen der Formeln (III-1) bis (III-25), 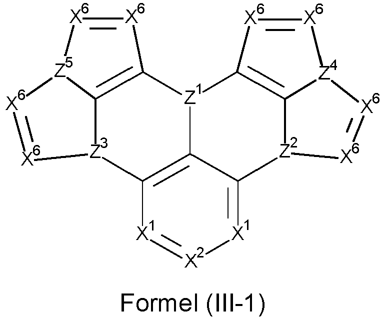 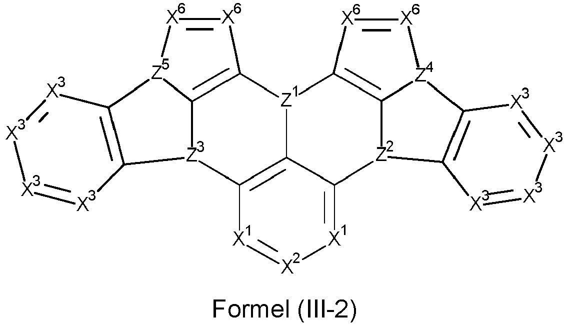 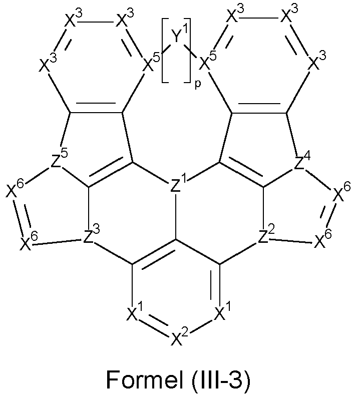 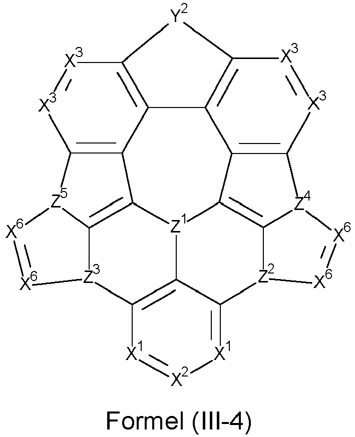 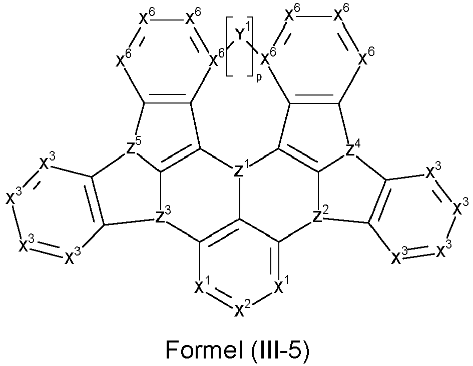 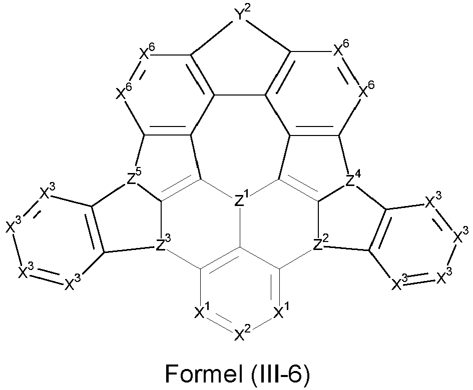 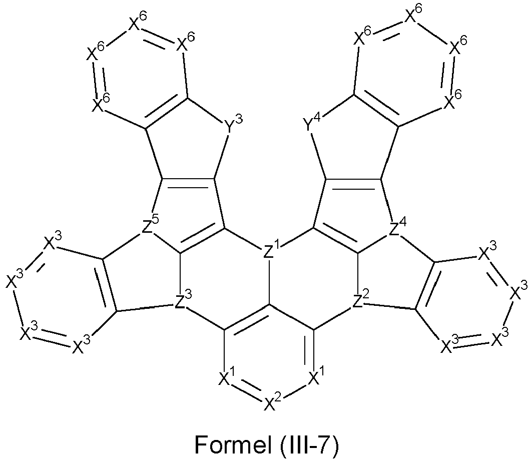 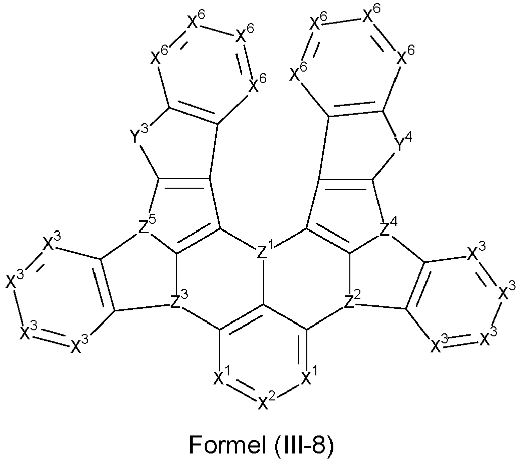 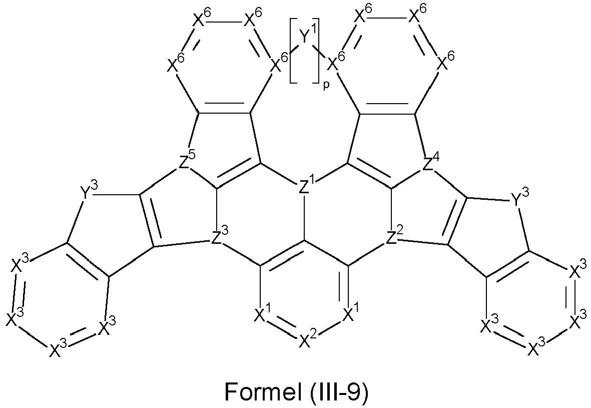 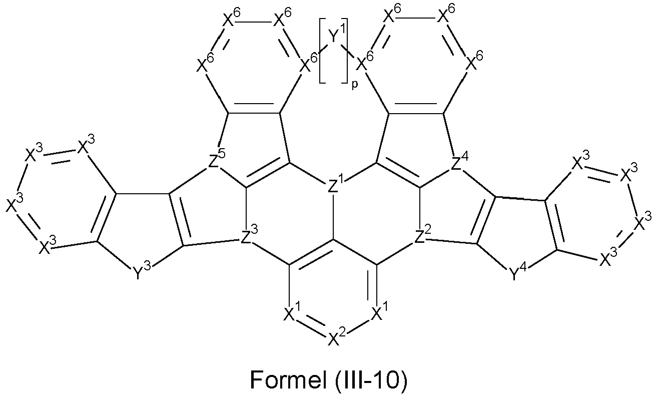 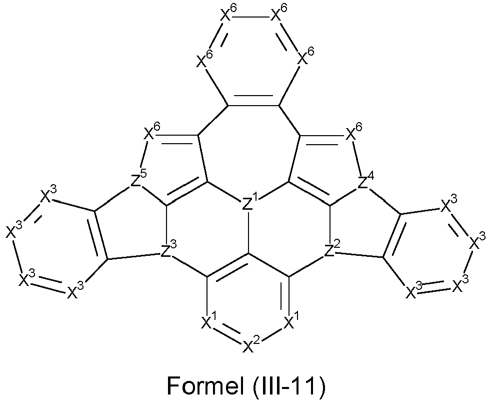 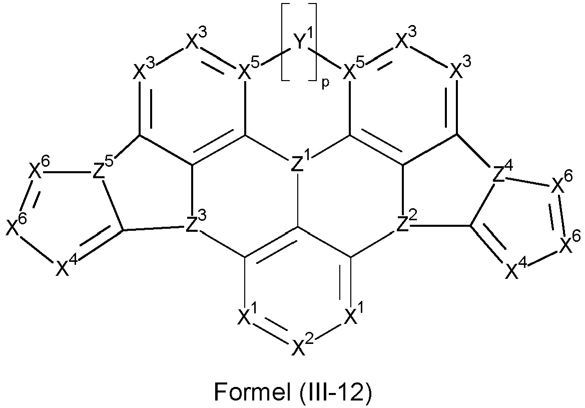 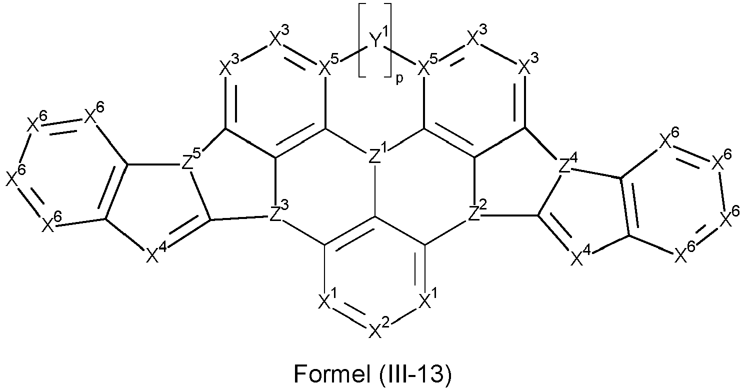 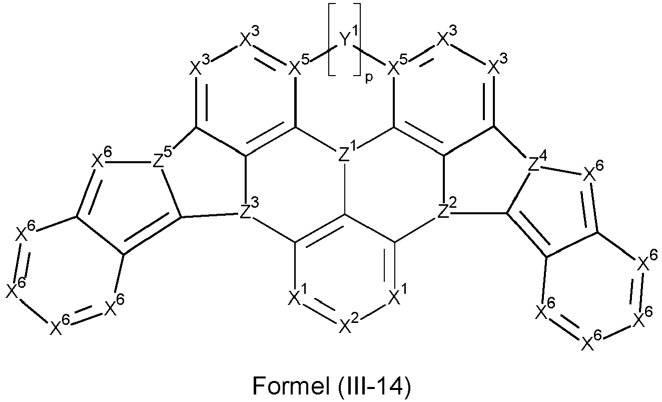 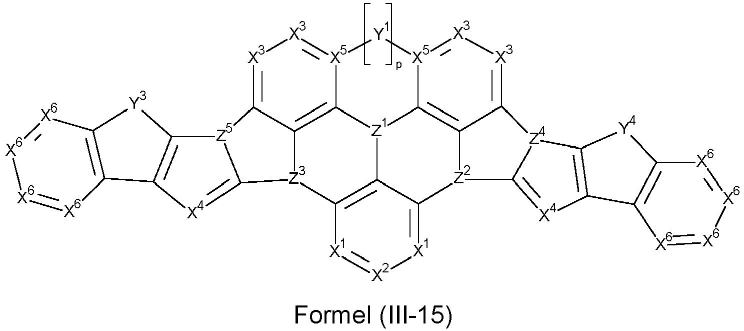 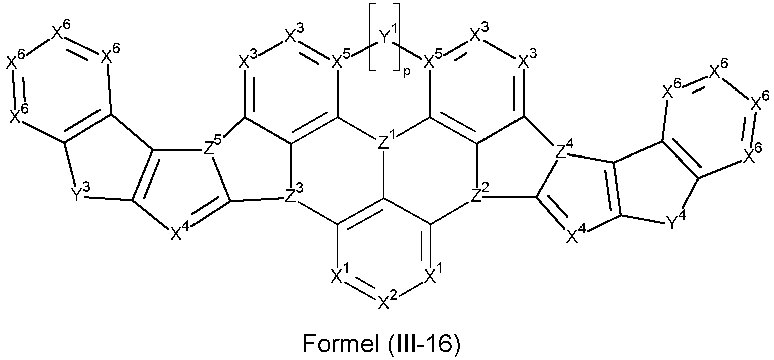 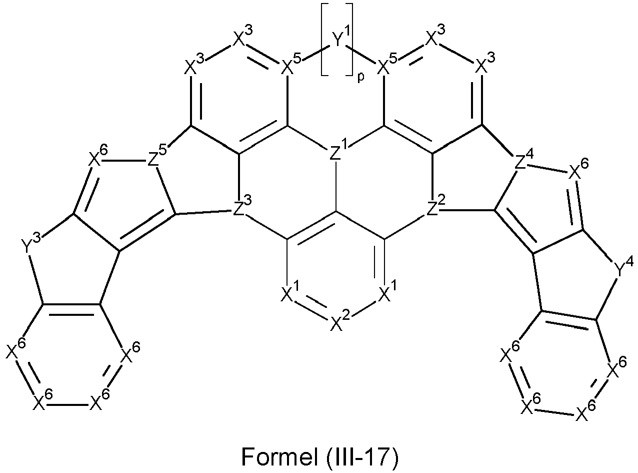 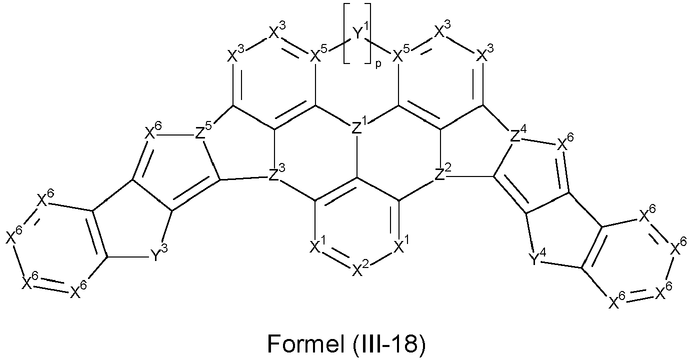 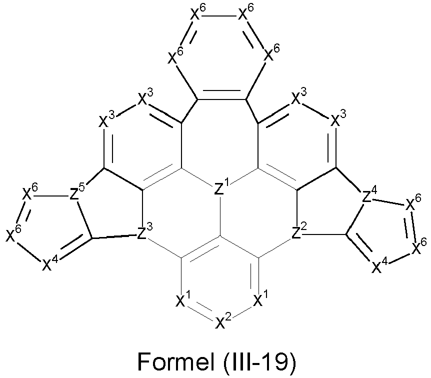 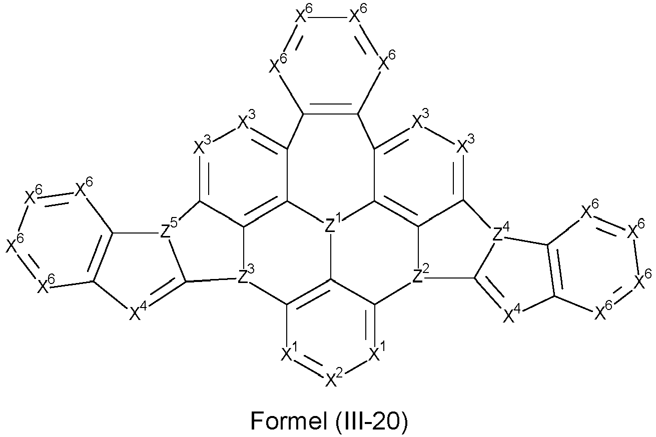 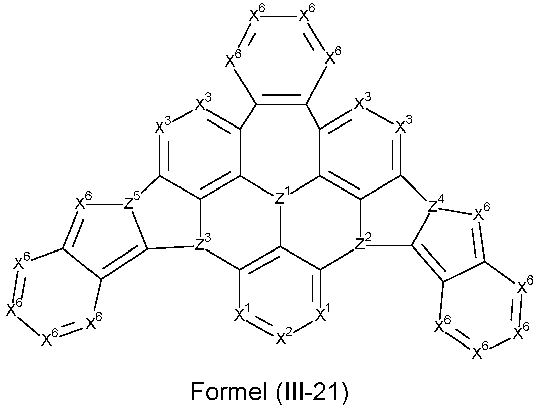 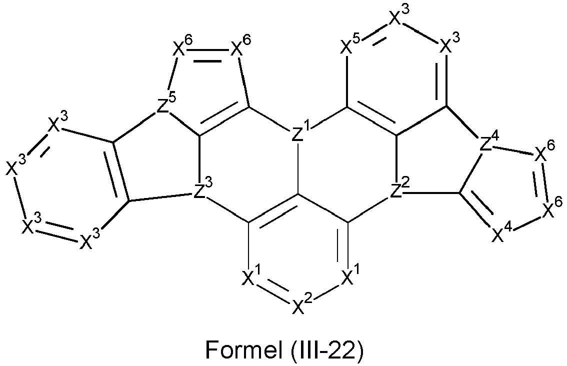 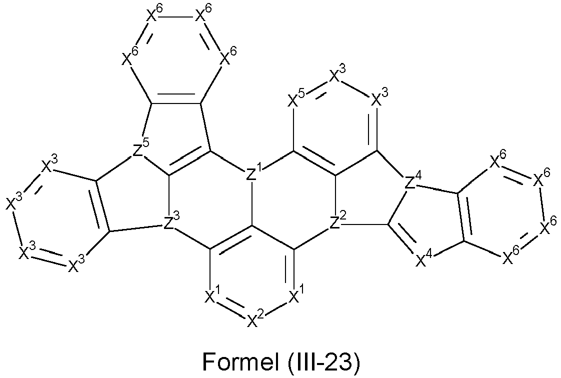 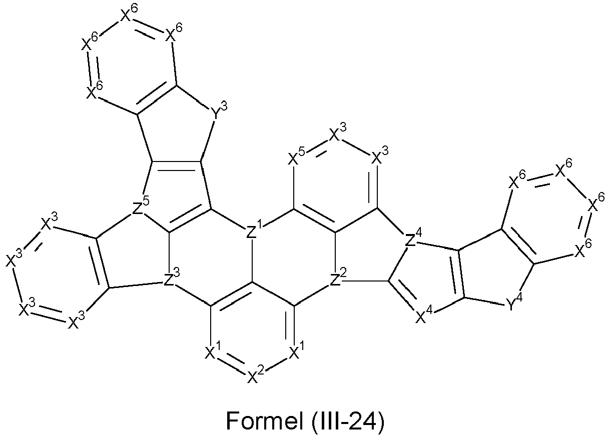 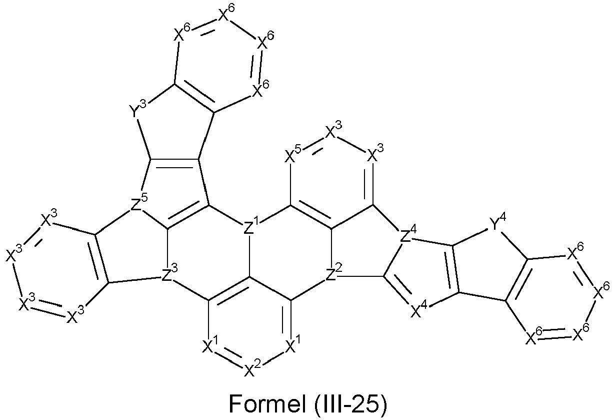
wobei die Symbole Z¹ , Z² , Z³ , X¹ , X² , Z⁴, Z⁵ , X³ , X⁴ , X⁵ und X⁶ die in Anspruch 1 genannten Bedeutungen aufweisen und die weiteren Symbole die folgende Bedeutung aufweisen:
p ist 0 oder 1,
Y¹ ist bei jedem Auftreten gleich oder verschieden eine Bindung, N(Ar'), N(R), P(Ar'), P(R), P(=O)Ar', P(=O)R, P(=S)Ar', P(=S)R, B(Ar'), B(R), Al(Ar'), Al(R), Ga(Ar'), Ga(R), C=O, C(R)2 , Si(R)2 , C=NR, C=NAr', C=C(R)2 , O, S, Se, S=O, oder SO2 , wobei die Symbole R und Ar' die in Anspruch 1 dargelegte Bedeutung aufweisen;
Y² , Y³ , Y⁴ ist bei jedem Auftreten gleich oder verschieden N(Ar'), N(R), P(Ar'), P(R), P(=O)Ar', P(=O)R, P(=S)Ar', P(=S)R, B(Ar'), B(R), AI(Ar'), Al(R), Ga(Ar'), Ga(R), C=O, C(R)2 , Si(R)2 , C=NR, C=NAr', C=C(R)₂ , O, S, Se, S=O, oder SO₂ , wobei die Symbole R und Ar' die in Anspruch 1 dargelegte Bedeutung aufweisen.

Hierbei sind Strukturen/Verbindungen der Formel (III-1) bis (III-21) bevorzugt, Strukturen/Verbindungen der Formel (III-1) bis (III-8) und (III-12) bis (III-18) besonders bevorzugt.

Vorzugsweise kann vorgesehen sein, dass in Formel (IIa) bis (IIe) und/oder (III-1) bis (III-25) nicht mehr als vier, vorzugsweise nicht mehr als zwei Gruppen X¹, X², X³, X⁴, X⁵ und X⁶ für N stehen, besonders bevorzugt alle Gruppen X¹, X², X³, X⁴, X⁵ und X⁶ für CR, CR^{a}, CR^{b}, R^{c}, R^{d}, R^{e}, R^{f} oder, im Falle Gruppe X¹, X⁵ und X⁶, für C stehen, falls die Gruppe X¹, X⁵ und X⁶ durch eine Bindung ein Ringsystem bildet.

Wie in den Beispielen gezeigt, fallen bei einigen Verfahren zur Herstellung erfindungsgemäßer Verbindungen Mischungen an, die im Allgemeinen zu einem verbreiterten Emissionsspektrum führen und daher nicht bevorzugt sind. Aus diesem Grund kann es zweckmäßig sein, diese Mischungen aufzutrennen. Die Vorteile eines schmalen Emissionsspektrums können durch geeignete Substitution erreicht werden, wie dies in den Beispielen gezeigt ist. Hierbei ist insbesondere eine Substitution an Position X³ oder X⁴ zweckmäßig. Ferner kann dieser Vorteil dadurch erreicht werden, dass X³ oder X⁴ an dieser Position für N steht

Vorzugsweise kann vorgesehen sein, dass die Gruppe X⁴ für CR^{d} steht und R^{d} kein H oder D darstellt, vorzugsweise eine geradkettige Alkyl-, Alkoxy- oder Thioalkoxygruppe mit 1 bis 40 C-Atomen oder eine Alkenyl-oder Alkinylgruppe mit 2 bis 40 C-Atomen oder eine verzweigte oder cyclische Alkyl-, Alkoxy- oder Thioalkoxygruppe mit 3 bis 20 C-Atomen ist, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann. Vorzugsweise kann weiterhin vorgesehen sein, dass die Gruppe X⁴ für N steht.

Weiterhin kann vorgesehen sein, dass mindestens eine Gruppe X³ für CR^{c} steht und R^{c} kein H oder D darstellt, vorzugsweise eine geradkettige Alkyl-, Alkoxy- oder Thioalkoxygruppe mit 1 bis 40 C-Atomen oder eine Alkenyl-oder Alkinylgruppe mit 2 bis 40 C-Atomen oder eine verzweigte oder cyclische Alkyl-, Alkoxy- oder Thioalkoxygruppe mit 3 bis 20 C-Atomen ist, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann. Vorzugsweise befindet sich die Gruppe X³, die für CR^{c} steht und R^{c} kein H oder D darstellt, in ortho-Stellung zur Gruppe Z² und/oder Z³. Vorzugsweise kann weiterhin vorgesehen sein, dass die Gruppe X³ für N steht, wobei sich die Gruppe X³, die für N steht, vorzugsweise in ortho-Stellung zur Gruppe Z² und/oder Z³.

In einer weiterhin bevorzugten Ausgestaltung kann vorgesehen sein, dass die erfindungsgemäßen Verbindungen eine Struktur der Formel (IV-1) bis (IV-38) umfassen, wobei die erfindungsgemäßen Verbindungen besonders bevorzugt ausgewählt sein können aus den Verbindungen der Formeln (IV-1) bis (IV-38), 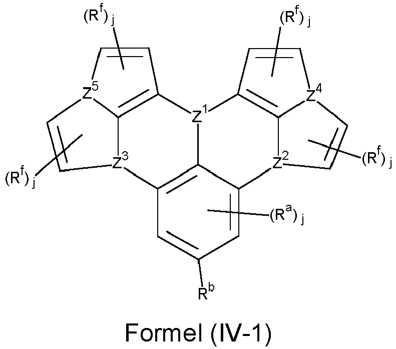 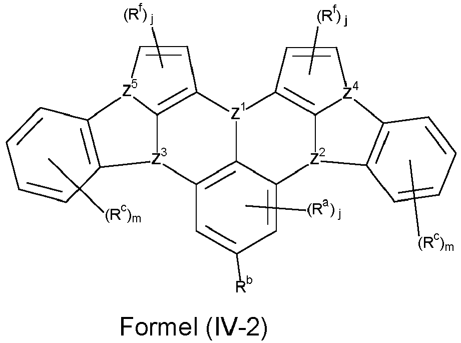 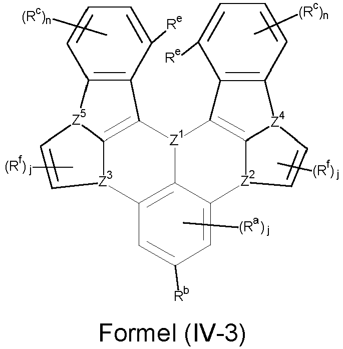 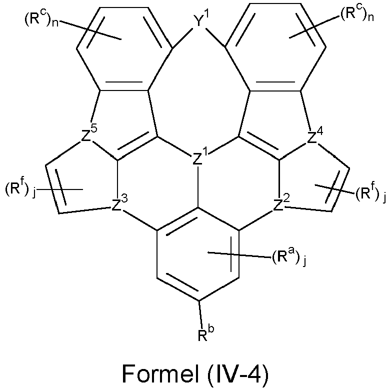 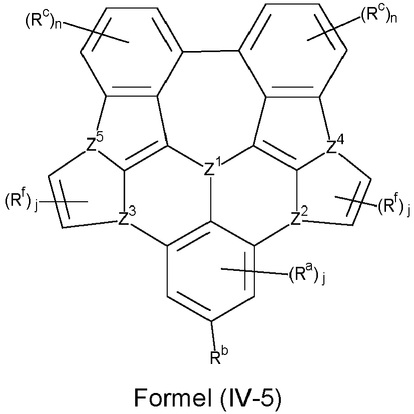 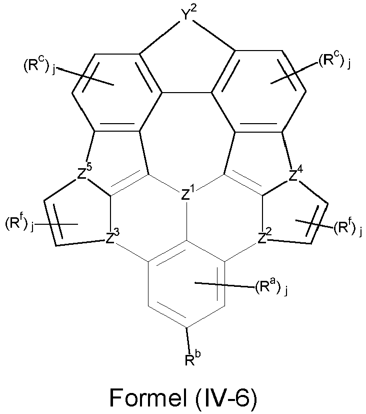 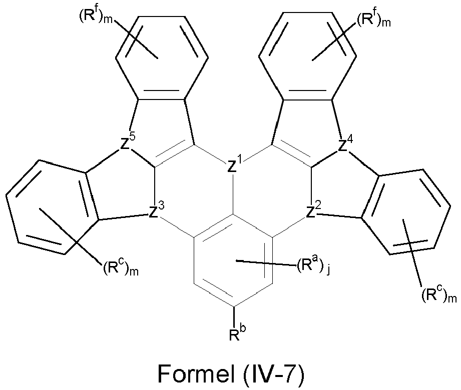 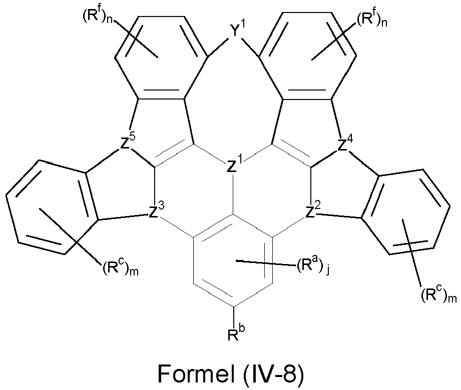 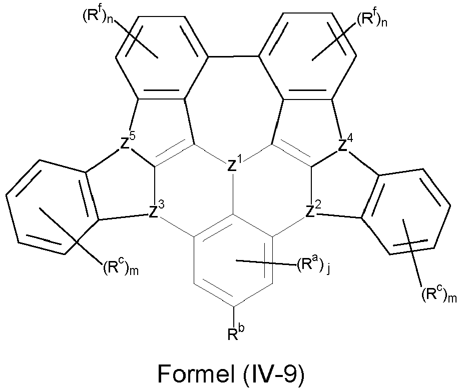 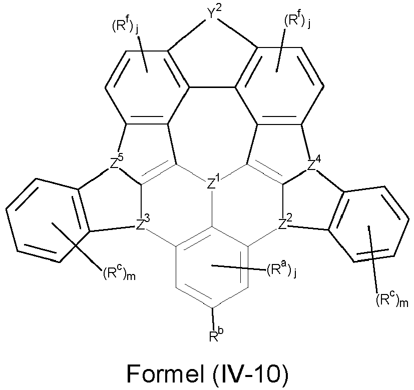 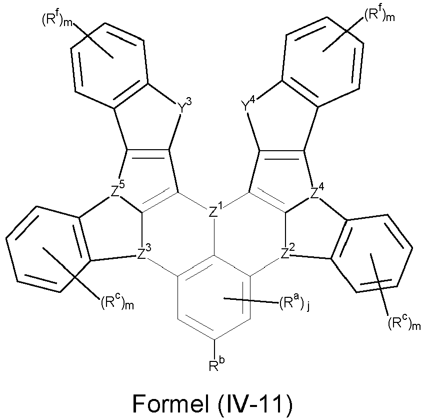 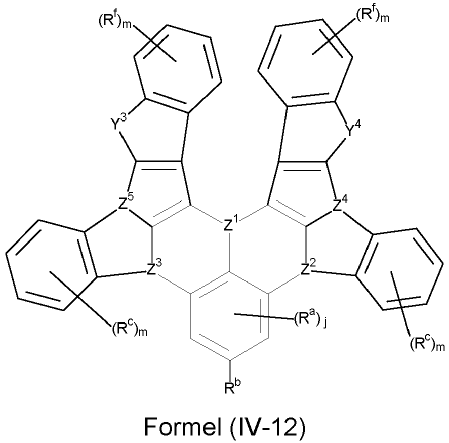 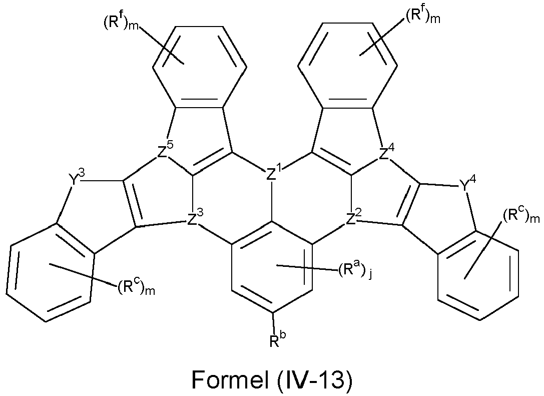 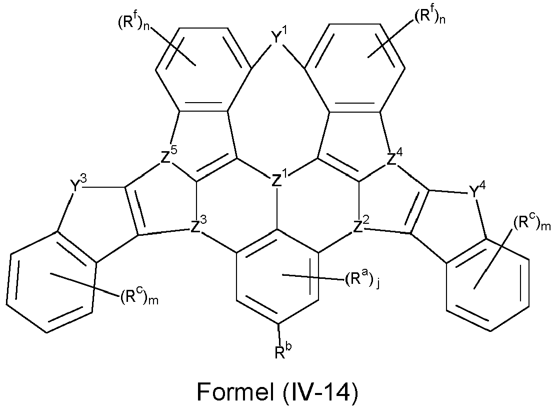 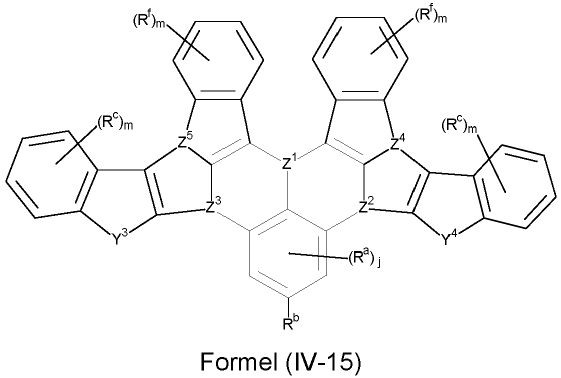 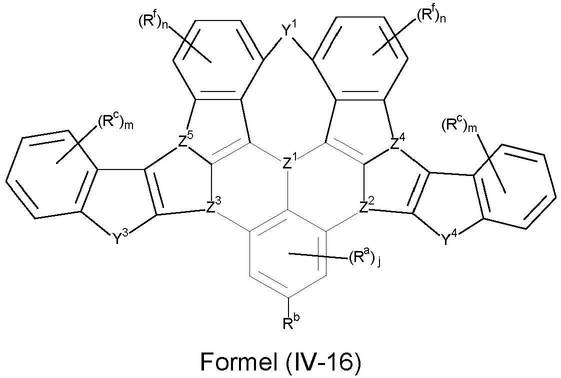 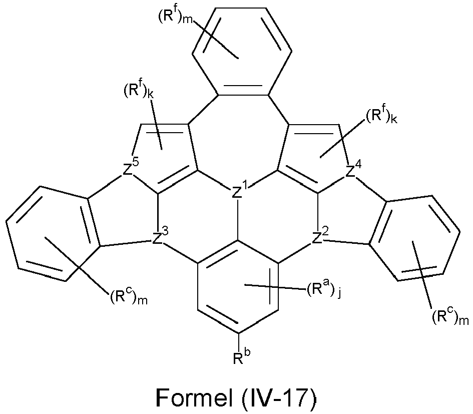 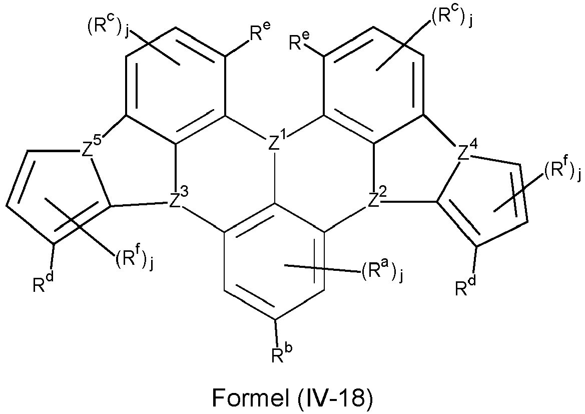 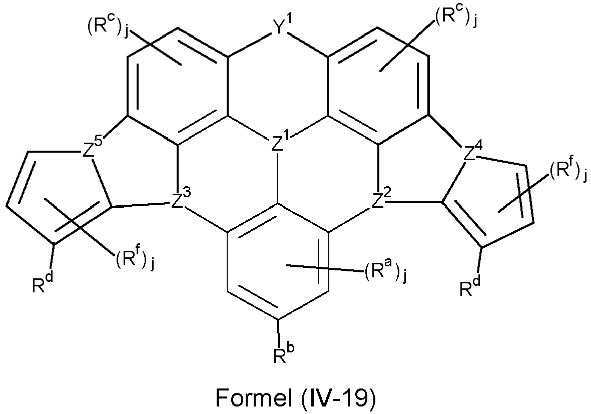 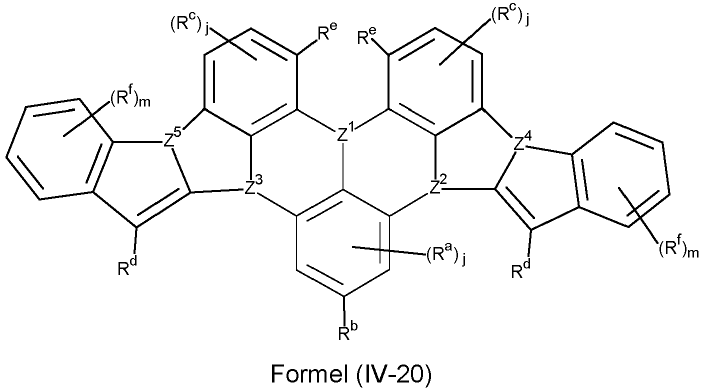 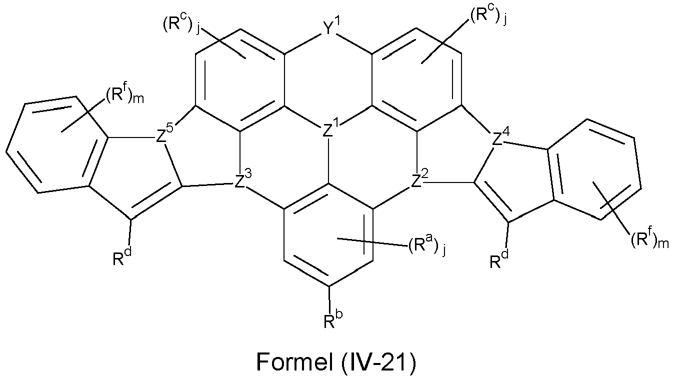 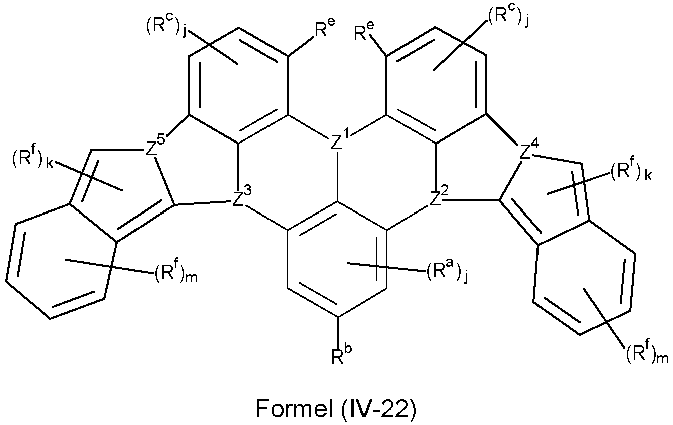 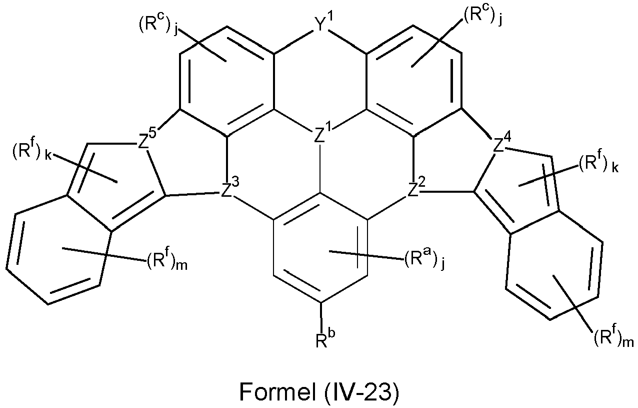 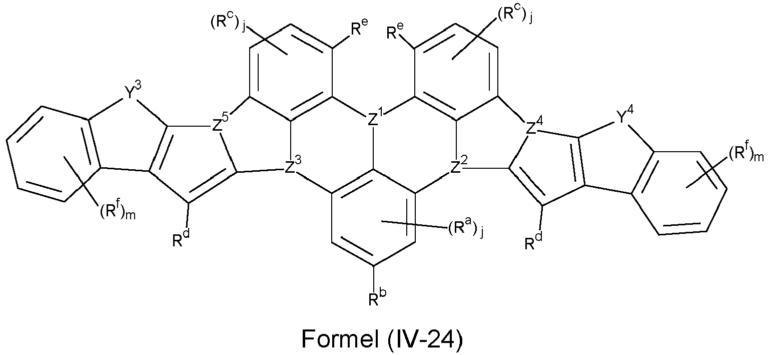 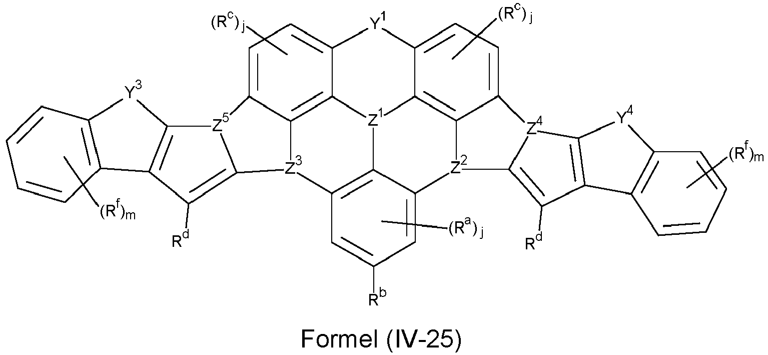 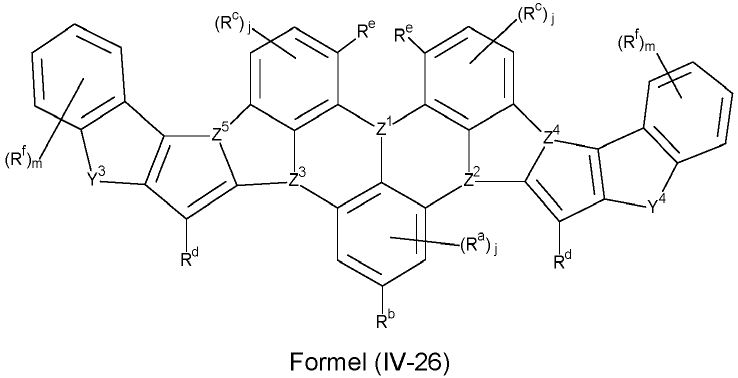 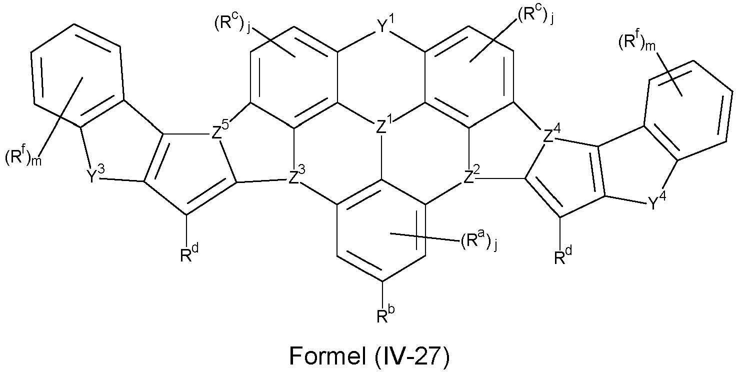 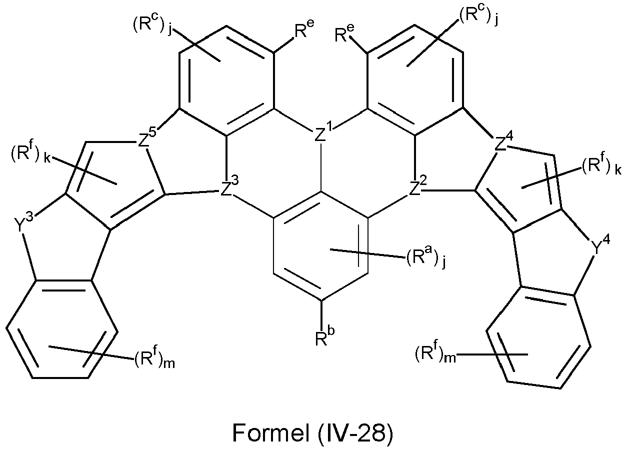 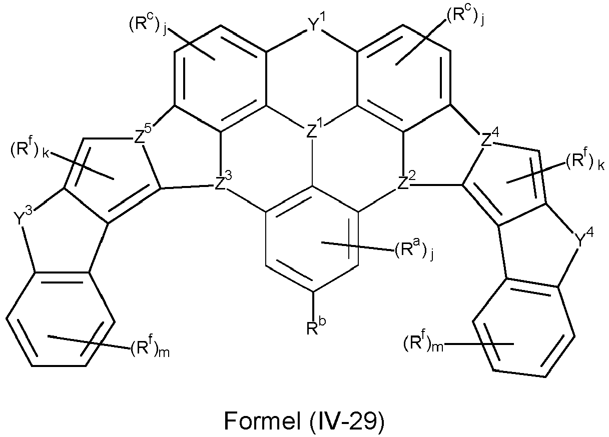 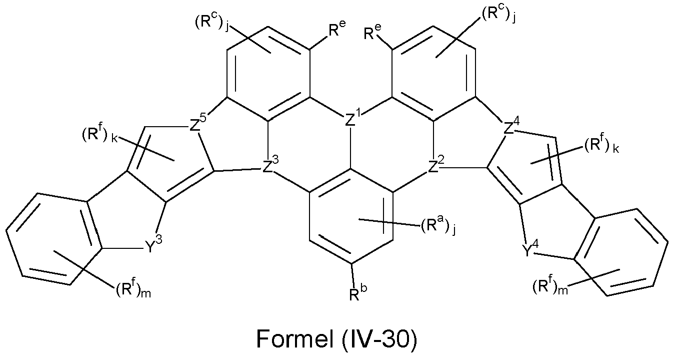 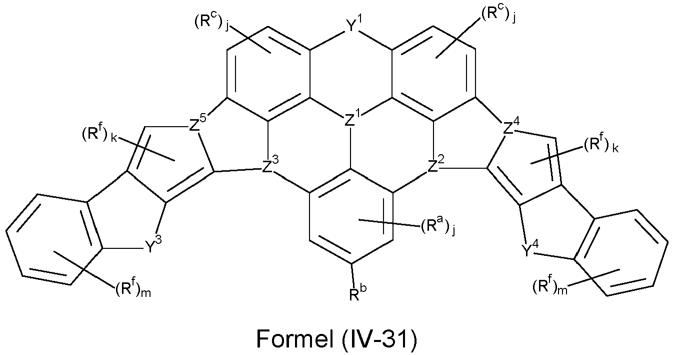 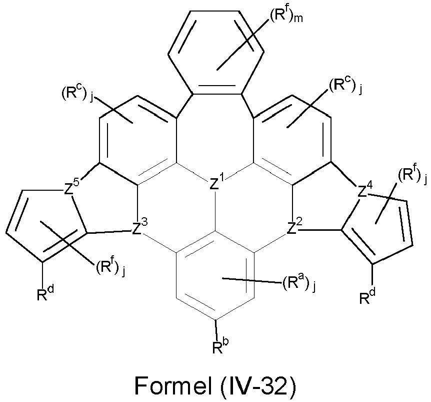 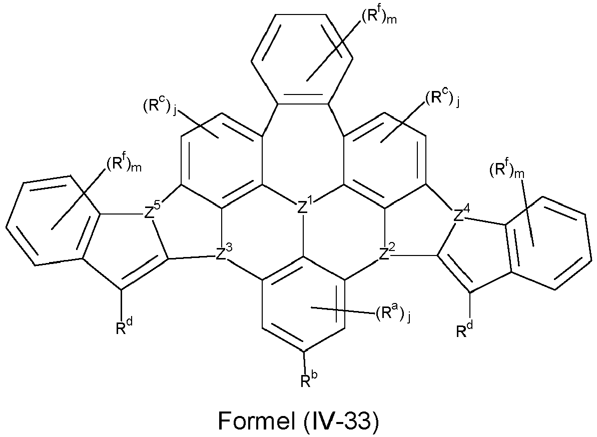 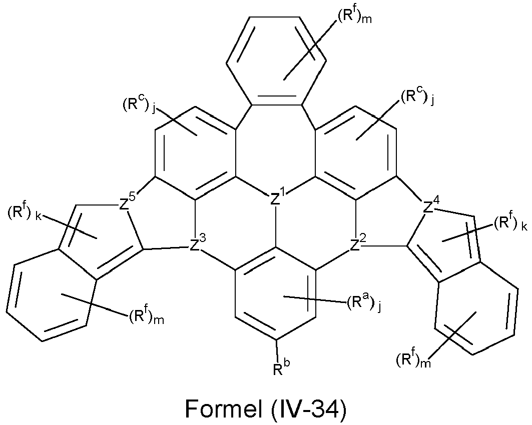 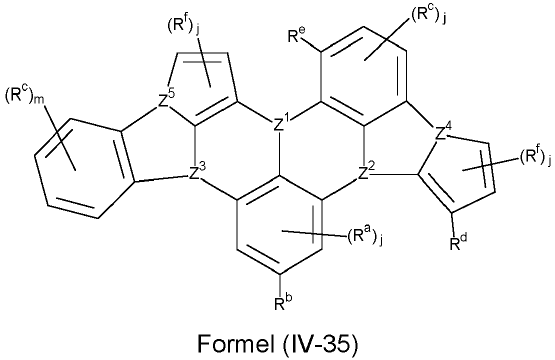 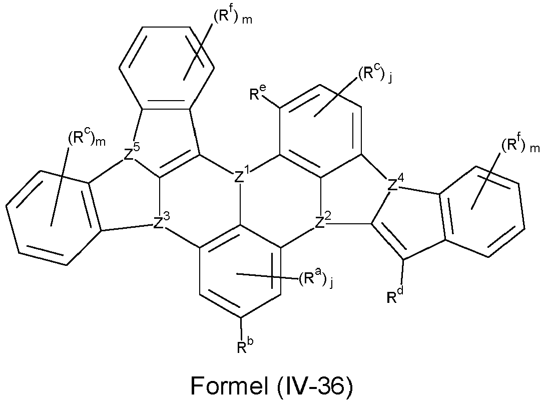 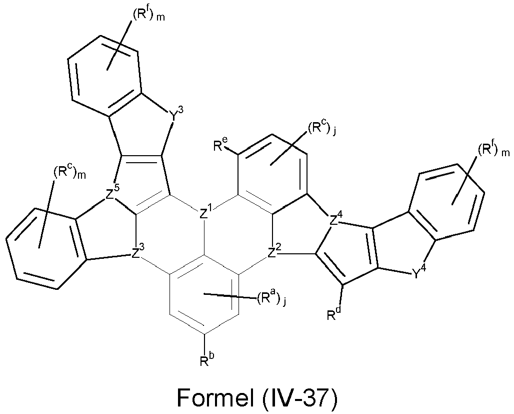 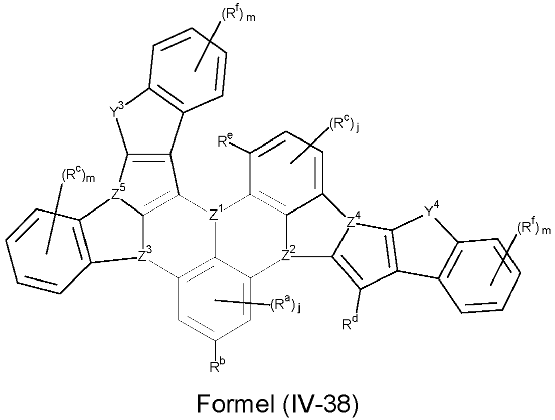 wobei die Symbole Z¹ , Z² , Z³ , R^{a} , R^{b} Z⁴ , Z⁵ , R^{c} , R^{d} , R^{e} und R^{f} die in Anspruch 1 genannten Bedeutungen aufweisen, die Symbole Y¹ , Y² , Y³ und Y⁴ die in Anspruch 2 aufweisen und die weiteren Symbole die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1;
- k: ist 0 oder 1.

Hierbei sind Strukturen/Verbindungen der Formel (IV-1) bis (IV-34) bevorzugt, Strukturen/Verbindungen der Formel (IV-1) bis (IV-12) und (IV-18) bis (IV-31) besonders bevorzugt.

Die Summe der Indices k, j, m und n in Strukturen/Verbindungen der Formeln (IV-1) bis (IV-38) beträgt vorzugsweise höchstens 10, vorzugsweise höchstens 8, insbesondere bevorzugt höchstens 6 und besonders bevorzugt höchstens 4.

Weiterhin kann unter anderem in Formeln (IIa) bis (Ile), (III-1) bis (III-25), (IV-1) bis (IV-38) und/oder den nachfolgend dargelegten bevorzugten Ausführungsformen dieser Formeln, vorgesehen sein, dass Z¹ N ist und mindestens eine, vorzugsweise zwei der Gruppen Z², Z³, Z⁴, Z⁵ für B steht/stehen. Ausgestaltungen, bei denen Z¹ für N steht und mindestens eine, vorzuasweise zwei der Gruppen Z², Z³, Z⁴, Z⁵ für B steht/stehen. können mit Vorteil als Emitter eingesetzt werden.

Ferner kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass Z¹ N ist und mindestens eine, vorzugsweise zwei der Gruppen Z², Z³, Z⁴, Z⁵ für N steht/stehen.

Ausführungsformen, bei denen viele, bevorzugt alle der Gruppen Z¹, Z², Z³, Z⁴, Z⁵ für N steht, können mit Vorteil insbesondere als Lochleitermaterial eingesetzt werden.

In einer weiteren Ausgestaltung kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass Z¹ B ist, und mindestens eine, vorzugsweise zwei der Gruppen Z², Z³, Z⁴, Z⁵ für N steht/stehen. Ausgestaltungen, bei denen Z¹ für B steht und mindestens eine, vorzugsweise zwei der Gruppen Z², Z³, Z⁴, Z⁵ für N steht/stehen, können mit Vorteil als Emitter eingesetzt werden.

In einer weiteren Ausgestaltung kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass Z¹ B ist, und mindestens eine, vorzugsweise zwei der Gruppen Z², Z³, Z⁴, Z⁵ für B steht/stehen.

Ausführungsformen, bei denen viele, bevorzugt alle der Gruppen Z¹, Z², Z³, Z⁴, Z⁵ für B stehen, können mit Vorteil insbesondere als Elektronentransportmaterial eingesetzt werden.

In einer Weitergestaltung kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass mindestens eine, vorzugsweise zwei der Gruppen Z², Z³ N ist/sind und mindestens eine, vorzugsweise zwei der Gruppen Z⁴, Z⁵ für B steht/stehen.

Ferner kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass mindestens eine, vorzugsweise zwei der Gruppen Z², Z³ B ist/sind und mindestens eine, vorzugsweise zwei der Gruppen Z⁴, Z⁵ für N steht/stehen.

Weiterhin kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass mindestens eine, vorzugsweise zwei der Gruppen Z², Z³ N ist/sind und mindestens eine, vorzugsweise zwei der Gruppen Z⁴, Z⁵ für N steht/stehen.

In einer weiteren Ausgestaltung kann unter anderem in Formeln (IIa) bis (IIe), (III-1) bis (III-25), (IV-1) bis (IV-38) und/oder den nachfolgend dargelegten bevorzugten Ausführungsformen dieser Formeln vorgesehen sein, dass mindestens eine, vorzugsweise zwei der Gruppen Z², Z³ B ist/sind und mindestens eine, vorzugsweise zwei der Gruppen Z⁴, Z⁵ für B steht/stehen.

In einer weiteren Ausführungsform kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass Z² B ist, Z³ N ist und mindestens eine, vorzugsweise zwei der Gruppen Z⁴, Z⁵ für N steht/stehen.

In einer Weitergestaltung kann unter anderem in Formeln (IIa) bis (Ile), (III-1) bis (III-25), (IV-1) bis (IV-38) und/oder den nachfolgend dargelegten bevorzugten Ausführungsformen dieser Formeln vorgesehen sein, dass Z² B ist, Z³ N ist und mindestens eine, vorzugsweise zwei der Gruppen Z⁴, Z⁵ für B steht/stehen.

Ferner kann unter anderem in Formeln (III-1) bis (III-25), (IV-1) bis (IV-38) und/oder den nachfolgend dargelegten bevorzugten Ausführungsformen dieser Formeln vorgesehen sein, dass p=1 ist und die Gruppe Y¹ für eine Bindung steht.

Weiterhin kann unter anderem in Formeln (III-1) bis (III-25), (IV-1) bis (IV-38) und/oder den nachfolgend dargelegten bevorzugten

Ausführungsformen dieser Formeln vorgesehen sein, dass die Gruppen Y³ und Y⁴ gleich sind.

Darüber hinaus kann unter anderem in Formeln (III-1) bis (III-25), (IV-1) bis (IV-38) und/oder den nachfolgend dargelegten bevorzugten Ausführungsformen dieser Formeln vorgesehen sein, dass die Gruppen Y³ und Y⁴ verschieden sind.

In einer bevorzugten Weiterbildung der vorliegenden Erfindung kann vorgesehen sein, dass mindestens zwei Reste R, R^{a}, R^{b}, R^{c}, R^{d}, R^{e}, R^{f} mit den weiteren Gruppen, an die die zwei Reste R, R^{a}, R^{b}, R^{c}, R^{d}, R^{e}, R^{f} binden, einen kondensierten Ring bilden, wobei die zwei Reste R, R^{a}, R^{b}, R^{c}, R^{d}, R^{e}, R^{f} mindestens eine Struktur der Formeln (RA-1) bis (RA-12) formen 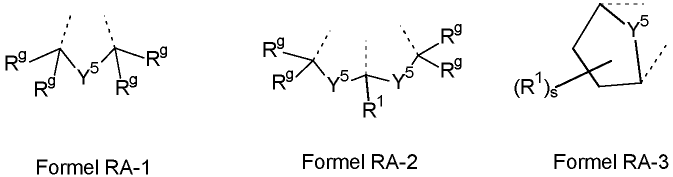 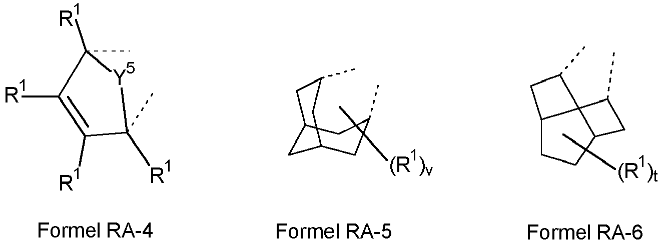 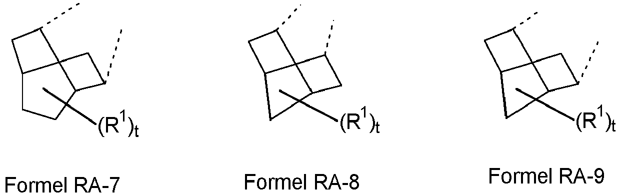 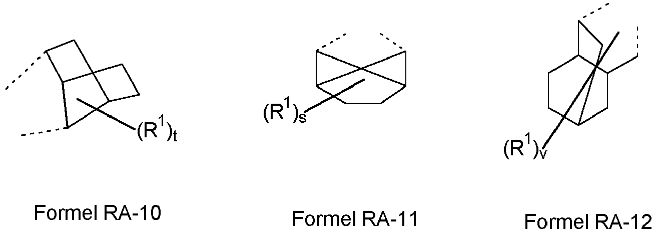 wobei R¹ die zuvor dargelegte Bedeutung hat, die gestrichelten Bindungen die Anbindungsstellen darstellen, über die die zwei Reste R, R^{a}, R^{b}, R^{c}, R^{d}, R^{e}, R^{f} binden, und die weiteren Symbole die folgende Bedeutung aufweisen:
- Y⁵: ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
- R^{g}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{g} auch miteinander oder ein Rest R^{g} mit einem Rest R¹ oder mit einer weiteren Gruppe ein Ringsystem bilden;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

In einer bevorzugten Ausführungsform der Erfindung bilden die mindestens zwei Reste R, R^{a}, R^{b}, R^{c}, R^{d}, R^{e}, R^{f} mit den weiteren Gruppen, an die die zwei Reste R, R^{a}, R^{b}, R^{c}, R^{d}, R^{e}, R^{f} binden, einen kondensierten Ring, wobei die zwei Reste R, R^{a}, R^{b}, R^{c}, R^{d}, R^{e}, R^{f} vorzugsweise mindestens eine der Strukturen der Formeln (RA-1a) bis (RA-4f) formen 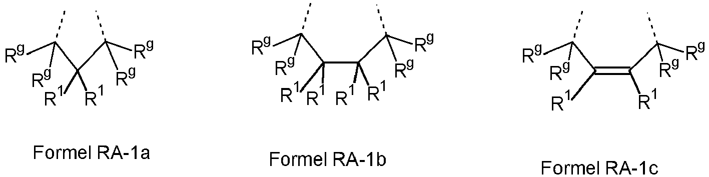 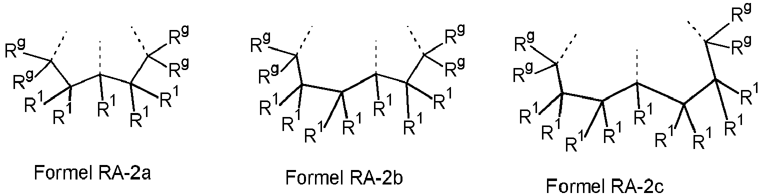 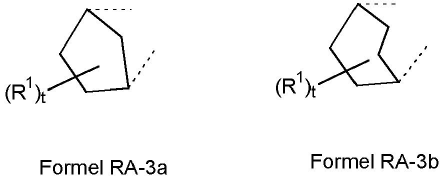 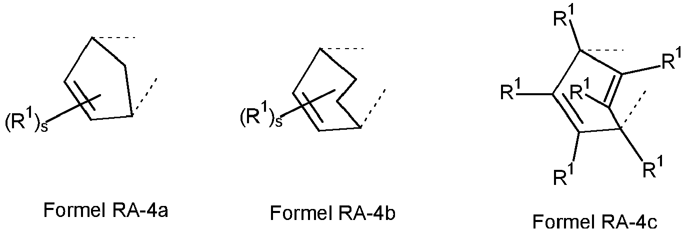 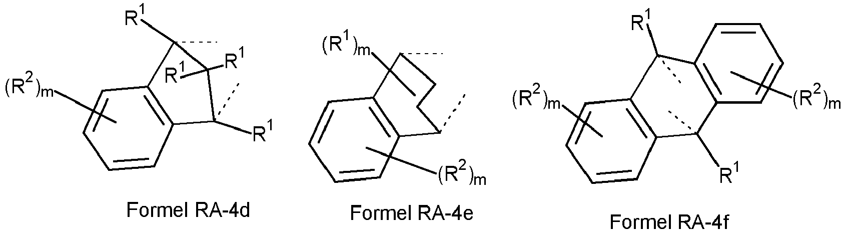 wobei die gestrichelten Bindungen die Anbindungsstellen darstellen, über die die zwei Reste R, R^{a}, R^{b}, R^{c}, R^{d}, R^{e}, R^{f} binden, , der Index m 0, 1, 2, 3 oder 4, vorzugsweise 0, 1 oder 2 ist und die Symbole R¹, R², R^{g} und die Indices s, und t die zuvor, insbesondere für Formeln (RA-1) bis (RA-12) dargelegte Bedeutung haben.

Ferner kann vorgesehen sein, dass die mindestens zwei Reste R, R^{a}, R^{b}, R^{c}, R^{d}, R^{e}, R^{f}, die Strukturen der Formeln (RA-1) bis (RA-12) und/oder (RA-1a) bis (RA-4f) formen und einen kondensierten Ring bilden, Reste R^{a}, R^{b}, R^{c}, R^{d}, R^{e}, R^{f} aus benachbarten Gruppen X¹, X², X³, X⁴, X⁵, X⁶ darstellen oder Reste R darstellen, die jeweils an benachbarte C-Atome binden, wobei diese C-Atome vorzugsweise über eine Bindung verbunden sind

In einer weiterhin bevorzugten Ausgestaltung bilden mindestens zwei Reste R, R^{a}, R^{b}, R^{c}, R^{d}, R^{e}, R^{f} mit den weiteren Gruppen, an die die zwei Reste R, R^{a}, R^{b}, R^{c}, R^{d}, R^{e}, R^{f} binden, einen kondensierten Ring, wobei die zwei Reste R, R^{a}, R^{b}, R^{c}, R^{d}, R^{e}, R^{f} Strukturen der Formel (RB), formen 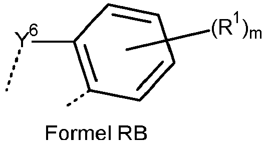 wobei R¹ die in Anspruch 1 genannte Bedeutung aufweist, die gestrichelten Bindungen die Anbindungsstellen darstellen, über die die zwei Reste R, R^{a}, R^{b}, R^{c}, R^{d}, R^{e}, R^{f} binden, der Index m 0, 1, 2, 3 oder 4, vorzugsweise 0, 1 oder 2 ist, und Y⁶ C(R¹)₂, NR¹, NAr', BR¹, BAr', O oder S ist, vorzugsweise C(R¹)₂, NAr' oder O.

Hierbei kann vorgesehen sein, dass die mindestens zwei Reste R, R^{a}, R^{b}, R^{c}, R^{d}, R^{e}, R^{f}, die Strukturen der Formel (RB) formen und einen kondensierten Ring bilden, Reste R^{a}, R^{b}, R^{c}, R^{d}, R^{e}, R^{f} aus benachbarten Gruppen X¹, X², X³, X⁴, X⁵, X⁶ darstellen oder Reste R darstellen, die jeweils an benachbarte C-Atome binden, wobei diese C-Atome vorzugsweise über eine Bindung miteinander verbunden sind.

Besonders bevorzugt umfassen die Verbindungen mindestens eine Struktur der Formeln (V-1) bis (V-10), besonders bevorzugt sind die Verbindungen ausgewählt aus Verbindungen der Formeln (V-1) bis (V-10), wobei die Verbindungen mindestens einen kondensierten Ring aufweisen 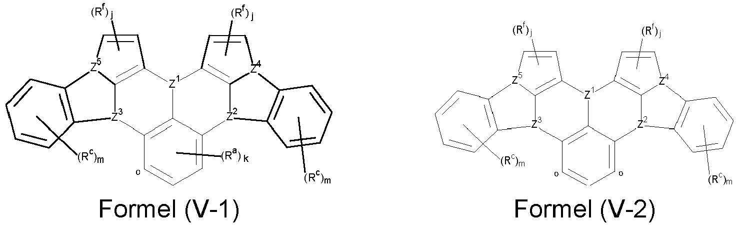 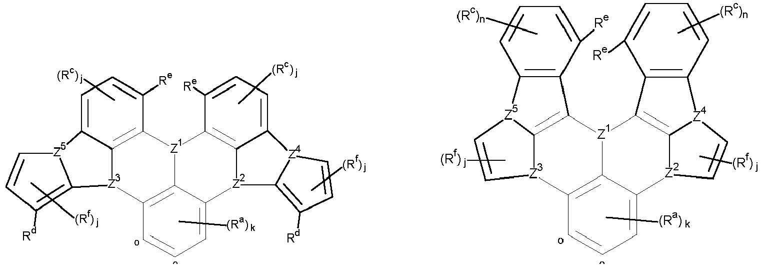 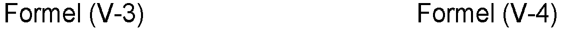 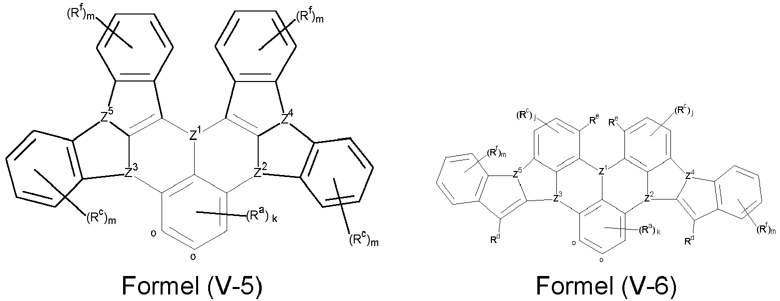 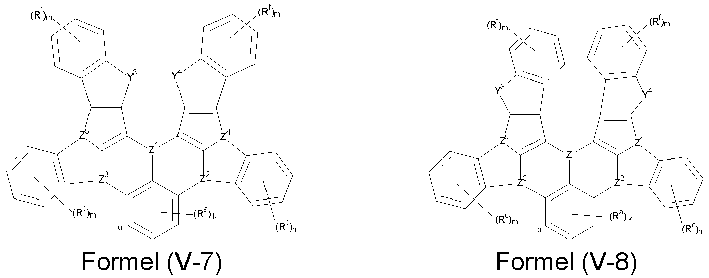 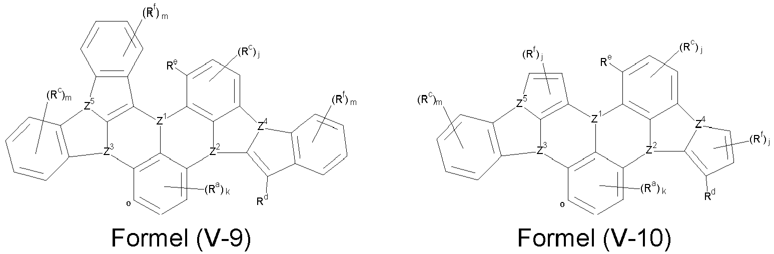 wobei die Symbole Z¹ , Z² , Z³ , R^{a} , R^{b} , Z⁴ , Z⁵ , R^{c} , R^{d} , R^{e} und R^{f} die in Anspruch 1 genannten Bedeutungen aufweisen, die Symbole Y³ und Y⁴ die in Anspruch 2 genannten Bedeutungen aufweisen, das Symbol o für die Anbindungsstellen steht und die weiteren Symbole die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1;
- k: ist 0 oder 1.

Hierbei sind Strukturen/Verbindungen der Formel (V-1) bis (V-8) bevorzugt.

Besonders bevorzugt umfassen die Verbindungen mindestens eine Struktur der Formeln (VI-1) bis (VI-9), besonders bevorzugt sind die Verbindungen ausgewählt aus Verbindungen der Formeln (VI-1) bis (VI-9), wobei die Verbindungen mindestens einen kondensierten Ring aufweisen 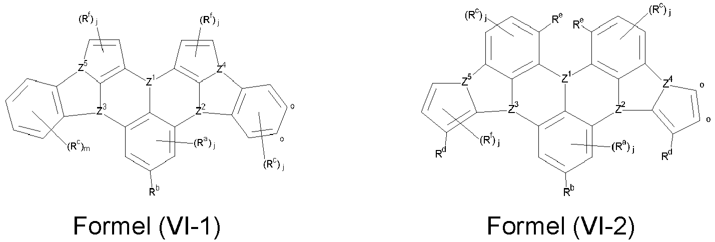 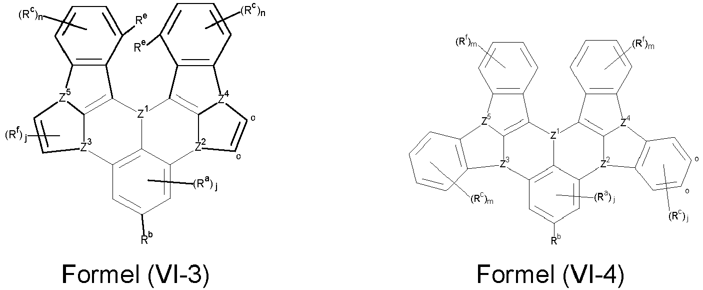 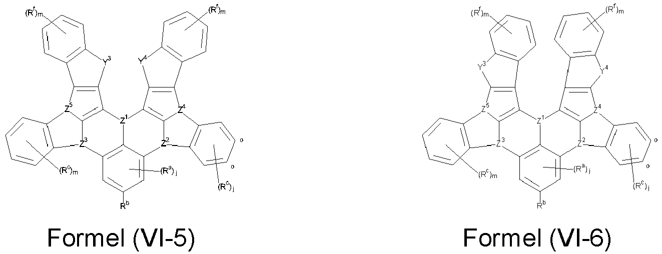 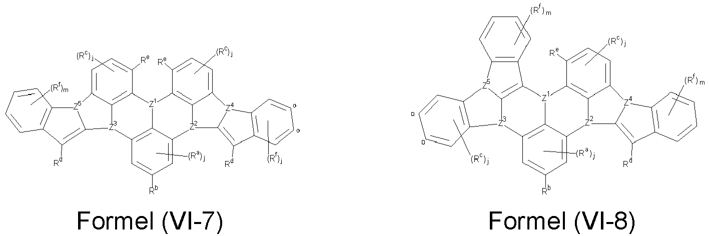 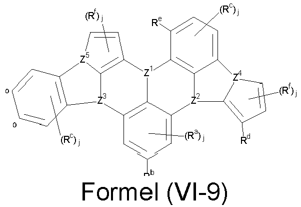 wobei die Symbole Z¹ , Z² , Z³ , R^{a} , R^{b}, Z⁴ , Z⁵ , R^{c} , R^{d} , R^{e} und R^{f} die in Anspruch 1 genannten Bedeutungen aufweisen, die Symbole , die Symbole Y³ und Y⁴ die in Anspruch 2 genannten Bedeutungen aufweisen, das Symbol o für die Anbindungsstellen steht und die weiteren Symbole die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1.

Hierbei sind Strukturen/Verbindungen der Formel (VI-1) bis (VI-7) bevorzugt.

Besonders bevorzugt umfassen die Verbindungen mindestens eine Struktur der Formeln (VII-1) bis (VII-9), besonders bevorzugt sind die Verbindungen ausgewählt aus Verbindungen der Formeln (VII-1) bis (VII-9), wobei die Verbindungen mindestens einen kondensierten Ring aufweisen 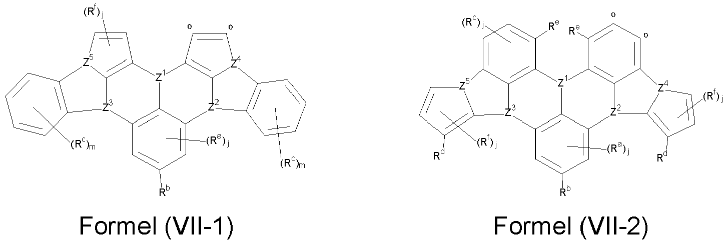 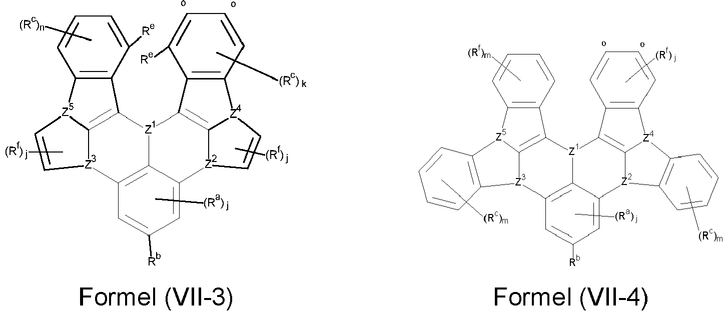 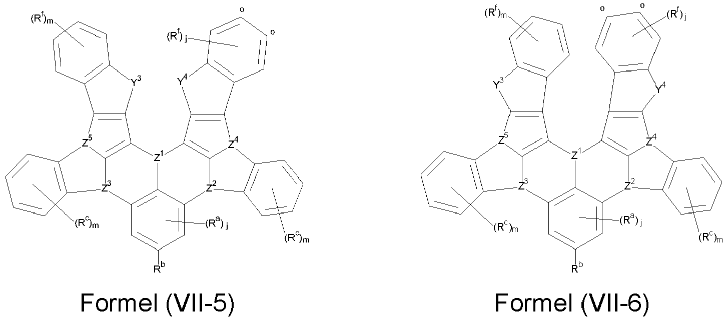 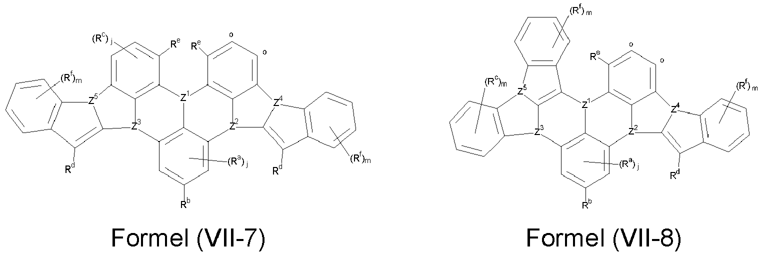 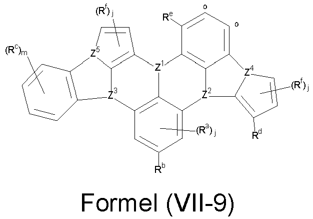 wobei die Symbole Z¹ , Z² , Z³ , R^{a} , R^{b}, Z⁴ , Z⁵ , R^{c} , R^{d} , R^{e} und R^{f} die in Anspruch 1 genannten Bedeutungen aufweisen, die Symbole Y³ und Y⁴ die in Anspruch 2 genannten Bedeutungen aufweisen, das Symbol o für die Anbindungsstellen steht und die weiteren Symbole die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1.

Hierbei sind Strukturen/Verbindungen der Formel (VII-1) bis (VII-7) bevorzugt.

Bevorzugt wird der kondensierte Ring, insbesondere in Formeln (V-1) bis (V-10), (VI-1) bis (VI-9) und/oder (VII-1) bis (VII-9), durch mindestens zwei Reste R, R^{a}, R^{b}, R^{c}, R^{d}, R^{e}, R^{f} und den weiteren Gruppen, an die die zwei Reste R, R^{a}, R^{b}, R^{c}, R^{d}, R^{e}, R^{f} binden, gebildet, wobei die mindestens zwei Reste R, R^{a}, R^{b}, R^{c}, R^{d}, R^{e}, R^{f} Strukturen der Formeln (RA-1) bis (RA-12) und/oder der Formel (RB) formen, vorzugsweise Strukturen der Formeln (RA-1) bis (RA-12).

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-12) und/oder (RA-1a) bis (RA-4f) gebildet ist und ein weiterer Ring durch Strukturen der Formeln (RA-1) bis (RA-12), (RA-1a) bis (RA-4f) oder (RB) gebildet ist, wobei die Verbindungen mindestens eine Struktur der Formeln (VIII-1) bis (VIII-20) umfassen, vorzugsweise die Verbindungen ausgewählt sind aus den Verbindungen der Formeln (VIII-1) bis (VIII-20), 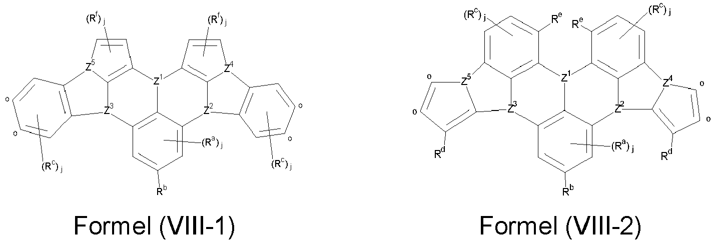 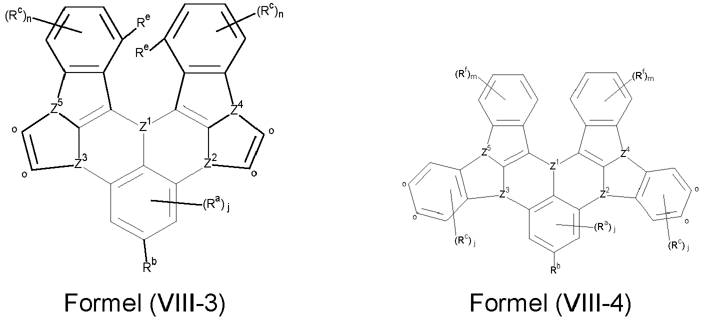 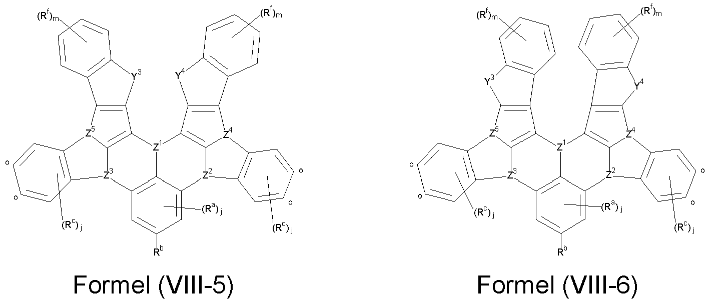 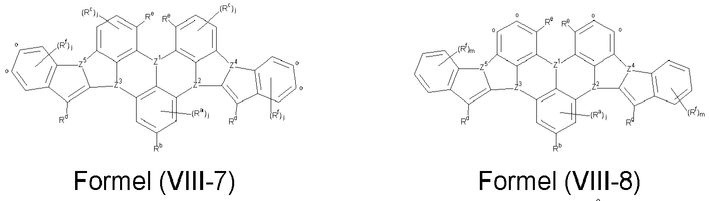 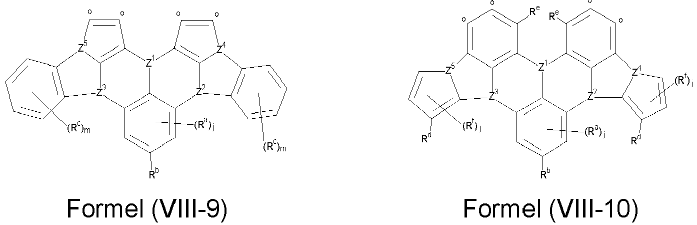 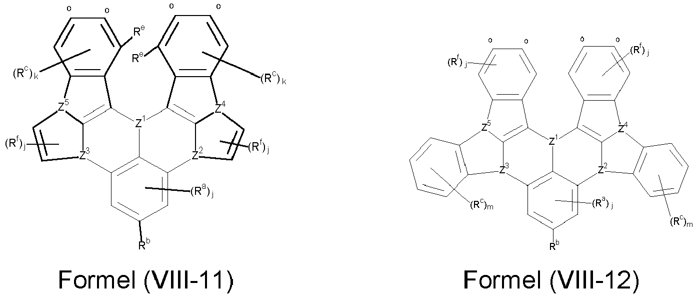 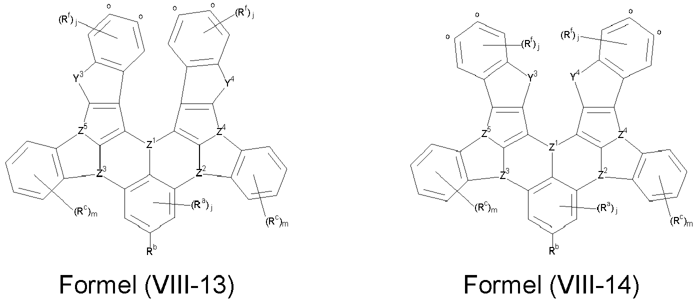 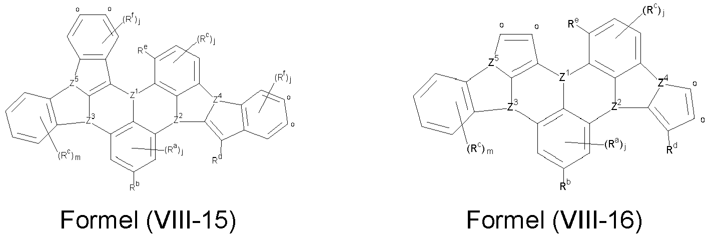 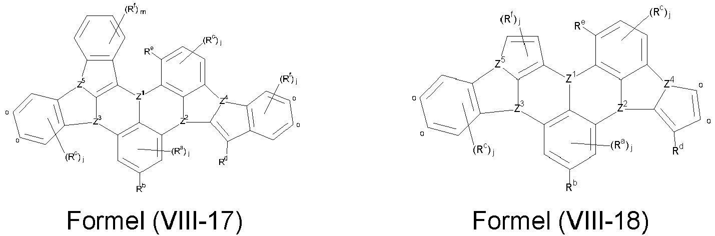 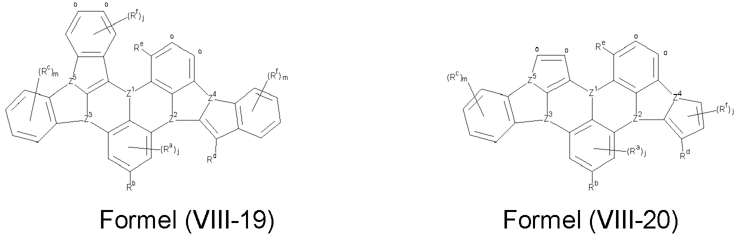 wobei die Symbole Z¹ , Z² , Z³ , R^{a} , R^{b} , Z⁴ , Z⁵ , R^{c} , R^{d} , R^{e} und R^{f} die in Anspruch 1 genannten Bedeutungen aufweisen, die Symbole Y³ und Y⁴ die in Anspruch 2 genannten Bedeutungen aufweisen, das Symbol o für die Anbindungsstellen steht und die weiteren Symbole die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1.

Hierbei sind Strukturen/Verbindungen der Formel (VIII-1) bis (VIII-16) bevorzugt, Strukturen/Verbindungen der Formel (VIII-1) bis (VIII-14) besonders bevorzugt.

Insbesondere in den Formeln (V-1) bis (V-10), (VI-1) bis (VI-9), (VII-1) bis (VII-9) und/oder (VIII-1) bis (VIII-20) ist die Summe der Indices k, j, l, m und n vorzugsweise 0, 1, 2 oder 3, besonders bevorzugt 1 oder 2. Hierbei kann vorgesehen sein, dass die Formeln (VIII-1) bis (VIII-20) mindestens zwei kondensierte Ringe, aufweisen, wobei die kondensierten Ringe gleich sind und der durch zwei Reste R, R^{a}, R^{b}, R^{c}, R^{d}, R^{e}, R^{f} gebildete Teil durch mindestens eine Struktur der Formeln (RA-1) bis (RA-12) und/oder (RA-1a) bis (RA-4f) darstellbar sind.

Weiterhin kann vorgesehen sein, dass die Formeln (VIII-1) bis (VIII-20) mindestens zwei kondensierte Ringe, aufweisen, wobei die kondensierten Ringe verschieden sind und der durch zwei Reste R, R^{a}, R^{b}, R^{c}, R^{d}, R^{e}, R^{f} gebildete Teil jeweils durch mindestens eine Struktur der Formeln (RA-1) bis (RA-12) und/oder (RA-1a) bis (RA-4f) darstellbar sind.

Darüber hinaus kann vorgesehen sein, dass die Formeln (VIII-1) bis (VIII-20) mindestens zwei kondensierte Ringe, aufweisen, wobei die kondensierten Ringe verschieden sind und einer der zwei kondensierten Ringe einen durch zwei Reste R gebildete Teil aufweist, der durch mindestens eine der Strukturen der Formeln (RA-1) bis (RA-12) und/oder (RA-1a) bis (RA-4f) darstellbar ist und einer der zwei kondensierte Ringe einen durch zwei Reste R, R^{a}, R^{b}, R^{c}, R^{d}, R^{e}, R^{f} gebildeten Teil aufweist, der durch eine der Strukturen der Formel (RB) darstellbar ist.

Weiterhin kann vorgesehen sein, dass die Substituenten R, R^{a}, R^{b}, R^{c}, R^{d}, R^{e}, R^{f} und R^{g}, R¹ und R² gemäß obigen Formeln mit den Ringatomen des Ringssystems, an das die R, R^{a}, R^{b}, R^{c}, R^{d}, R^{e}, R^{f} und R^{g}, R¹ und R² binden, kein kondensiertes aromatisches oder heteroaromatisches Ringsystem bilden. Dies schließt die Bildung eines kondensierten aromatischen oder heteroaromatischen Ringsystems mit möglichen Substituenten R¹ und R² ein, die an die Reste R, R^{a}, R^{b}, R^{c}, R^{d}, R^{e}, R^{f}, R^{g} und R¹ gebunden sein können.

Wenn zwei Reste, die insbesondere ausgewählt sein können aus R, R^{a}, R^{b}, R^{c}, R^{d}, R^{e}, R^{f}, R^{g}, R¹ und/oder R², miteinander ein Ringsystem bilden, so kann dieses mono- oder polycyclisch, aliphatisch, heteroaliphatisch, aromatisch oder heteroaromatisch sein. Dabei können die Reste, die miteinander ein Rinasystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R^{a}, R^{b}, R^{c}, R^{d}, R^{e}, R^{f}, R^{g}, R¹ und/oder R² versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R^{a}, R^{b}, R^{c}, R^{d}, R^{e}, R^{f}, R^{g}, R¹ und/oder R² versehen.

Gemäß einer bevorzugten Ausgestaltung ist eine erfindungsgemäße Verbindung durch mindestens eine der Strukturen gemäß Formel (I), (la), (IIa) bis (IIe), (III-1) bis (III-25), (IV-1) bis (IV-38), (V-1) bis (V-10), (VI-1) bis (VI-9), (VII-1) bis (VII-9) und/oder (VIII-1) bis (VIII-20) darstellbar. Vorzugsweise weisen erfindungsgemäße Verbindungen, bevorzugt umfassend Strukturen gemäß Formel (IIa) bis (Ile), (III-1) bis (III-25), (IV-1) bis (IV-38), (V-1) bis (V-10), (VI-1) bis (VI-9), (VII-1) bis (VII-9) und/oder (VIII-1) bis (VIII-20) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Bevorzugte aromatische bzw. heteroaromatische Ringsysteme R, R^{a}, R^{b}, R^{c}, R^{d}, R^{e}, R^{f}, Ar' und/oder Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3-oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ oder R substituiert sein können.

Vorzugsweise kann vorgesehen sein, dass mindestens ein Substituent R, R^{a}, R^{b}, R^{c}, R^{d}, R^{e}, R^{f} gleich oder verschieden bei jedem Auftreten ausgewählt ist aus der Gruppe bestehend aus H, D, einer verzweigte oder cyclische Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-75, wobei die Substituenten R, R^{a}, R^{b}, R^{c}, R^{d}, R^{e}, R^{f} vorzugsweise entweder einen Ring gemäß den Strukturen der Formeln (RA-1) bis (RA-12), (RA-1a) bis (RA-4f) oder (RB) bilden oder der Substituent R, R^{a}, R^{b}, R^{c}, R^{d}, R^{e}, R^{f} gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-75, und/oder die Gruppe Ar' gleich oder verschieden bei jedem Auftreten ausgewählt ist aus den Gruppen der folgenden Formeln Ar-1 bis Ar-75, 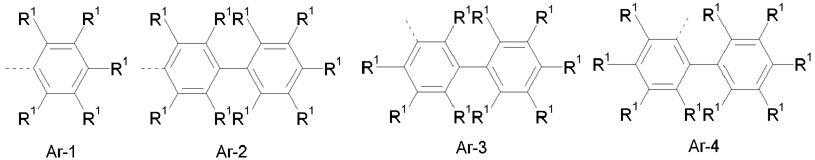 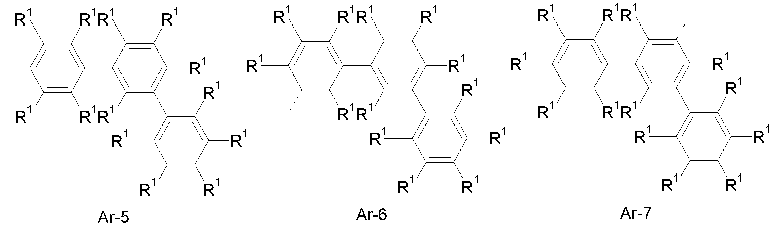 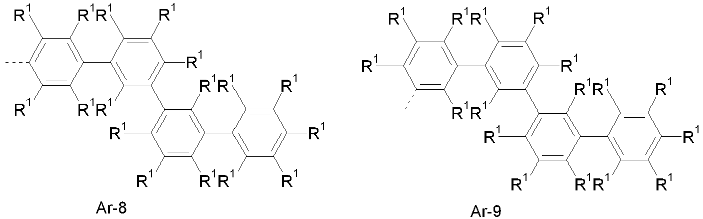 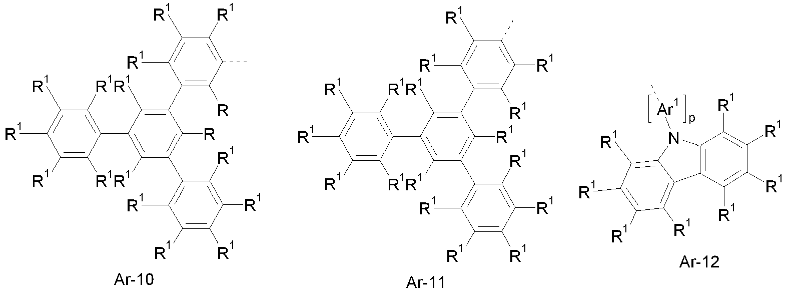 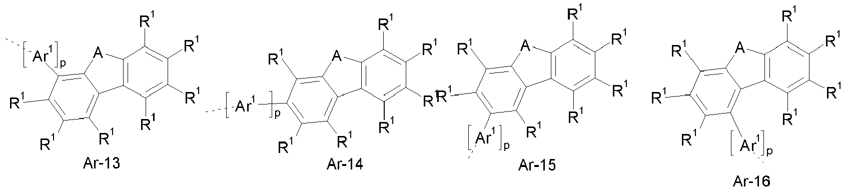 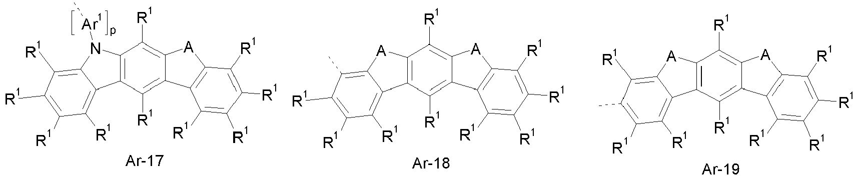 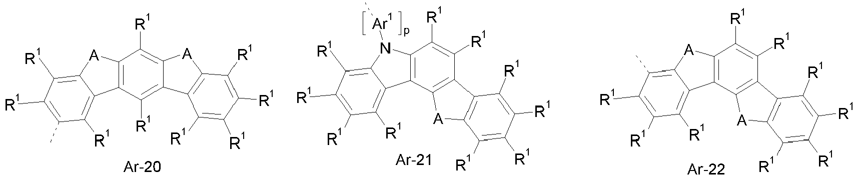 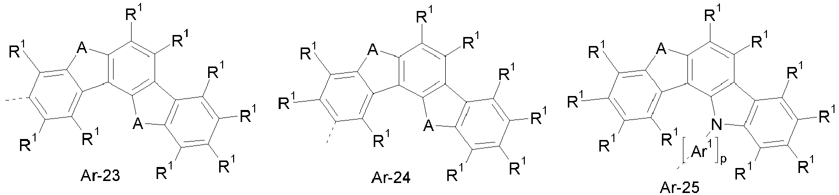 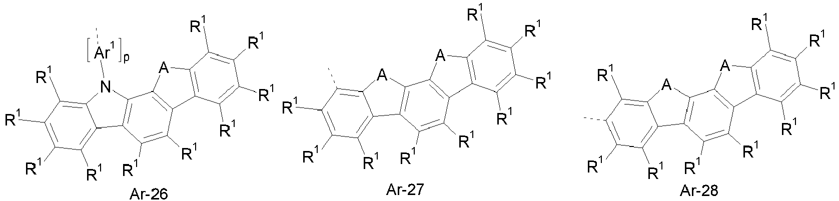 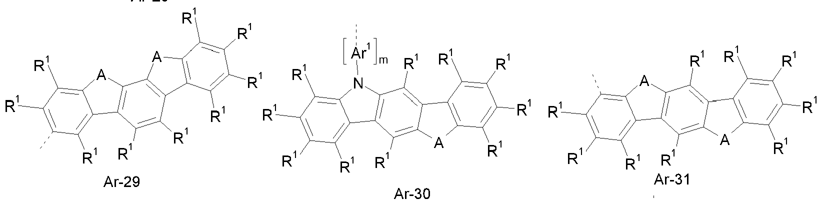 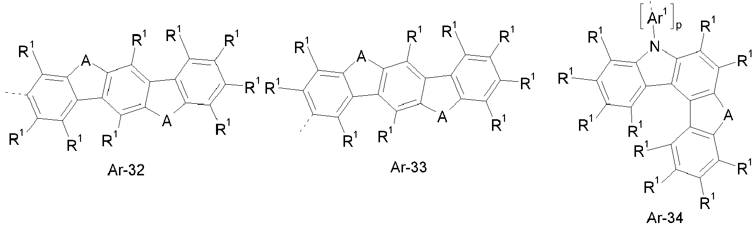 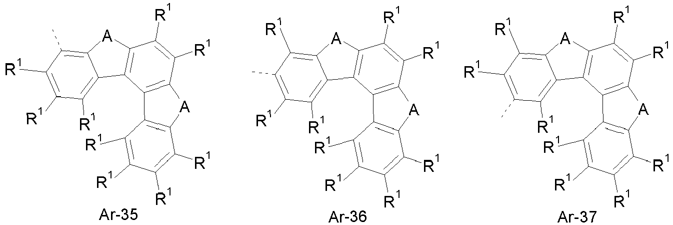 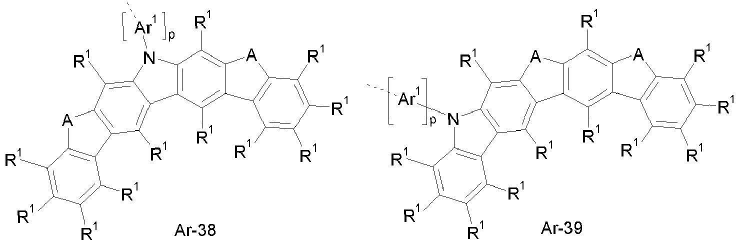 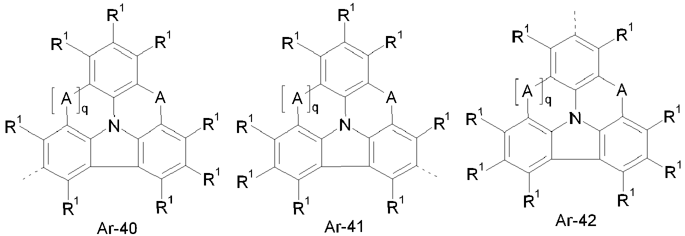 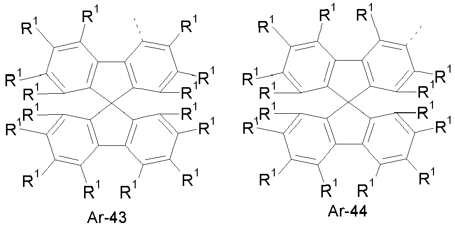 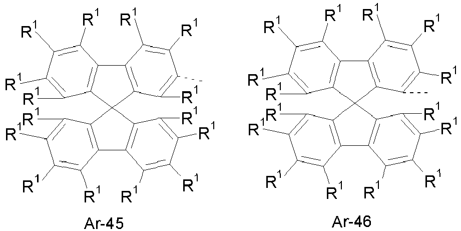 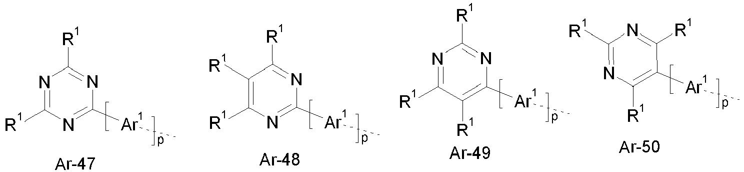 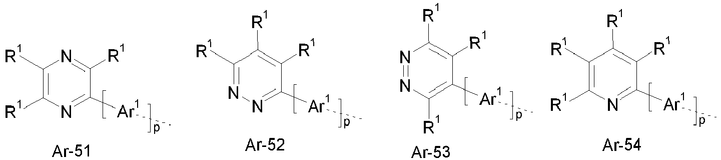 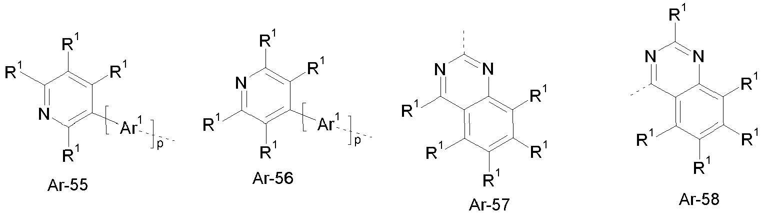 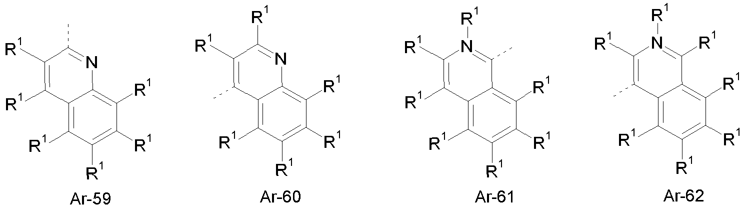 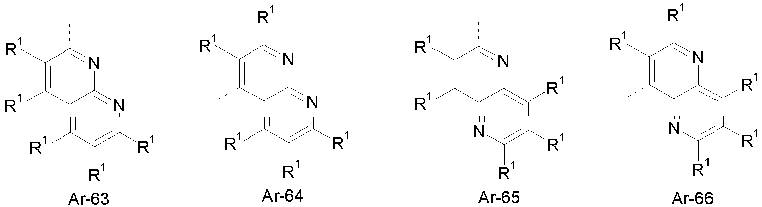 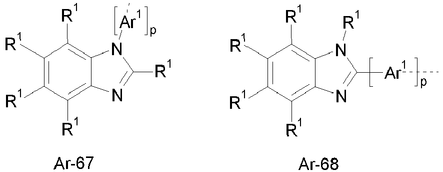 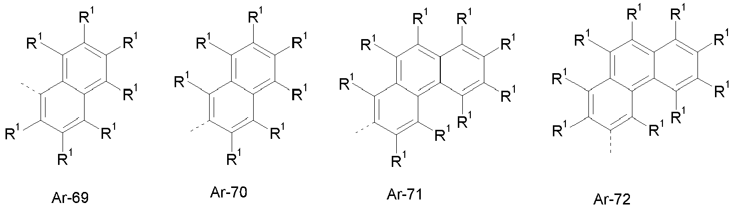 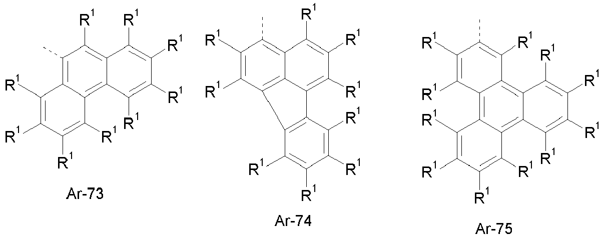 wobei R¹ die oben genannten Bedeutungen aufweist, die gestrichelte Bindung die Anbindungstelle darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an HetAr gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Wenn die oben genannten Gruppen für Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Im Folgenden werden bevorzugte Substituenten R, R^{a}, R^{b}, R^{c}, R^{d}, R^{e}, R^{f} und R^{g} beschrieben.

In einer bevorzugten Ausführungsform der Erfindung ist R, R^{a}, R^{b}, R^{c}, R^{d}, R^{e}, R^{f} gleich oder verschieden bei jedem Auftreten ausgewählt aus der Gruppe bestehend aus H, D, F, CN, NO₂, Si(R¹)₃, B(OR¹)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist Substituent R, R^{a}, R^{b}, R^{c}, R^{d}, R^{e}, R^{f} gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

Ferner kann vorgesehen sein, dass mindestens ein Substituent Substituent R, R^{a}, R^{b}, R^{c}, R^{d}, R^{e}, R^{f} gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. In einer weiterhin bevorzugten Ausführungsform der Erfindung bilden die Substituenten R entweder einen Ring gemäß den Strukturen der Formeln (RA-1) bis (RA-12), (RA-1a) bis (RA-4f) oder (RB) oder R ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar)₂. Besonders bevorzugt ist Substituent R, R^{a}, R^{b}, R^{c}, R^{d}, R^{e}, R^{f} gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

In einer bevorzugten Ausführungsform der Erfindung ist R^{g} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist R^{g} gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R¹ substituiert sein kann, einem aromatischen oder heteroaromatischen Ringsystem mit 6 bis 30 aromatischen Ringatomen, welches mit einem oder mehreren Resten R¹ substituiert sein kann. Besonders bevorzugt ist R^{a} gleich oder verschieden bei jedem Auftreten ausgewählt aus der Gruppe bestehend aus einer geradkettigen Alkylgruppe mit 1 bis 5 C-Atomen oder einer verzweigten oder cyclischen Alkylgruppe mit 3 bis 5 C-Atomen, wobei die Alkylgruppe jeweils mit einem oder mehreren Resten R¹ substituiert sein kann oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

In einer bevorzugten Ausführungsform der Erfindung ist R^{g} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R¹ substituiert sein kann, oder einem aromatischen oder heteroaromatischen Ringsystem mit 6 bis 24 aromatischen Ringatomen, das jeweils durch einen oder mehrere Reste R¹ substituiert sein kann; dabei können zwei Reste R^{g} auch miteinander ein Ringsystem bilden. Besonders bevorzugt ist R^{g} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R¹ substituiert sein kann, bevorzugt aber unsubstituiert ist; dabei können zwei Reste R^{g} miteinander ein Ringsystem bilden. Ganz besonders bevorzugt ist R^{g} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{g} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme Substituent R, R^{a}, R^{b}, R^{c}, R^{d}, R^{e}, R^{f}, R^{g} beziehungsweise Ar oder Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R, R¹ beziehungsweise R² substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-75 ist festzuhalten, dass diese mit einem Substituenten R¹ dargestellt sind. Im Falle der Ringsysteme Ar sind diese Substituenten R¹ durch R und im Falle R^{g} sind diese Substituenten R¹ durch R² zu ersetzen.

Weitere geeignete Gruppen R, R^{a}, R^{b}, R^{c}, R^{d}, R^{e}, R^{f} sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁵ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁵ substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formel (I), (IIa) bis (Ile), (III-1) bis (III-25), (IV-1) bis (IV-38), (V-1) bis (V-10), (VI-1) bis (VI-9), (VII-1) bis (VII-9) und/oder (VIII-1) bis (VIII-20) umfasst.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf. Beispiele für bevorzugte Verbindungen gemäß den oben aufgeführten Ausführungsformen sind die in der folgenden Tabelle aufgeführten Verbindungen:

| | |
|---|---|
| 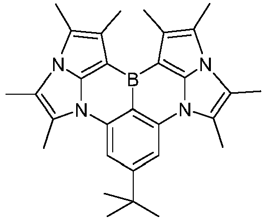 | 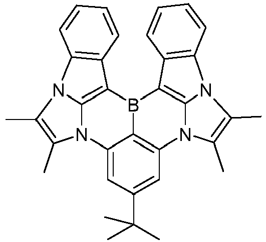 |
| 1 | 2 |
| 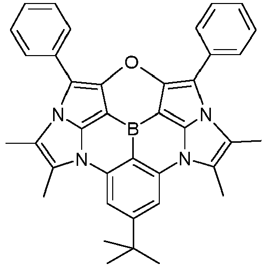 | 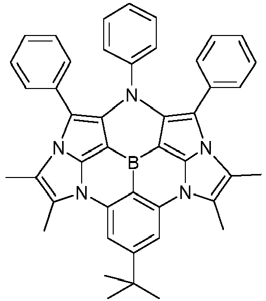 |
| 3 | 4 |
| 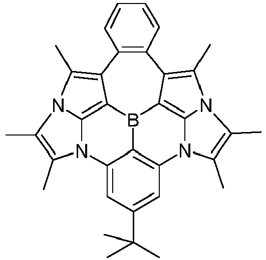 | 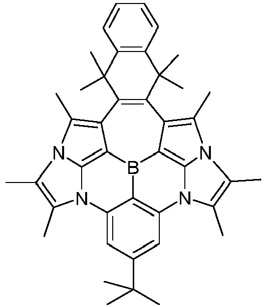 |
| 5 | 6 |
| 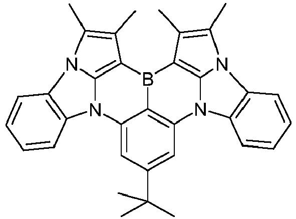 | 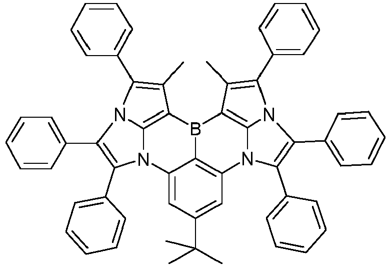 |
| 7 | 8 |
| 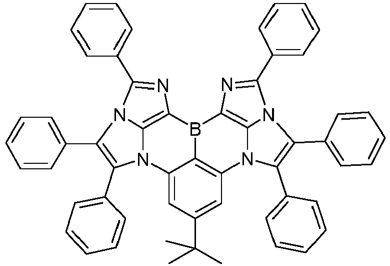 | 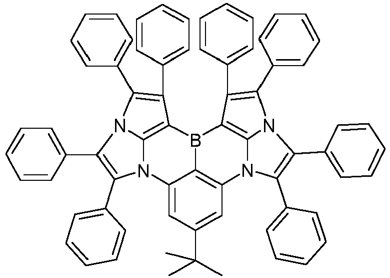 |
| 9 | 10 |
| 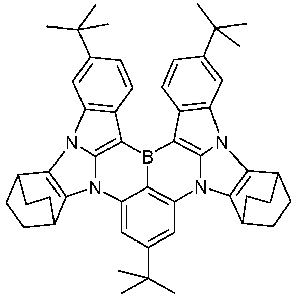 | 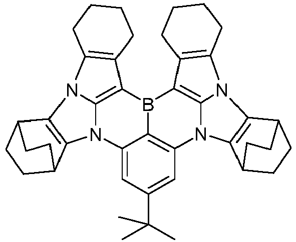 |
| 11 | 12 |
| 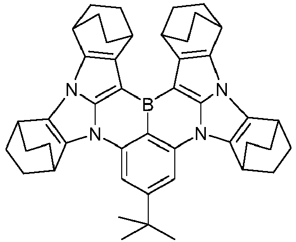 | 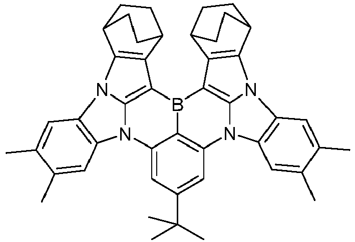 |
| 13 | 14 |
| 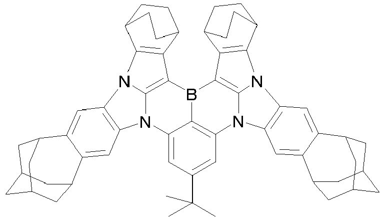 | 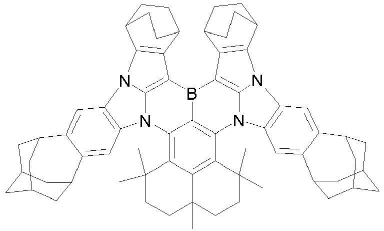 |
| 15 | 16 |
| 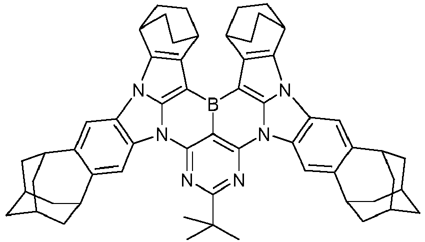 | 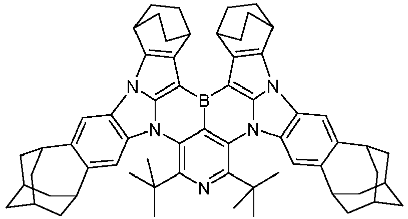 |
| 17 | 18 |
| 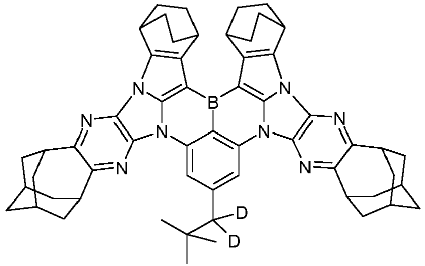 | 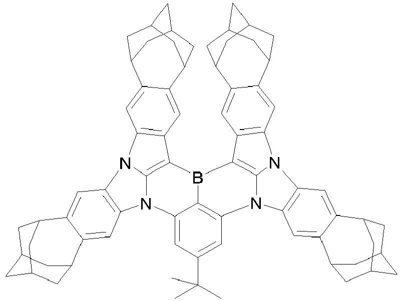 |
| 19 | 20 |
| 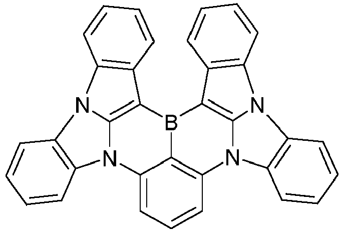 | 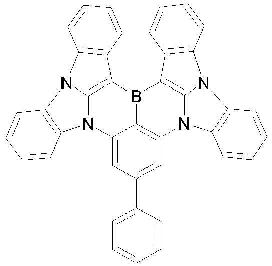 |
| 21 | 22 |
| 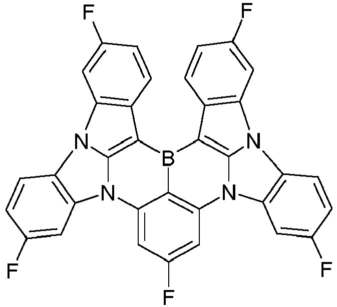 | 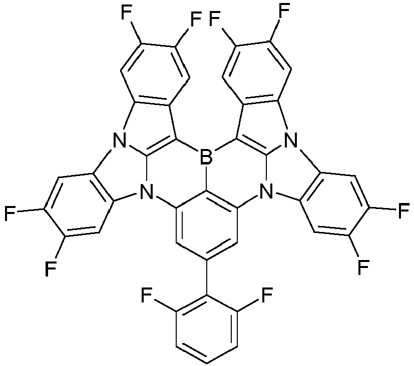 |
| 23 | 24 |
| 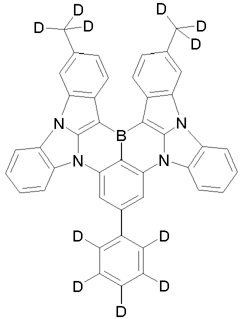 | 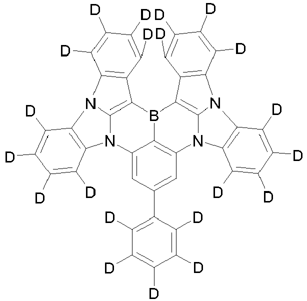 |
| 25 | 26 |
| 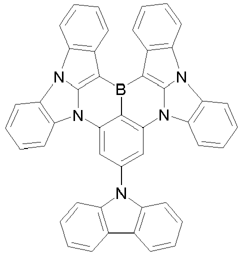 | 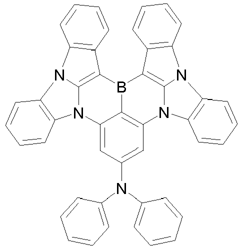 |
| 27 | 28 |
| 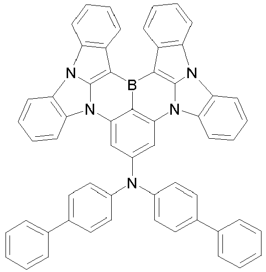 | 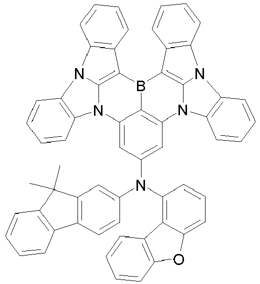 |
| 29 | 30 |
| 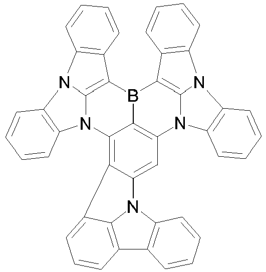 | 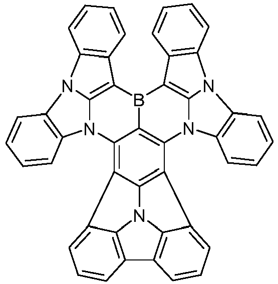 |
| 31 | 32 |
| 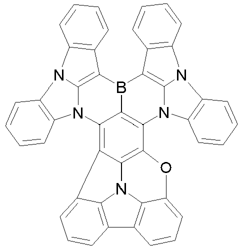 | 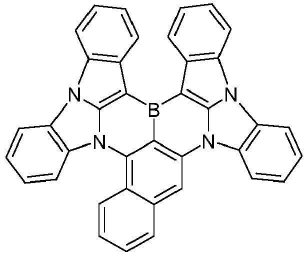 |
| 33 | 34 |
| 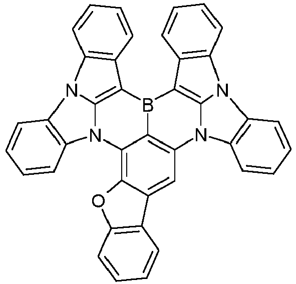 | 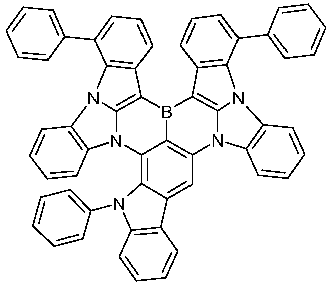 |
| 35 | 36 |
| 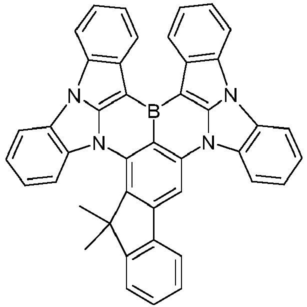 | 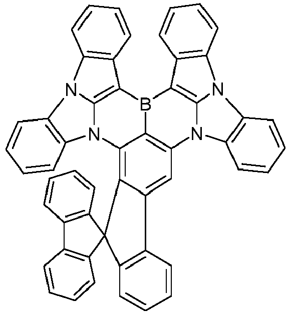 |
| 37 | 38 |
| 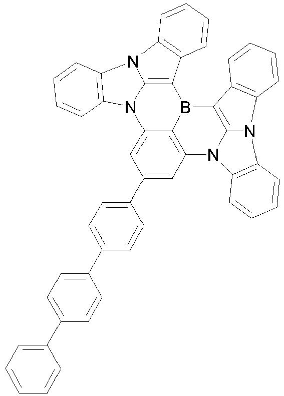 | 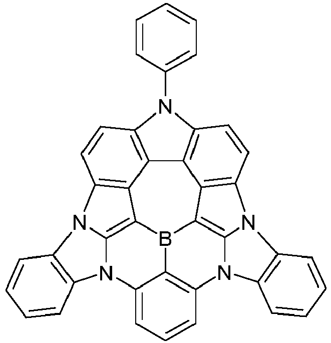 |
| 39 | 40 |
| 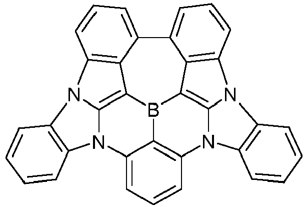 | 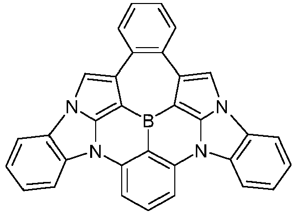 |
| 41 | 42 |
| 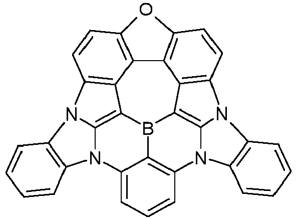 | 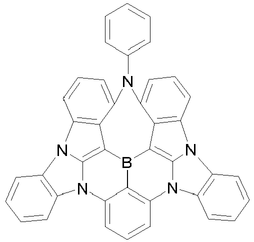 |
| 43 | 44 |
| 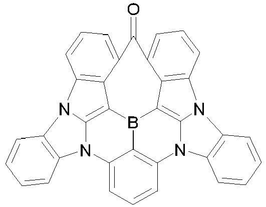 | 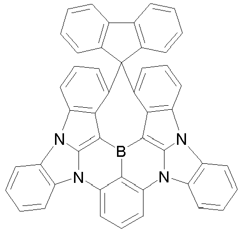 |
| 45 | 46 |
| 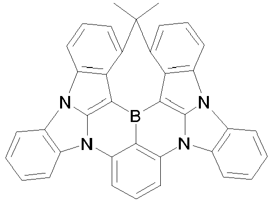 | 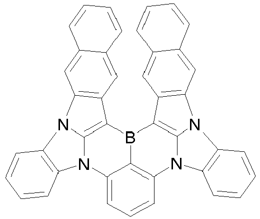 |
| 47 | 48 |
| 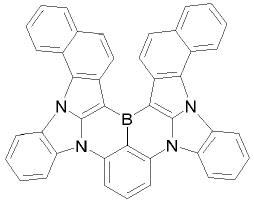 | 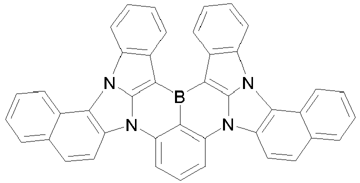 |
| 49 | 50 |
| 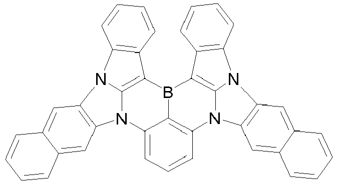 | 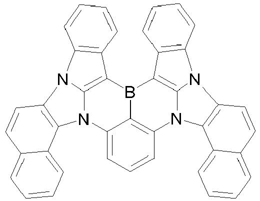 |
| 51 | 52 |
| 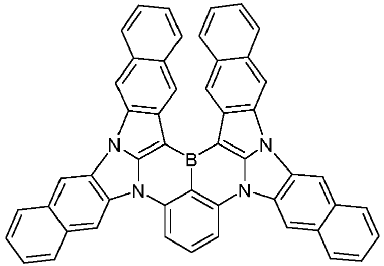 | 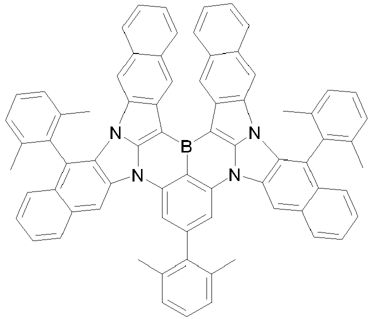 |
| 53 | 54 |
| 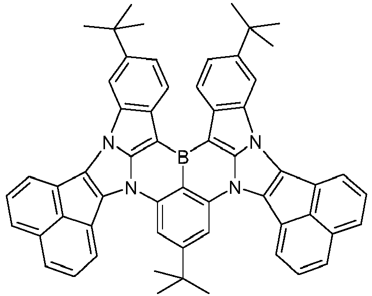 | 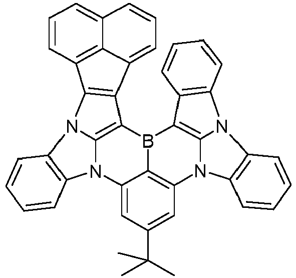 |
| 55 | 56 |
| 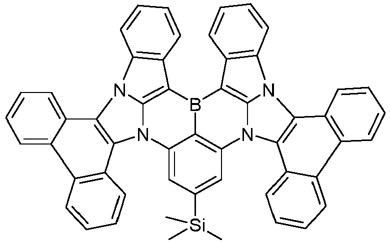 | 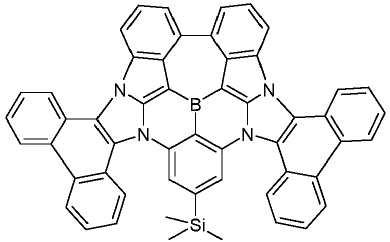 |
| 57 | 58 |
| 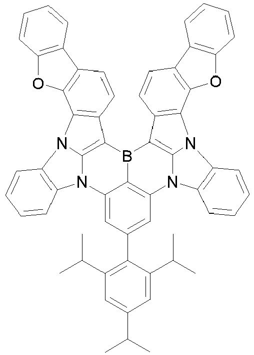 | 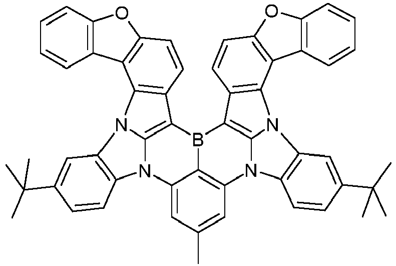 |
| 59 | 60 |
| 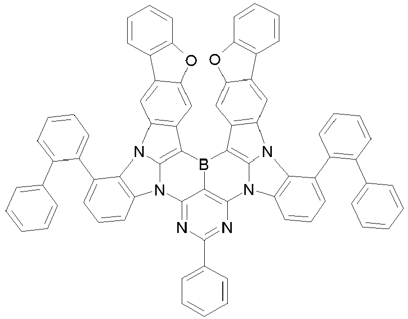 | 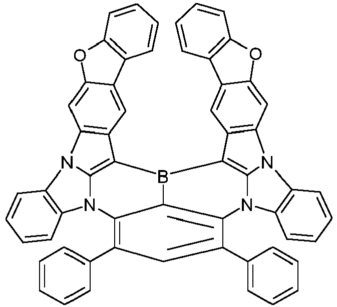 |
| 61 | 62 |
| 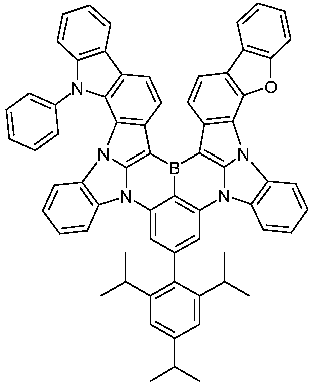 | 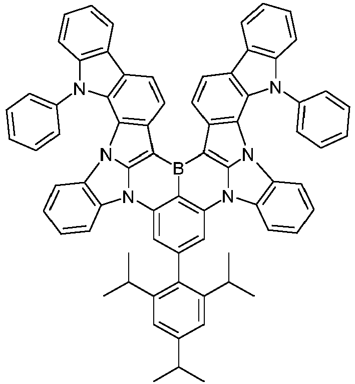 |
| 63 | 64 |
| 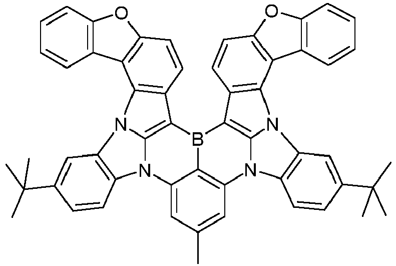 | 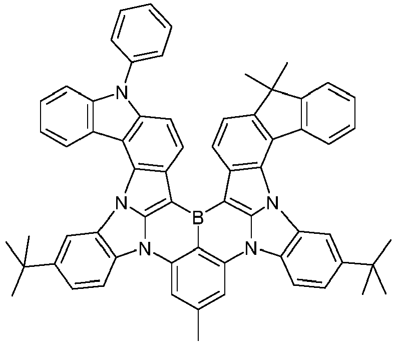 |
| 65 | 66 |
| 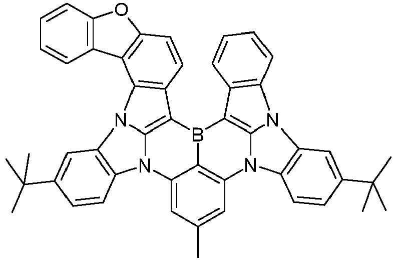 | 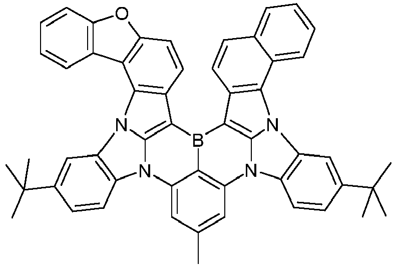 |
| 67 | 68 |
| 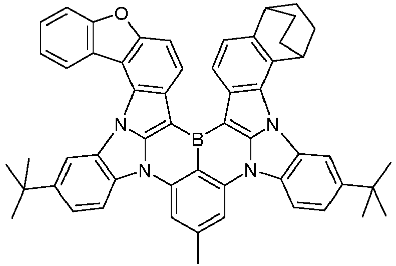 | 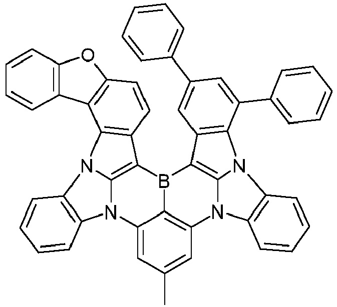 |
| 69 | 70 |
| 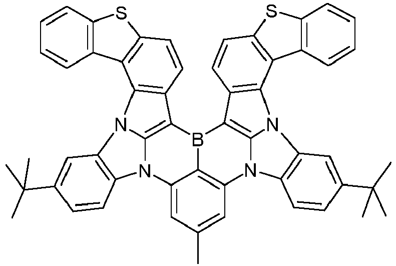 | 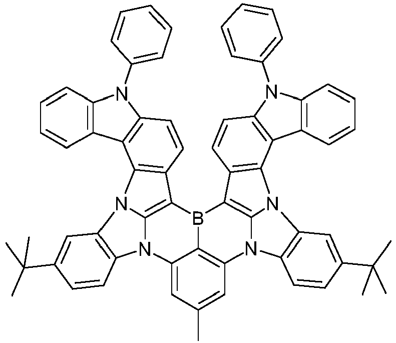 |
| 71 | 72 |
| 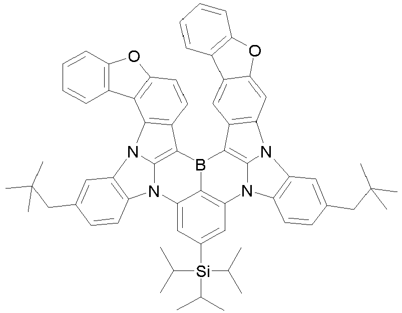 | 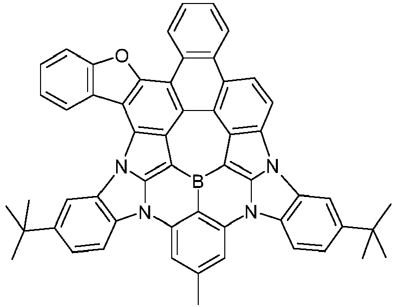 |
| 73 | 74 |
| 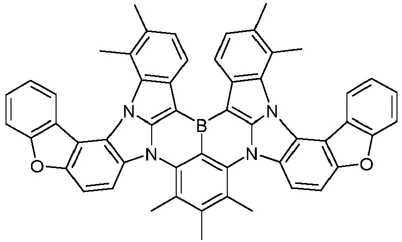 | 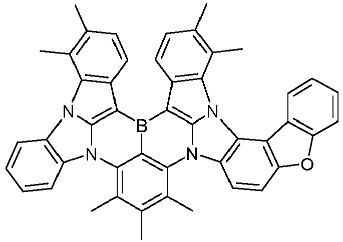 |
| 75 | 76 |
| 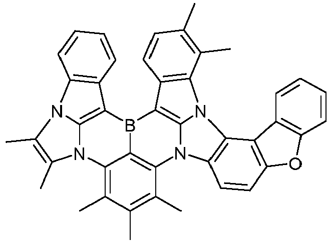 | 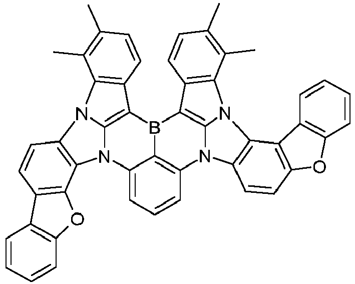 |
| 77 | 78 |
| 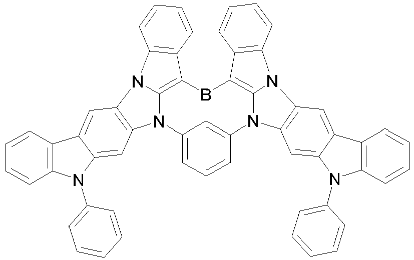 | 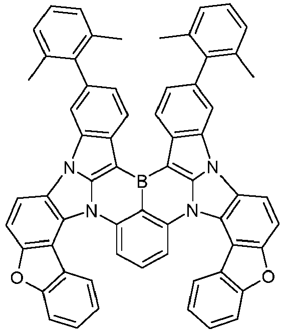 |
| 79 | 80 |
| 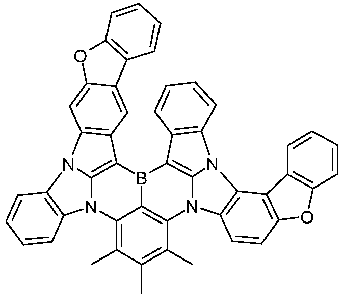 | 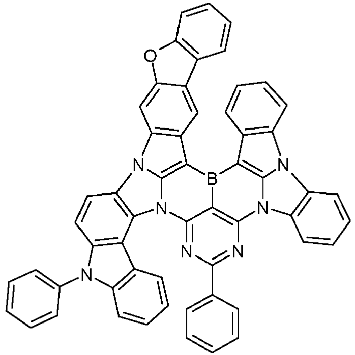 |
| 81 | 82 |
| 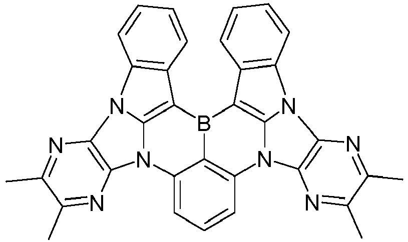 | 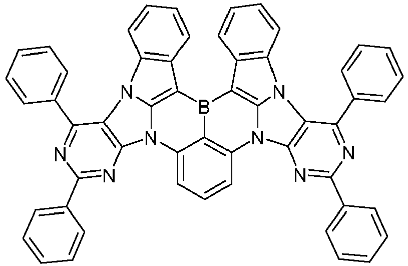 |
| 83 | 84 |
| 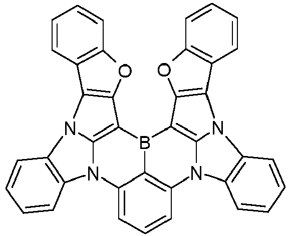 | 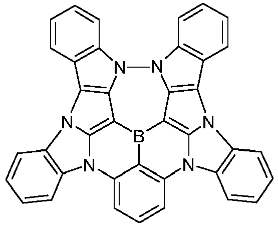 |
| 85 | 86 |
| 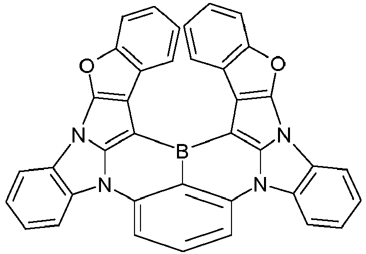 | 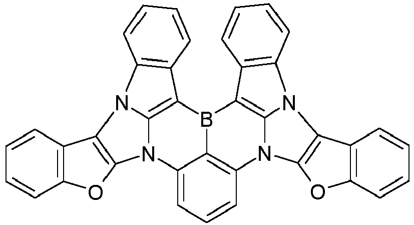 |
| 87 | 88 |
| 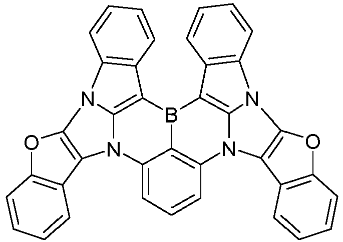 | 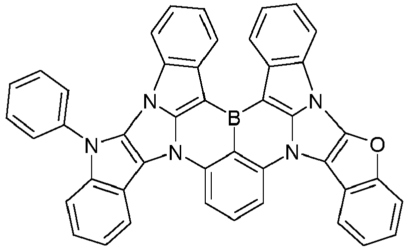 |
| 89 | 90 |
| 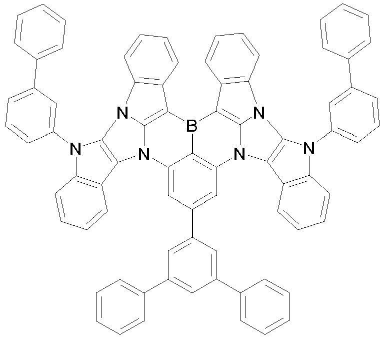 | 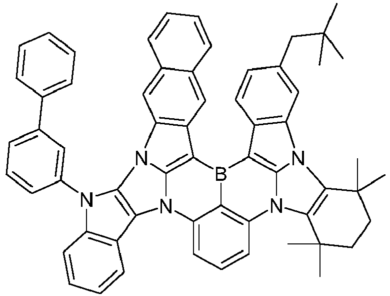 |
| 91 | 92 |
| 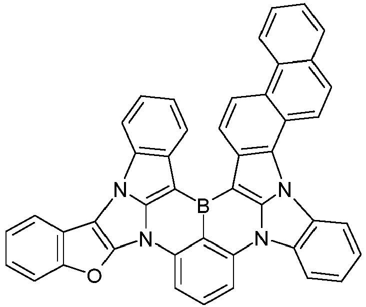 | 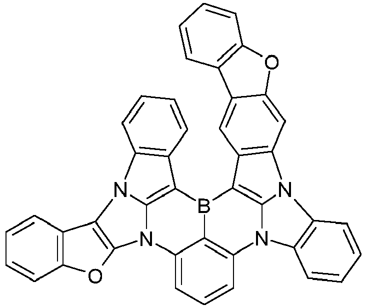 |
| 93 | 94 |
| 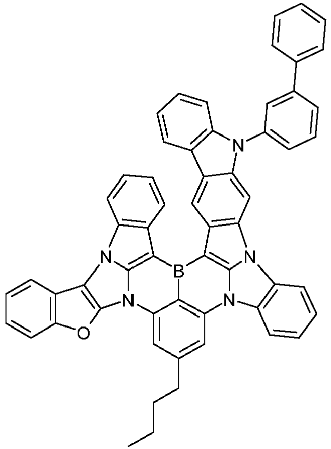 | 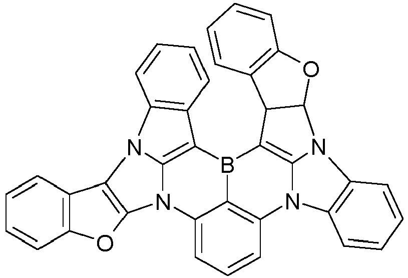 |
| 95 | 96 |
| 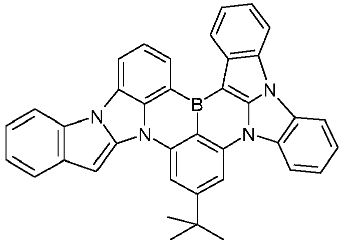 | 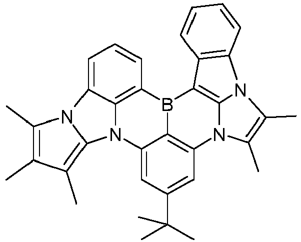 |
| 97 | 98 |
| 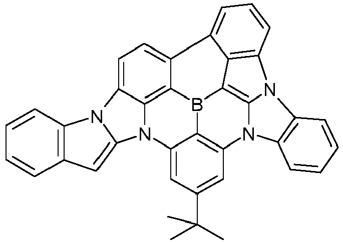 | 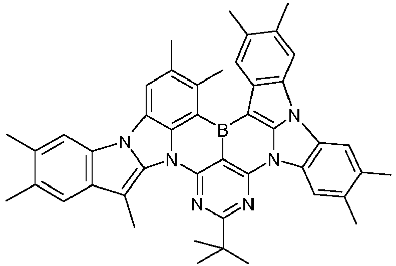 |
| 99 | 100 |
| 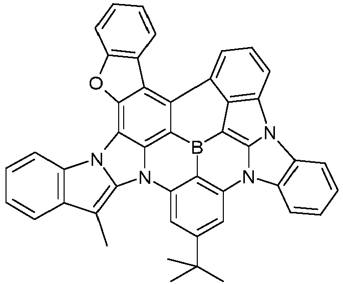 | 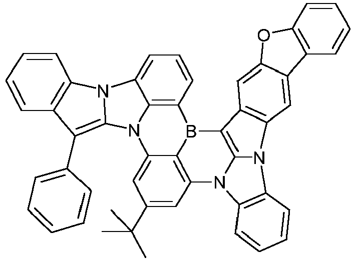 |
| 101 | 102 |
| 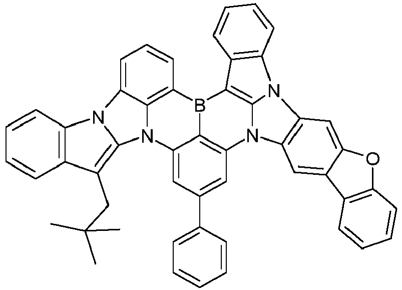 | 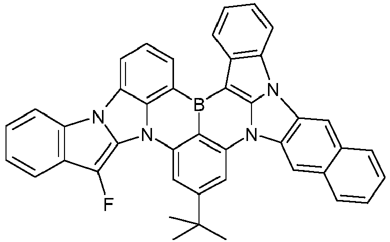 |
| 103 | 104 |
| 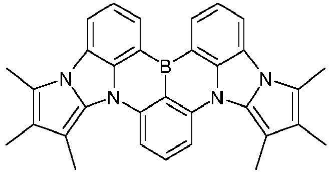 | 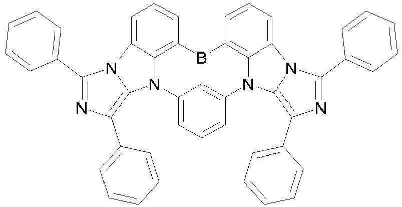 |
| 105 | 106 |
| 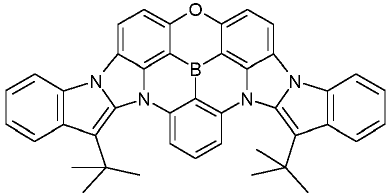 | 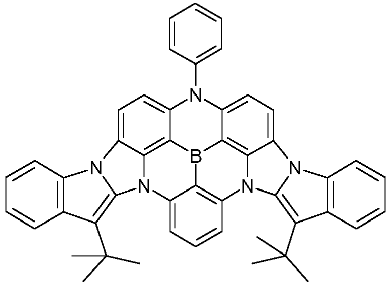 |
| 107 | 108 |
| 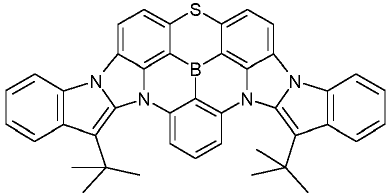 | 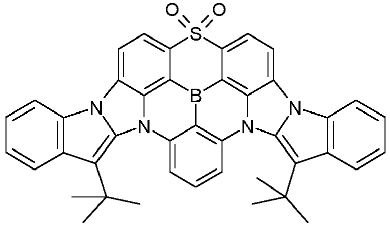 |
| 109 | 110 |
| 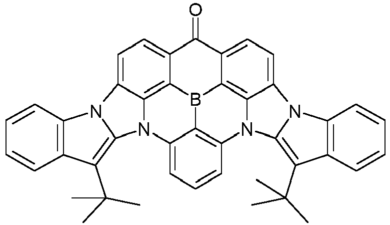 | 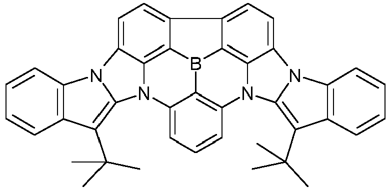 |
| 111 | 112 |
| 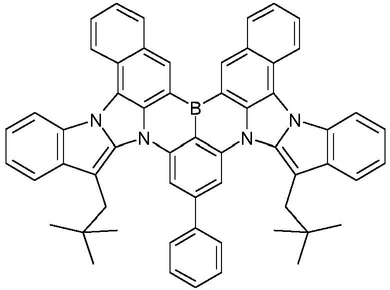 | 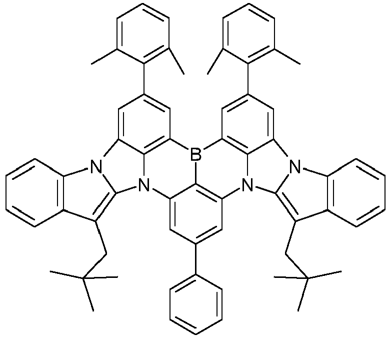 |
| 113 | 114 |
| 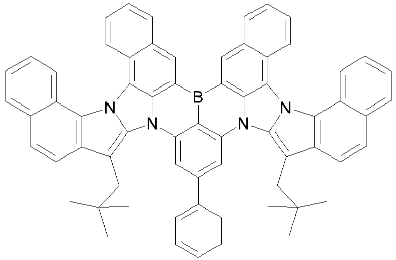 | 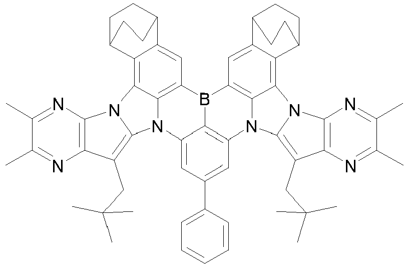 |
| 115 | 116 |
| 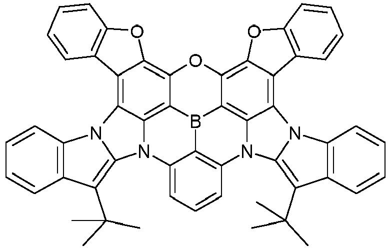 | 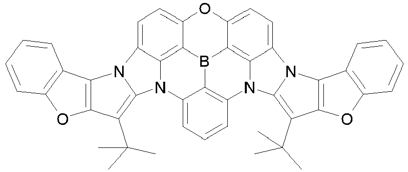 |
| 117 | 118 |
| 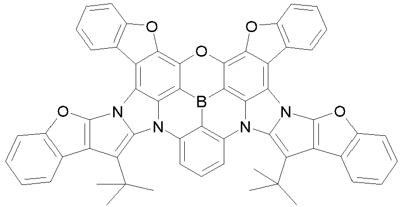 | 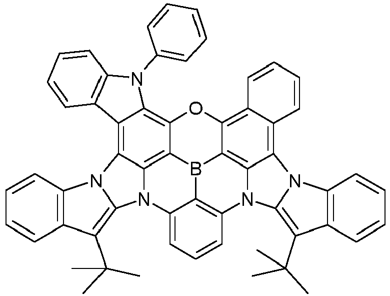 |
| 119 | 120 |
| 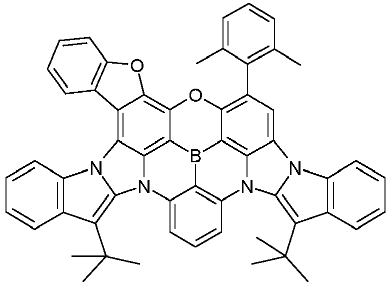 | 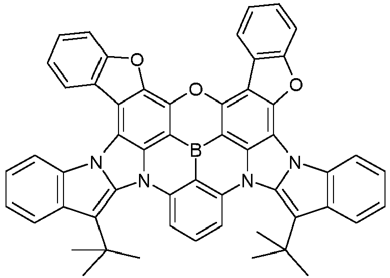 |
| 121 | 122 |
| 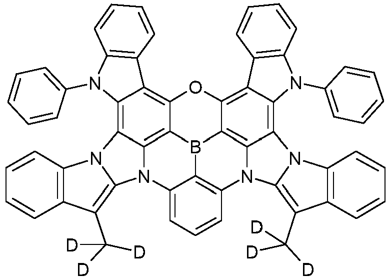 | 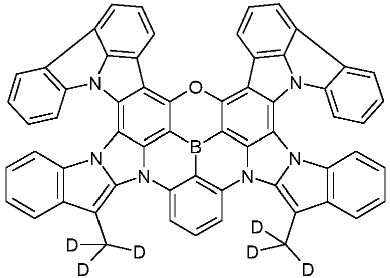 |
| 123 | 124 |
| 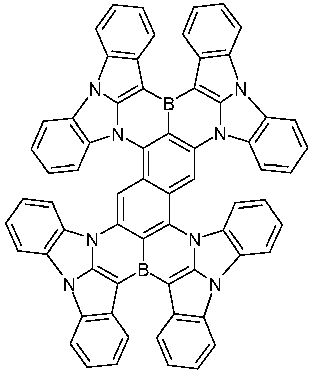 | 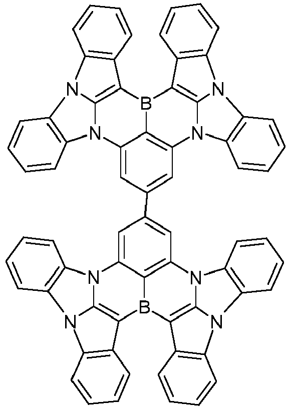 |
| 125 | 126 |
| 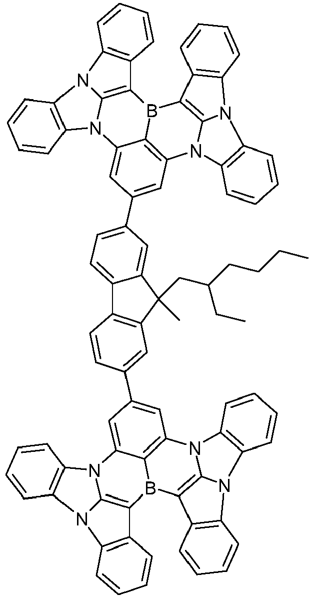 | 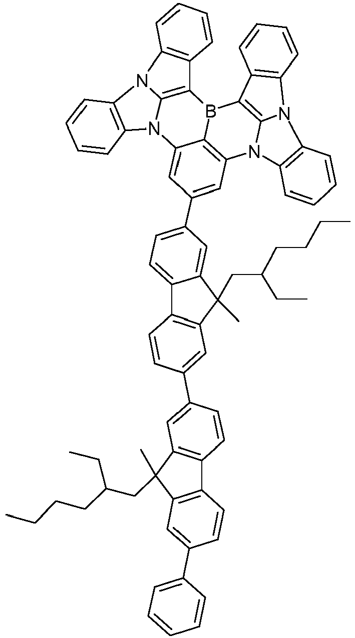 |
| 127 | 128 |
| 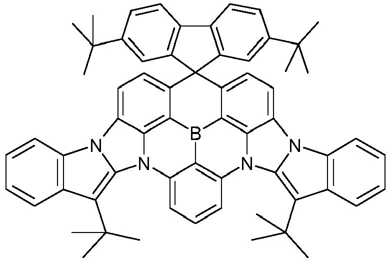 | 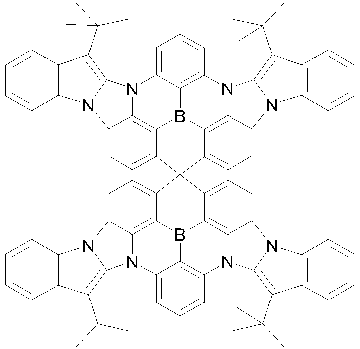 |
| 129 | 130 |
| 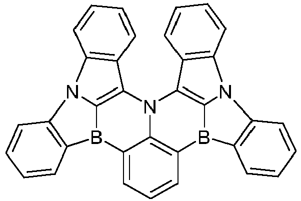 | 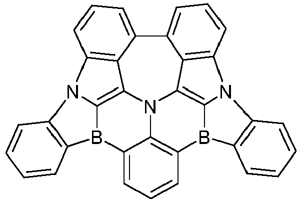 |
| 131 | 132 |
| 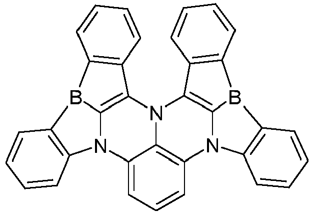 | 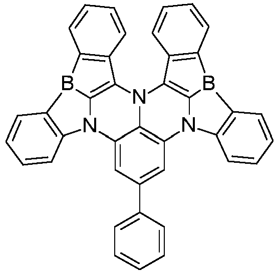 |
| 133 | 134 |
| 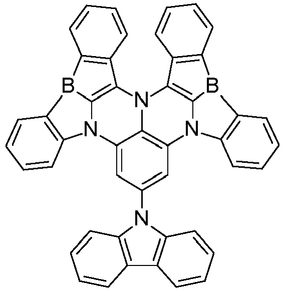 | 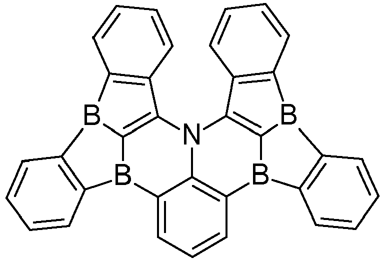 |
| 135 | 136 |
| 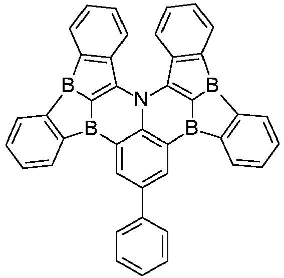 | 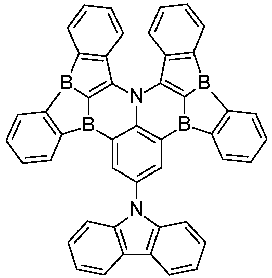 |
| 137 | 138 |
| 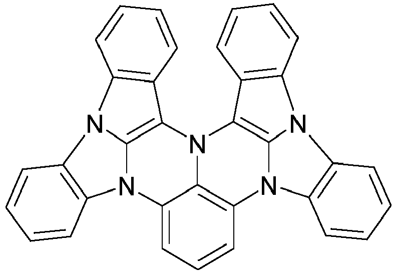 | 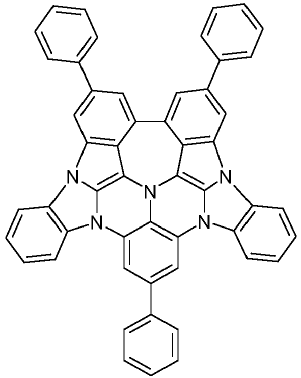 |
| 139 | 140 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem ein Grundgerüst mit mindestens einer der Gruppen Z², Z³ oder einem Vorläufer einer der Gruppe Z², Z³ synthetisiert wird und die Gruppe Z¹ mittels einer Metallierungsreaktion, einer nukleophilen aromatischen Substitutionsreaktion oder einer Kupplungsreaktion eingeführt wird.

Geeignete Verbindungen, umfassend ein Grundgerüst mit einer Gruppe Z², Z³ können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (IIa)-(IIe) und bevorzugten Ausführungsformen dieser Formel oder erfindungsgemäße Verbindungen, wobei ein oder mehrere Bindungen der erfindungsgemäßen Verbindungen oder der Strukturen der Formel (IIa)-(IIe) und bevorzugten Ausführunosforman dieser Formal zum Polymer Oligomer oder Dendrimer vorhanden sind. Je nach Verknüpfung der Strukturen der (IIa)-(IIe) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können koniuaiert, teilkoniuaiert oder nicht-koniuaiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (IIa)-(IIe) bzw. die zuvor und nachfolgend ausgeführten bevorzuaten Ausführunasformen zu 0.01 bis 99.9 mol%, bevorzuat 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formel (IIa)-(IIe) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cvmol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise ein Emitter und/oder ein Matrixmaterial, wobei sich diese Verbindungen von den erfindungsgemäßen Verbindungen unterscheiden. Geeignete Emitter und Matrixmaterialien sind hinten im Zusammenhang mit der organischen Elektrolumineszenzvorrichtung aufgeführt. Die weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung, vorzugsweise als Emitter, besonders bevorzugt als grüner, roter oder blauer Emitter. Hierbei zeigen erfindungsgemäße Verbindungen bevorzugt fluorezierende Eigenschaften und stellen somit bevorzugt fluoreszierenden Emitter bereit. Ferner können erfindungsgemäße Verbindungen als Host-Materialien, Elektronentransportmaterialien und/oder Lochleitermaterialien. Hierbei können insbesondere erfindungsgemäße Verbindungen, bei denen viele, bevorzugt alle der Gruppen Z¹, Z², Z³, Z⁴, Z⁵ für N steht, mit Vorteil als Lochleitermaterial eingesetzt werden. Ferner können insbesondere erfindungsgemäße Verbindungen, bei denen viele, bevorzugt alle der Gruppen Z¹, Z², Z³, Z⁴, Z⁵ für B stehen, mit Vorteil als Elektronentransportmaterial eingesetzt werden.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus Besonders bevorzugt ist elektronische Vorrichtung ausgewählt aus der Gruppe bestehend aus organischen Elektrolumineszenzvorrichtungen (OLEDs, sOLED, PLEDs, LECs, etc.),vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enhalten Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem- Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung (IIa)-(IIe) gemäß Formel bzw. die oben ausgeführten bevorzugten Ausführungsformen in einer emittierenden Schicht als Emitter, vorzugsweise roter, grüner oder blauer Emitter.

Wenn die erfindungsgemäße Verbindung als Emitter in einer emittierenden Schicht eingesetzt wird, wird bevorzugt ein geeignetes Matrixmaterial eingesetzt, welches als solches bekannt ist.

Eine bevorzugte Mischung aus der erfindungsgemäßen Verbindung und einem Matrixmaterial enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% an Matrixmaterial bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

In einer bevorzugten Ausgestaltung wird eine erfindungsgemäße Verbindung, die als Emitter verwendet wird, vorzugsweise in Kombination mit einem oder mehreren phosphoreszierenden Materialien (Triplettemitter) und/oder einer Verbindung eingesetzt, die ein TADF-Hostmaterial (thermally activated delayed fluorescence) darstellt. Hierbei wird vorzugsweise ein Hyperfluoreszenz- und/oder Hyperphosphoreszenz-System gebildet.

In WO 2015/091716 A1 und in WO 2016/193243 A1 werden OLEDs offenbart, die in der Emissionsschicht sowohl eine phosphoreszierende Verbindung als auch einen fluoreszierenden Emitter enthalten, wobei die Energie von der phosphoreszierenden Verbindung auf den fluoreszierenden Emitter übertragen wird (Hyperphosphoreszenz). Die phosphoreszierende Verbindung verhält sich in diesem Zusammenhang demnach wie ein Host-Material. Wie der Fachmann weiß, haben Hostmaterialien höhere Singulett und Triplett-Energien im Vergleich zu dem Emittern, damit die Energie des Host-Materials auch möglichst optimal auf den Emitter übertragen werden. Die im Stand der Technik offenbarten Systeme weisen genau solch eine Energierelation auf.

Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/001990, WO 2018/019687, WO 2018/019688, WO 2018/041769, WO 2018/054798, WO 2018/069196, WO 2018/069197, WO 2018/069273, WO 2018/178001, WO 2018/177981, WO 2019/020538, WO 2019/115423, WO 2019/158453 und WO 2019/179909 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine erfindungsgemäße Verbindung kann vorzugsweise in Kombination mit einem TADF-Hostmaterial und/oder einem TADF-Emitter eingesetzt werden, wie dies zuvor dargelegt ist.

Der als thermisch aktivierte verzögerte Fluoreszenz (TADF = "thermally activated delayed fluorescence") bezeichnete Vorgang wird beispielsweise von B. H. Uoyama et al., Nature 2012, Vol. 492, 234 beschrieben. Um diesen Prozess zu ermöglichen, ist im Emitter ein vergleichsweise kleiner Singulett-Triplett-Abstand ΔE(S₁ - T₁) von zum Beispiel weniger als etwa 2000 cm⁻¹ nötig. Um den an sich spin-verbotenen Übergang T₁ → S₁ zu öffnen, kann neben dem Emitter eine weitere Verbindung in der Matrix vorgesehen werden, die eine starke Spin-Bahn-Kopplung aufweist, sodass über die räumliche Nähe und die damit mögliche Wechselwirkung zwischen den Molekülen ein Inter-System-Crossing ermöglicht wird, oder die Spin-Bahn-Kopplung wird über ein im Emitter enthaltenes Metallatom erzeugt.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Ferner ist eine organische Elektrolumineszenzvorrichtung bevorzugt, ist eine organische Elektrolumineszenzvorrichtung, enthaltend eine Verbindung gemäß Formel (IIa)-(IIe) bzw. die oben ausgeführten bevorzugten Ausführungsformen in einer lochleitenden Schicht als Lochleitermaterial. Hierbei sind insbesondere Verbindungen bevorzugt, bei denen Z¹ N ist und mindestens eine, vorzugsweise zwei der Gruppen Z², Z³, Z⁴, Z⁵ für N steht/stehen. Ferner sind hierbei insbesondere Verbindungen bevorzugt, bei denen mindestens eine, vorzugsweise zwei der Gruppen Z², Z³ N ist/sind und mindestens eine, vorzugsweise zwei der Gruppen Z⁴, Z⁵ für N steht/stehen.

Weiterhin ist eine organische Elektrolumineszenzvorrichtung bevorzugt, enthaltend eine Verbindung gemäß Formel (IIa)-(IIe) bzw. die oben ausgeführten bevorzugten Ausführungsformen in einer elektronenleitenden Schicht als Elektronentransportmaterial. Hierbei sind insbesondere Verbindungen bevorzugt, bei denen Z¹ B ist und mindestens eine, vorzugsweise zwei der Gruppen Z², Z³, Z⁴, Z⁵ für B steht/stehen. Ferner sind hierbei insbesondere Verbindungen bevorzugt, bei denen mindestens eine, vorzugsweise zwei der Gruppen Z², Z³ B ist/sind und mindestens eine, vorzugsweise zwei der Gruppen Z⁴, Z⁵ für B steht/stehen

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (IIa)-(IIe) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (IIa)-(IIe) oder deren oder deren zuvor dargelegten bevorzugten Ausführungsformen sind neu Ein weiterer Gegenstand der vorliegenden Erfindung ist daher eine Formulierungen, enthaltend mindestens ein Lösungsmittel und eine Verbindung gemäß Formel(IIa)-(IIe) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen gemäß Formel (IIa)-(IIe) bzw. die zuvor und nachfolgend ausgeführten bevorzugten Ausführungsformen als Emitter weisen sehr schmal Emissionsbanden mit geringen FWHM-Werten (Full Width Half Maximum) auf und führen zu besonders Farb-reiner Emission, erkennbar an den kleinen CIE-y-Werten. Besonders überraschend ist hierbei, dass sowohl blaue Emitter mit geringen FWHM-Werten als auch Emitter mit aerinaen FWHM-Werten bereitgestellt werden, die im grünen, gelben oder roten Bereich des Farbspektrums emittieren.
2. Elektronische Vorrichtungen, insbesondere organische Elektrolumineszenzevorrichtungen enthaltend Verbindungen gemäß bzw. die zuvor und nachfolgend ausgeführten bevorzugten Ausführungsformen, insbesondere als Emitter, als Lochleitermaterial und/oder als Elektronentransportmaterial weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
3. Elektronische Vorrichtungen, insbesondere organische Elektrolumineszenzvorrichtungen enthaltend Verbindungen gemäß Formel (IIa)-(IIe) bzw. die zuvor und nachfolgend ausgeführten bevorzugten Ausführungsformen als Emitter, als Lochleitermaterial und/oder als Elektronentransportmaterial weisen eine hervorragende Effizienz auf. Hierbei bewirken erfindungsgemäße Verbindungen gemäß Formel (IIa)-(IIe) bzw. die zuvor und nachfolgend ausgeführten bevorzugten Ausführungsformen eine geringe Betriebsspannung bei Verwendung in elektronischen Vorrichtungen.
4. Die erfindungsgemäßen Verbindungen gemäß Formel (IIa)-(IIe) bzw. die zuvor und nachfolgend ausgeführten bevorzugten Ausführungsformen zeigen eine sehr hohe Stabilität und Lebensdauer.
5. Mit Verbindungen gemäß Formel (IIa)-(IIe) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus
6. Verbindungen gemäß Formel (IIa)-(IIe) bzw. die zuvor und nachfolgend ausgeführten bevorzugten Ausführungsformen weisen eine ausgezeichnete Glasfilmbildung auf.
7. Verbindungen gemäß Formel (IIa)-(IIe) bzw. die zuvor und nachfolgend ausgeführten bevorzugten Ausführungsformen bilden aus Lösungen sehr gute Filme und zeigen eine ausgezeichnete Löslichkeit

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbinden die mehrere enantiomere, diastereomere oder tautomere Formen aufweisen können wird eine Form stellvertretend gezeigt.

### Synthese von Synthonen S:

### Beispiel S1:

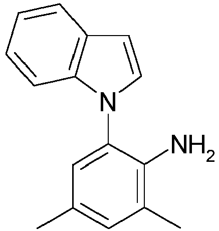

Darstellung nach W. Wu et al., Organic Chemistry Frontiers, 2019, 6(13), 2200 unter Einsatz von Indol [120-72-9] und 2-lod-4,6-dimethyl-anilin [4102-54-9], Ausbeute: 64 %; Reinheit: ca. 95 %ig n. ¹H-NMR.

Analog können folgende Verbindungen dargestellt werden.

| **Bsp.** | **Edukt** | **Produkt** | **Ausbeute** |
|---|---|---|---|
| S2 | 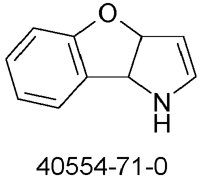 | 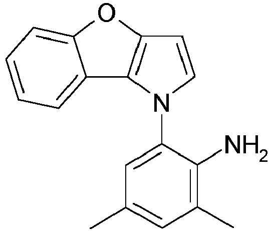 | 59 % |
| S3 | 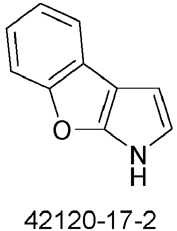 | 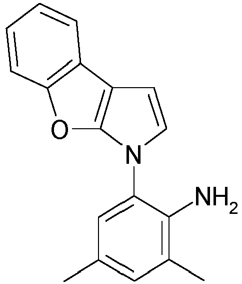 | 69 % |
| S4 | 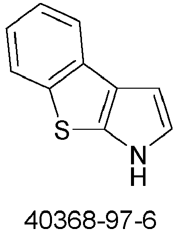 | 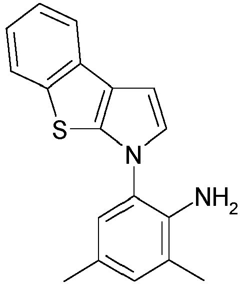 | 56 % |
| S5 | 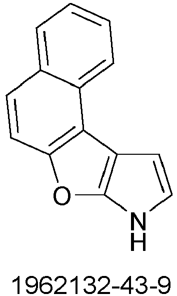 | 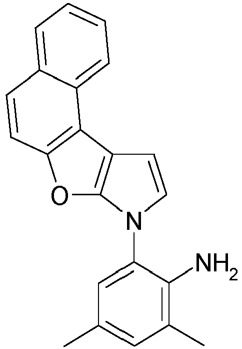 | 60 % |
| S6 | 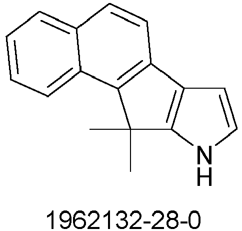 | 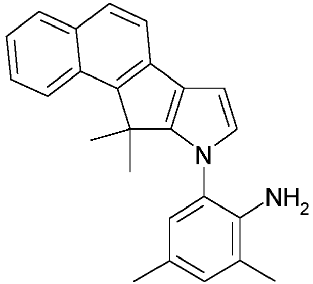 | 67 % |
| S7 | 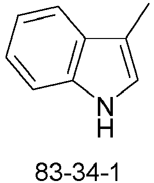 | 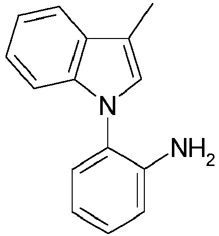 | 49 % |
| | 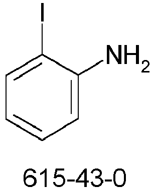 | | |

### Beispiel S10:

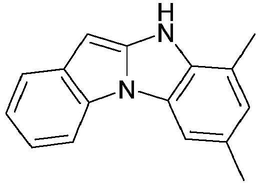

Darstellung nach US2019/0252623, S. 62. Einsatz von S1 anstelle von Verbindung A, Durchführung von Schritt 1 bis 3, Ausbeute: 38 %; Reinheit: ca. 95 %ig n. ¹H-NMR.

Analog können folgende Verbindungen dargestellt werden.

| **Bsp ·** | **Edukt** | **Produkt** | **Ausbeut e** |
|---|---|---|---|
| S11 | 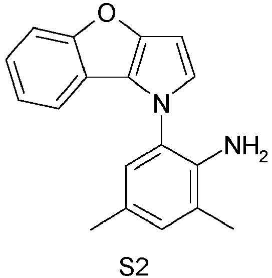 | 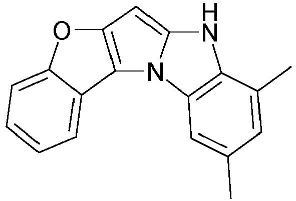 | 40 % |
| S12 | 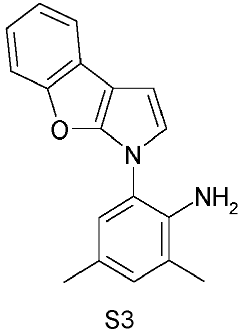 | 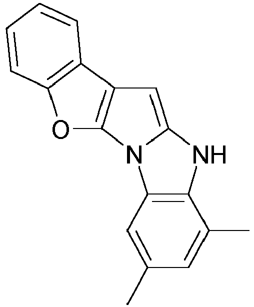 | 40 % |
| S13 | 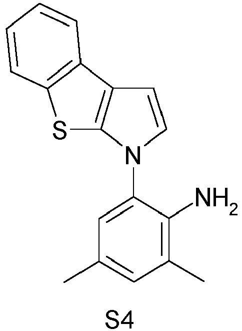 | 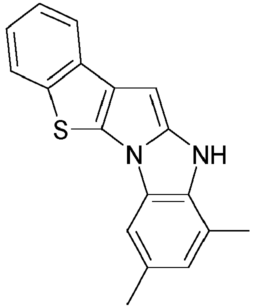 | 35 % |
| S14 | 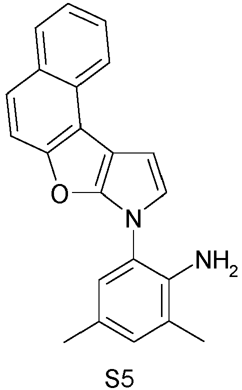 | 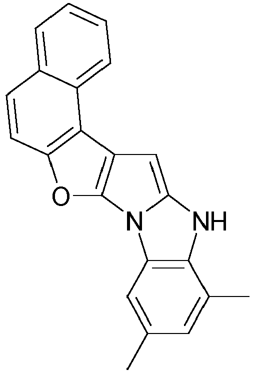 | 38 % |
| S15 | 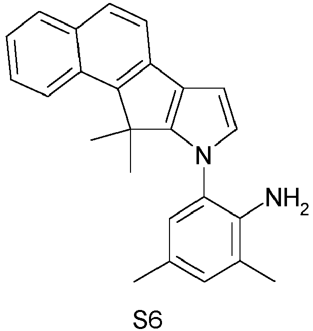 | 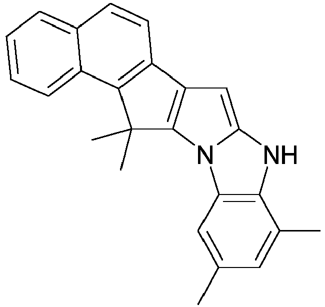 | 42 % |
| S16 | 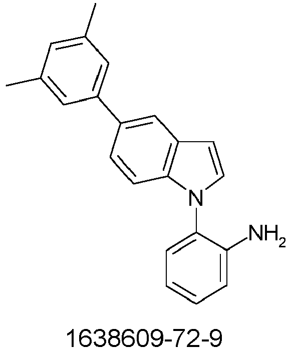 | 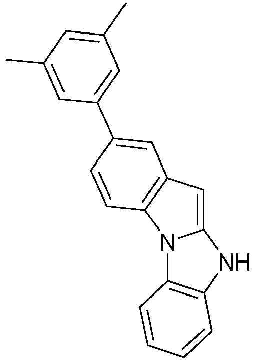 | 35 % |
| S17 | 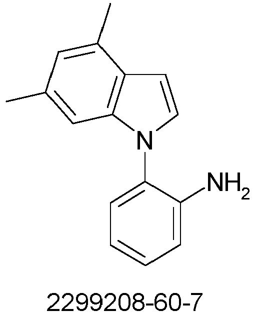 | 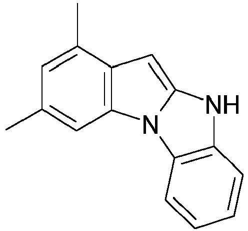 | 38 % |
| S18 | 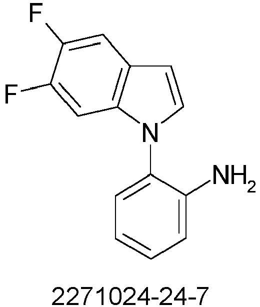 | 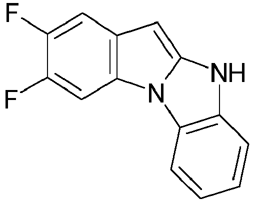 | 41 % |
| S19 | 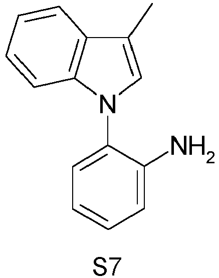 | 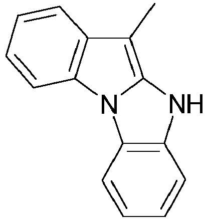 | 50 % |

### Beispiel S100:

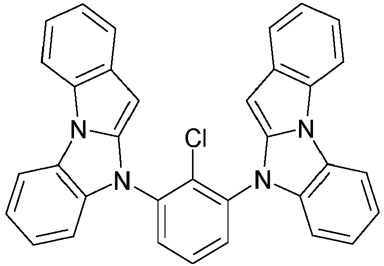

Ein Gemisch aus 22.7 g (110 mmol) 10H-Indolo[1,2-a]benzimidazole [2345630-10-4], 11.3 g (50 mmol) 1-Brom-2,3-dichlorbenzol [56691-77-4], 12.5 g (130 mmol) Natrium-tert-butanolat [865-48-5], 354 mg (0.5 mmol) (Amphos)₂PdCl₂ [887919-35-9] und 200 ml o-Xylol wird 3 h bei 80 °C und dann 6 h bei 130 °C gerührt. Nach Erkalten wird die Reaktionsmischung mit 500 ml Ethylacetat und 500 ml Wasser versetzt, die org. Phase wird abgetrennt, einmal mit 500 ml Wasser und zweimal mit je 300 ml ges. Kochsalzlösung gewaschen und über Magnesiumsulfat getrocknet. Man filtriert über ein mit Ethylacetat vorgeschlämmtes Kieselgelbett ab, engt das Filtrat zur Trockene ein, kocht den Rückstand mit 300 ml Ethanol aus, filtriert vom Feststoff ab, wäscht diesen zweimal mit 50 ml Ethanol, trocknet im Vakuum und kristallisiert aus Dichlormethan / Acetonitril um. Ausbeute: 15.2 g (29 mmol) 58 %; Reinheit: ca. 95 % n. ¹H-NMR.

Analog können folgende Verbindungen dargestellt werden:

| Bsp. | Edukt | Produkt | Ausbeute |
|---|---|---|---|
| S101 | 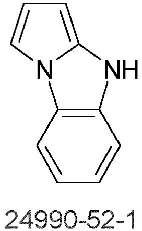 | 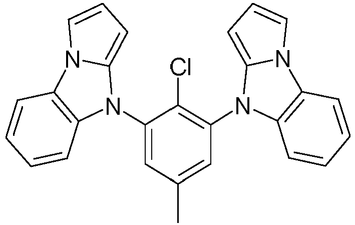 | 30 % |
| | 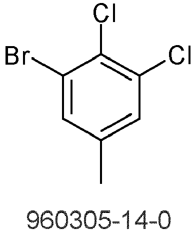 | | |
| S102 | 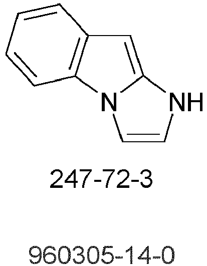 | 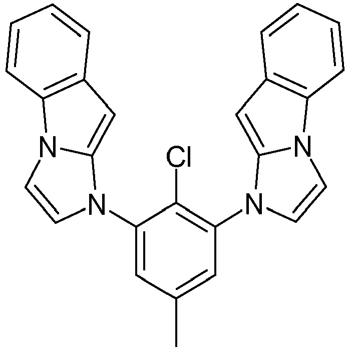 | 27 % |
| S103 | 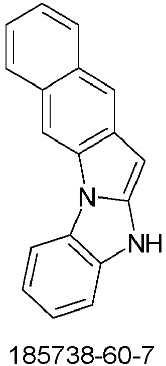 | 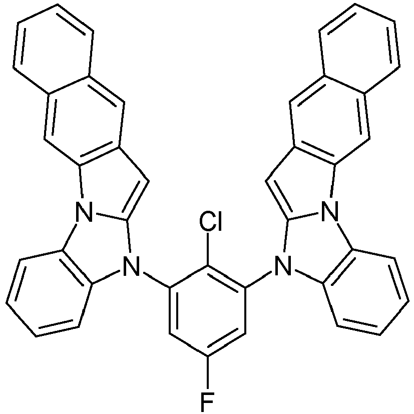 | 51 % |
| | 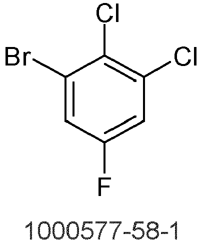 | | |
| S104 | 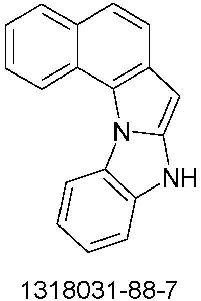 | 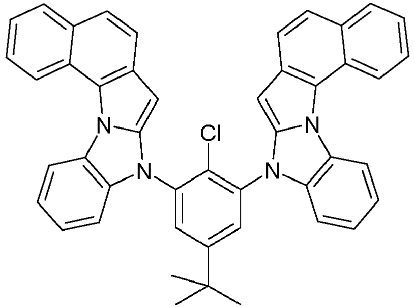 | 49 % |
| | 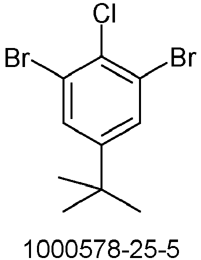 | | |
| S105 | 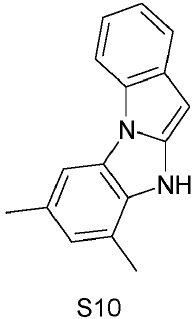 | 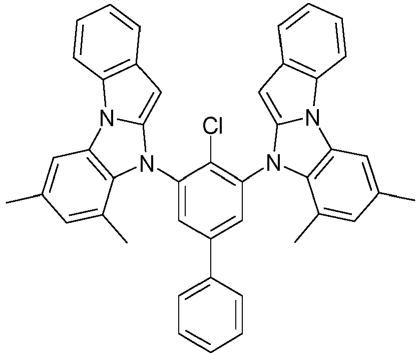 | 55 % |
| | 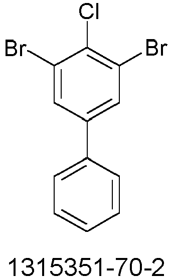 | | |
| S106 | 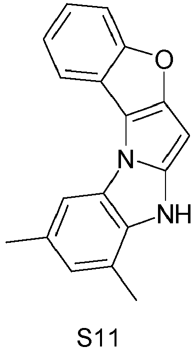 1315351-70-2 | 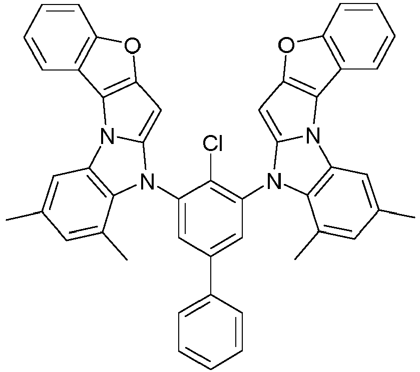 | 43 % |
| S107 | 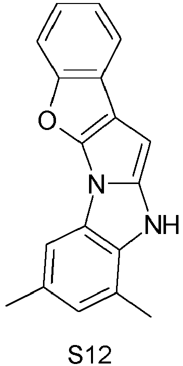 56691-77-4 | 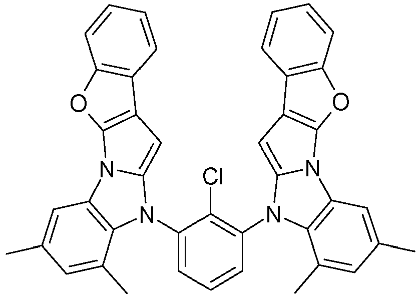 | 48 % |
| S108 | 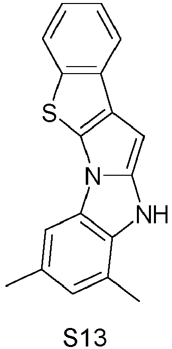 1000578-25-5 | 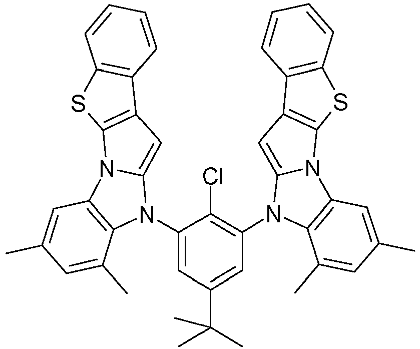 | 45 % |
| S109 | 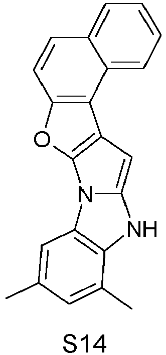 1000578-25-5 | 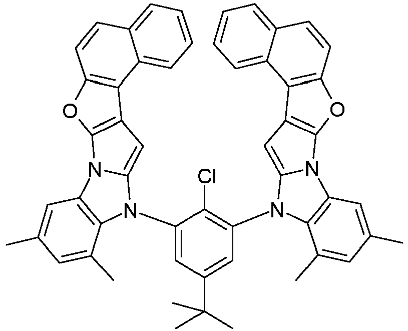 | 47 % |
| S110 | 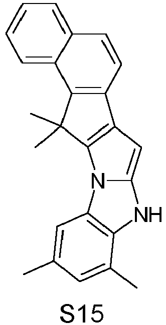 1315351-70-2 | 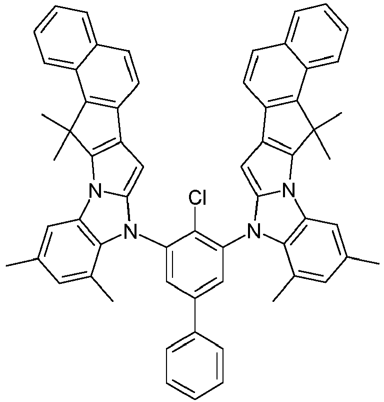 | 50 % |
| S111 | 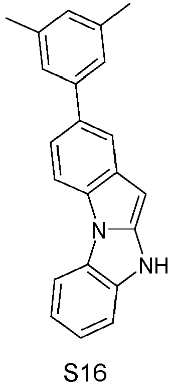 56691-77-4 | 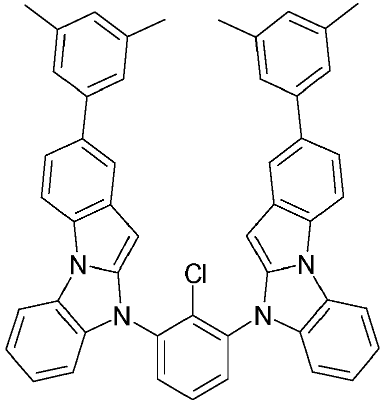 | 53 % |
| S112 | 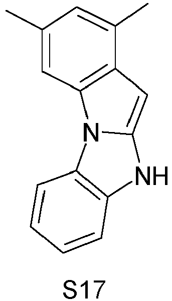 56691-77-4 | 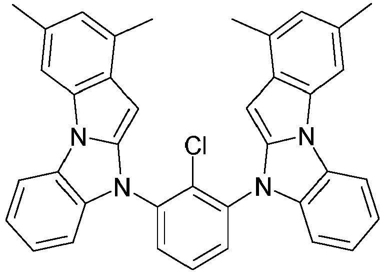 | 57 % |
| S113 | 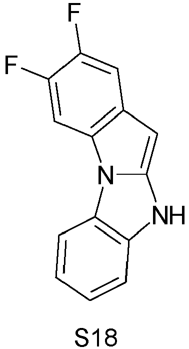 56691-77-4 | 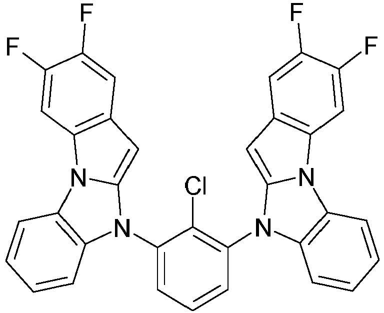 | 46 % |
| S114 | 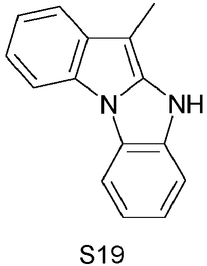 1315351-70-2 | 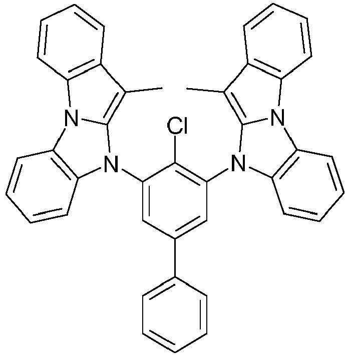 | 43 % |

### Beispiel Dotand D100A und D100B und D100C:

### Schritt 1: Lithiierung von S100

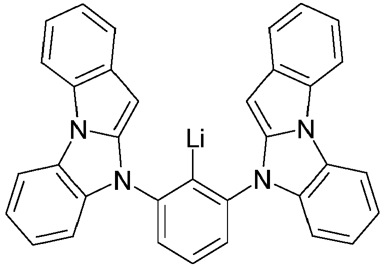

### Intermediat nicht isoliert

In einen ausgeheizten, mit Argon inertisierten Vierhalskolben mit Magnetrührkern, Tropftrichter, Wasserabscheider, Rückflusskühler und Argonüberlagerung werden 26.1 g (50 mmol) S100 und 200 ml tert-Butylbenzol vorgelegt und auf - 40 °C gekühlt. Das Gemisch wird tropfenweise während 10 min. mit 64.7 ml (110 mmol) tert-BuLi, 1.7 M in *n*-Pentan versetzt. Man lässt die Reaktionsmischung auf Raumtemperatur erwärmen und rührt 3 h bei 60 °C nach, wobei das *n*-Pentan über den Wasserabscheider abdestilliert wird.

### Schritt 2: Transmetallierung und Cyclisierung

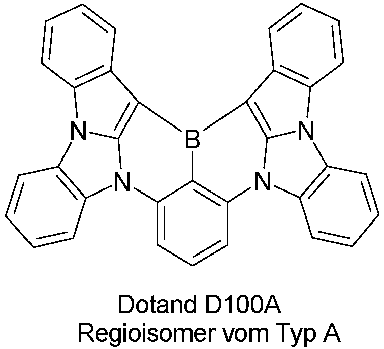

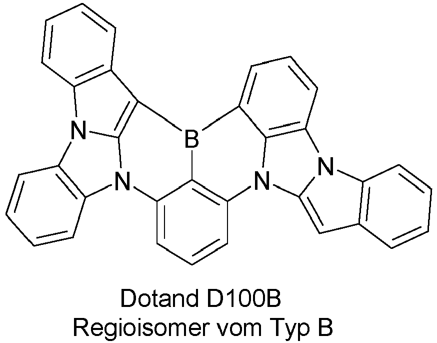

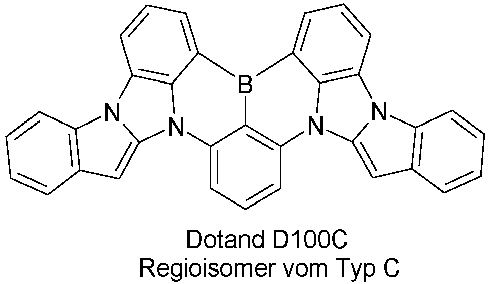

Die Reaktionsmischung wird wieder auf - 40 °C abgekühlt. Über einen Zeitraum von ca. 10 min. werden 5.7 ml (60 mmol) Bortribromid zugetropft. Nach erfolgter Zugabe wird die Reaktionsmischung 1 h bei RT gerührt. Dann wird die Reaktionsmischung auf 0 °C abgekühlt und über einen Zeitraum von ca. 30 min. tropfenweise mit 21.0 ml (120 mmol) Di-isopropylethylamin versetzt. Anschließend wird die Reaktionsmischung 5 h bei 130 °C gerührt. Nach Erkalten verdünnt man mit 500 ml Toluol, hydrolysiert durch Zugabe von 300 ml wässriger 10 Gew.-% iger Kaliumacetat-Lösung, trennt die org. Phase ab und engt diese im Vakuum zur Trockene ein. Der ölige Rückstand wird mit DCM auf ISOLUTE^{®} absorbiert und mit einem *n*-Pentan-DCM Gemisch (10:1) heiß über ein Kieselgel-Bett filtriert. Man engt das Filtrat zur Trockene ein. Der Rückstand wird flash-chromatographiert, Kieselgel, *n*-Heptan/Ethylacetat, Gradient, Säulenautomat Torrent der Fa. A. Semrau, wodurch die drei Isomeren Dotanden D100A, D100B und D100C getrennt werden. Jeder einzelne Dotand kann erneut durch Flash-Chromatographie weiter gereinigt werden. Die weitere Reinigung erfolgt durch wiederholte Heißextraktions-kristallisation mit DCM/Acetonitril-Gemischen und abschließende fraktionierte Sublimation oder Tempern im Hochvakuum. Ausbeuten: D100A: 1.98 g (4.0 mmol) 8 %, D100B: 4.60 g (9.3 mmol) 19 %, D100C: 3.56 g (7.2 mmol) 14 %; Reinheit: ca. 99.9 % n. ¹H-NMR.

Analog können folgende Verbindungen dargestellt werden:

| Bsp. | Edukt | Produkte | Ausbeute |
|---|---|---|---|
| D101A | S101 | 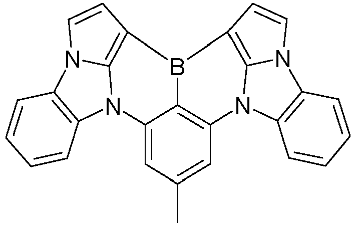 | 4 % |
| D101B | | 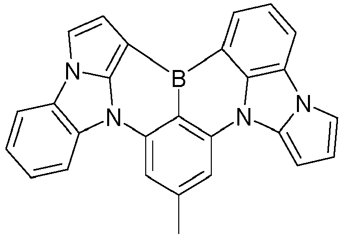 | 9 % |
| D101C | | 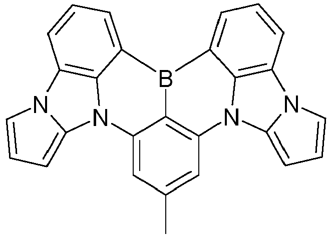 | 5 % |
| D102A | S102 | 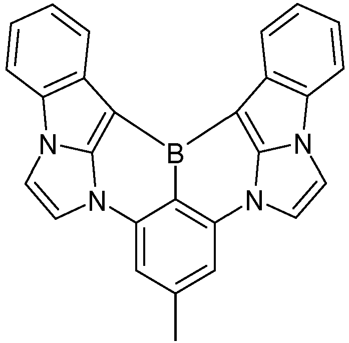 | 3 % |
| D103A | S103 | 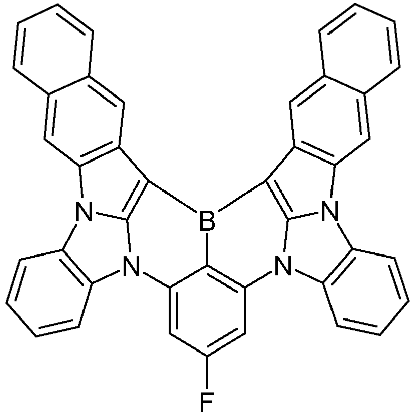 | 10 % |
| D103B | | 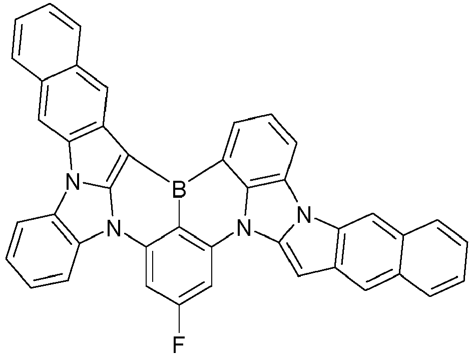 | 17 % |
| D103C | | 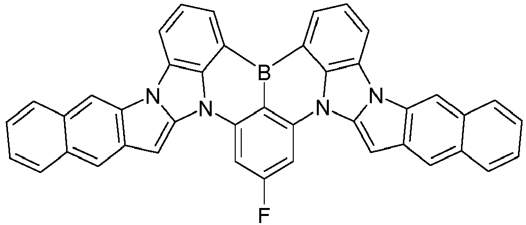 | 17 % |
| D104A | S104 | 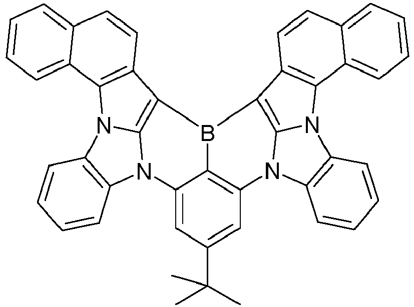 | 12 % |
| D104B | | 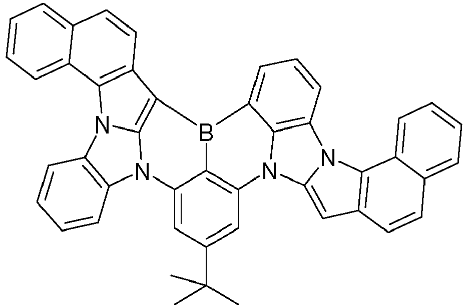 | 22 % |
| D104C | | 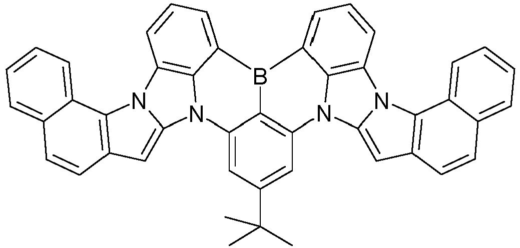 | 19 % |
| D105A | S105 | 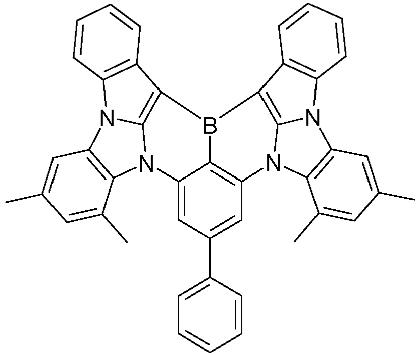 | 36 % |
| D106A | S106 | 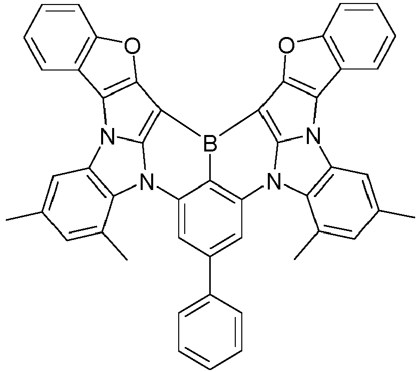 | 32 % |
| D107A | S107 | 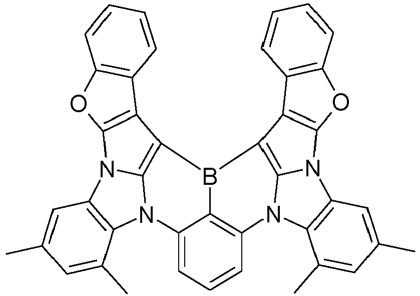 | 33 % |
| D108A | S108 | 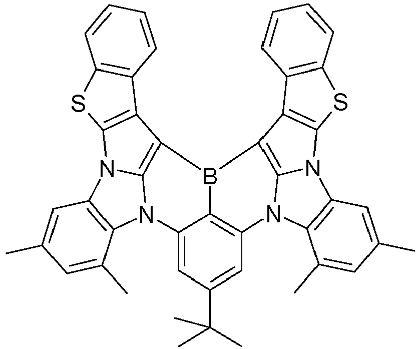 | 30 % |
| D109A | S109 | 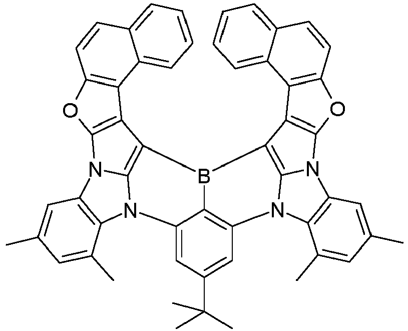 | 18 % |
| D110A | S110 | 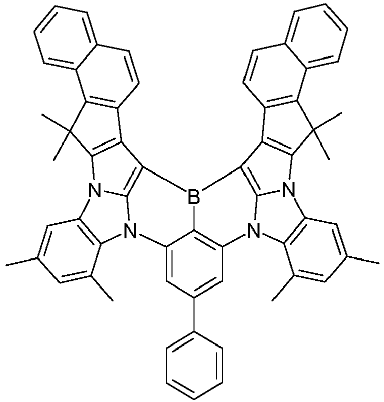 | 35 % |
| D111A | S111 | 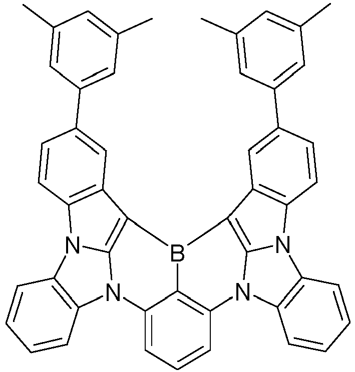 | 10 % |
| D111B | | 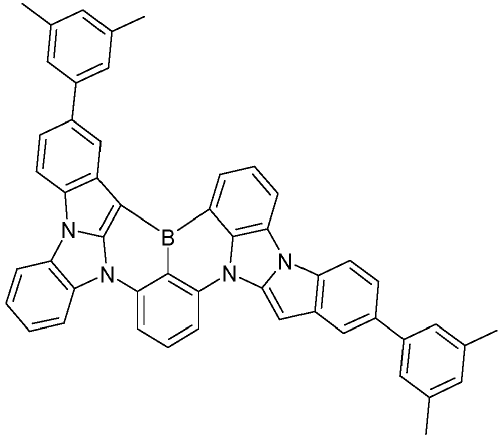 | 18 % |
| D111C | | 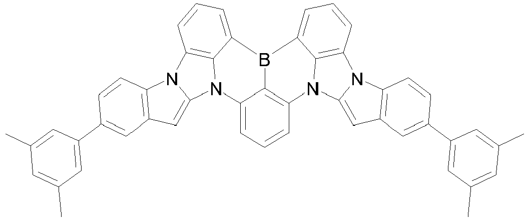 | 17 % |
| D112A | S112 | 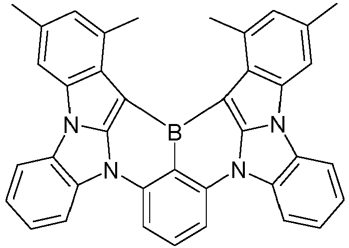 | 6% |
| D112B | | 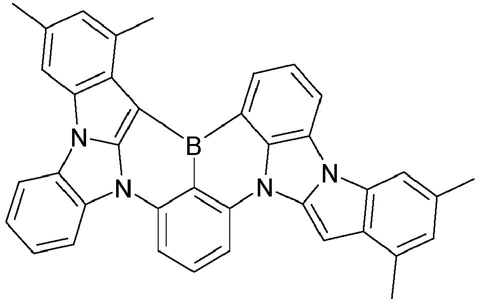 | 18 % |
| D112C | | 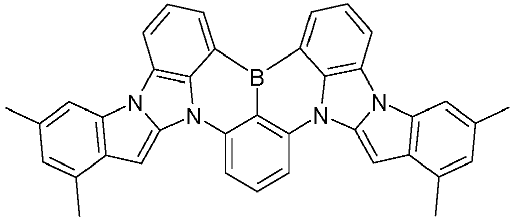 | 22 % |
| D113A | S113 | 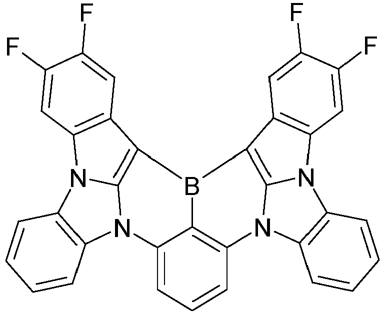 | 12 % |
| D113B | | 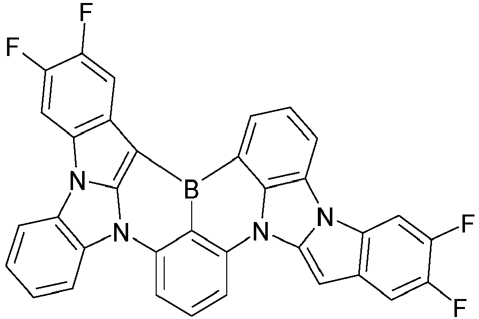 | 23 % |
| D113C | | 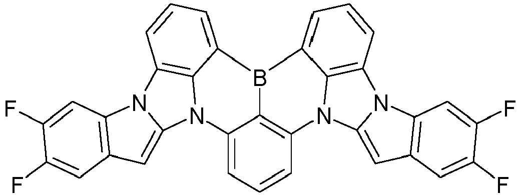 | 18 % |
| D114C | S114 | 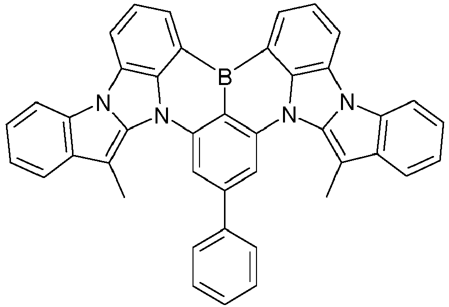 | 40 % |
| D115C | 237339 3-86-1 | 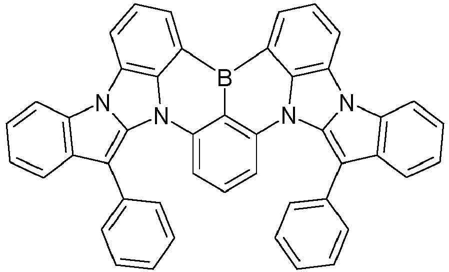 | 37 % |

### Beispiel Dotand D200A

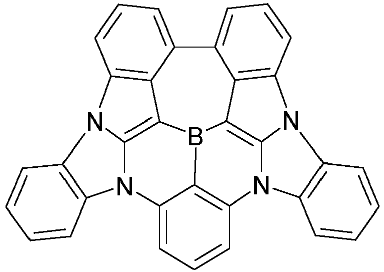

Darstellung aus D100A durch Flash-Vakuum-Pyrolyse, Trägergas Argon, Vakuum ca. 10⁻² torr, Temperatur Pyrolysezone 600 ° C, Kontakt 5 % PdO auf Aluminiumoxid. Ausbeute 25 %.

### Beispiel Dotand D215C

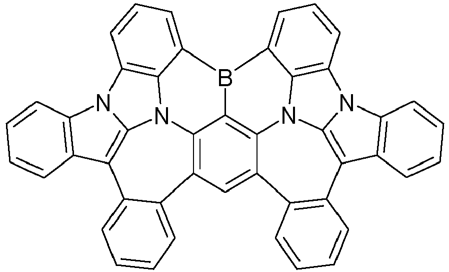

Darstellung aus D115C durch Flash-Vakuum-Pyrolyse, Trägergas Argon, Vakuum ca. 10⁻² torr, Temperatur Pyrolysezone 550 ° C, Kontakt 5 % PdO auf Aluminiumoxid. Ausbeute 30 %.

### Herstellung von vakuumprozessierten OLED-Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylenedioxy-thiophene) poly(styrenesulfonate), bezogen als CLEVIOS^{™} P VP AI 4083 von Heraeus Precious Metals GmbH Deutschland, aus wässriger Lösung aufgeschleudert) und anschließend bei 180°C 10 min. lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochinjektionsschicht 1 (HIL1) bestehend aus Ref-HTM1 dotiert mit 5 % NDP-9 (kommerziell erhältlich von der Fa. Novaled), 20 nm / Lochstransportschicht 1 (HTL1) aus: 150 nm HTM1 für UV & Blaue OLEDs; 50 nm für Grüne & Gelbe OLEDs; 110 mn für Rote OLEDs / Lochstransportschicht 2 (HTL2) aus: 10 nm für Blaue OLEDs; 20 nm für Grüne & Gelbe OLEDs; 10 mn für Rote OLEDs / Emissionsschicht (EML): 25 nm für Blaue OLEDs; 40 nm für Grüne & Gelbe OLEDs; 35 nm für Rote OLEDs / Lochblockierschicht (HBL) 10 nm / Elektronentransportschicht (ETL) 30 nm / Elektroneninjektionsschicht (EIL) aus 1 nm ETM2 / und abschließend eine Kathode. Die Kathode wird durch eine 100 nm dicke Aluminiumschicht gebildet.

Zunächst werden vakuum-prozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie SMB1:D1 (95:5%) bedeutet hierbei, dass das Material SEB1 in einem Volumenanteil von 95% und D1 in einem Anteil von 5% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 3 gezeigt.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 1000 cd/m² bestimmt und daraus die Emissionsfarbe und die EL-FWHM-Werte (ELectroluminescence - Full Width Half Maximum - Breite der EL-Emissionsspektren auf halber Peakhöhe in eV, zur besseren Vergleichbarkeit über den gesamten Spektralbereich) entnommen.

Verwendung von erfindungsgemäßen Verbindungen als Materialien in OLEDs

Die erfindungsgemäßen Verbindungen lassen sich unter anderem als Dotand in der Emissionsschicht in OLEDs einsetzen. Als Vergleich gemäß dem Stand der Technik wird die Verbindungen D-Ref.1 gemäß Tabelle 3 verwendet. Die Ergebnisse der OLEDs sind in Tabelle 2 zusammengefasst.

**Tabelle 1: Aufbau der OLEDs**

| **Bsp.** | **EML** | **HBL** | **ETL** |
|---|---|---|---|
| **Blaue OLEDs (420 - 499 nm)** | | | |
| D-Ref.1 | SMB1:D-Ref.1 (95%: 5%) | ETM1 | ETM1:ETM2 (50%:50%) |
| D-D100A | SMB1:D-D100A (95%:5%) | ETM1 | ETM1:ETM2 (50%:50%) |
| D-D100B | SMB1:D-D100B (95%:5%) | ETM1 | ETM1:ETM2 (50%:50%) |
| D-D101A | SMB4:D-D101A (95%:5%) | ETM1 | ETM1:ETM2 (50%:50%) |
| D-D101B | SMB1:D-D101B (95%:5%) | ETM1 | ETM1:ETM2 (50%:50%) |
| D-D102A | SMB1:D-D102A (95%:5%) | ETM1 | ETM1:ETM2 (50%:50%) |
| D-D103A | SMB1:D-D103A (95%:5%) | ETM1 | ETM1:ETM2 (50%:50%) |
| D-D104A | SMB1:D-D104A (95%:5%) | ETM1 | ETM1:ETM2 (50%:50%) |
| D-D104B | SMB1:D-D104B (95%:5%) | ETM1 | ETM1:ETM2 (50%:50%) |
| D-D105A | SMB1:D-D105A (95%:5%) | ETM1 | ETM1:ETM2 (50%:50%) |
| D-D106A | SMB1:D-D106A (95%:5%) | ETM1 | ETM1:ETM2 (50%:50%) |
| D-D107A | SMB1:D-D107A (95%:5%) | ETM1 | ETM1:ETM2 (50%:50%) |
| D-D108A | SMB1:D-D108A (95%:5%) | ETM1 | ETM1:ETM2 (50%:50%) |
| D-D109A | SMB1:D-D109A (95%:5%) | ETM1 | ETM1:ETM2 (50%:50%) |
| D-D110A | SMB1:D-D110A (95%:5%) | ETM1 | ETM1:ETM2 (50%:50%) |
| D-D111A | SMB1:D-D111A (95%:5%) | ETM1 | ETM1:ETM2 (50%:50%) |
| D-D111B | SMB1:D-D111 B (95%:5%) | ETM1 | ETM1:ETM2 (50%:50%) |
| D-D112A | SMB1:D-D112A (95%:5%) | ETM1 | ETM1:ETM2 (50%:50%) |
| D-D113A | SMB1:D-D113A (95%:5%) | ETM1 | ETM1:ETM2 (50%:50%) |
| D-D113B | SMB1:D-D113B (95%:5%) | ETM1 | ETM1:ETM2 (50%:50%) |

| **Grüne & Gelbe OLEDs (500 - 590 nm)** | | | |
|---|---|---|---|
| D-D100C | SMB1:D-D100C (95%:5%) | ETM1 | ETM1:ETM2 (50%:50%) |
| D-D101C | SMB1:D-D101C (95%:5%) | ETM1 | ETM1:ETM2 (50%:50%) |
| D-D103B | SMB1:D-D103B (95%:5%) | ETM1 | ETM1:ETM2 (50%:50%) |
| D-D103C | SMB1:D-D103C (95%:5%) | ETM1 | ETM1:ETM2 (50%:50%) |
| D-D104C | SMB1:D-D104C (95%:5%) | ETM1 | ETM1:ETM2 (50%:50%) |
| D-D111C | SMB1:D-D111C (95%:5%) | ETM1 | ETM1:ETM2 (50%:50%) |
| D-D112B | SMB1:D-D112B (95%:5%) | ETM1 | ETM1:ETM2 (50%:50%) |
| D-D112C | SMB1:D-D112C | ETM1 | ETM1:ETM2 |
| | (95%:5%) | | (50%:50%) |
| D-D 113C | SMB2:D-C113C (95%:5%) | ETM1 | ETM1 :ETM2 (50%:50%) |
| D-D 114C | SMB3:D-D114C (95%:5%) | ETM1 | ETM1:ETM2 (50%:50%) |
| D-D 115C | SMB4:D-D115C (93%:7%) | ETM1 | ETM1:ETM2 (50%:50%) |
| D-D210A | SMB1:D-D210A (95%:5%) | ETM1 | ETM1:ETM2 (50%:50%) |

| **Rote OLEDs (591 - 700 nm)** | | | |
|---|---|---|---|
| D-D215C | SMB1:D-D116C (97%:3%) | ETM1 | ETM1:ETM2 (50%:50%) |

**Tabelle 2: Ergebnisse der Vakuum-prozessierten OLEDs**

| **Bsp.** | **EQE [%]** | **Spannung [V]** | **EL-max. [nm]** | **EL-FWHM [eV]** |
|---|---|---|---|---|
| Ref.1 | 6.3 | 4.6 | 464 | 0.17 |
| D-D100A | 6.0 | 4.6 | 438 | 0.17 |
| D-D100B | 7.4 | 4.4 | 484 | 0.31 |
| D-D100C | 7.9 | 4.3 | 540 | 0.18 |
| D-D101A | 5.1 | 4.6 | 423 | 0.15 |
| D-D101B | 7.2 | 4.4 | 476 | 0.28 |
| D-D101C | 7.8 | 4.3 | 527 | 0.19 |
| D-D102A | 6.3 | 4.5 | 442 | 0.15 |
| D-D103A | 7.1 | 4.5 | 462 | 0.14 |
| D-D103B | 6.7 | 4.4 | 502 | 0.30 |
| D-D103C | 7.0 | 4.5 | 564 | 0.19 |
| D-D104A | 6.8 | 4.5 | 456 | 0.16 |
| D-D104B | 6.9 | 4.4 | 499 | 0.28 |
| D-D104C | 7.5 | 4.1 | 550 | 0.27 |
| D-D105A | 6.7 | 4.4 | 477 | 0.16 |
| D-D106A | 6.8 | 4.4 | 482 | 0.15 |
| D-D107A | 6.2 | 4.5 | 441 | 0.15 |
| D-D108A | 6.3 | 4.5 | 445 | 0.16 |
| D-D109A | 6.3 | 4.5 | 440 | 0.16 |
| D-D110A | 6.6 | 4.4 | 462 | 0.15 |
| D-D111A | 6.5 | 4.4 | 458 | 0.16 |
| D-D111B | 6.9 | 4.3 | 486 | 0.29 |
| D-D111C | 7.4 | 4.2 | 542 | 0.20 |
| D-D112A | 6.9 | 4.5 | 447 | 0.16 |
| D-D112B | 7.3 | 4.3 | 533 | 0.30 |
| D-D112C | 7.6 | 3.9 | 596 | 0.20 |
| D-D113A | 5.9 | 4.5 | 436 | 0.16 |
| D-D113B | 6.7 | 4.4 | 482 | 0.29 |
| D-D113C | 7.5 | 4.1 | 537 | 0.19 |
| D-D 114C | 7.7 | 3.9 | 573 | 0.18 |
| D-D115C | 7.0 | 4.3 | 564 | 0.18 |
| D-D210A | 6.1 | 4.2 | 540 | 0.35 |
| D-D215C | 3.8 | 3.5 | 686 | 0.22 |

**Tabelle 3: Strukturformeln der verwendeten Materialien**

| | |
|---|---|
| 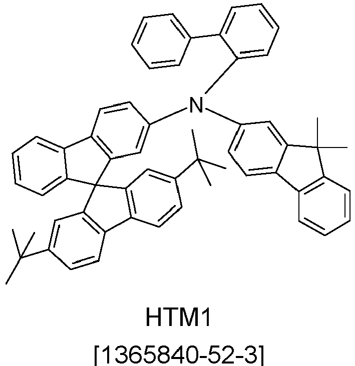 | 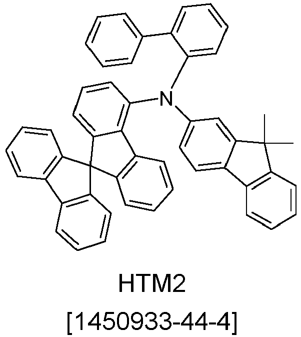 |
| 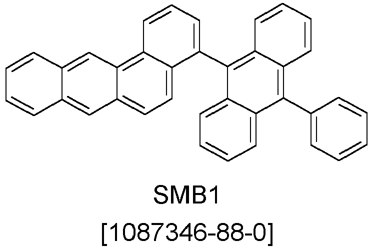 | 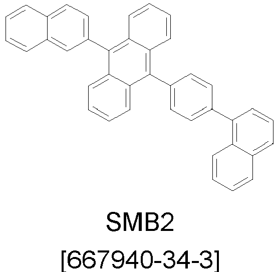 |
| 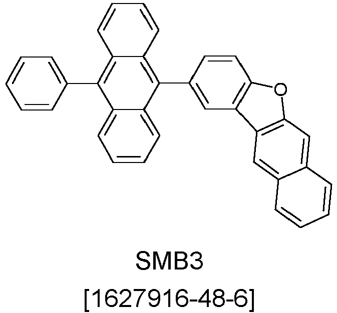 | 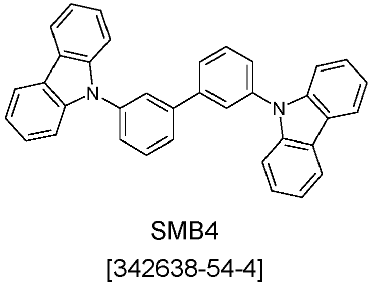 |
| 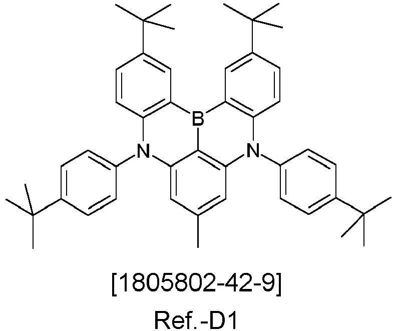 | |
| 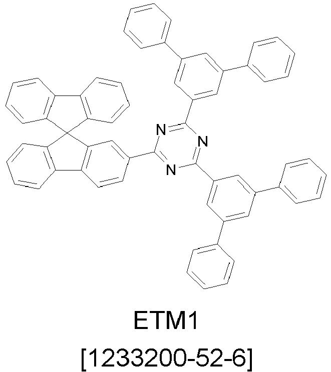 | 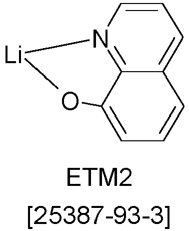 |

Mit den erfindungsgemäßen Verbindungen vom Regioisomeren-Typ A sind blaue und tiefblaue OLEDs mit besserer Effizienz und Betriebsspannung zugänglich, was zu Bauteilen mit verbesserter Leistungseffizienz führt. Außerdem sind tiefblaue OLEDs mit Emissionsmaxima um 440 nm und darunter sind mit den Verbindungen nach dem SdT nicht zugänglich. Zudem zeichnen sich die erfindungsgemäßen Verbindungen vom Regioisomeren-Typ A durch Emissionsspenkten mit sehr geringer Halbwertsbreite EL-FWHM (**EL**ectro luminescent **F**ull **W**idth **H**alf **M**ax) aus, was zu sehr farbreiner Emission und damit zu sehr guten CIE X und Y Werten führt.

Erfindungsgemäße Verbindungen vom Regioisomeren-Typ C zeichnen sich durch Emissionsspenkten mit sehr geringer Halbwertsbreite EL-FWHM (**E**lectro **L**uminescent **F**ull **W**idth **H**alf **M**ax) aus, was zu sehr farbreiner Emission und damit zu sehr guten CIE X und Y Werten führt. Mit ihnen sind neben dem Blauen auch der Grüne und Gelbe Spektralbereich zugänglich. Die hohen EQE-Werte bei verringerten Betriebsspannungen führt zu Bauteilen mit sehr guter Leistungseffizienz.

Erfindungsgemäße Verbindungen vom Regioisomeren-Typ B zeigen verbesserte Effizienz und Betriebsspannung. Außerdem sind mit diesen Verbindungen Grüne, Gelbe und Rote Bauteile realisierbar.

## Patentansprüche

1. Verbindung umfassend mindestens eine Struktur der Formel (IIa), (IIb), (IIc), (IId), (IIe), 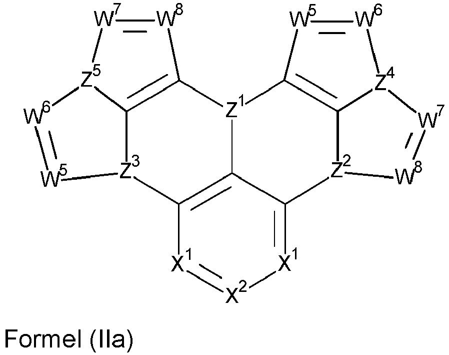 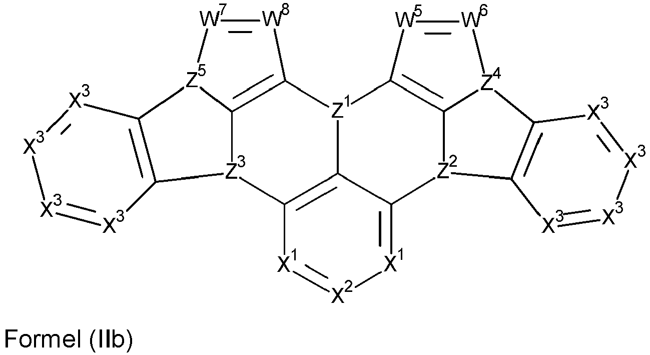 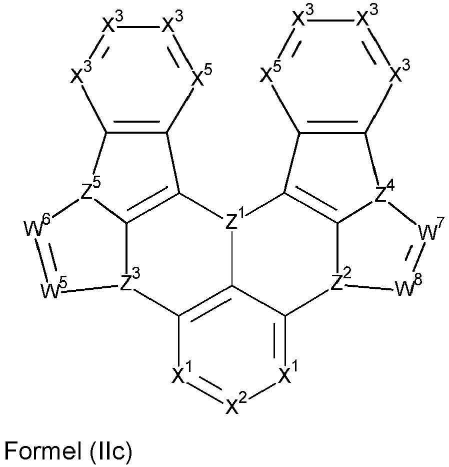 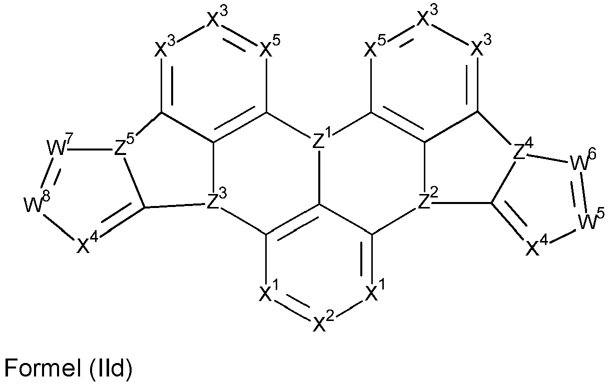 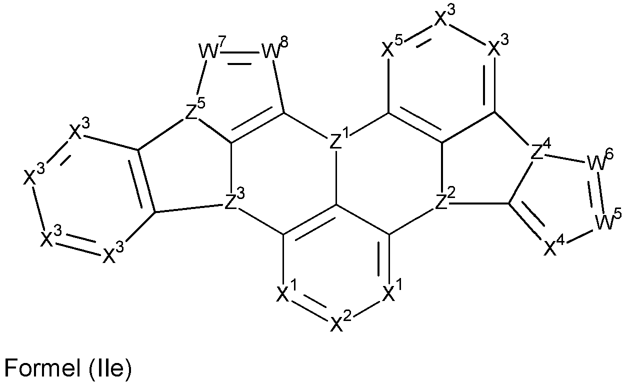 wobei für die verwendeten Symbole und Indizes gilt:
Z¹ ist B;
Z², Z³, Z⁴, Z⁵ ist bei jedem Auftreten gleich oder verschieden N oder B; wobei zwei der Gruppen Z², Z³, Z⁴, Z⁵ für N stehen,
W⁵, W⁶, W⁷, W⁸ steht bei jedem Auftreten gleich oder verschieden für C(Ar) oder X⁶;
Ar ist bei jedem Auftreten gleich oder verschieden ein aromatisches oder heteroaromatisches Ringsystem mit 5 bis 60 aromatischen Ringatomen, das mit einem oder mehreren Resten R substituiert sein kann, hierbei kann die Gruppe Ar mit mindestens einer Gruppe Ar, X¹, R oder einer weiteren Gruppe ein Ringsystem bilden;
X¹ steht bei jedem Auftreten gleich oder verschieden für N, CR^{a} oder C, falls durch eine Bindung mit einem der Ringe Ar^{c}, Ar^{d} oder einer weiteren Gruppe ein Ringsystem gebildet wird, mit der Maßgabe, dass nicht mehr als zwei der Gruppen X¹, X² in einem Cyclus für N stehen;
X² steht bei jedem Auftreten gleich oder verschieden für N oder CR^{b}, mit der Maßgabe, dass nicht mehr als zwei der Gruppen X¹, X² in einem Cyclus für N stehen;
X³ steht bei jedem Auftreten gleich oder verschieden für N oder CR^{c} mit der Maßgabe, dass nicht mehr als zwei der Gruppen X³, X⁵ in einem Cyclus für N stehen;
X⁴ steht bei jedem Auftreten gleich oder verschieden für N oder CR^{d} mit der Maßgabe, dass nicht mehr als zwei der Gruppen X⁴, X⁶ in einem Cyclus für N stehen;
X⁵ steht bei jedem Auftreten gleich oder verschieden für N, CR^{e} oder C, falls durch eine Bindung mit einer Gruppe Ar oder einer Gruppe X⁵ oder einer Gruppe X⁶ ein Ringsystem gebildet wird, mit der Maßgabe, dass nicht mehr als zwei der Gruppen X³, X⁵ in einem Cyclus für N stehen;
X⁶ steht bei jedem Auftreten gleich oder verschieden für N, CR^{f} oder C, falls durch eine Bindung mit einer Gruppe Ar, einer Gruppe X⁵ oder einer Gruppe X⁶ ein Ringsystem gebildet wird, mit der Maßgabe, dass nicht mehr als zwei der Gruppen X⁴, X⁶ in einem Cyclus für N stehen;
R, R^{a}, R^{b} ist bei jedem Auftreten gleich oder verschieden H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R, R^{a}, R^{b} auch miteinander oder einer weiteren Gruppe ein Ringsystem bilden;
R^{c}, R^{d}, R^{e}, R^{f} ist bei jedem Auftreten gleich oder verschieden H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 CAtomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{c}, R^{d}, R^{e}, R^{f} auch miteinander oder einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)OAr", C(=O)OR², C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere Reste R¹ miteinander ein Ringsystem bilden, dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, CI. Br, I oder CN ersetzt sein können und das durch ein oder mehrere Alkylgruppen mit jeweils 1 bis 4 Kohlenstoffatomen substituiert sein kann, dabei können zwei oder mehrere Substituenten R² miteinander ein Ringsystem bilden.

2. Verbindung nach Anspruch 1, umfassend mindestens eine Struktur der Formel (III-1) bis (III-25), 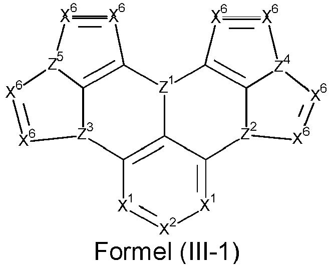 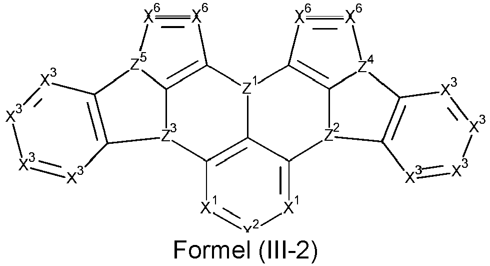 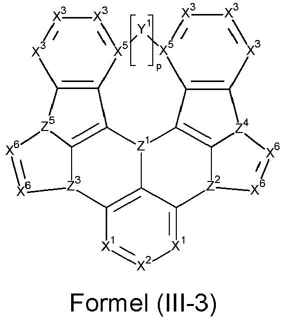 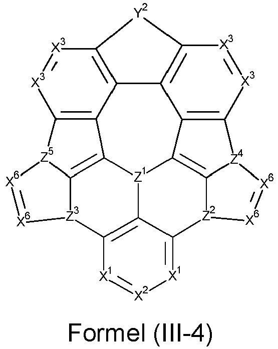 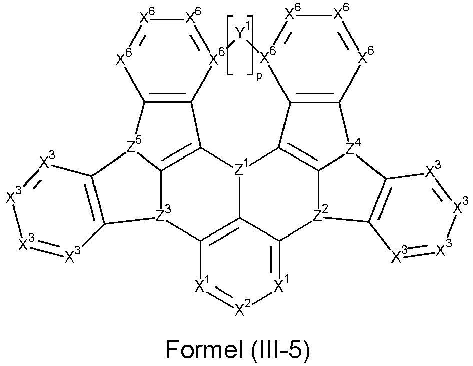 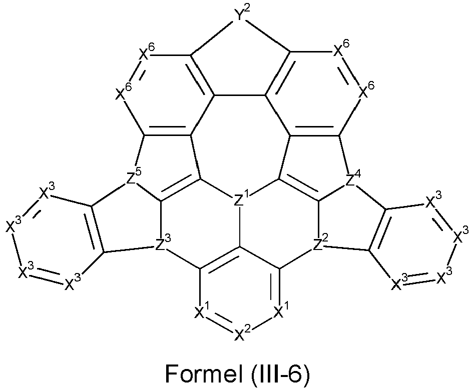 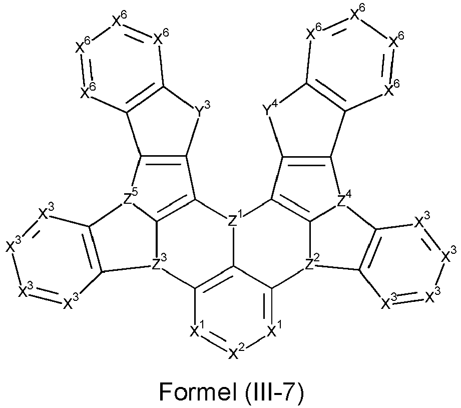 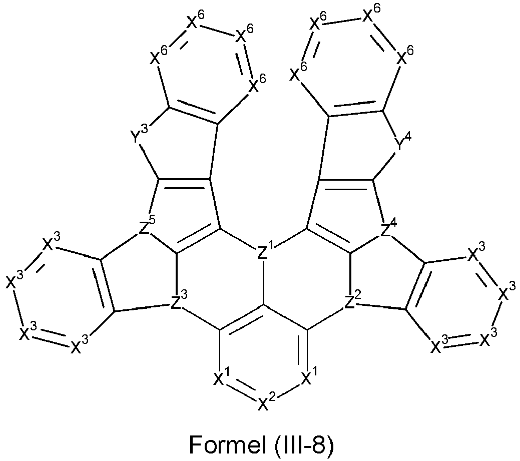 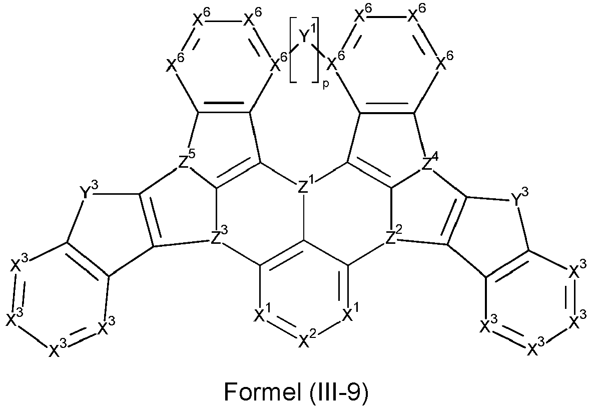 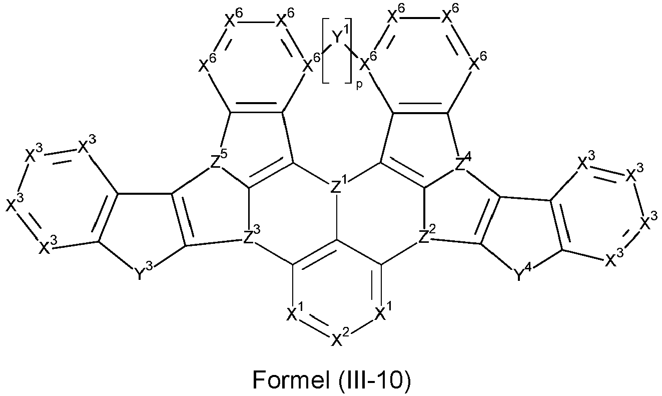 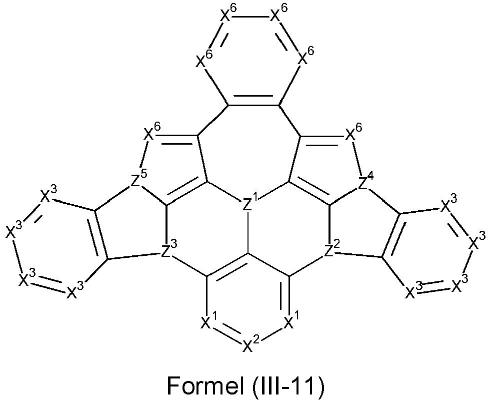 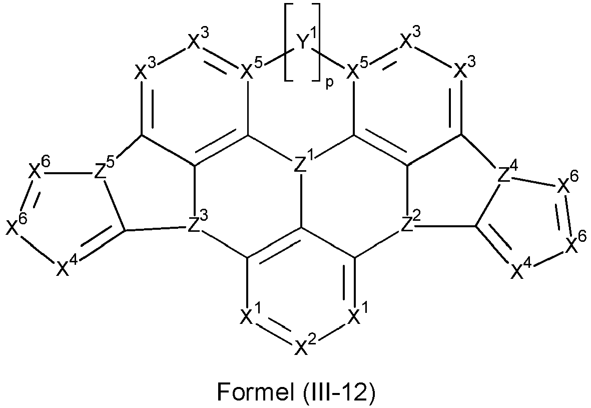 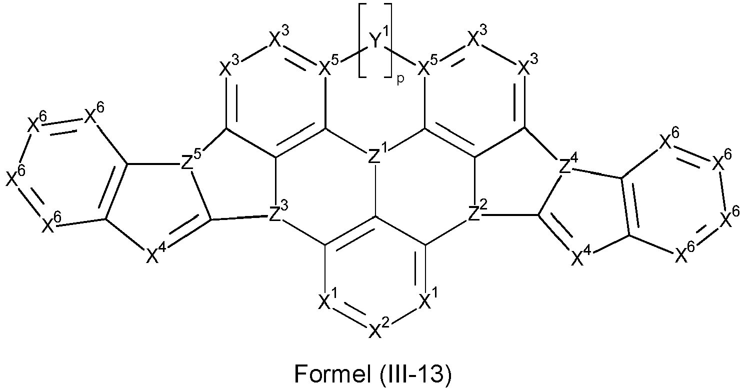 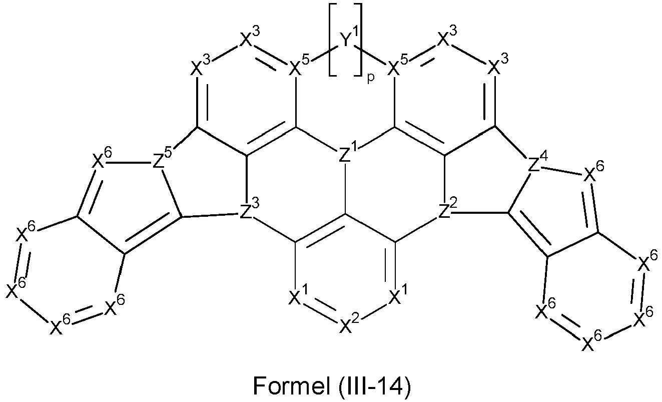 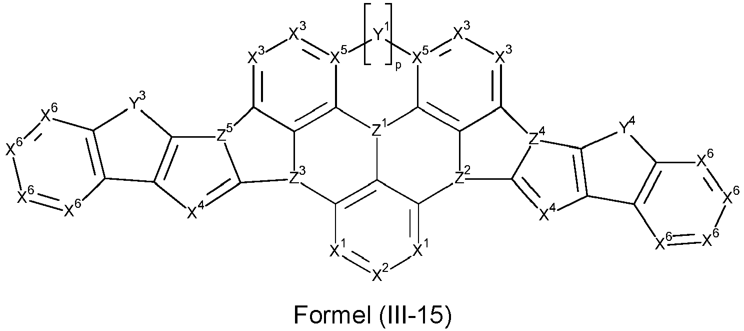 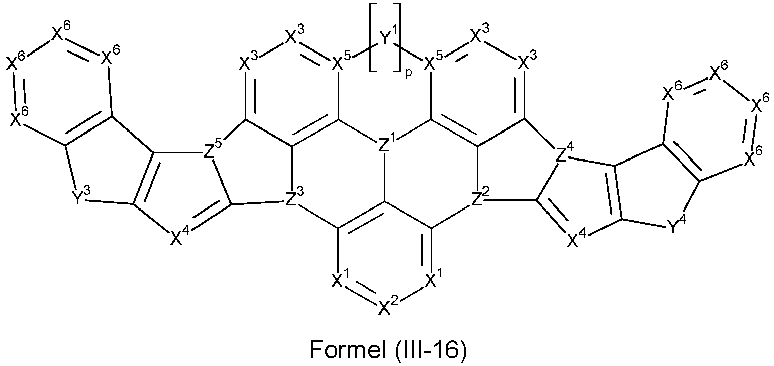 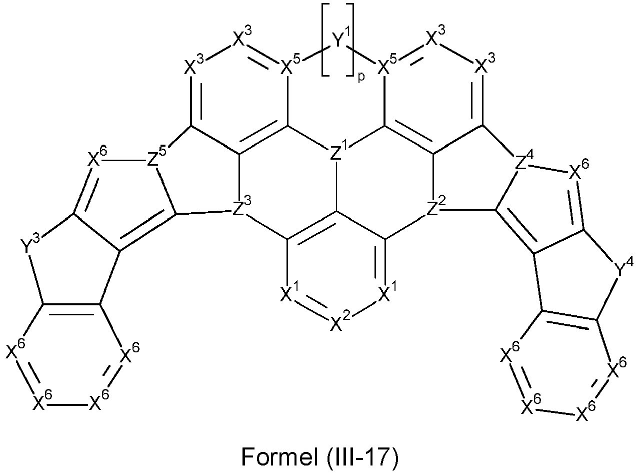 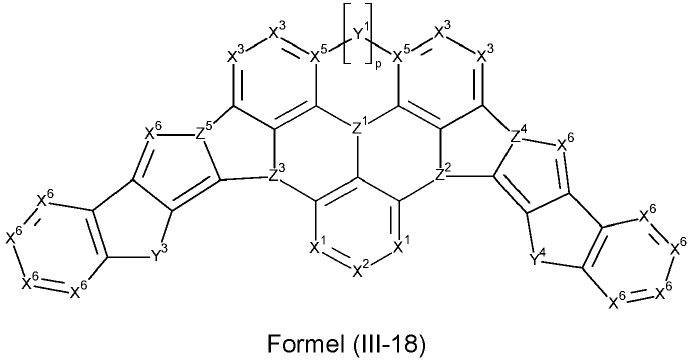 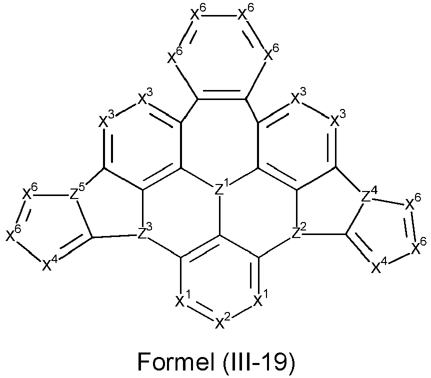 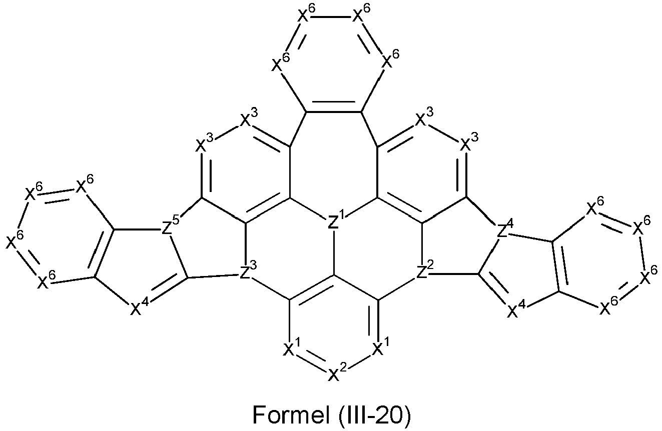 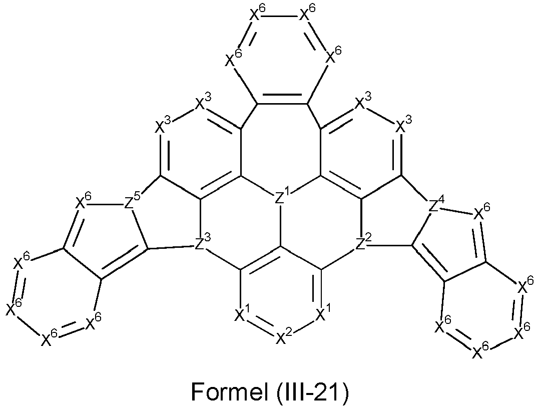 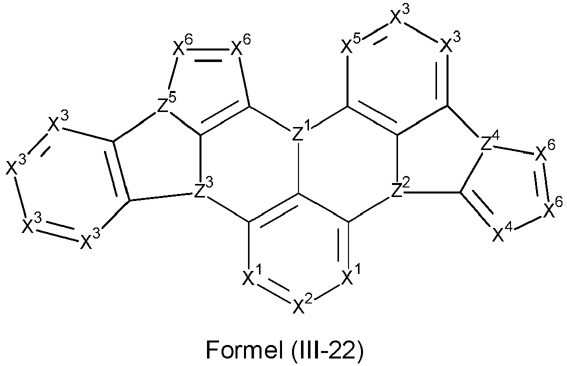 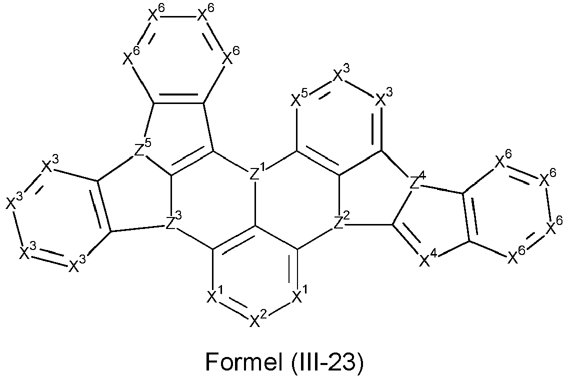 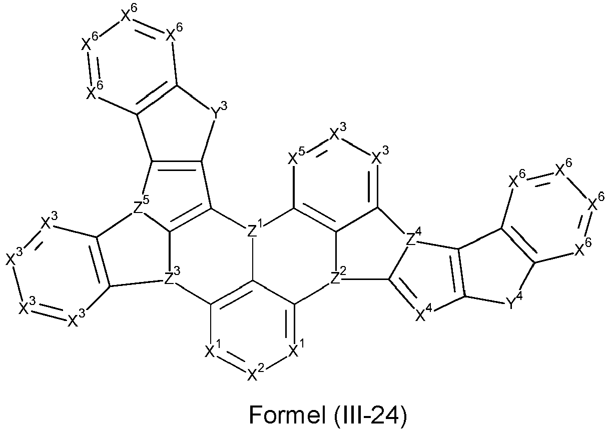 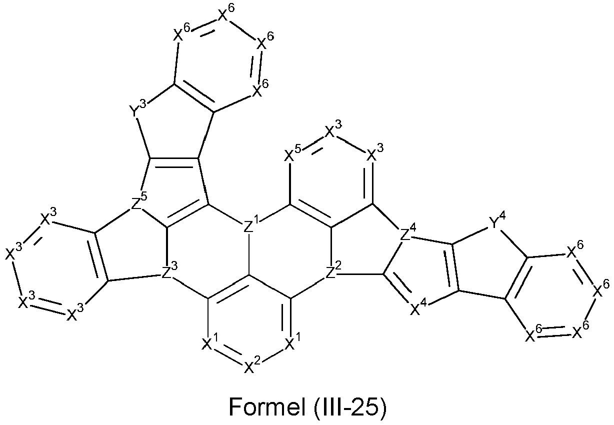 wobei die Symbole Z¹, Z², Z³, X¹, X², Z⁴, Z⁵, X³, X⁴, X⁵ und X⁶ die in Anspruch 1 genannten Bedeutungen aufweisen und die weiteren Symbole die folgende Bedeutung aufweisen:
p ist 0 oder 1,
Y¹ ist bei jedem Auftreten gleich oder verschieden eine Bindung, N(Ar'), N(R), P(Ar'), P(R), P(=O)Ar', P(=O)R, P(=S)Ar`, P(=S)R, B(Ar'), B(R), Al(Ar'), AI(R), Ga(Ar'), Ga(R), C=O, C(R)₂, Si(R)₂, C=NR, C=NAr', C=C(R)₂, O, S, Se, S=O, oder SO₂, wobei die Symbole R und Ar' die in Anspruch 1 dargelegte Bedeutung aufweisen;
Y², Y³, Y⁴ ist bei jedem Auftreten gleich oder verschieden N(Ar`), N(R), P(Ar'), P(R), P(=O)Ar`, P(=O)R, P(=S)Ar', P(=S)R, B(Ar'), B(R), Al(Ar'), AI(R), Ga(Ar'), Ga(R), C=O, C(R)₂, Si(R)₂, C=NR, C=NAr', C=C(R)₂, O, S, Se, S=O, oder SO₂, wobei die Symbole R und Ar' die in Anspruch 1 dargelegte Bedeutung aufweisen.

3. Verbindung nach Anspruch 1 oder 2, umfassend mindestens eine Struktur der Formel (IV-1) bis (IV-38), 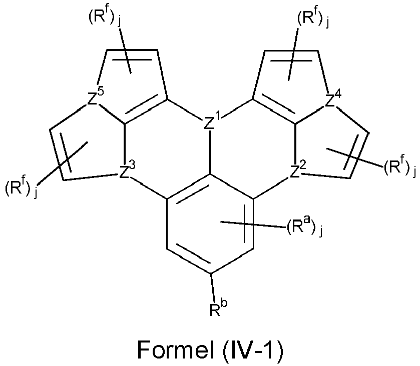 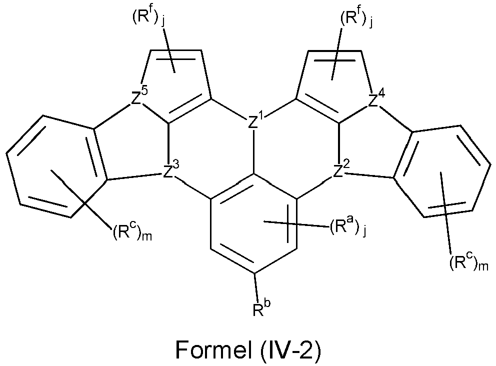 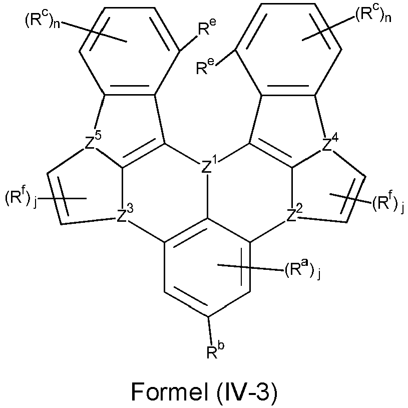 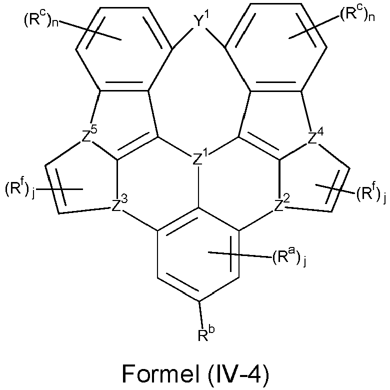 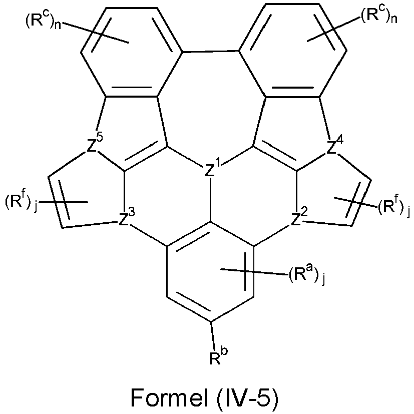 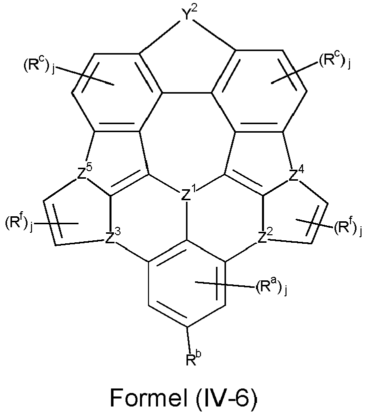 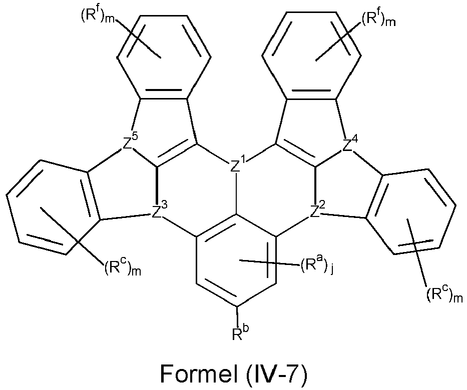 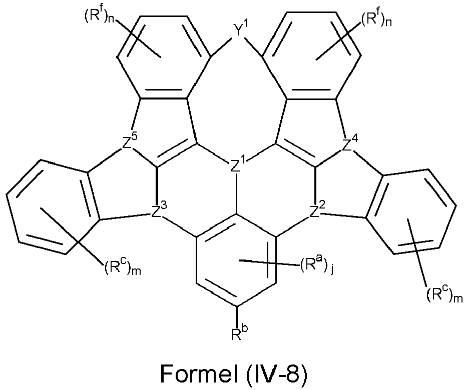 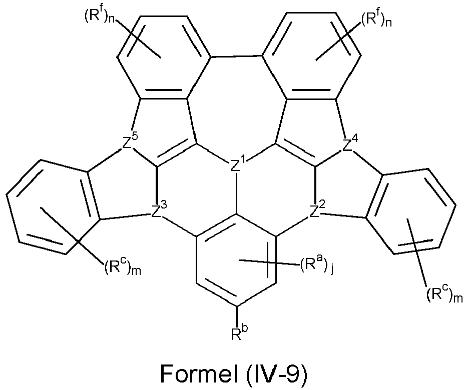 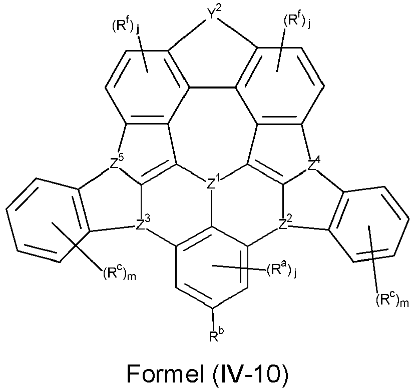 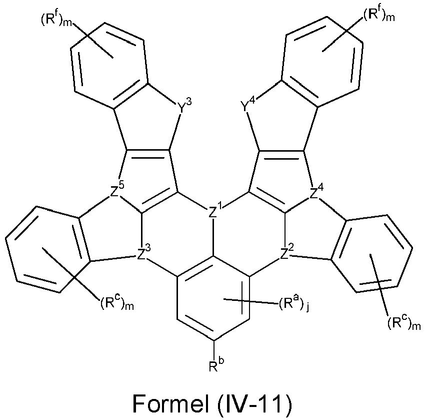 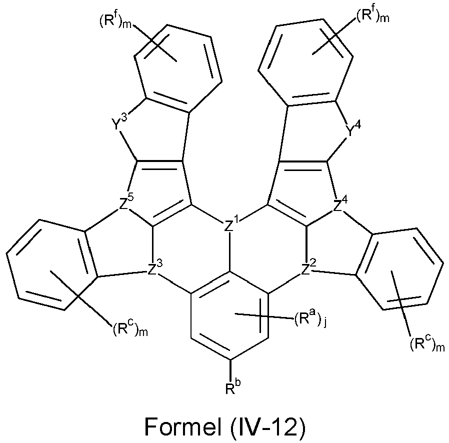 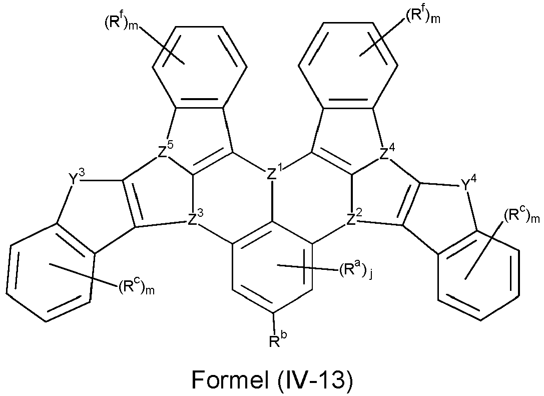 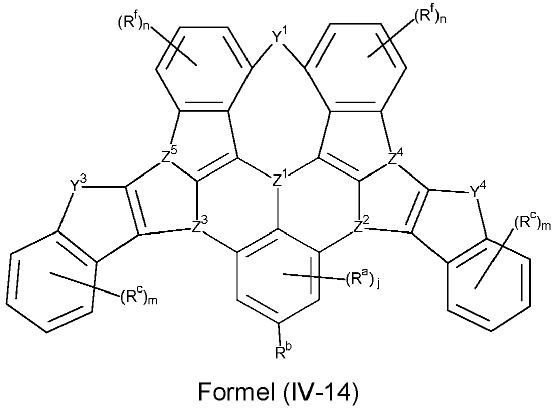 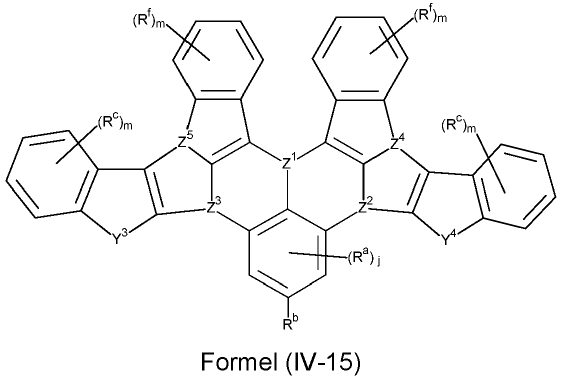 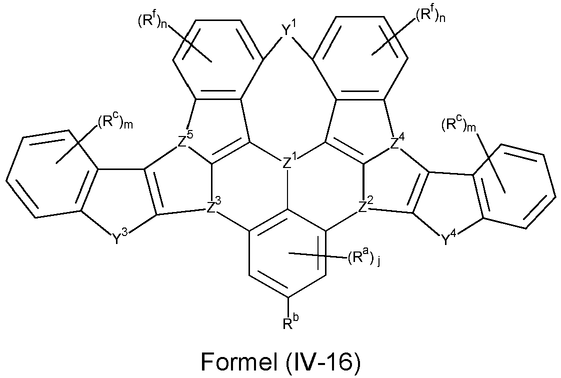 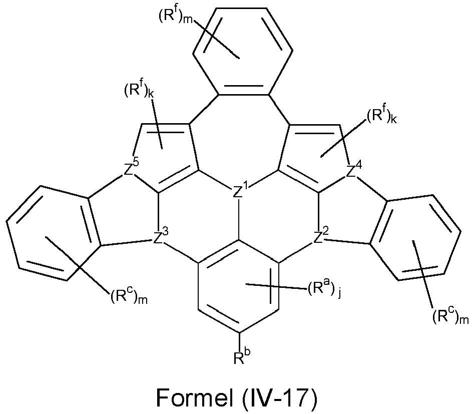 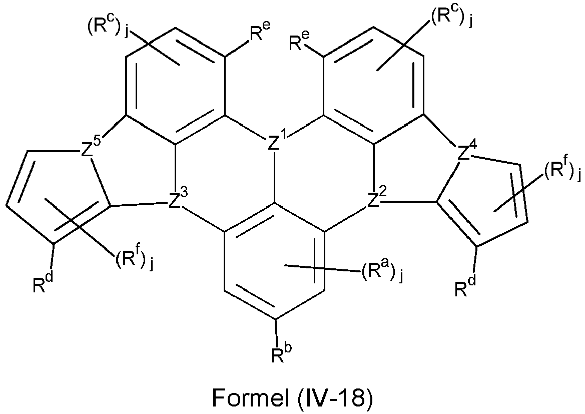 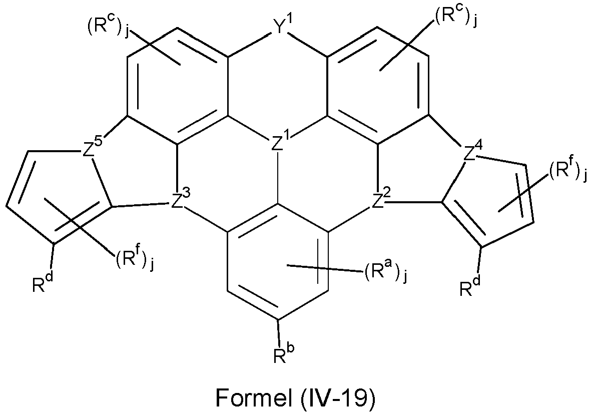 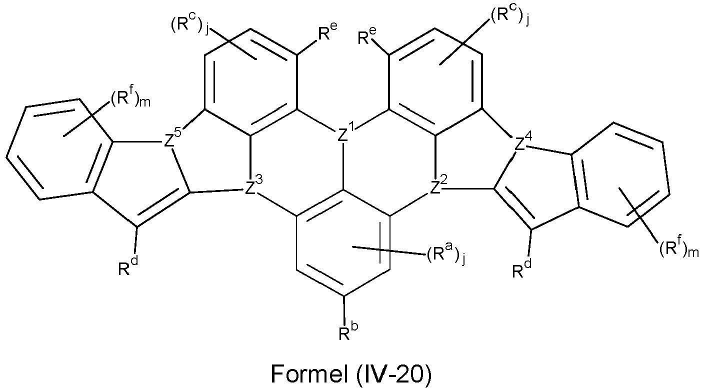 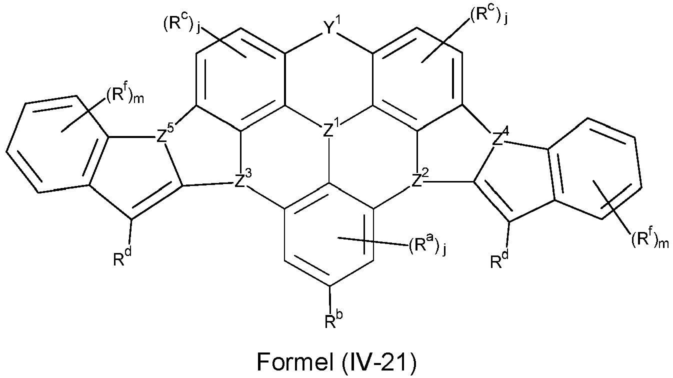 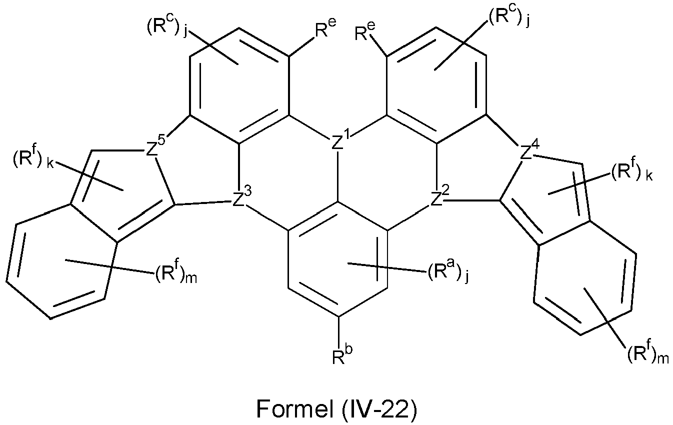 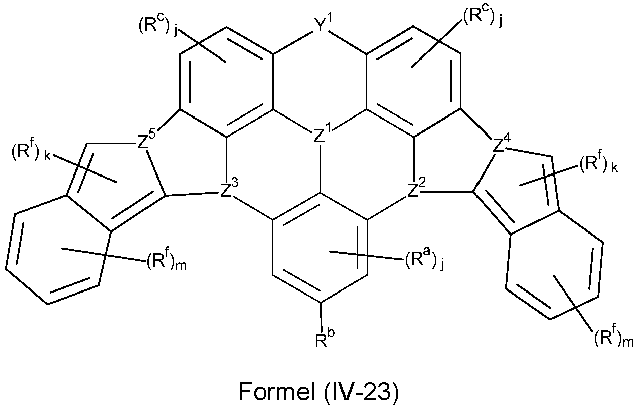 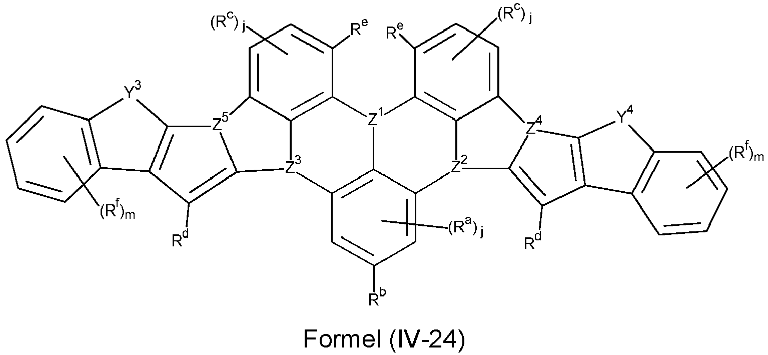 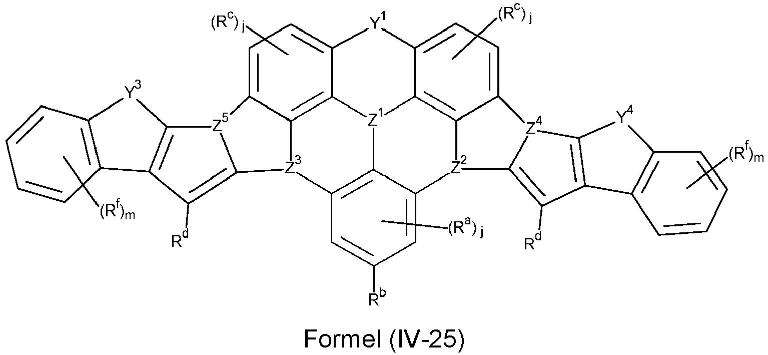 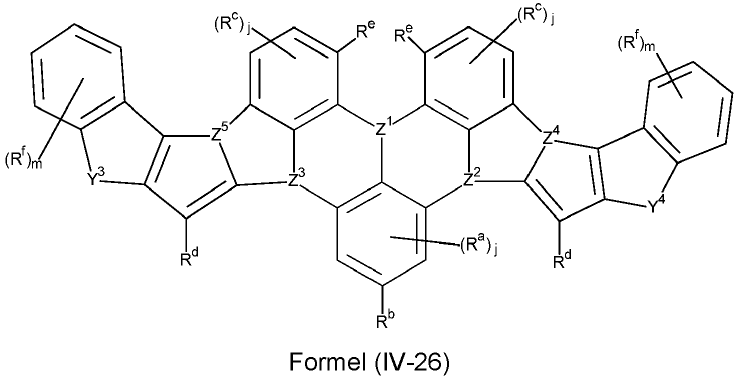 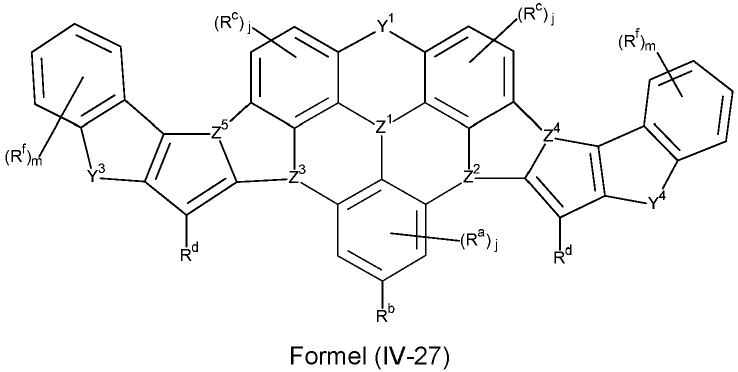 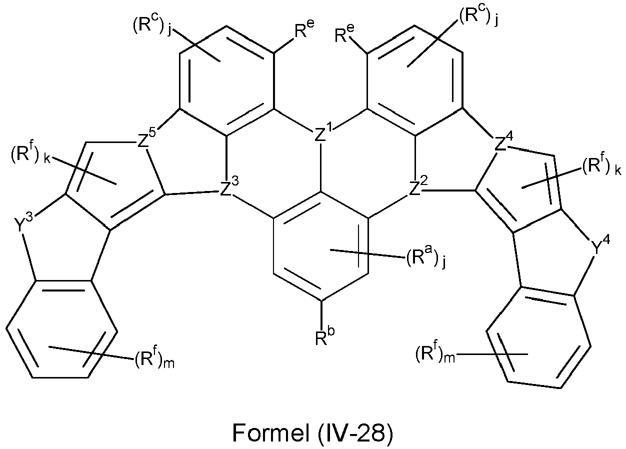 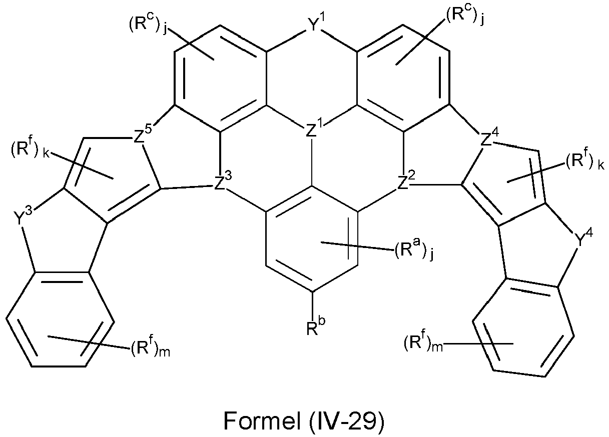 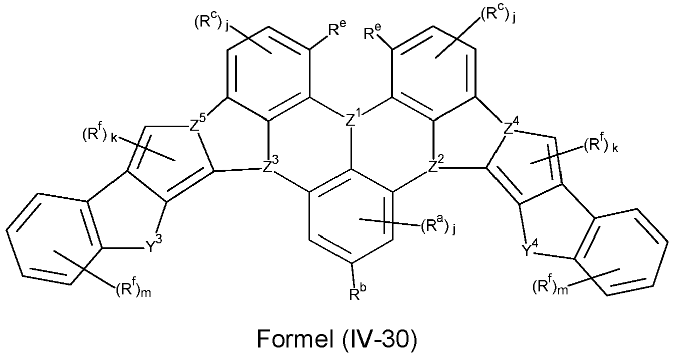 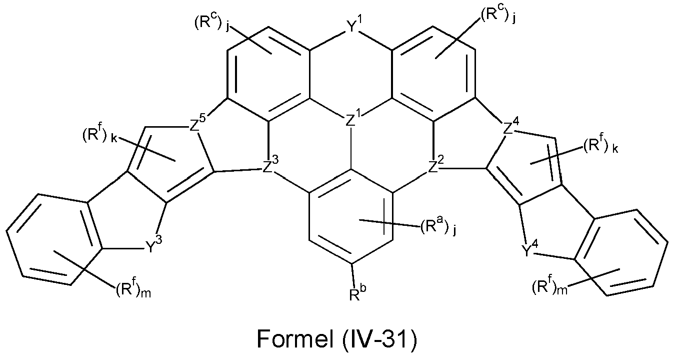 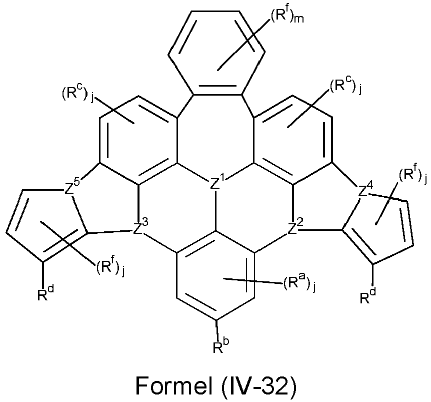 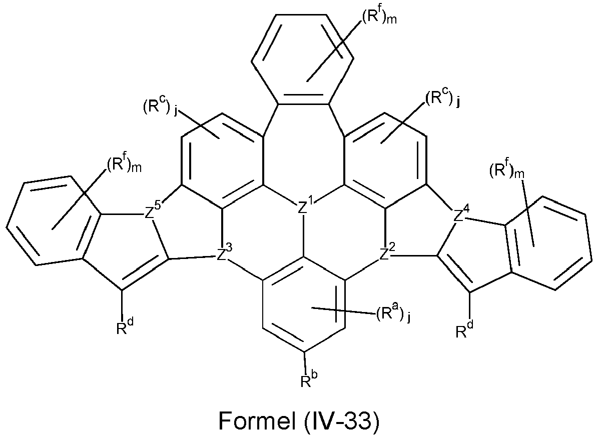 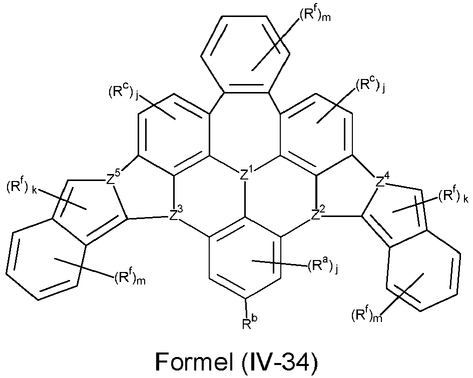 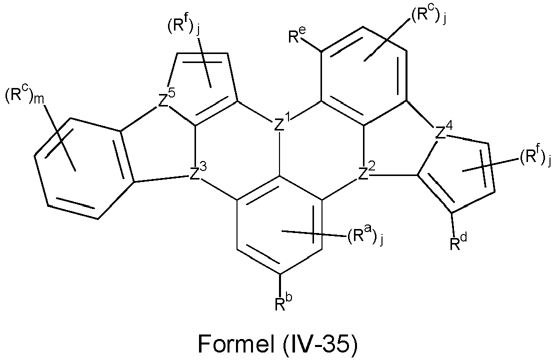 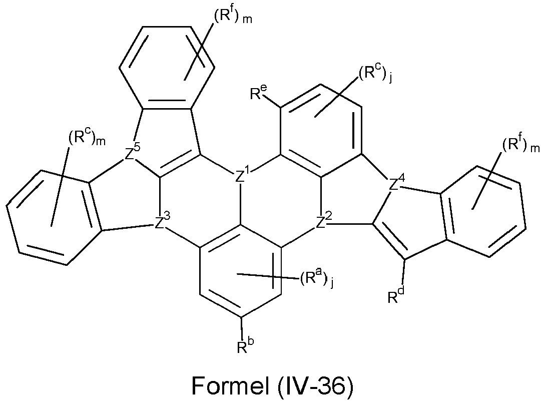 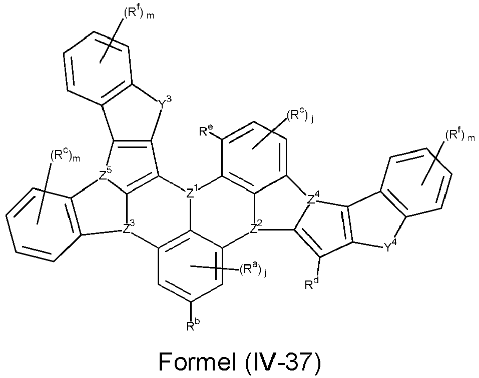 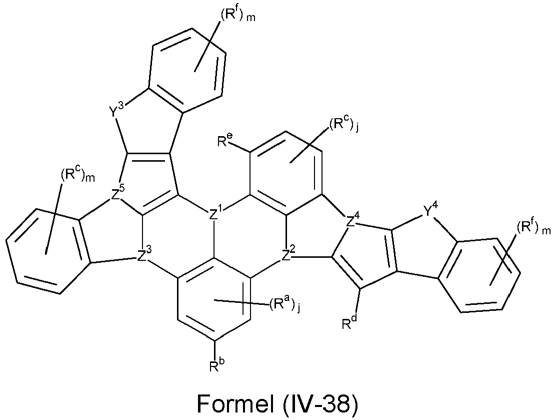 wobei die Symbole Z¹, Z², Z³, R^{a}, R^{b} Z⁴, Z⁵, R^{c}, R^{d}, R^{e} und R^{f} die in Anspruch 1 genannten Bedeutungen aufweisen, die Symbole Y¹, Y², Y³ und Y⁴ die in Anspruch 2 aufweisen und die weiteren Symbole die folgende Bedeutung haben:
m ist 0, 1, 2, 3 oder 4;
n ist 0, 1, 2 oder 3;
j ist 0, 1 oder 2;
k ist 0 oder 1.

4. Verbindung nach einem oder mehreren der Ansprüche 1 bis 3, **dadurch gekennzeichnet, dass** mindestens zwei Reste R, R^{a}, R^{b}, R^{c}, R^{d}, R^{e}, R^{f} mit den weiteren Gruppen, an die die zwei Reste R, R^{a}, R^{b}, R^{c}, R^{d}, R^{e}, R^{f} binden, einen kondensierten Ring bilden, wobei die zwei Reste R, R^{a}, R^{b}, R^{c}, R^{d}, R^{e}, R^{f} mindestens eine Struktur der Formeln (RA-1) bis (RA-12) formen 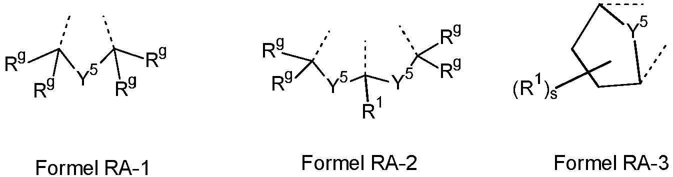 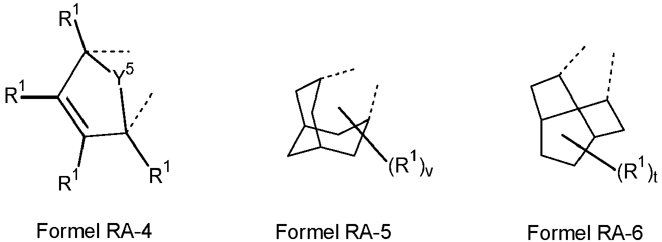 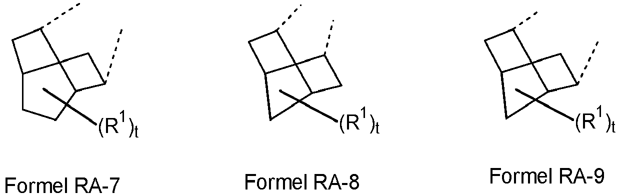 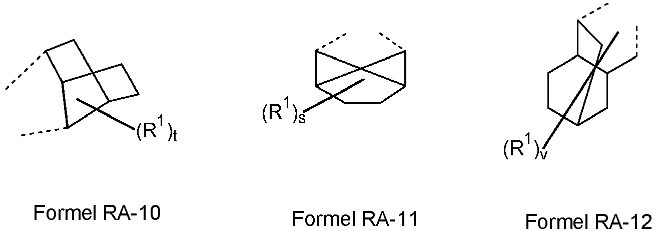 wobei R¹ die zuvor dargelegte Bedeutung hat, die gestrichelten Bindungen die Anbindungsstellen an die Atome der Gruppen, an die die zwei Reste R, R^{a}, R^{b}, R^{c}, R^{d}, R^{e}, R^{f} binden, darstellen, und die weiteren Symbole die folgende Bedeutung aufweisen:
Y⁵ ist bei jedem Auftreten gleich oder verschieden C(R¹)₂, (R¹)₂C-C(R¹)₂, (R¹)C=C(R¹), NR¹, NAr', O oder S;
R^{g} ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{g} auch miteinander oder ein Rest R^{g} mit einem Rest R¹ oder mit einer weiteren Gruppe ein Ringsystem bilden;
s ist 0, 1, 2, 3, 4, 5 oder 6;
t ist 0, 1, 2, 3, 4, 5, 6, 7 oder 8;
v ist 0, 1, 2, 3, 4, 5, 6, 7, 8 oder 9.

5. Verbindung nach einem oder mehreren der Ansprüche 1 bis 4, dass mindestens zwei Reste R, R^{a}, R^{b}, R^{c}, R^{d}, R^{e}, R^{f} mit den weiteren Gruppen, an die die zwei Reste R, R^{a}, R^{b}, R^{c}, R^{d}, R^{e}, R^{f} binden, einen kondensierten Ring bilden, wobei die zwei Reste R, R^{a}, R^{b}, R^{c}, R^{d}, R^{e}, R^{f} der Strukturen der Formel (RB) formen, 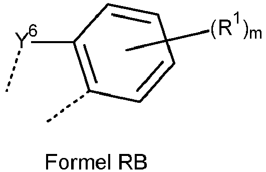 wobei R¹ die in Anspruch 1 dargelegte Bedeutung aufweist, die gestrichelten Bindungen die Anbindungsstellen darstellen, an die die zwei Reste R, R^{a}, R^{b}, R^{c}, R^{d}, R^{e}, R^{f} binden, der Index m 0, 1, 2, 3 oder 4 ist und Y⁶ C(R¹)₂, NR¹, NAr', BR¹, BAr', O oder S ist.

6. Verbindung nach einem oder mehreren der Ansprüche 1 bis 5, umfassend mindestens eine Struktur der Formeln (V-1) bis (V-10), wobei die Verbindungen mindestens einen kondensierten Ring aufweisen, 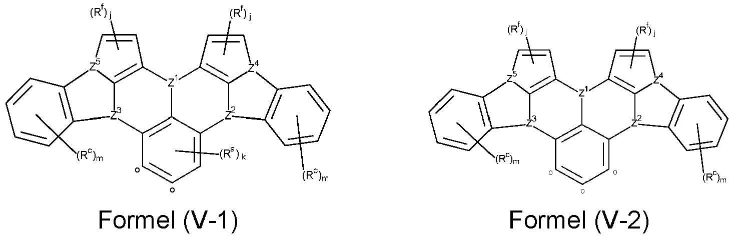 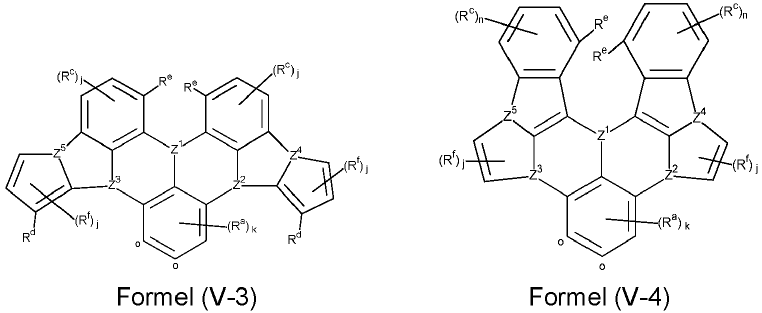 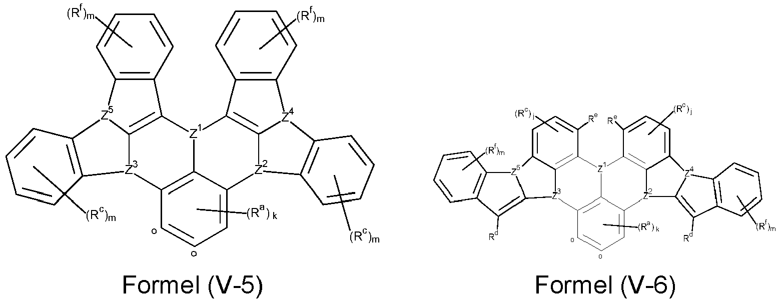 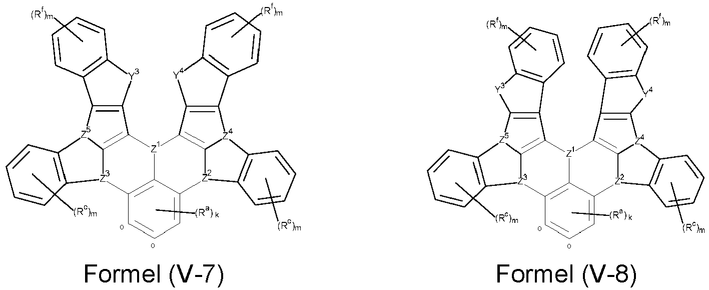 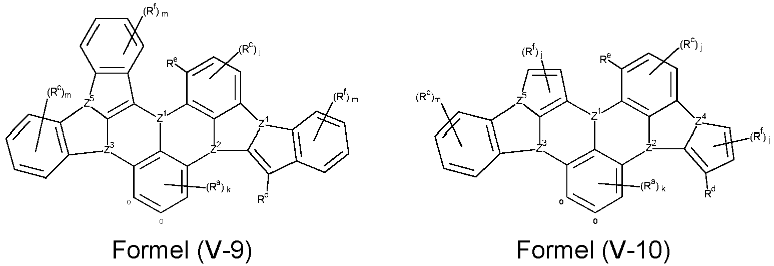 wobei die Symbole Z¹, Z², Z³, R^{a}, R^{b}, Z⁴, Z⁵, R^{c}, R^{d}, R^{e} und R^{f} die in Anspruch 1 genannten Bedeutungen aufweisen, die Symbole Y³ und Y⁴ die in Anspruch 2 genannten Bedeutungen aufweisen, das Symbol o für die Anbindungsstellen steht und die weiteren Symbole die folgende Bedeutung haben:
m ist 0, 1, 2, 3 oder 4;
n ist 0, 1, 2 oder 3;
j ist 0, 1 oder 2.

7. Verbindung nach einem oder mehreren der Ansprüche 1 bis 6, umfassend mindestens eine Struktur der Formeln (VI-1) bis (VI-9), wobei die Verbindungen mindestens einen kondensierten Ring aufweisen 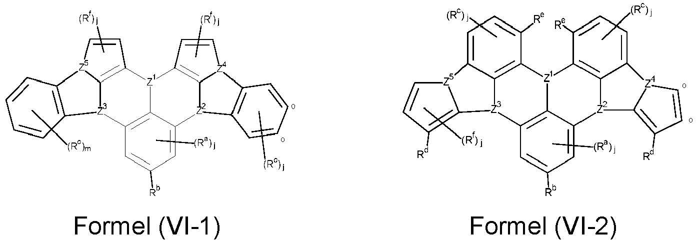 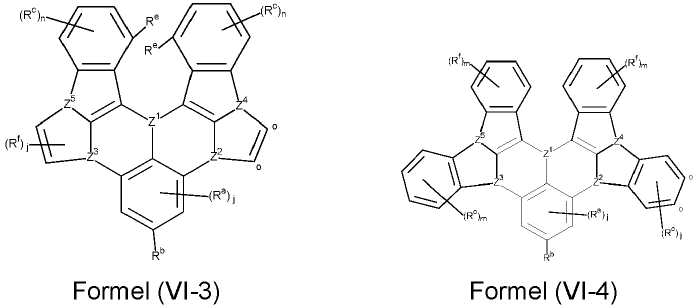 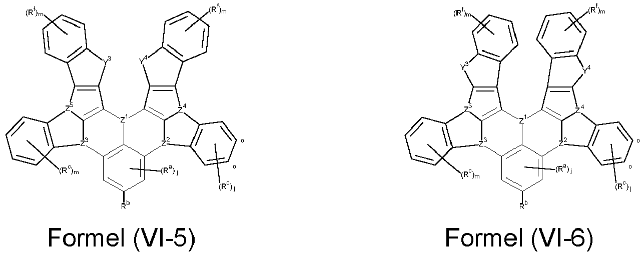 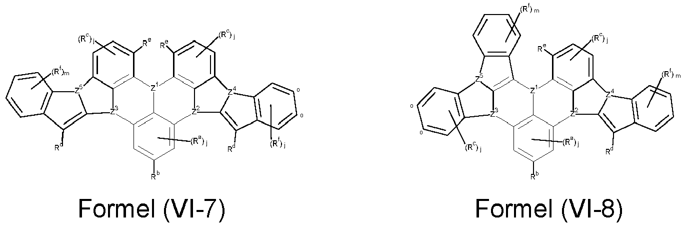 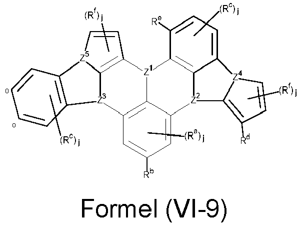 wobei die Symbole Z¹, Z², Z³, R^{a}, R^{b}, Z⁴, Z⁵, R^{c}, R^{d}, R^{e} und R^{f} die in Anspruch 1 genannten Bedeutungen aufweisen, die Symbole , die Symbole Y³ und Y⁴ die in Anspruch 2 genannten Bedeutungen aufweisen, das Symbol o für die Anbindungsstellen steht und die weiteren Symbole die folgende Bedeutung haben:
m ist 0, 1, 2, 3 oder 4;
n ist 0, 1, 2 oder 3;
j ist 0, 1 oder 2.

8. Verbindung nach einem oder mehreren der Ansprüche 1 bis 7, umfassend mindestens eine Struktur der Formeln (VII-1) bis (VII-9), wobei die Verbindungen mindestens einen kondensierten Ring aufweisen 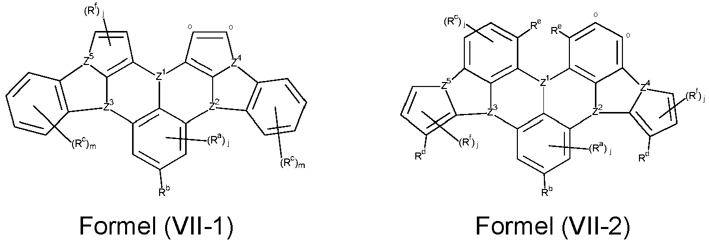 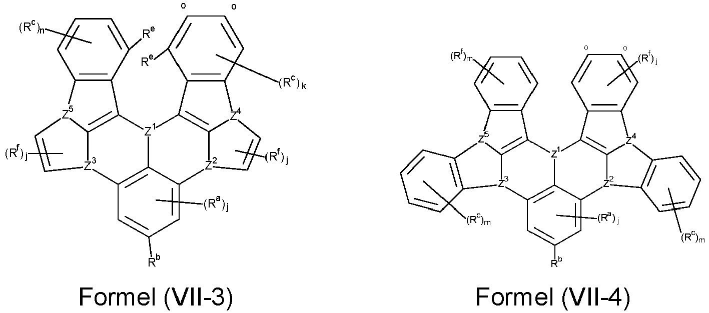 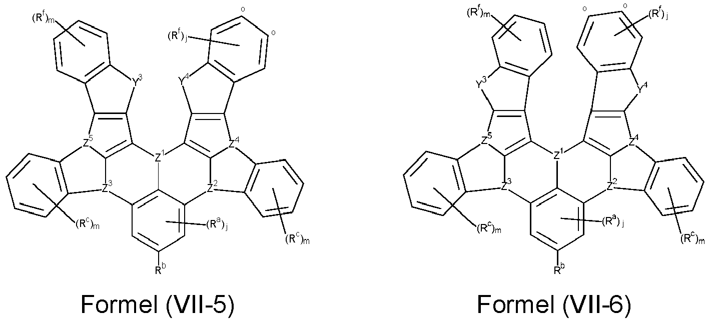 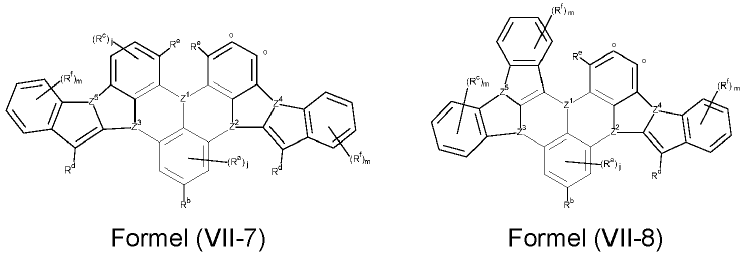 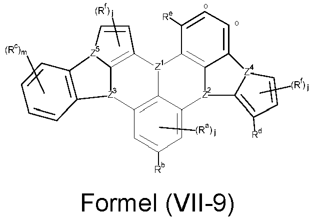 wobei die Symbole Z¹, Z², Z³, R^{a}, R^{b}, Z⁴, Z⁵, R^{c}, R^{d}, R^{e} und R^{f} die in Anspruch 1 genannten Bedeutungen aufweisen, die Symbole Y³ und Y⁴ die in Anspruch 2 genannten Bedeutungen aufweisen, das Symbol o für die Anbindungsstellen steht und die weiteren Symbole die folgende Bedeutung haben:
m ist 0, 1, 2, 3 oder 4;
n ist 0, 1, 2 oder 3;
j ist 0, 1 oder 2.

9. Oligomer, Polymer oder Dendrimer enthaltend eine oder mehrere Verbindungen nach einem der Ansprüche 1 bis 8, wobei statt eines Wasserstoffatoms oder eines Substituenten eine oder mehrere Bindungen der Verbindungen zum Polymer, Oligomer oder Dendrimer vorhanden sind.

10. Formulierung, enthaltend mindestens eine Verbindung nach einem oder mehreren der Ansprüche 1 bis 8 oder ein Oligomer, Polymer oder Dendrimer nach Anspruch 9 und mindestens eine weitere Verbindung, wobei die weitere Verbindung bevorzugt ausgewählt ist aus einem oder mehreren Lösemitteln.

11. Zusammensetzung, enthaltend mindestens eine Verbindung nach einem oder mehreren der Ansprüche 1 bis 8 oder ein Oligomer, Polymer oder Dendrimer nach Anspruch 9 und mindestens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

12. Verfahren zur Herstellung einer Verbindung nach einem oder mehreren der Ansprüche 1 bis 8, **dadurch gekennzeichnet, dass** ein Grundgerüst mit mindestens einer der Gruppen Z², Z³ oder einem Vorläufer einer der Gruppe Z², Z³ synthetisiert wird und die Gruppe Z¹ mittels einer Metallierungsreaktion, einer nukleophilen aromatischen Substitutionsreaktion oder einer Kupplungsreaktion eingeführt wird.

13. Verwendung einer Verbindung nach einem oder mehreren der Ansprüche 1 bis 8 oder ein Oligomer, Polymer oder Dendrimer nach Anspruch 9 in einer elektronischen Vorrichtung.

14. Elektronische Vorrichtung enthaltend mindestens eine Verbindung nach einem oder mehreren der Ansprüche 1 bis 8 oder ein Oligomer, Polymer oder Dendrimer nach Anspruch 9.

## Claims

1. A compound comprising at least one structure of formula (IIa), (IIb), (IIc), (Ild), (Ile), 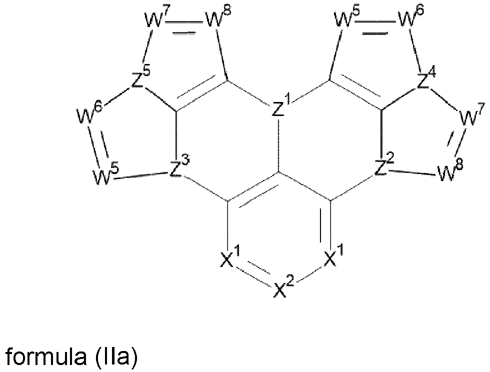 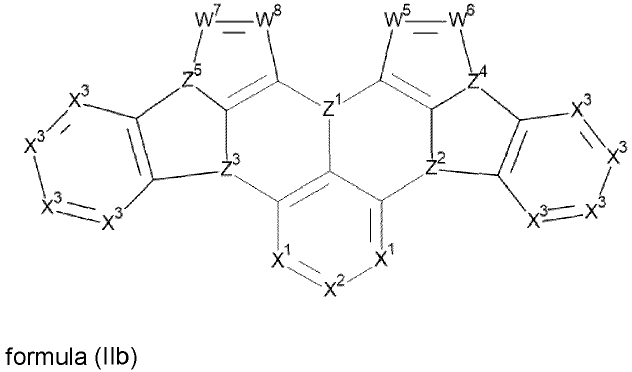 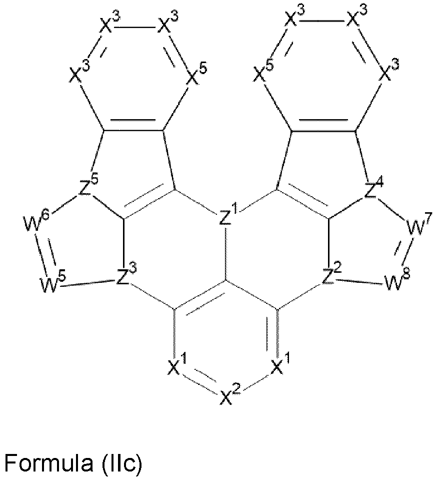 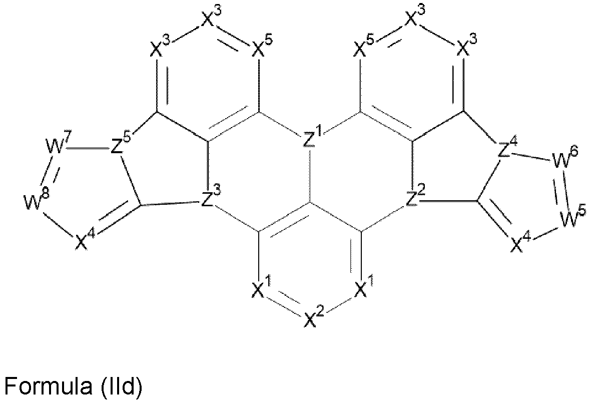 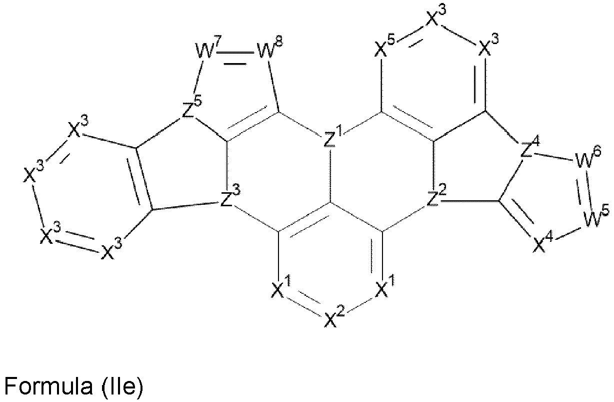 wherein the following applies to the symbols and indices used Z¹ is B;
Z², Z³, Z⁴, Z⁵ is N or B, whichever is the same or different at each occurrence; wherein two of the groups Z², Z³, Z⁴, Z⁵ are N,
W⁵, W⁶, W⁷, W⁸ is the same or different for C(Ar) or X⁶ at each occurrence;
Ar is, in each occurrence, identically or differently, an aromatic or heteroaromatic ring system with 5 to 60 aromatic ring atoms, which can be substituted with one or more radicals R, in which case the group Ar can form a ring system with at least one group Ar, X¹, R or a further group;
X¹ represents N, CR^{a} or C, identically or differently in each occurrence, if a ring system is formed by a bond with one of the rings Ar^{c}, Ar^{d} or a further group, with the proviso that no more than two of the groups X¹, X² in a cycle represent N;
X² is the same or different for N or CR^{b} at each occurrence, with the proviso that no more than two of the groups X¹, X² are in a cycle for N;
X³ is the same or different for each occurrence for N or CR^{c} with the proviso that no more than two of the groups X³, X⁵ are in a cycle for N;
X⁴ is the same or different for each occurrence for N or CR^{d} with the proviso that no more than two of the groups X⁴, X⁶ are in a cycle for N;
X⁵ represents N, CR^{e} or C, identically or differently for each occurrence, if a ring system is formed by a bond with a group Ar or a group X⁵ or a group X⁶, with the proviso that no more than two of the groups X³, X⁵ in a cycle represent N;
X⁶ in each occurrence is the same or different for N, CR^{f} or C, if a ring system is formed by a bond with a group Ar, a group X⁵ or a group X⁶, with the proviso that no more than two of the groups X⁴, X⁶ in a cycle are N;
R, R^{a}, R^{b} is in each occurrence the same or different H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, wherein the alkyl, alkoxy, thioalkoxy, thioalkoxy or alkynyl group has 1 to 40 carbon atoms, alkoxy, thioalkoxy, alkenyl or alkynyl group may each be substituted with one or more radicals R¹ wherein one or more non-adjacent CH₂ groups may be replaced by R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system with 5 to 60 aromatic ring atoms, each of which may be substituted by one or more radicals R¹, or an aryloxy or heteroaryloxy group with 5 to 60 aromatic ring atoms, which may be substituted by one or more radicals R¹; two radicals R, R^{a}, R^{b} can also form a ring system with each other or with another group;
R^{c}, R^{d}, R^{e}, R^{f} is in each occurrence the same or different H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, wherein the alkyl, alkoxy, thioalkoxy, thioalkoxy or alkynyl group has 1 to 40 carbon atoms, alkoxy, thioalkoxy, alkenyl or alkynyl group may each be substituted with one or more radicals R¹ wherein one or more non-adjacent CH₂ groups may be replaced by R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system with 5 to 60 aromatic ring atoms, each of which may be substituted by one or more radicals R¹, or an aryloxy or heteroaryloxy group with 5 to 60 aromatic ring atoms, which may be substituted by one or more radicals R¹; two radicals R^{c}, R^{d}, R^{e}, R^{f} can also form a ring system with each other or with another group;
Ar' is in each occurrence identical or different an aromatic or heteroaromatic ring system with 5 to 60 aromatic ring atoms, which can be substituted with one or more radicals R¹, whereby two radicals Ar', which bind to the same C atom, Si atom, N atom, P atom or B atom, can also be linked by a single bond or a bridge, selected from B(R¹), C(R¹)₂, Si(R¹)₂, C=O, C=NR¹, C=C(R¹)₂, O, S, S=O, SO₂, N(R¹), P(R¹) and P(=O)R';
R¹ is the same or different for each occurrence H, D, F, Cl, Br, I, CN, NO₂, N(Ar")₂, N(R²)₂, C(=O)OAr", C(=O)OR², C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, a straight-chain alkyl, alkoxy or thioalkoxy group with 1 to 40 C atoms or a branched or cyclic alkyl, alkoxy or thioalkoxy group with 1 to 40 C atoms, alkoxy or thioalkoxy group having 3 to 40 carbon atoms or an alkenyl group having 2 to 40 carbon atoms, each of which may be substituted by one or more radicals R², wherein one or more non-adjacent CH₂ groups are substituted by -R²C=CR²-, -C=C-, Si(R²)₂, C=O, C=S, C=Se, C=NR², -C(=O)O-, -C(=O)NR²-, NR², P(=O)(R²), -O-, -S-, SO or SO₂ and wherein one or more H atoms may be replaced by D, F, Cl, Br, I, CN or NO₂, or an aromatic or heteroaromatic ring system with 5 to 60 aromatic ring atoms, each of which may be substituted by one or more radicals R², or an aryloxy or heteroaryloxy group having 5 to 60 aromatic ring atoms, which may be substituted by one or more radicals R², or an aralkyl or heteroaralkyl group having 5 to 60 aromatic ring atoms, which may be substituted by one or more radicals R², or a combination of these systems; two or more radicals R¹ may form a ring system with one another, and one or more radicals R¹ may form a ring system with another part of the compound;
Ar" is an aromatic or heteroaromatic ring system with 5 to 30 aromatic ring atoms, which may be substituted with one or more radicals R², whereby two radicals Ar", which bind to the same C atom, Si atom, N atom, P atom or B atom, can also be linked by a single bond or a bridge, selected from B(R²), C(R²)₂, Si(R²)₂, C=O, C=NR², C=C(R²)₂, O, S, S=O, SO₂, N(R²), P(R²) and P(=O)R²;
R² is the same or different in each occurrence and is selected from the group consisting of H, D, F, CN, an aliphatic hydrocarbon radical having 1 to 20 carbon atoms or an aromatic or heteroaromatic ring system having 5 to 30 aromatic ring atoms, in which one or more H atoms may be replaced by D, F, Cl, Br, I or CN and which may be substituted by one or more alkyl groups each having 1 to 4 carbon atoms, whereby two or more substituents R² may form a ring system with one another.

2. A compound according to claim 1 comprising at least one structure of formula (III-1) to (III-25), 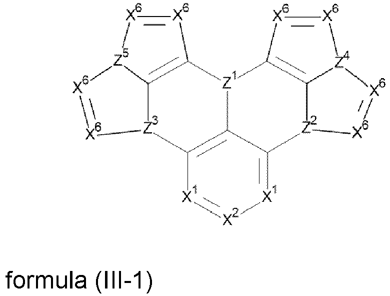 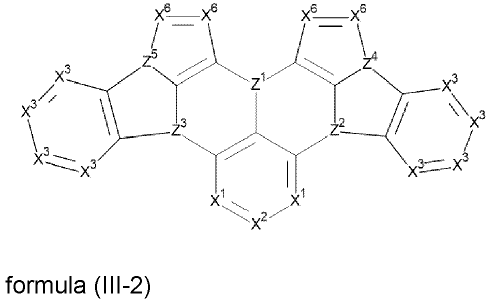 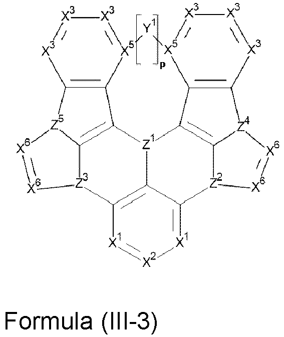 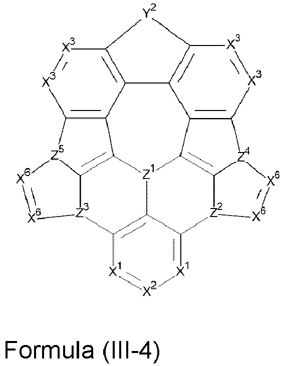 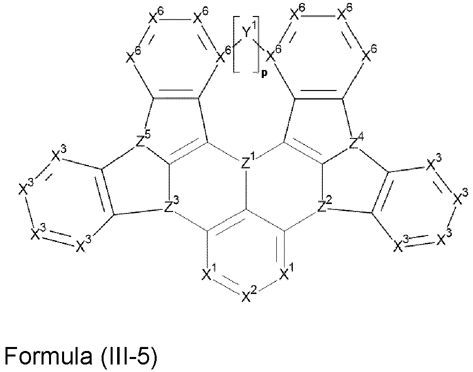 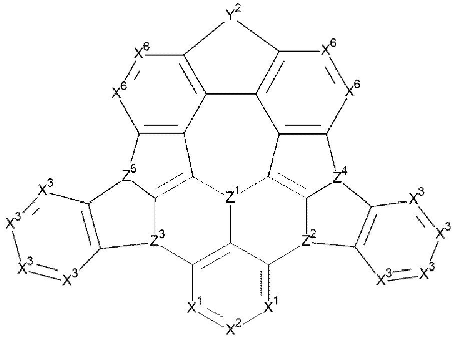 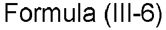 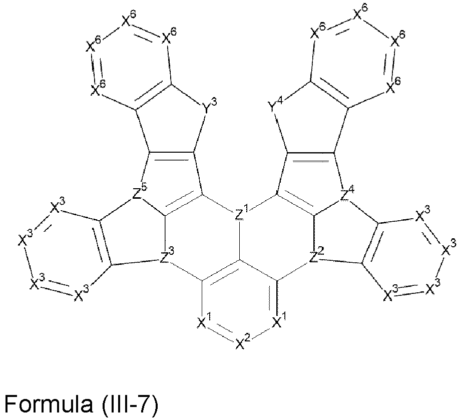 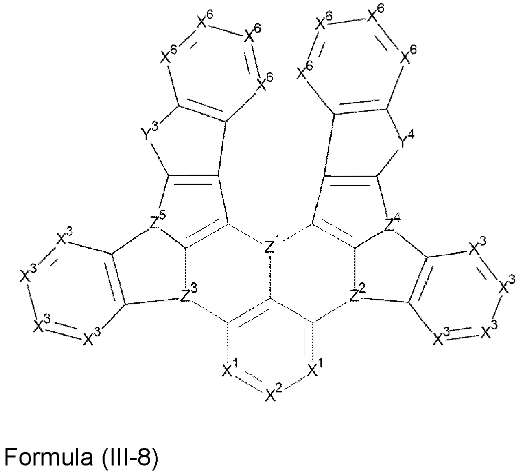 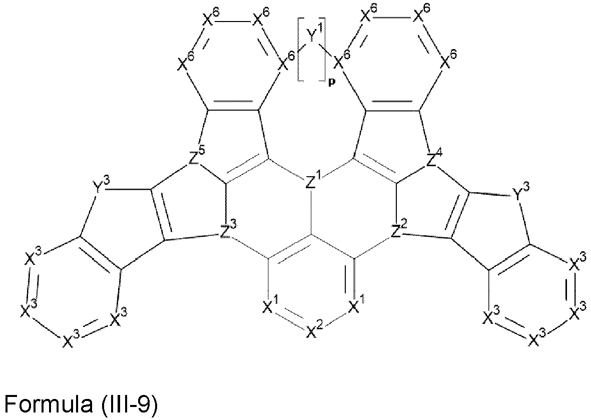 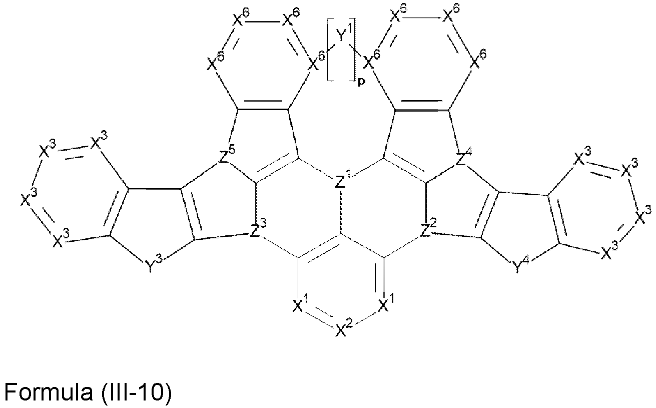 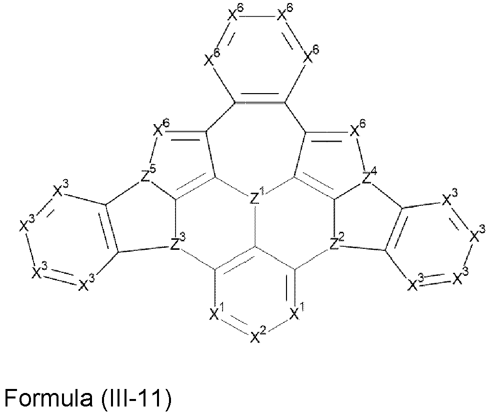 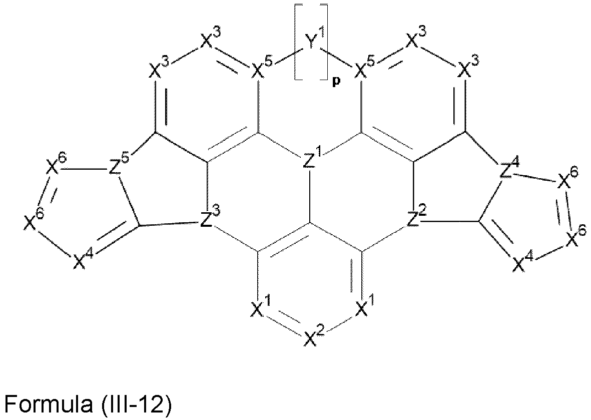 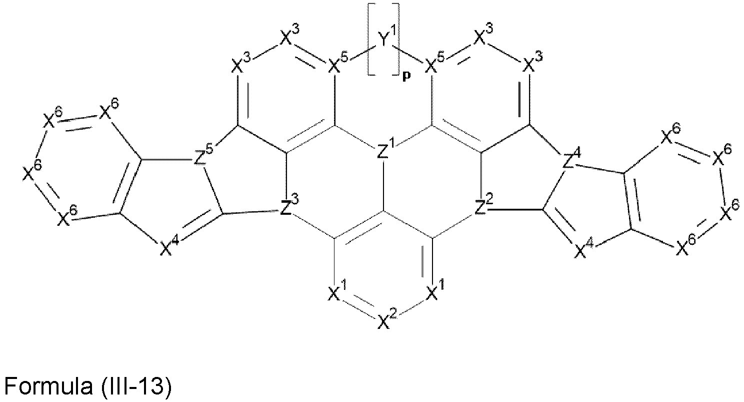 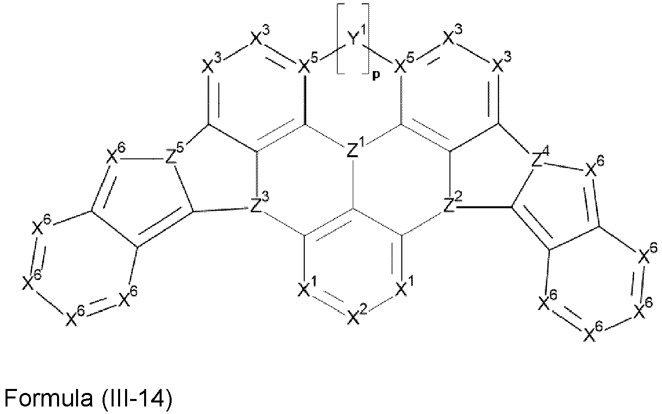 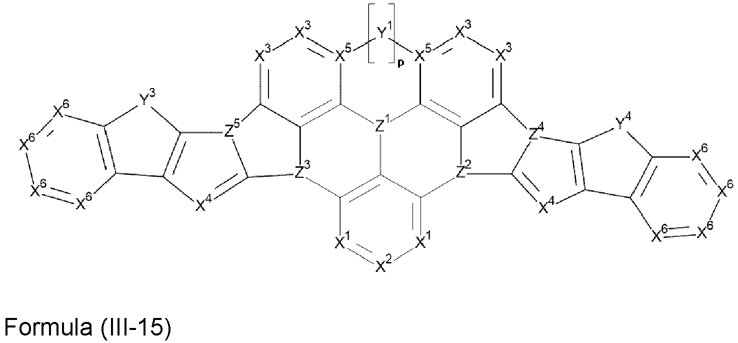 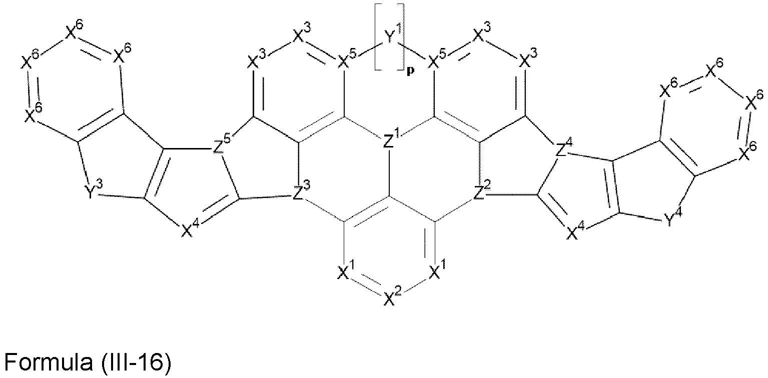 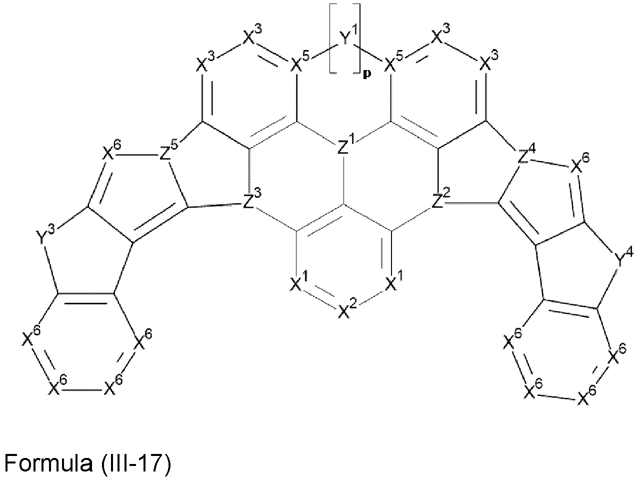 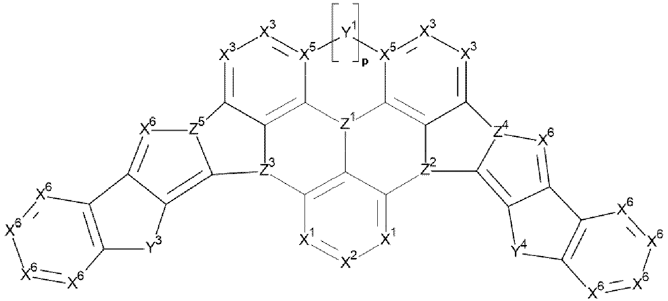 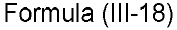 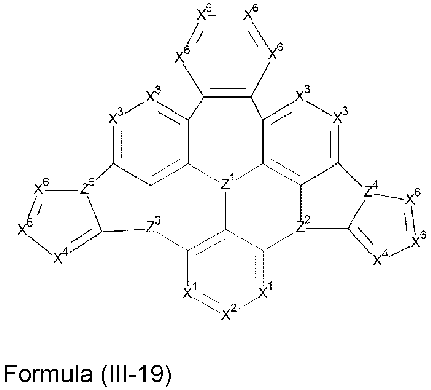 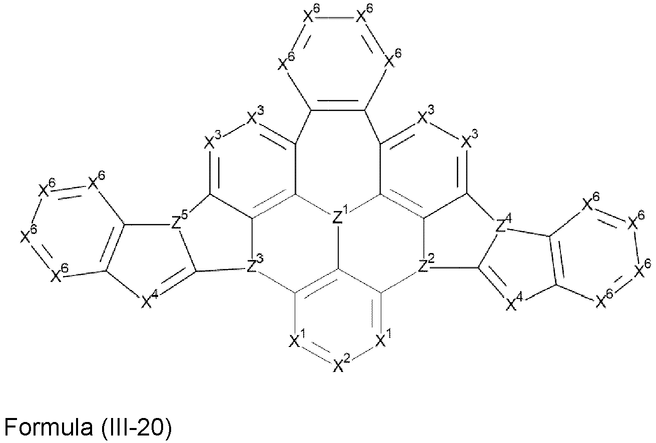 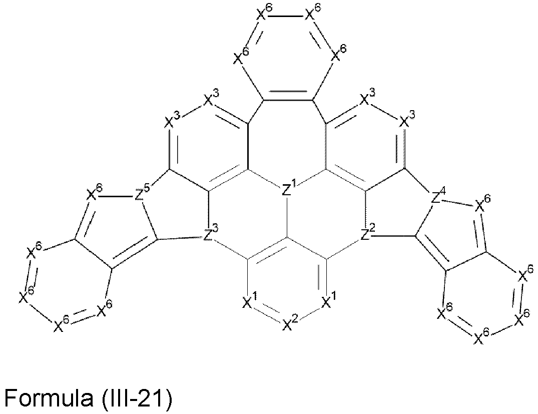 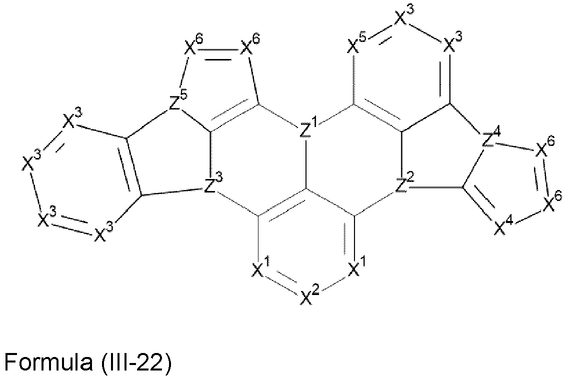 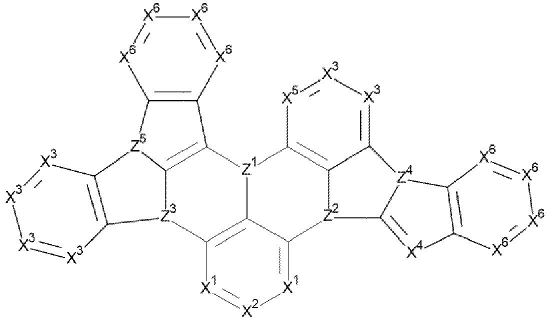 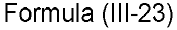 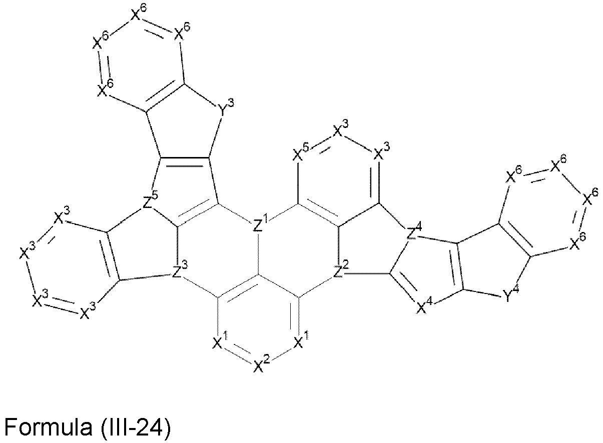 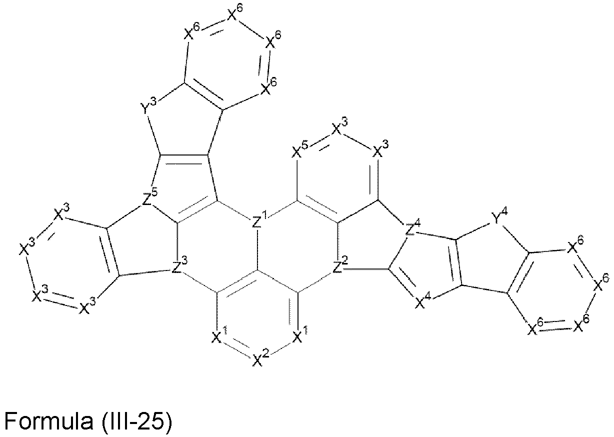 wherein the symbols Z¹, Z², Z³, X¹, X², Z⁴, Z⁵, X³, X⁴, X⁵ and X⁶ have the meanings given in claim 1 and the further symbols have the following meanings:
p is 0 or 1,
Y¹ is the same or different for each occurrence a bond, N(Ar'), N(R), P(Ar'), P(R), P(=O)Ar', P(=O)R, P(=S)Ar', P(=S)R, B(Ar'), B(R), AI(Ar'), AI(R), Ga(Ar'), Ga(R), C=O, C(R)₂, Si(R)₂, C=NR, C=NAr', C=C(R)₂, O, S, Se, S=O, or SO₂, wherein the symbols R and Ar' have the meaning set forth in claim 1;
Y², Y³, Y⁴ is at each occurrence the same or different N(Ar'), N(R), P(Ar'), P(R), P(=O)Ar', P(=O)R, P(=S)Ar', P(=S)R, B(Ar'), B(R), AI(Ar'), AI(R), Ga(Ar'), Ga(R), C=O, C(R)₂, Si(R)₂, C=NR, C=NAr', C=C(R)₂, O, S, Se, S=O, or SO₂, wherein the symbols R and Ar' have the meaning set forth in claim 1.

3. A compound according to claim 1 or 2 comprising at least one structure of formula (IV-1) to (IV-38), 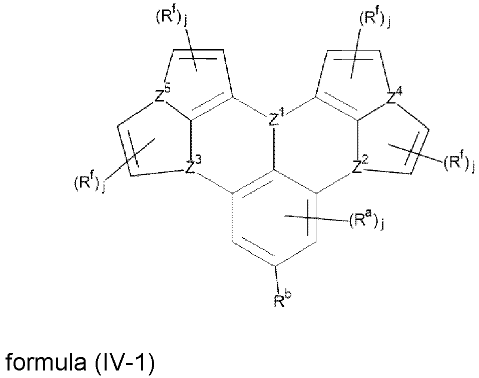 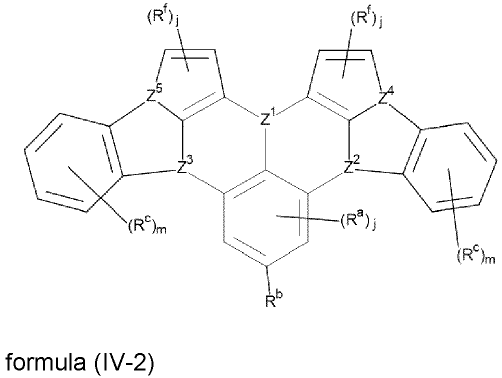 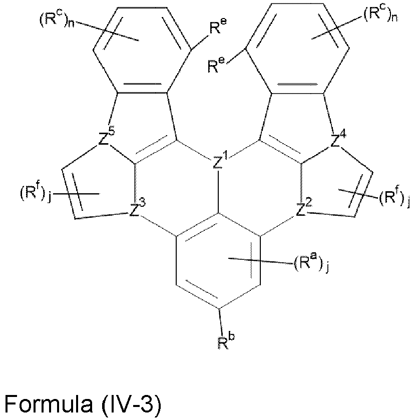 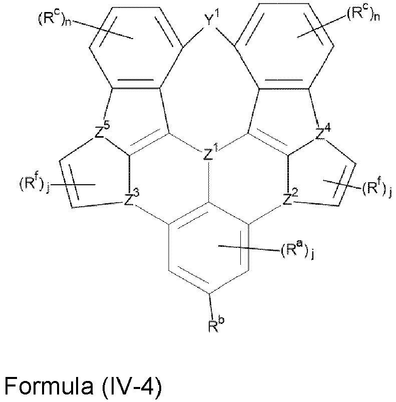 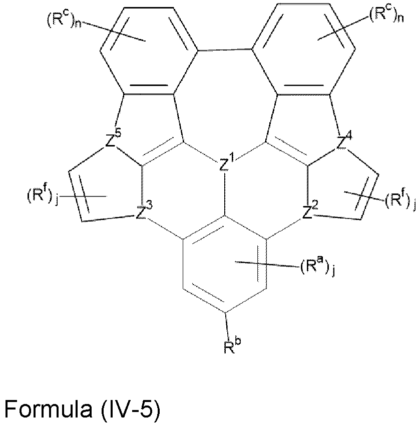 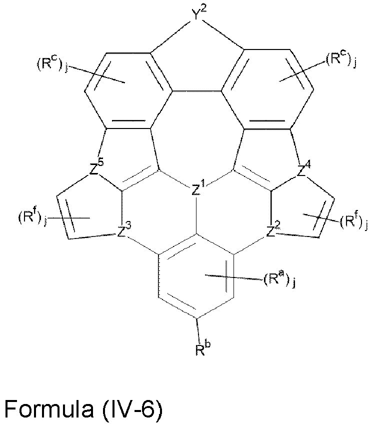 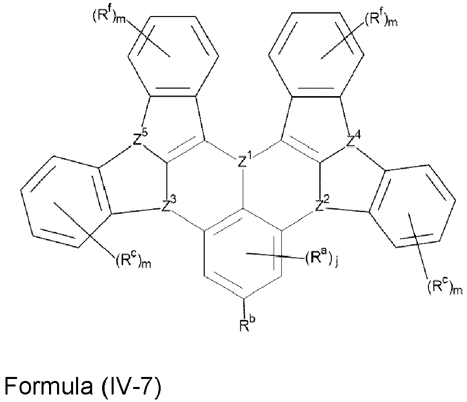 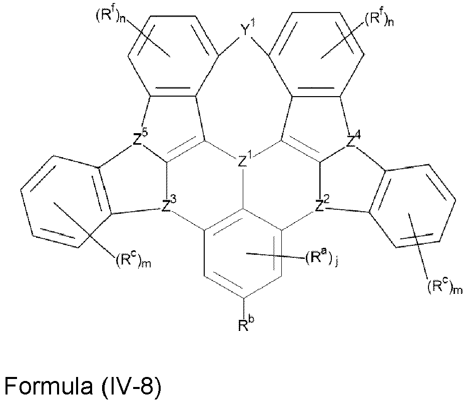 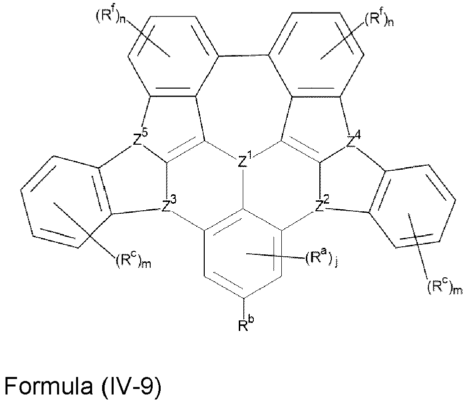 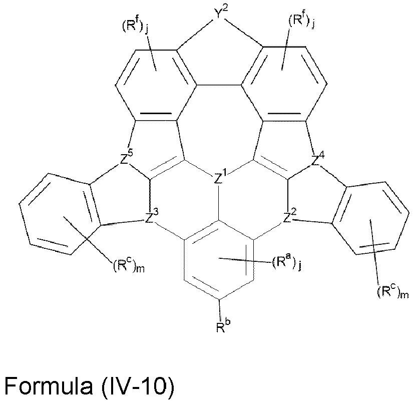 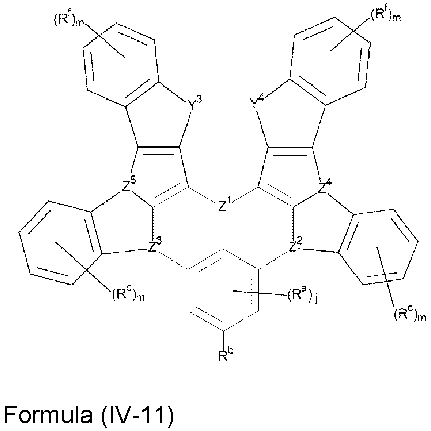 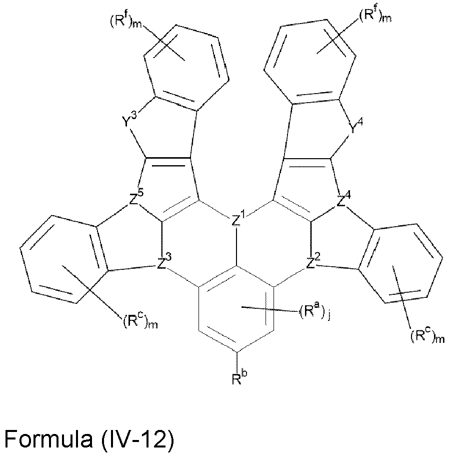 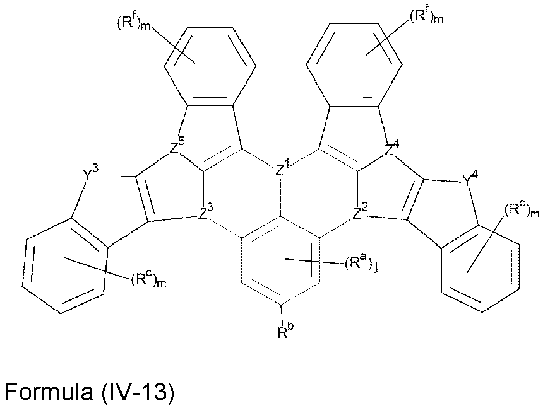 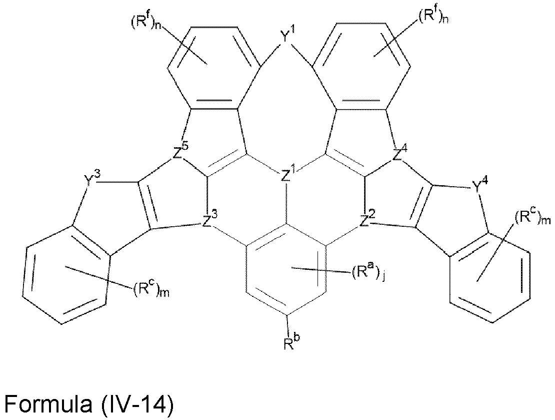 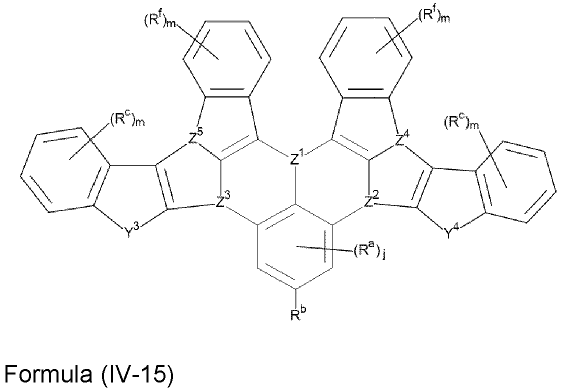 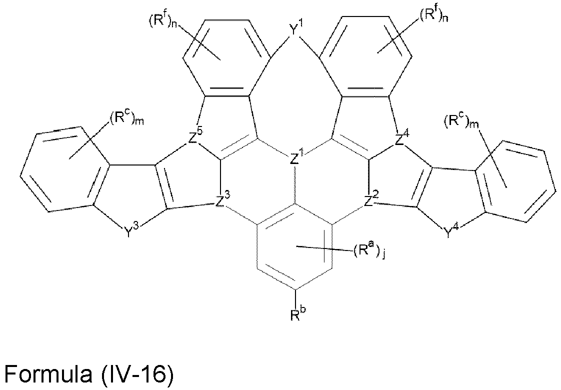 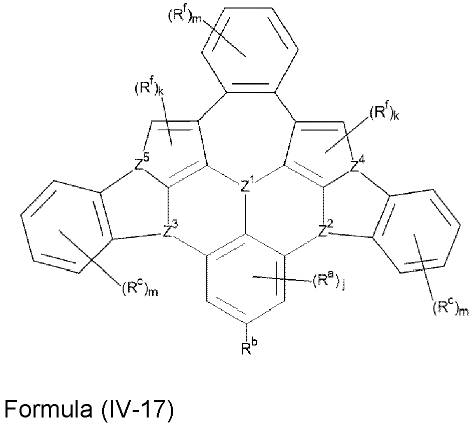 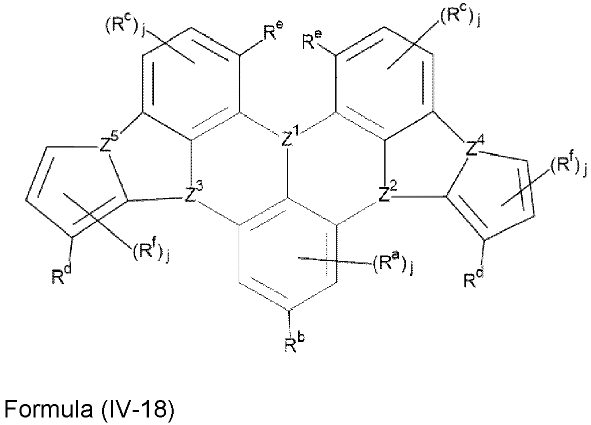 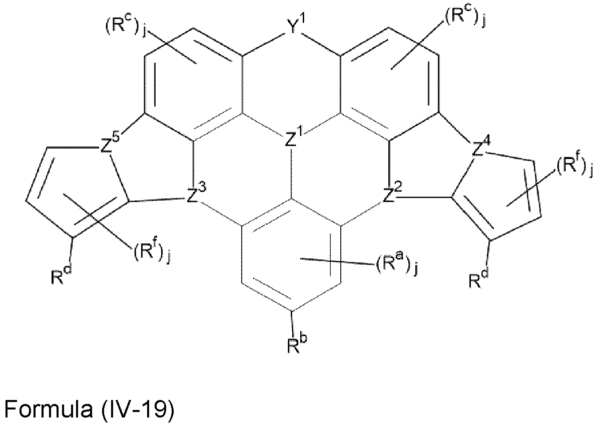 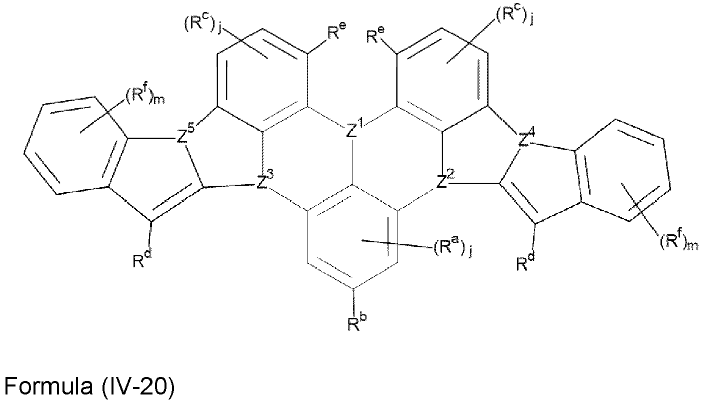 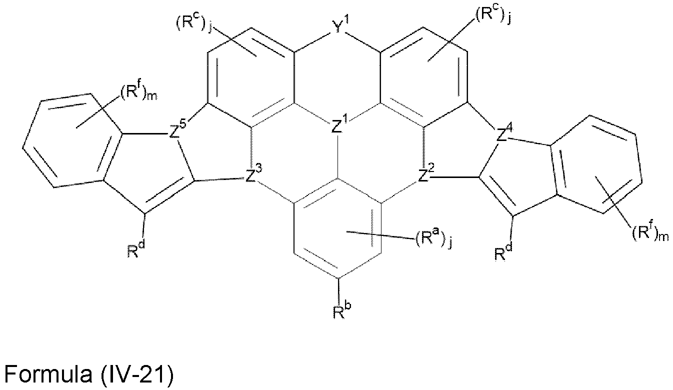 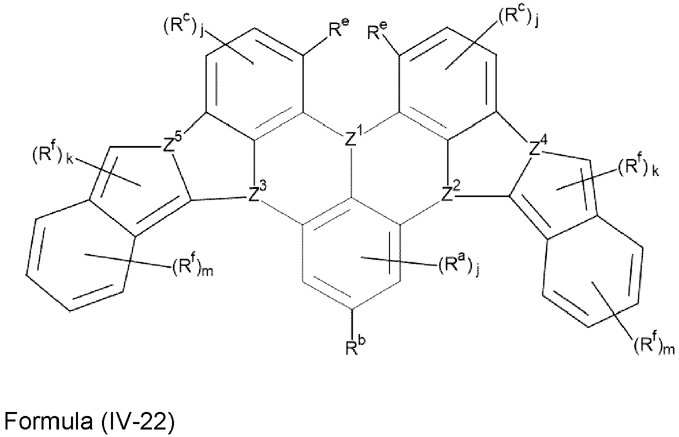 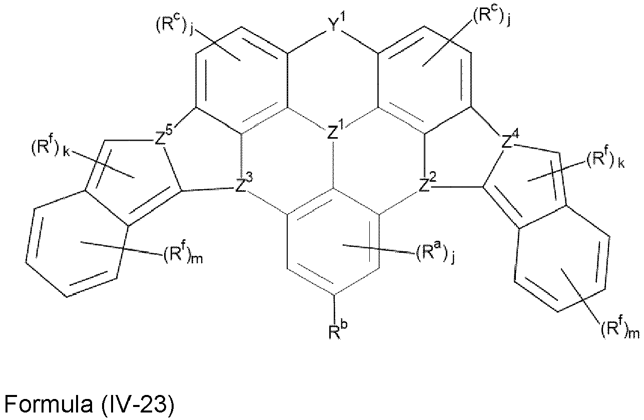 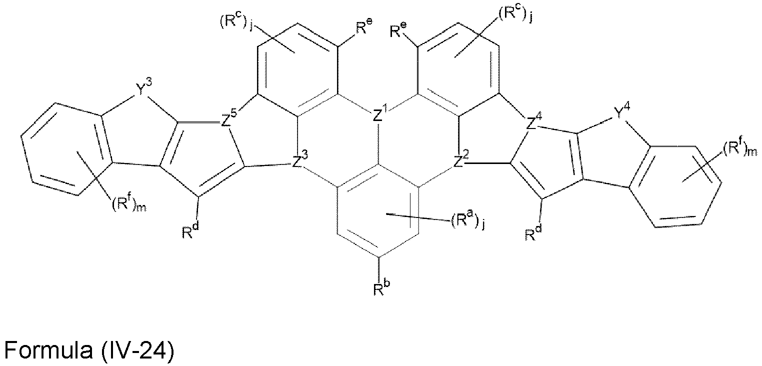 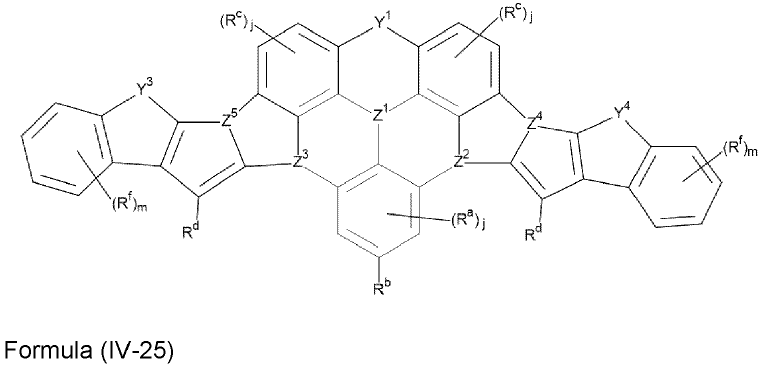 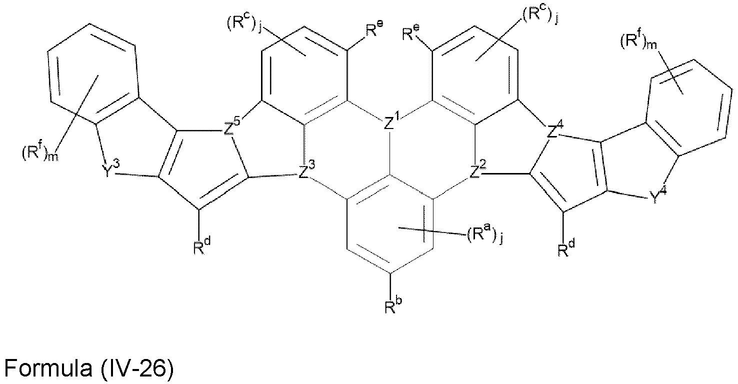 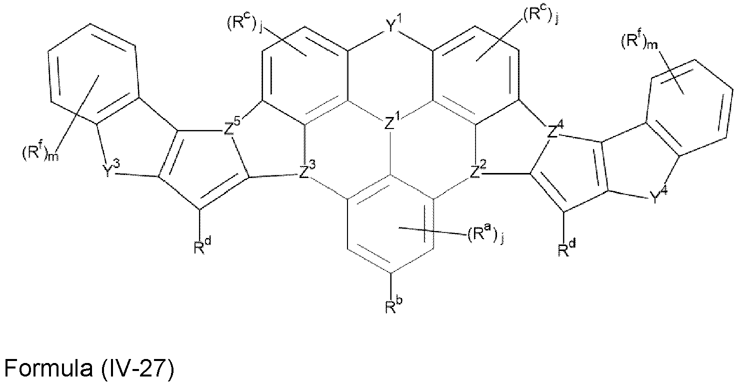 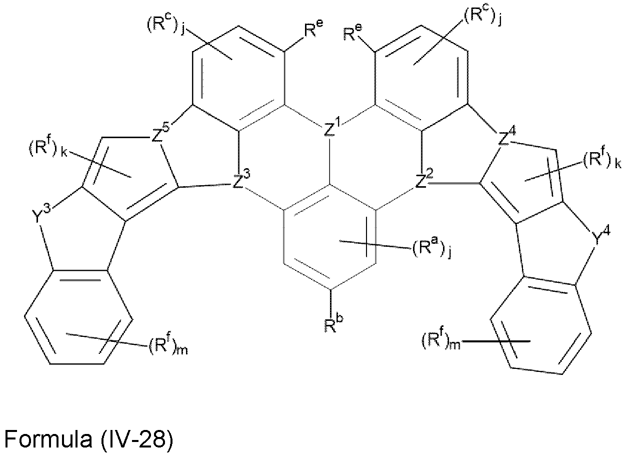 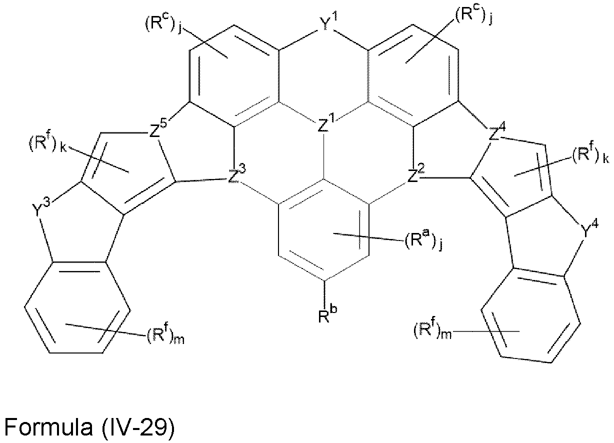 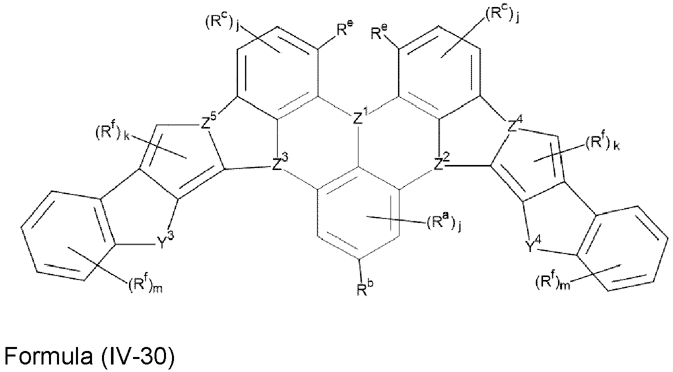 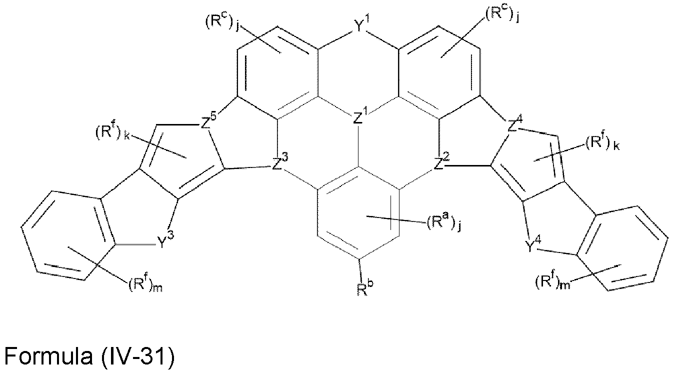 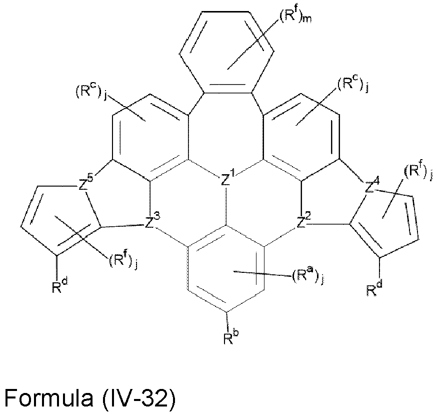 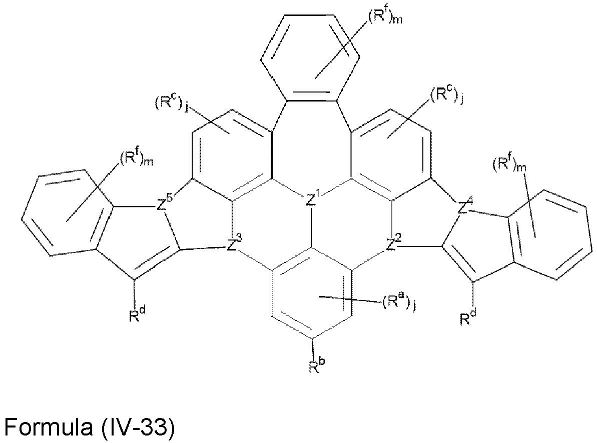 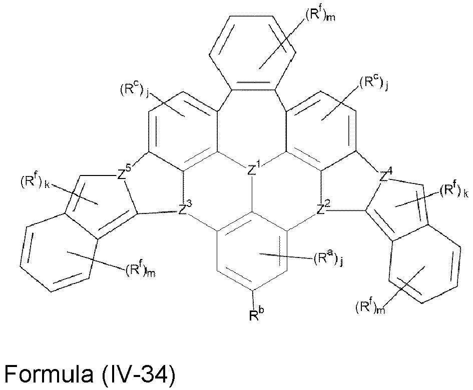 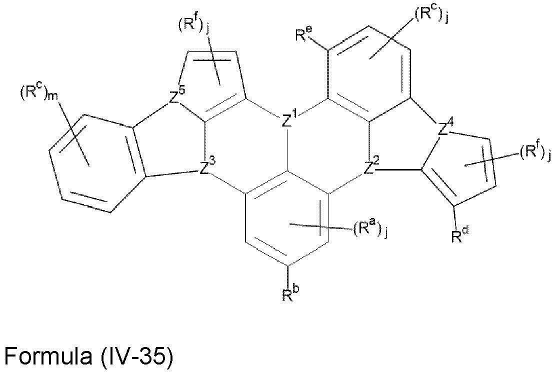 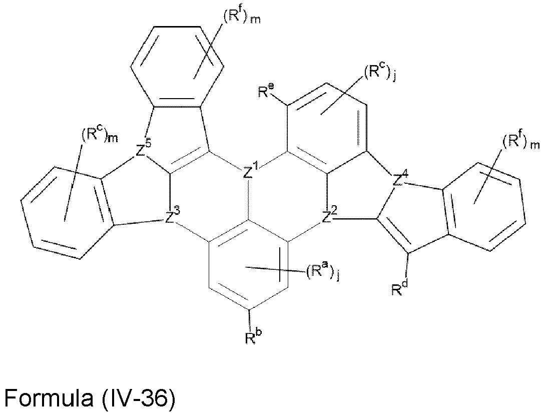 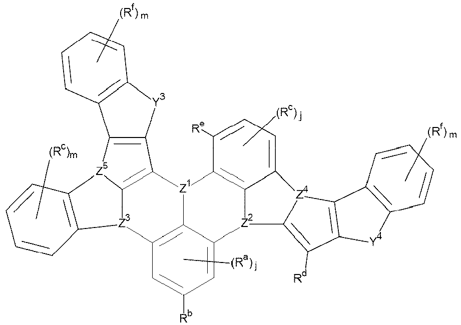 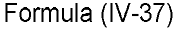 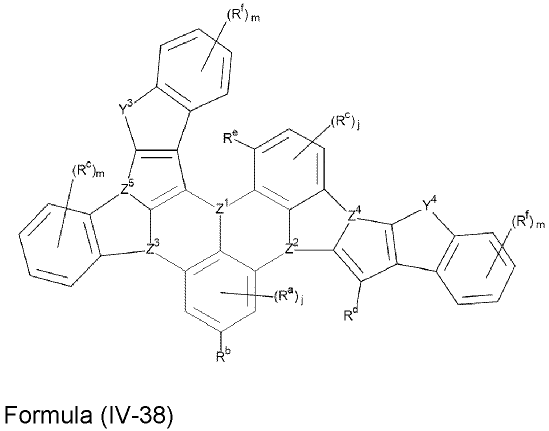 wherein the symbols Z¹, Z², Z³, R^{a}, R^{b} Z⁴, Z⁵, R^{c}, R^{d}, R^{e} and R^{f} have the meanings given in claim 1, the symbols Y¹, Y², Y³ and Y⁴ have the meanings given in claim 2 and the further symbols have the following meanings:
m is 0, 1, 2, 3 or 4;
n is 0, 1, 2 or 3;
j is 0, 1 or 2;
k is 0 or 1.

4. A compound according to one or more of claims 1 to 3, **characterised in that** at least two radicals R, R^{a}, R^{b}, R^{c}, R^{d}, R^{e}, R^{f} form a fused ring with the further groups to which the two radicals R, R^{a}, R^{b}, R^{c}, R^{d}, R^{e}, R^{f} form a condensed ring, wherein the two radicals R, R^{a}, R^{b}, R^{c}, R^{d}, R^{e}, R^{f} form at least one structure of the formulae (RA-1) to (RA-12) 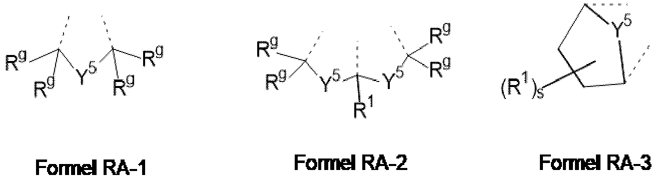 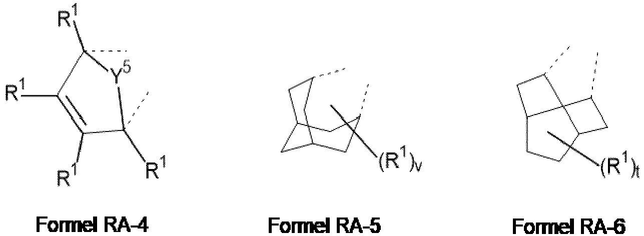 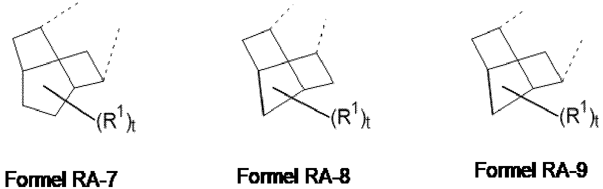 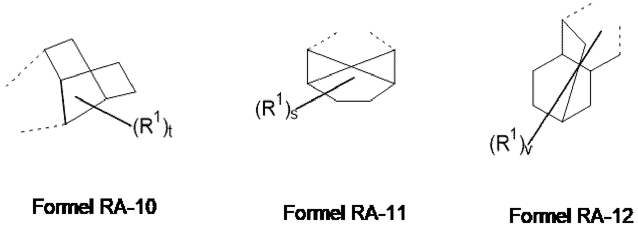 wherein R¹ has the meaning given above, the dashed bonds represent the binding sites to the atoms of the groups to which the two radicals R, R^{a}, R^{b}, R^{c}, R^{d}, R^{e}, R^{f} bind, and the other symbols have the following meaning:
Y⁵ is the same or different at each occurrence C(R¹)₂, (R¹)₂C-C(R¹)₂, (R¹)C=C(R¹), NR¹, NAr', O or S;
R^{g} is in each occurrence the same or different F, a straight-chain alkyl, alkoxy or thioalkoxy group with 1 to 40 C atoms or an alkenyl or alkynyl group with 2 to 40 C atoms or a branched or cyclic alkyl, alkoxy or thioalkoxy group with 1 to 40 C atoms or a branched or cyclic alkyl, alkoxy or thioalkoxy group with 2 to 40 C atoms, alkoxy or thioalkoxy group with 3 to 20 C atoms, wherein the alkyl, alkoxy, thioalkoxy, alkenyl or alkynyl group can each be substituted with one or more radicals R², wherein one or more non-adjacent CH₂ groups may be replaced by R²C=CR², C=C, Si(R²)₂, C=O, C=S, C=Se, C=NR², -C(=O)O-, -C(=O)NR²-, NR², P(=O)(R¹), -O-, -S-, SO or SO₂, or an aromatic or heteroaromatic ring system with 5 to 60 aromatic ring atoms, which may each be substituted by one or more radicals R², or an aryloxy or heteroaryloxy group having 5 to 60 aromatic ring atoms, which may be substituted by one or more radicals R²; two radicals R^{g} can also form a ring system with each other or a radical R^{g} with a radical R¹ or with a further group;
s is 0, 1, 2, 3, 4, 5 or 6;
t is 0, 1, 2, 3, 4, 5, 6, 7 or 8;
v is 0, 1, 2, 3, 4, 5, 6, 7, 8 or 9.

5. The compound according to one or more of claims 1 to 4, in that at least two radicals R, R^{a}, R^{b}, R^{c}, R^{d}, R^{e}, R^{f} form a fused ring with the further groups to which the two radicals R, R^{a}, R^{b}, R^{c}, R^{d}, R^{e}, R^{f} form a fused ring, wherein the two radicals R, R^{a}, R^{b}, R^{c}, R^{d}, R^{e}, R^{f} form the structures of formula (RB), 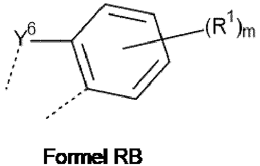 wherein R¹ has the meaning given in claim 1, the dotted bonds represent the binding sites to which the two radicals R, R^{a}, R^{b}, R^{c}, R^{d}, R^{e}, R^{f} bind, the index m is 0, 1, 2, 3 or 4 and Y⁶ is C(R¹)₂, NR¹, NAr', BR¹, BAr', O or S.

6. The compound according to one or more of claims 1 to 5, comprising at least one structure of formulae (V-1) to (V-10), wherein the compounds have at least one fused ring, 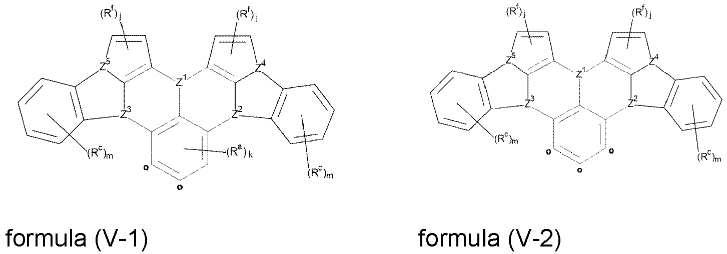 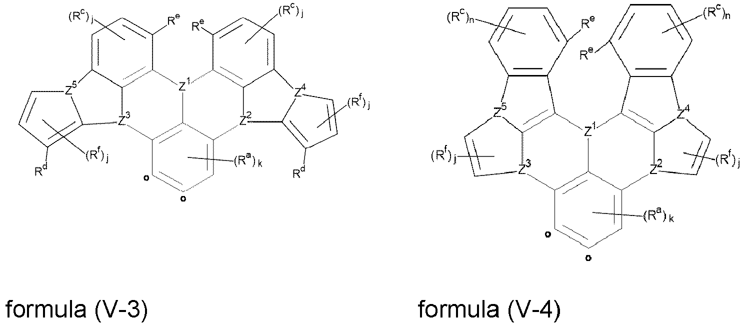 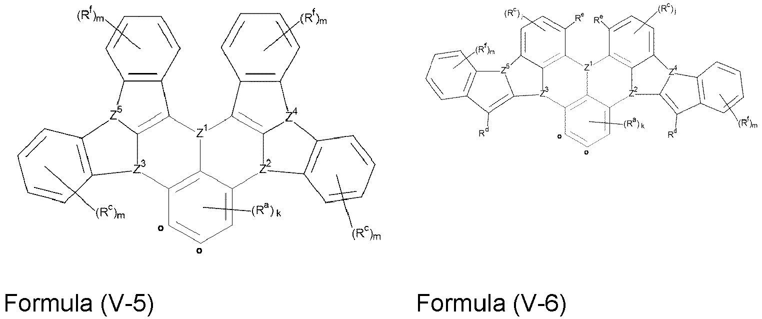 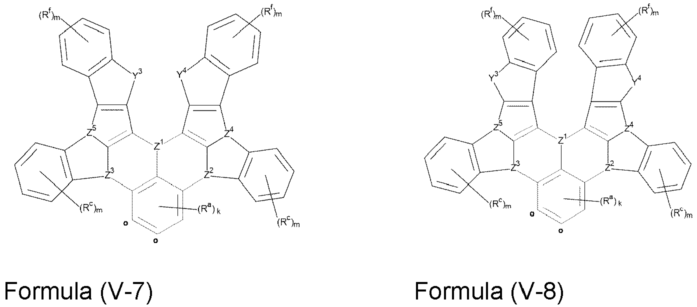 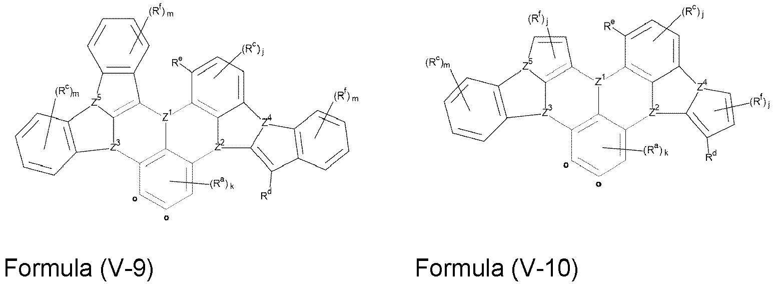 wherein the symbols Z¹, Z², Z³, R^{a}, R^{b}, Z⁴, Z⁵, R^{c}, R^{d}, R^{e} and R^{f} have the meanings given in claim 1, the symbols Y³ and Y⁴ have the meanings given in claim 2, the symbol o stands for the binding sites and the other symbols have the following meanings:
m is 0, 1, 2, 3 or 4;
n is 0, 1, 2 or 3;
j is 0, 1 or 2.

7. A compound according to one or more of claims 1 to 6, comprising at least one structure of formulae (VI-1) to (VI-9), wherein the compounds comprise at least one fused ring 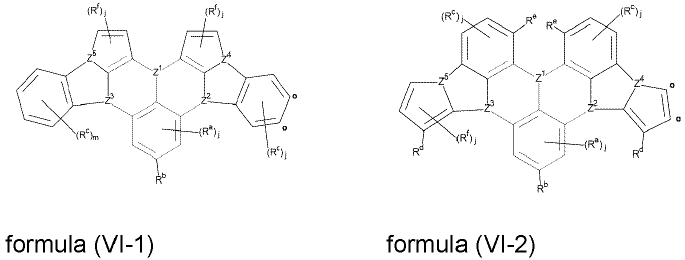 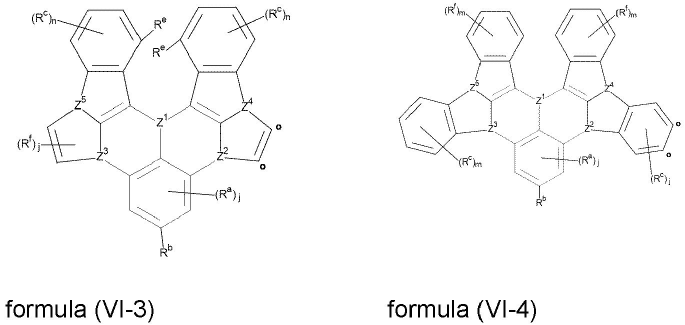 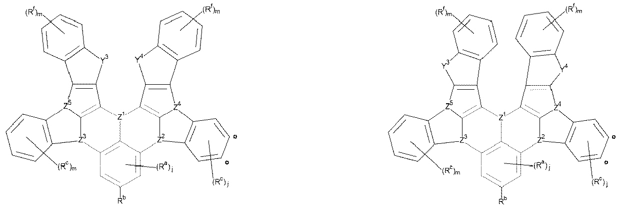 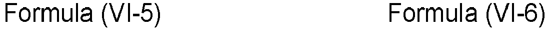 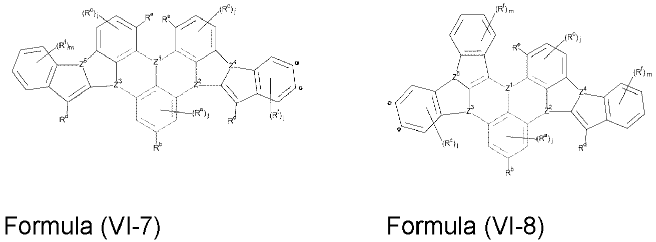 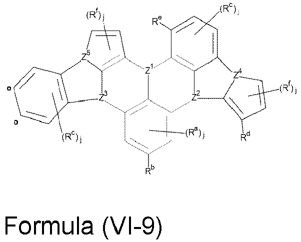 wherein the symbols Z¹, Z², Z³, R^{a}, R^{b}, Z⁴, Z⁵, R^{c}, R^{d}, R^{e} and R^{f} have the meanings given in claim 1, the symbols , the symbols Y³ and Y⁴ have the meanings given in claim 2, the symbol o stands for the binding sites and the further symbols have the following meanings:
m is 0, 1, 2, 3 or 4;
n is 0, 1, 2 or 3;
j is 0, 1 or 2.

8. A compound according to one or more of claims 1 to 7, comprising at least one structure of formulae (VII-1) to (VII-9), wherein the compounds comprise at least one fused ring 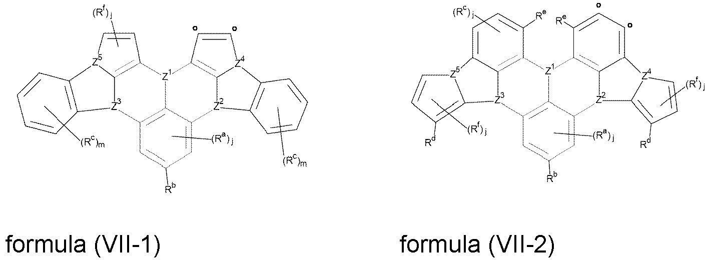 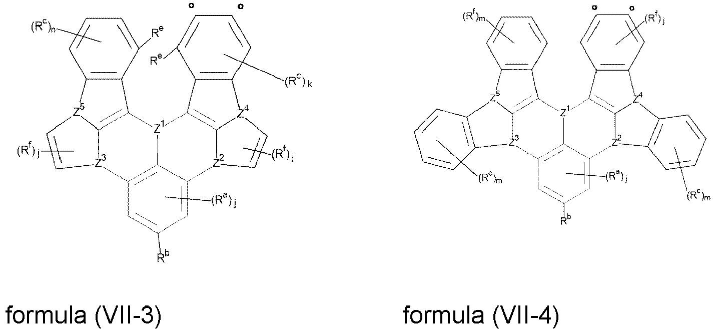 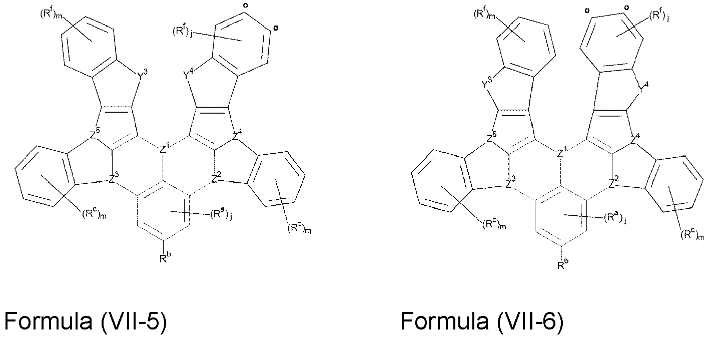 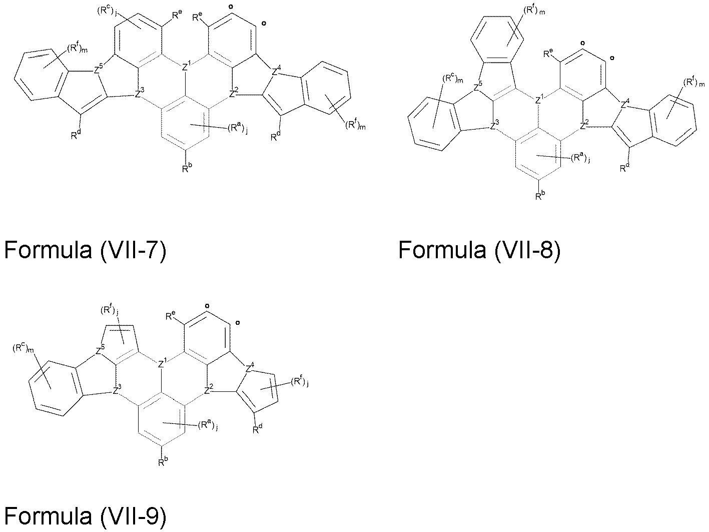 wherein the symbols Z¹, Z², Z³, R^{a}, R^{b}, Z⁴, Z⁵, R^{c}, R^{d}, R^{e} and R^{f} have the meanings given in claim 1, the symbols Y³ and Y⁴ have the meanings given in claim 2, the symbol o stands for the binding sites and the other symbols have the following meanings:
m is 0, 1, 2, 3 or 4;
n is 0, 1, 2 or 3;
j is 0, 1 or 2.

9. Oligomer, polymer or dendrimer containing one or more compounds according to one of claims 1 to 8, wherein instead of a hydrogen atom or a substituent one or more bonds of the compounds to the polymer, oligomer or dendrimer are present.

10. A formulation containing at least one compound according to one or more of claims 1 to 8 or an oligomer, polymer or dendrimer according to claim 9 and at least one further compound, wherein the further compound is preferably selected from one or more solvents.

11. A composition containing at least one compound according to one or more of claims 1 to 8 or an oligomer, polymer or dendrimer according to claim 9 and at least one further compound selected from the group consisting of fluorescent emitters, phosphorescent emitters, emitters showing TADF, host materials, electron transport materials, electron injection materials, hole conductor materials, hole injection materials, electron blocking materials and hole blocking materials.

12. A process for the preparation of a compound according to one or more of claims 1 to 8, **characterised in that** a backbone is synthesized with at least one of the groups Z², Z³ or a precursor of one of the groups Z², Z³ and the group Z¹ is introduced by means of a metallation reaction, a nucleophilic aromatic substitution reaction or a coupling reaction.

13. Use of a compound according to one or more of claims 1 to 8 or an oligomer, polymer or dendrimer according to claim 9 in an electronic device.

14. An electronic device containing at least one compound according to one or more of claims 1 to 8 or an oligomer, polymer or dendrimer according to claim 9.

## Revendications

1. Composé comprenant au moins une structure de formule (Ila), (IIb), (IIc), (IId), (Ile), 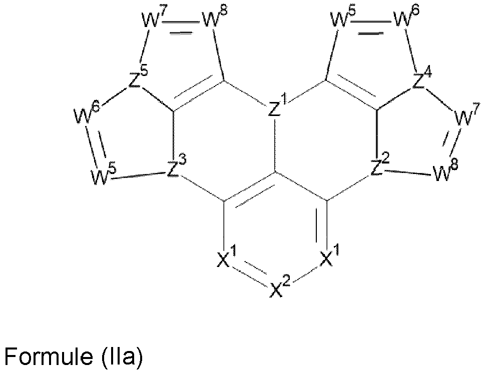 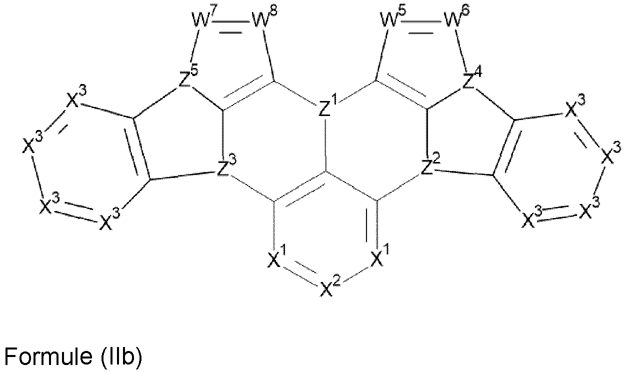 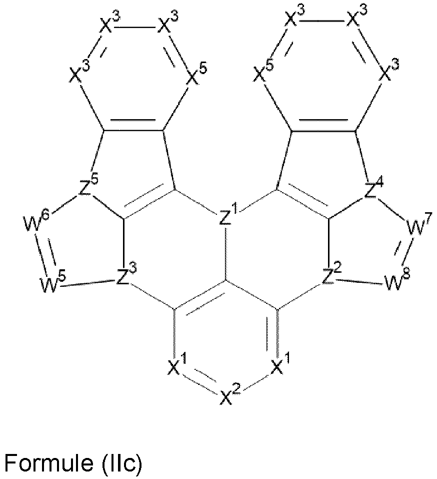 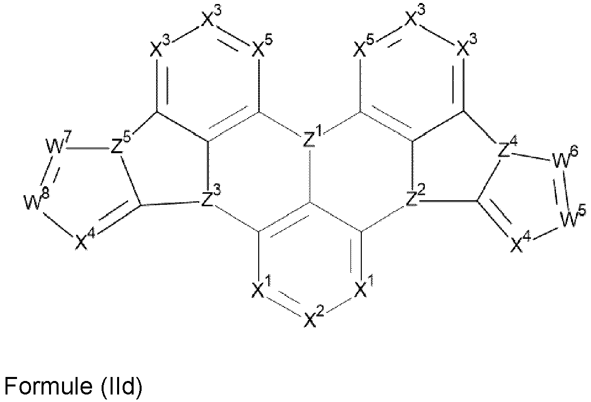 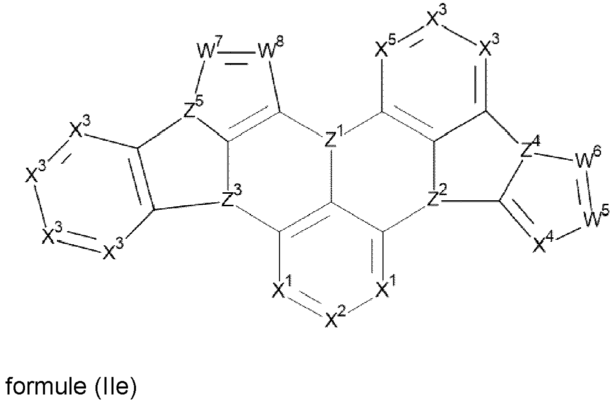 dans laquelle, pour les symboles et les indices utilisés, on a : Z¹ est B ;
Z², Z³, Z⁴, Z⁵ est à chaque occurrence identique ou différente N ou B ; dans lequel deux des groupes Z², Z³, Z⁴, Z⁵ sont N,
W⁵, W⁶, W⁷, W⁸, ou X⁶; identiques ou différents à chaque occurrence, représentent C(Ar)
Ar est, à chaque occurrence identique ou différente, un système cyclique aromatique ou hétéroaromatique comportant de 5 à 60 atomes dans le cycle aromatique, qui peut être substitué par un ou plusieurs radicaux R, le groupe Ar pouvant former un système cyclique avec au moins un groupe Ar, X¹, R ou un autre groupe ;
X¹ représente à chaque occurrence, de manière identique ou différente, N, CR^{a} ou C, si un système cyclique est formé par une liaison avec l'un des cycles Ar^{c}, Ar^{d} ou un autre groupe, à condition que pas plus de deux des groupes X¹, X² dans un cycle représentent N ;
X² représente à chaque occurrence, de manière identique ou différente, N ou CR^{b}, à condition que pas plus de deux des groupes X¹, X² dans un cycle représentent N ;
X³ est identique ou différent de N ou CR^{c} à chaque fois, à condition que pas plus de deux des groupes X³, X⁵ dans un cycle soient N
X⁴ est à chaque fois identique ou différent de N ou CR^{d}, à condition qu'il n'y ait pas plus de deux groupes X⁴, X⁶ dans un cycle pour N ;
X⁵ représente à chaque occurrence, de manière identique ou différente, N, CR^{e} ou C, si un système cyclique est formé par une liaison avec un groupe Ar ou un groupe X⁵ ou un groupe X⁶, à condition que pas plus de deux des groupes X³, X⁵ dans un cycle représentent N ;
X⁶ représente à chaque occurrence, de manière identique ou différente, N, CR^{f} ou C, si un système cyclique est formé par une liaison avec un groupe Ar, un groupe X⁵ ou un groupe X⁶, à condition que pas plus de deux des groupes X⁴, X⁶ dans un cycle représentent N ;
R, R^{a}, R^{b} est à chaque occurrence identique ou différente H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R', OSO₂Ar', OSO₂R¹, un groupe alkyle, alcoxy ou thioalcoxy à chaîne droite ayant de 1 à 40 atomes de carbone ou un groupe alcényle ou alcynyle ayant de 2 à 40 atomes de carbone ou un groupe alkyle, alcoxy ou thioalcoxy ramifié ou cyclique ayant de 3 à 20 atomes de carbone, dans lequel le groupe alkyle, alcoxy ou thioalcoxy a de 3 à 20 atomes de carbone, alcoxy, thioalcoxy, alcényle ou alcynyle peuvent chacun être substitués par un ou plusieurs radicaux R¹, dans laquelle un ou plusieurs groupes CH₂ non adjacents peuvent être remplacés par R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO ou SO₂, ou un système cyclique aromatique ou hétéroaromatique ayant de 5 à 60 atomes cycliques aromatiques, qui peuvent être substitués chacun par un ou plusieurs radicaux R¹, ou un groupe aryloxy ou hétéroaryloxy ayant de 5 à 60 atomes cycliques aromatiques, qui peuvent être substitués par un ou plusieurs radicaux R¹; deux radicaux R, R^{a}, R^{b} peuvent également former un système cyclique entre eux ou avec un autre groupe ;
R^{c}, R^{d}, R^{e}, R^{f} est à chaque occurrence identique ou différente H, D, OH, F, Cl, Br, I, CN, NO₂, N(Ar')₂, N(R¹)₂, C(=O)OAr', C(=O)OR¹, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, un groupe alkyle, alcoxy ou thioalcoxy à chaîne droite ayant de 1 à 40 atomes de carbone ou un groupe alcényle ou alcynyle ayant de 2 à 40 atomes de carbone ou un groupe alkyle, alcoxy ou thioalcoxy ramifié ou cyclique ayant de 3 à 20 atomes de carbone, dans lequel le groupe alkyle, alcoxy ou thioalcoxy a de 3 à 20 atomes de carbone, alcoxy, thioalcoxy, alcényle ou alcynyle peuvent chacun être substitués par un ou plusieurs radicaux R¹, dans laquelle un ou plusieurs groupes CH₂ non adjacents peuvent être remplacés par R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO ou SO₂, ou un système cyclique aromatique ou hétéroaromatique ayant de 5 à 60 atomes cycliques aromatiques, dont chacun peut être substitué par un ou plusieurs radicaux R¹, ou un groupe aryloxy ou hétéroaryloxy ayant de 5 à 60 atomes cycliques aromatiques, qui peut être substitué par un ou plusieurs radicaux R¹; deux radicaux R^{c}, R^{d}, R^{e}, R^{f} peuvent également former un système cyclique entre eux ou avec un autre groupe ;
Ar' est à chaque fois identique ou différent un système cyclique aromatique ou hétéroaromatique avec 5 à 60 atomes cycliques aromatiques, qui peut être substitué par un ou plusieurs radicaux R¹, deux radicaux Ar', qui se lient au même atome de C, de Si, de N, de P ou de B, peuvent également être reliés par une simple liaison ou un pont, choisi parmi B(R¹), C(R¹)₂, Si(R¹)₂, C=O, C=NR¹, C=C(R¹)₂, O, S, S=O, SO₂, N(R¹), P(R¹) et P(=O)R¹, sont pontés entre eux ;
R¹ est à chaque occurrence identique ou différente H, D, F, Cl, Br, I, CN, NO₂, N(Ar")₂, N(R²)₂, C(=O)OAr", C(=O)OR², C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, un groupe alkyle, alcoxy ou thioalcoxy à chaîne droite ayant de 1 à 40 atomes de carbone ou un groupe alkyle, alcoxy ou thioalcoxy à chaîne ramifiée ou cyclique, alcoxy ou thioalcoxy ayant de 3 à 40 atomes de carbone ou un groupe alcényle ayant de 2 à 40 atomes de carbone, dont chacun peut être substitué par un ou plusieurs radicaux R², un ou plusieurs groupes CH₂ non adjacents pouvant être remplacés par-R²C=CR²-, -C≡C-, Si(R²)₂, C=O, C=S, C=Se, C=NR², -C(=O)O-, -C(=O)NR²-, NR², P(=O)(R²), -O-, -S-, SO ou SO₂ et dans lequel un ou plusieurs atomes de H peuvent être remplacés par D, F, Cl, Br, I, CN ou NO₂, ou un système cyclique aromatique ou hétéroaromatique comportant 5 à 60 atomes cycliques aromatiques, dont chacun peut être substitué par un ou plusieurs radicaux R², ou un groupe aryloxy ou hétéroaryloxy ayant de 5 à 60 atomes de cycle aromatique, qui peut être substitué par un ou plusieurs radicaux R², ou un groupe aralkyle ou hétéroaralkyle ayant de 5 à 60 atomes de cycle aromatique, qui peut être substitué par un ou plusieurs radicaux R², ou une combinaison de ces systèmes ; où deux ou plusieurs radicaux R¹ peuvent former ensemble un système cyclique, où un ou plusieurs radicaux R¹ peuvent former un système cyclique avec une autre partie du composé ;
Ar" est à chaque fois identique ou différent un système cyclique aromatique ou hétéroaromatique avec 5 à 30 atomes cycliques aromatiques, qui peut être substitué par un ou plusieurs radicaux R², deux radicaux Ar", qui se lient au même atome de C, de Si, de N, de P ou de B, peuvent également être reliés par une simple liaison ou un pont, choisi parmi B(R²), C(R²)₂, Si(R²)₂, C=O, C=NR², C=C(R²)₂, O, S, S=O, SO₂, N(R²), P(R²) et P(=O)R², sont pontés entre eux ;
R² est choisi à chaque occurrence, de manière identique ou différente, dans le groupe constitué par H, D, F, CN, un radical hydrocarboné aliphatique comportant de 1 à 20 atomes de carbone ou un système cyclique aromatique ou hétéroaromatique comportant de 5 à 30 atomes cycliques aromatiques, dans lequel un ou plusieurs atomes de H peuvent être remplacés par D, F, Cl, Br, I ou CN et qui peut être substitué par un ou plusieurs groupes alkyle ayant chacun 1 à 4 atomes de carbone, deux ou plusieurs substituants R² pouvant former ensemble un système cyclique.

2. Composé selon la revendication 1, comprenant au moins une structure de formule (III-1) à (III-25), 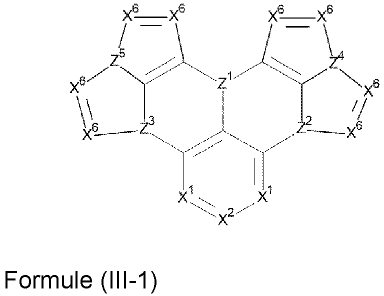 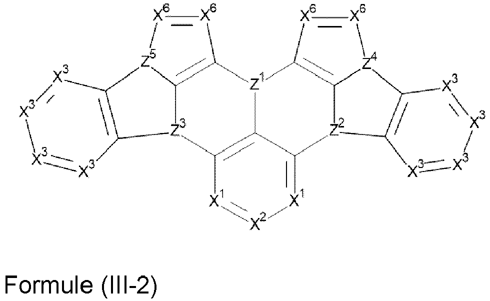 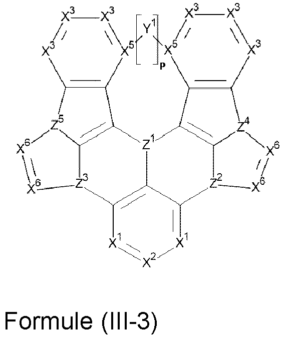 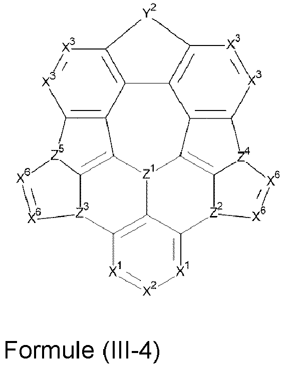 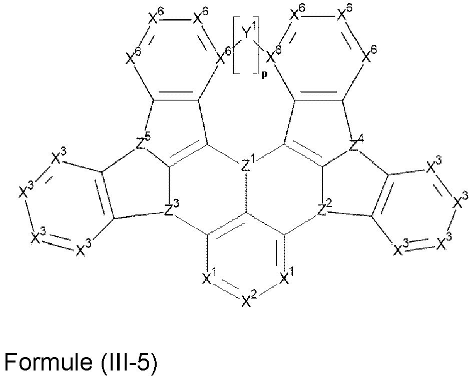 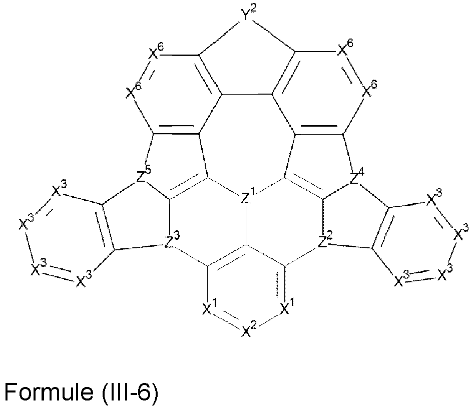 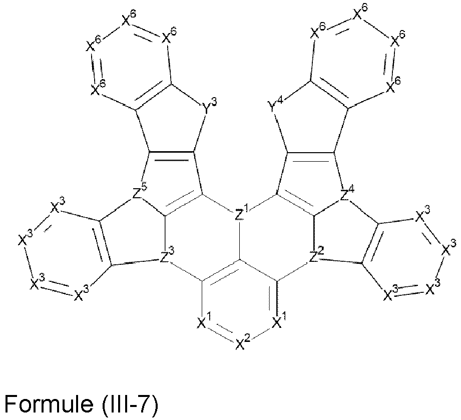 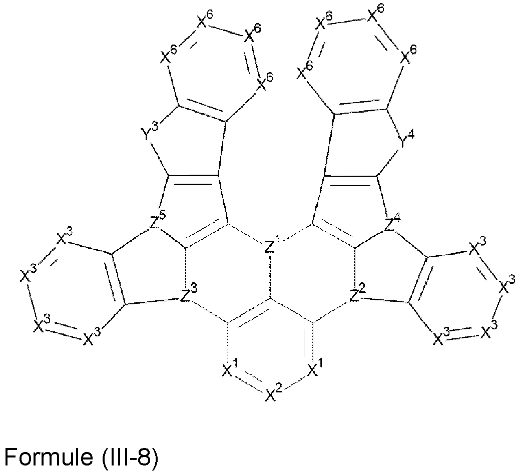 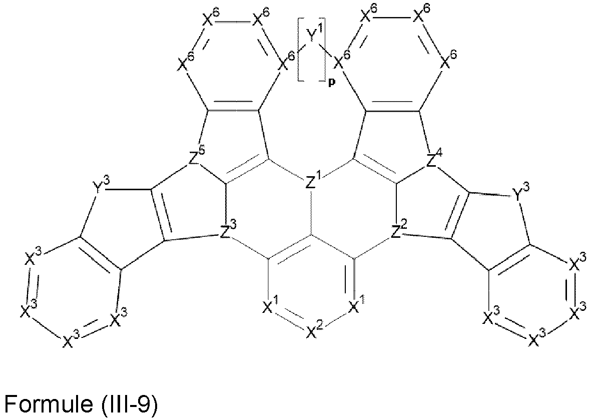 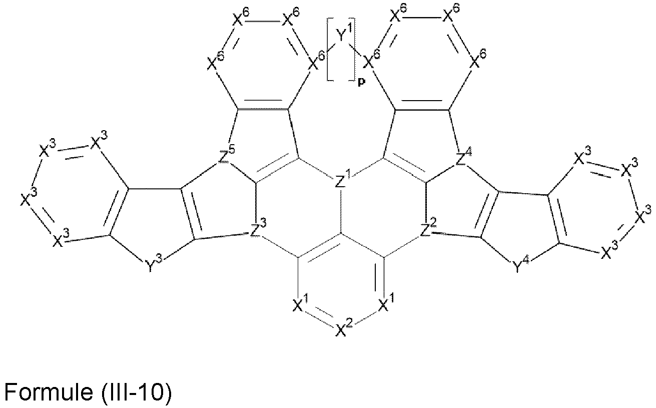 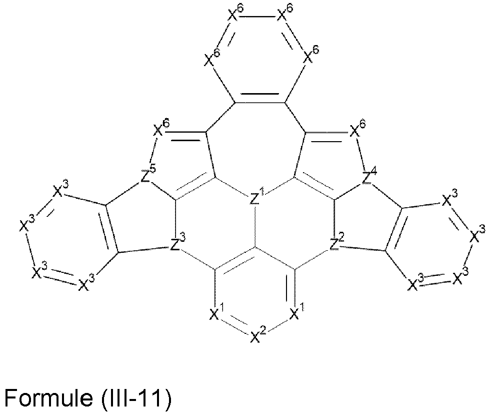 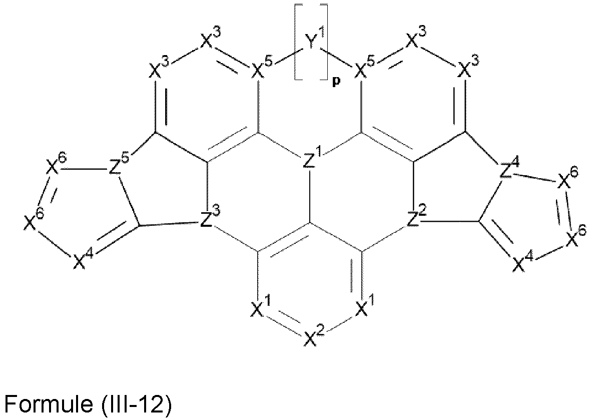 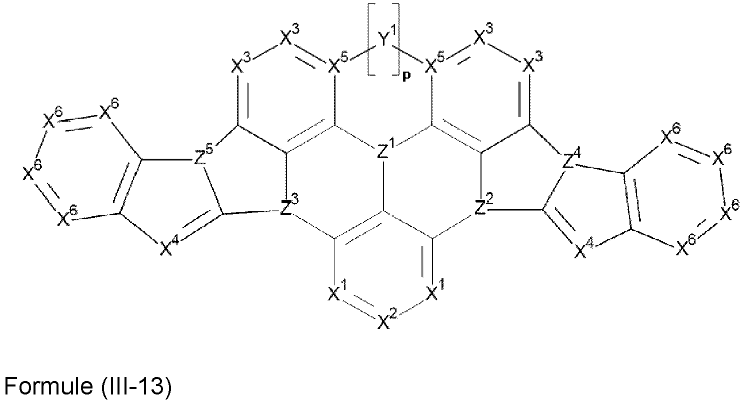 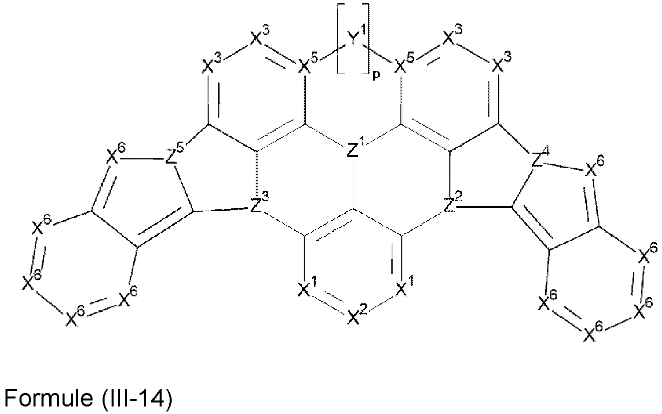 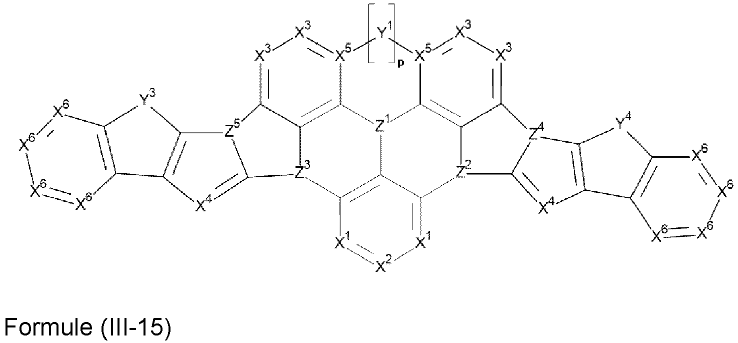 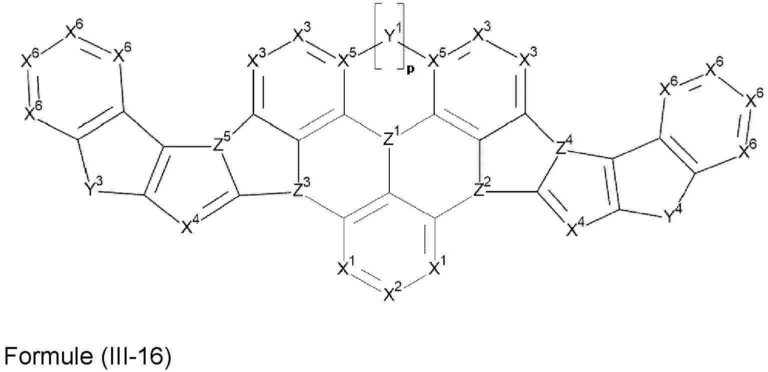 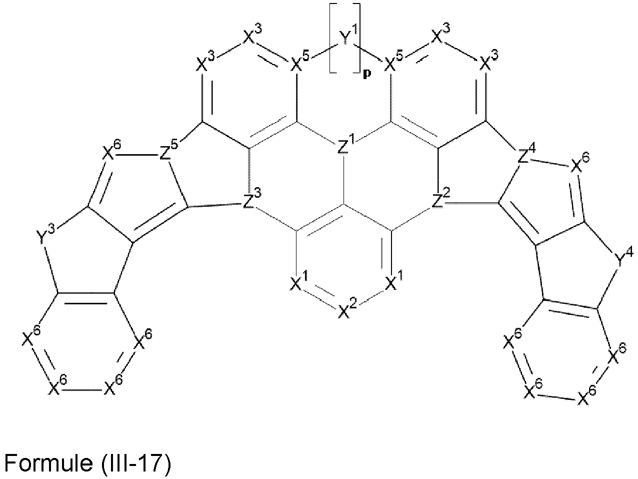 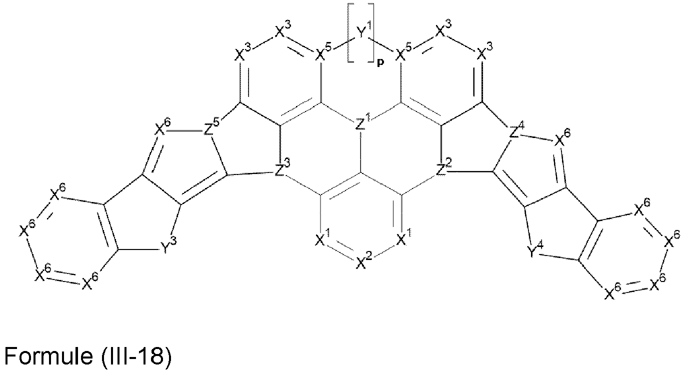 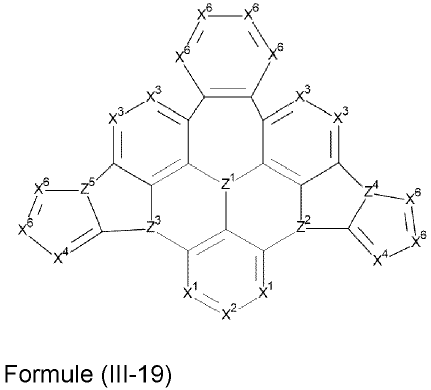 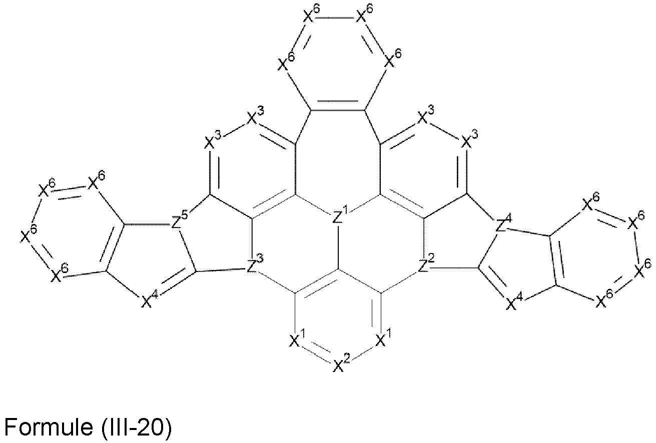 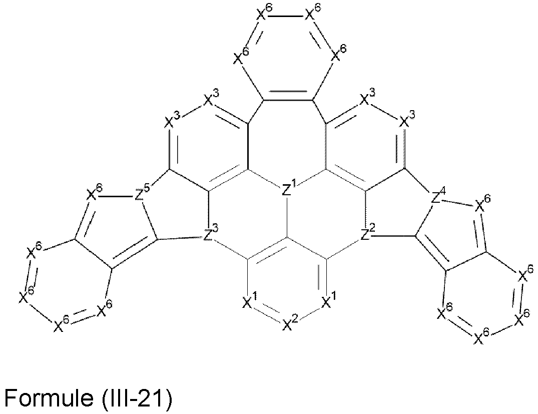 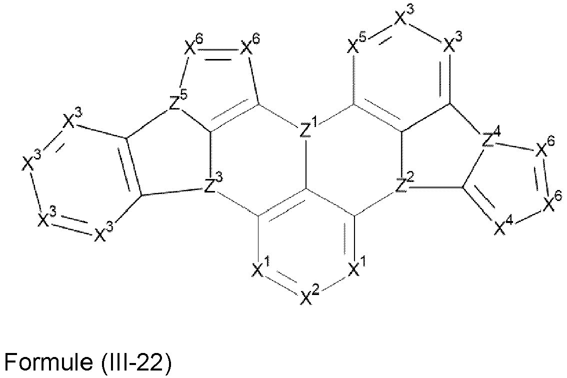 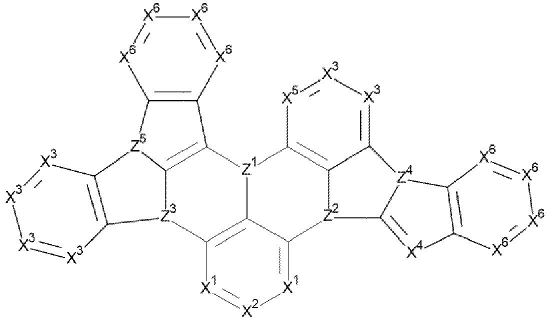 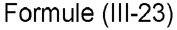 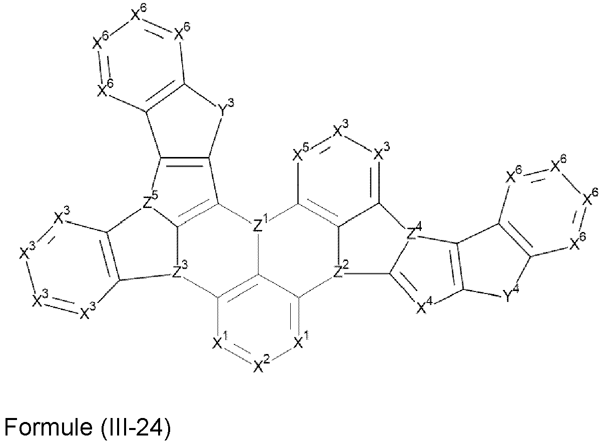 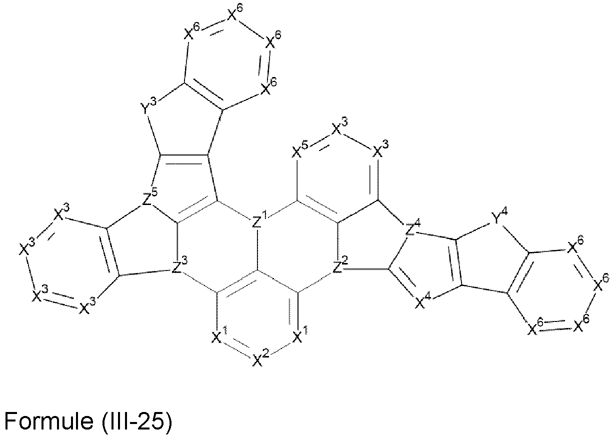 dans laquelle les symboles Z¹, Z², Z³, X¹, X², Z⁴, Z⁵, X³, X⁴, X⁵ et X⁶ ont les significations données dans la revendication 1 et les autres symboles ont la signification suivante :
p est 0 ou 1,
Y¹ est à chaque occurrence identique ou différente une liaison, N(Ar'), N(R), P(Ar'), P(R), P(=O)Ar', P(=O)R, P(=S)Ar', P(=S)R, B(Ar'), B(R), AI(Ar'), AI(R), Ga(Ar'), Ga(R), C=O, C(R)₂, Si(R)₂, C=NR, C=NAr', C=C(R)₂, O, S, Se, S=O, ou SO₂, dans lesquels les symboles R et Ar' ont la signification donnée dans la revendication 1 ;
Y², Y³, Y⁴ est à chaque occurrence identique ou différente N(Ar'), N(R), P(Ar'), P(R), P(=O)Ar', P(=O)R, P(=S)Ar', P(=S)R, B(Ar'), B(R), AI(Ar'), AI(R), Ga(Ar'), Ga(R), C=O, C(R)₂, Si(R)₂, C=NR, C=NAr', C=C(R)₂, O, S, Se, S=O, ou SO₂, dans lesquels les symboles R et Ar' ont la signification donnée dans la revendication 1.

3. Composé selon la revendication 1 ou 2, comprenant au moins une structure de formule (IV-1) à (IV-38), 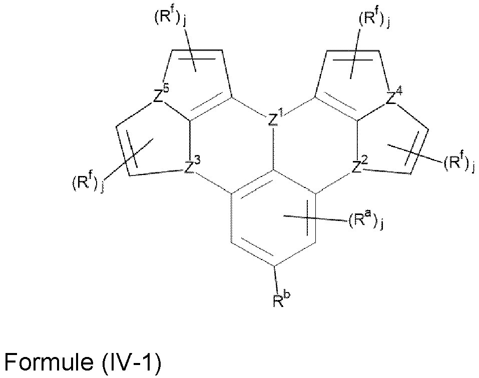 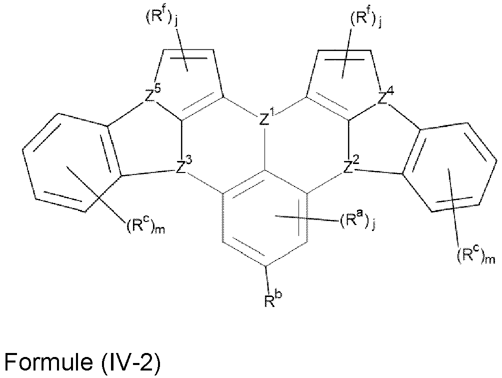 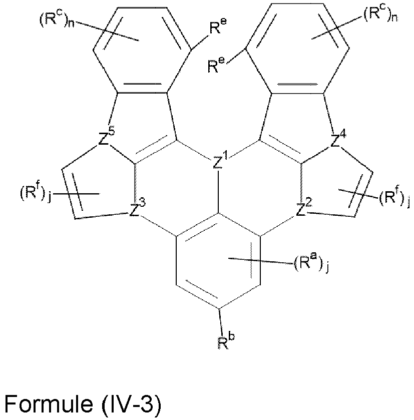 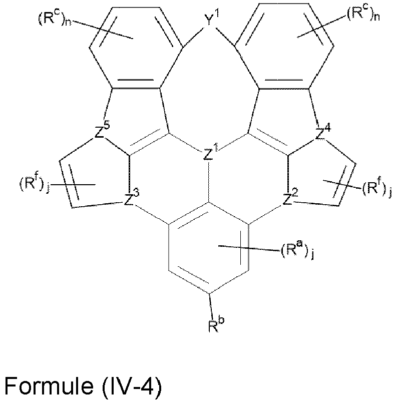 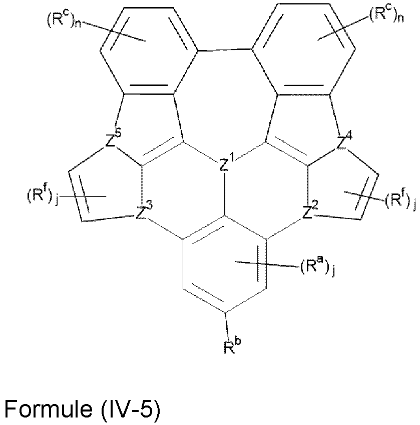 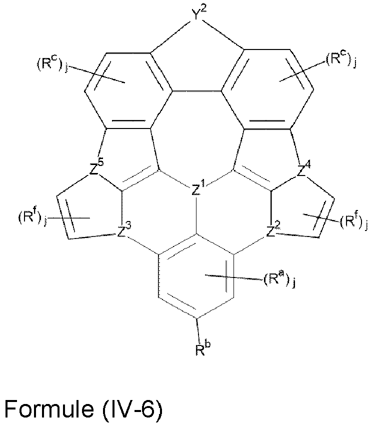 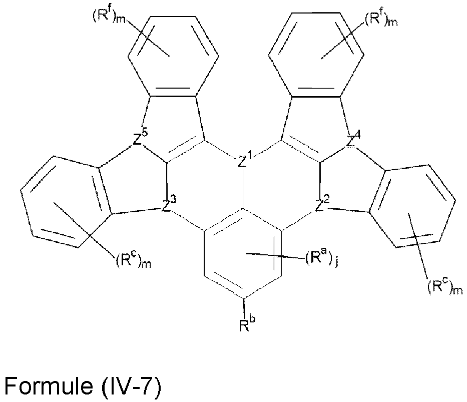 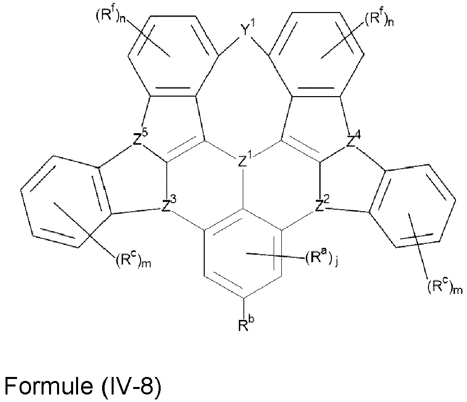 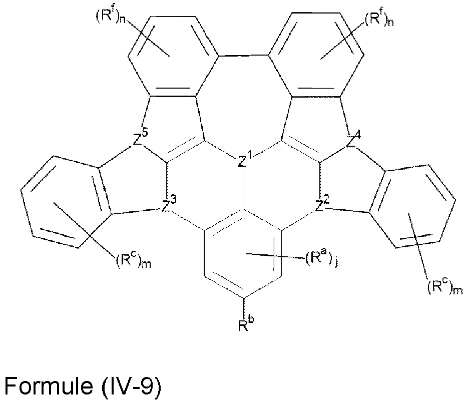 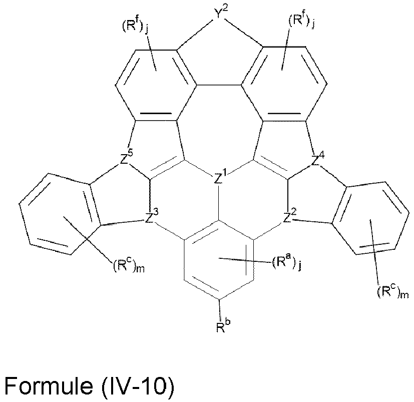 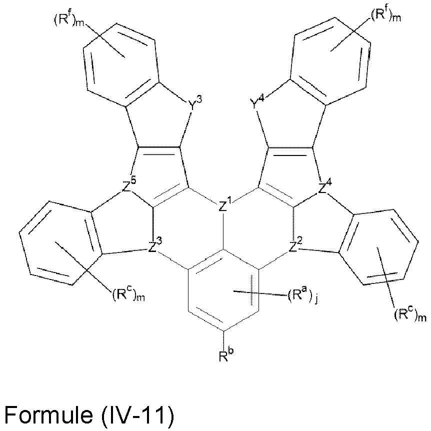 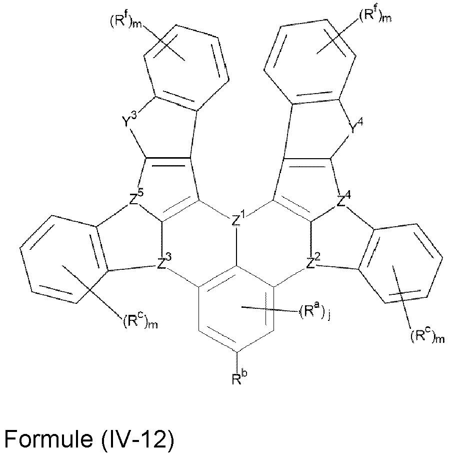 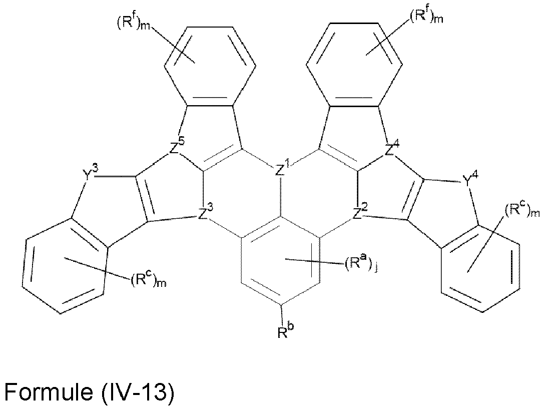 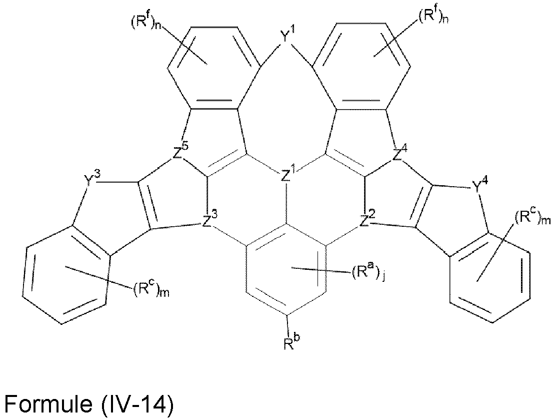 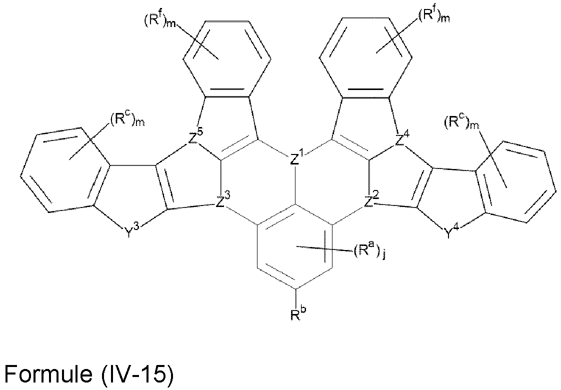 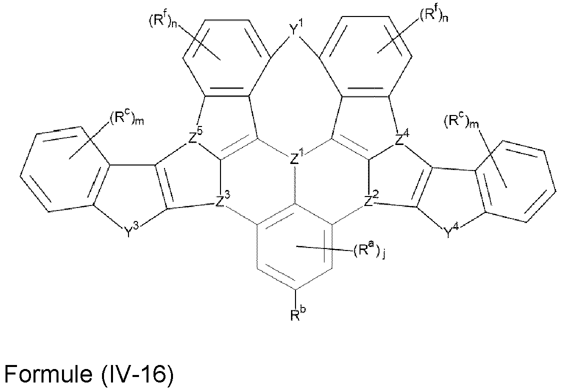 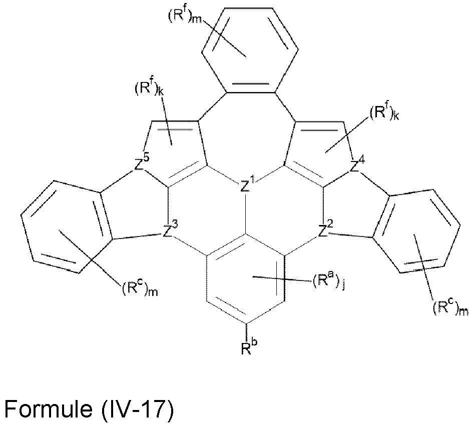 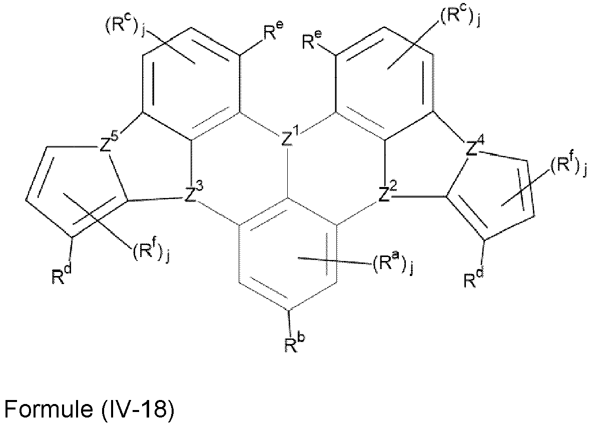 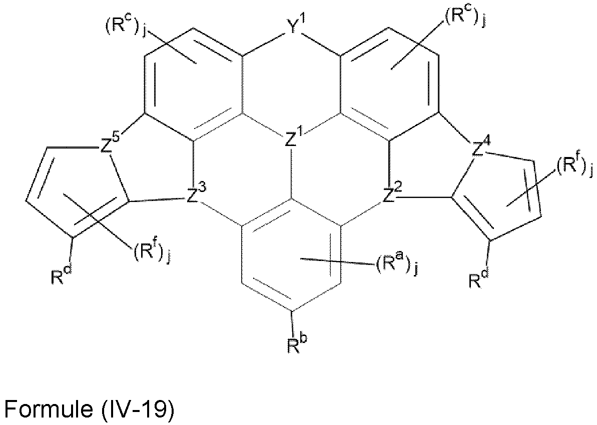 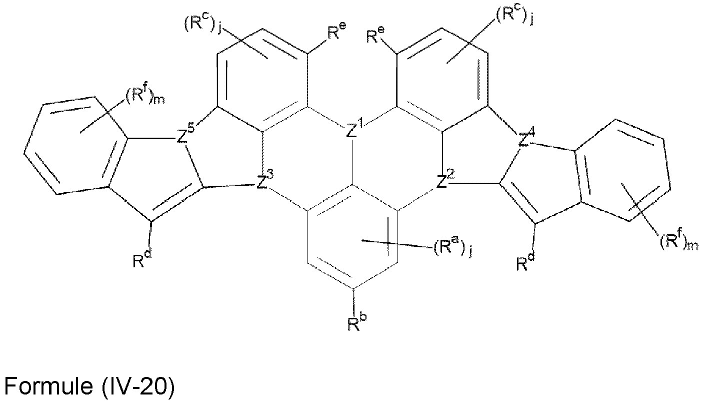 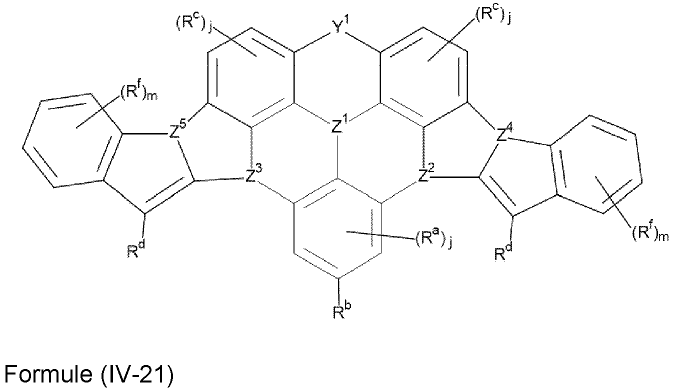 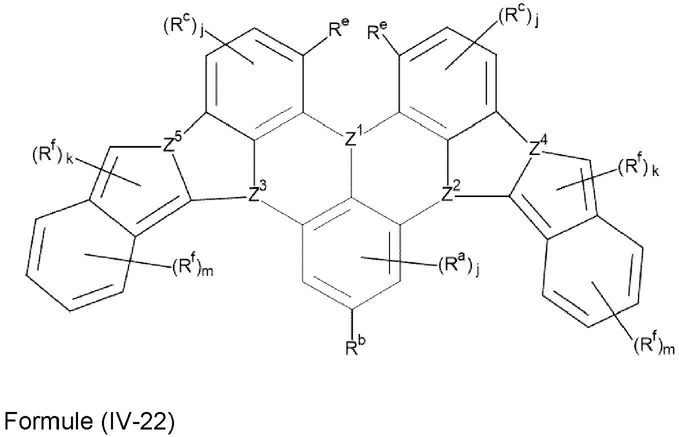 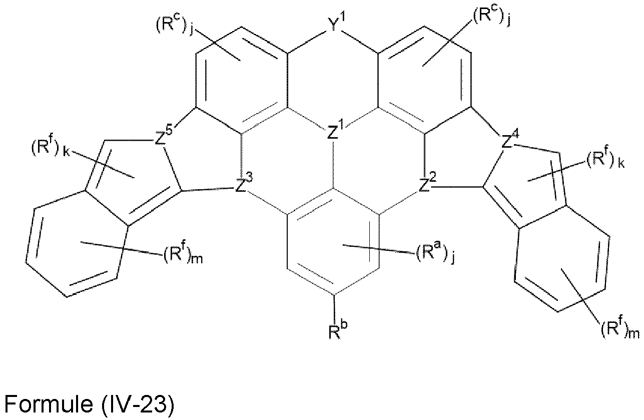 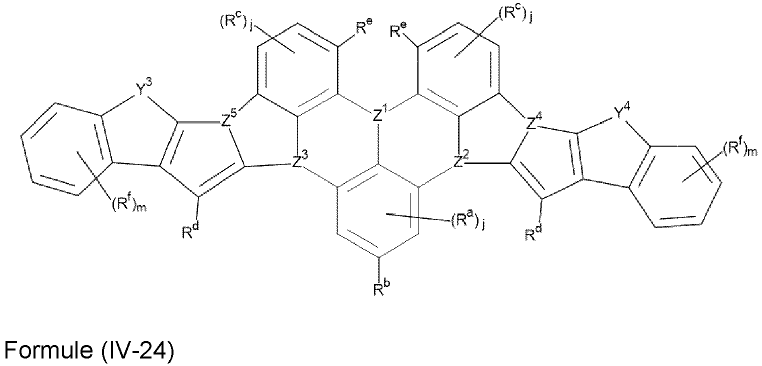 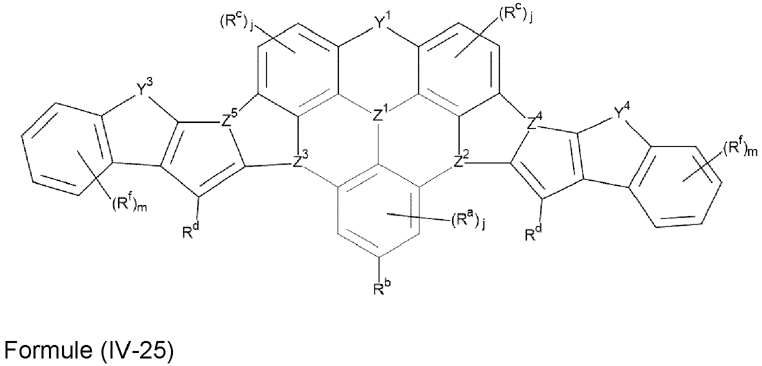 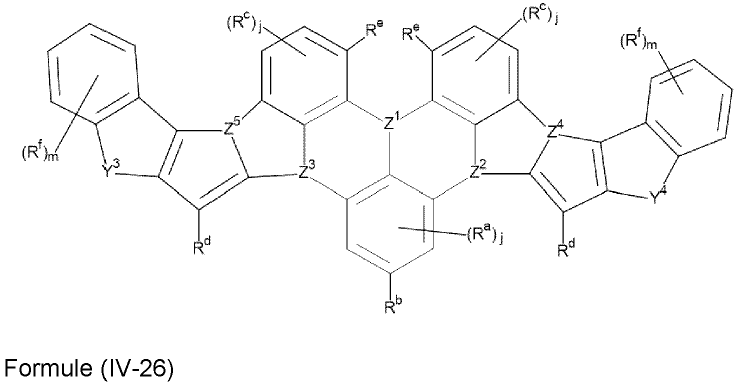 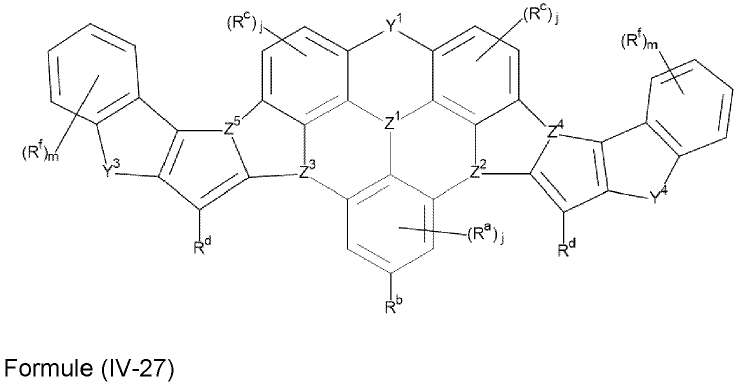 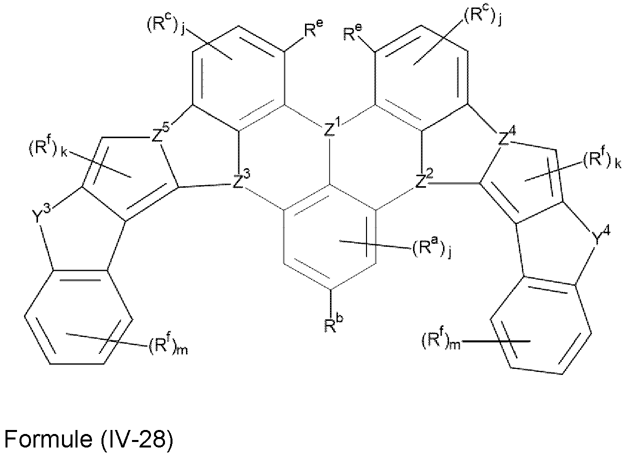 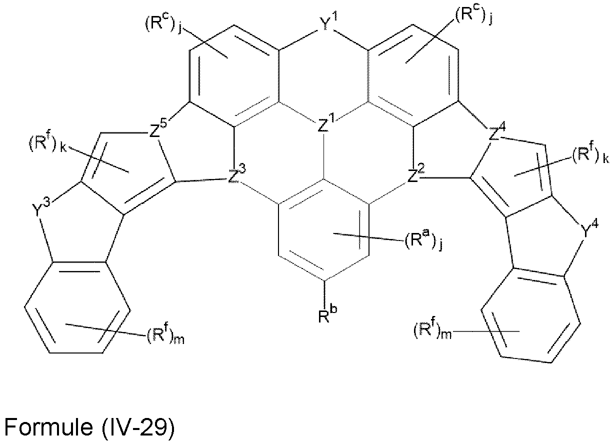 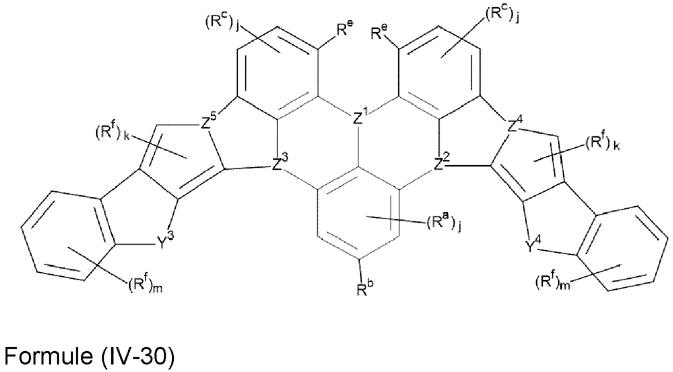 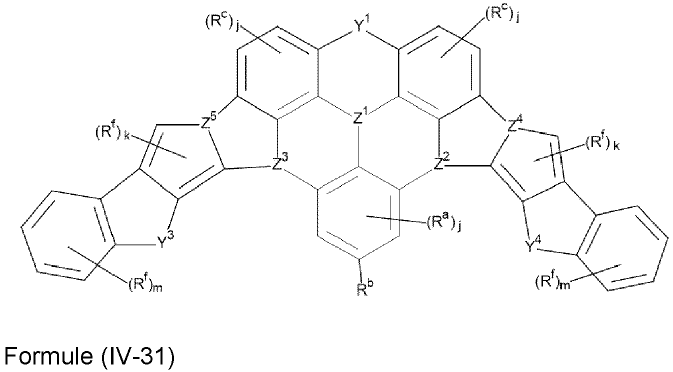 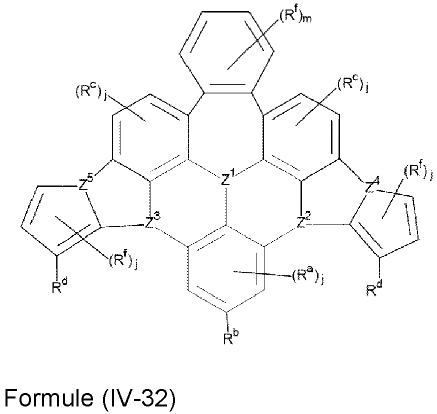 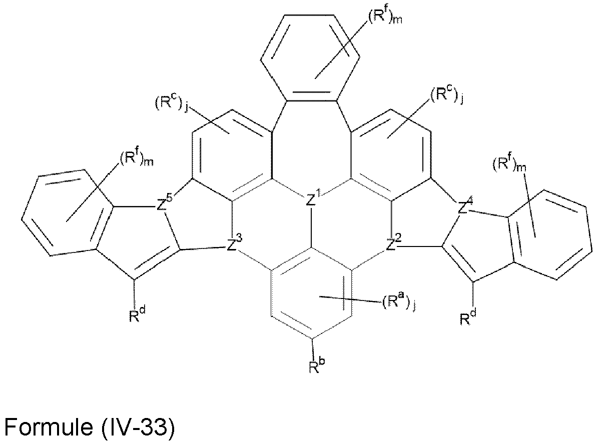 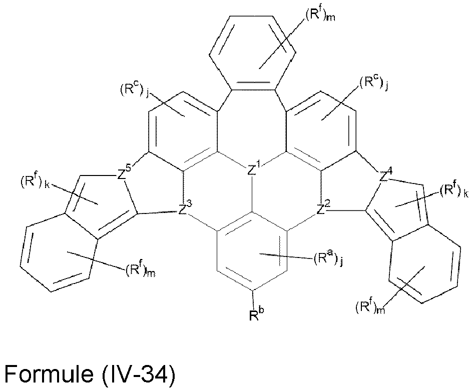 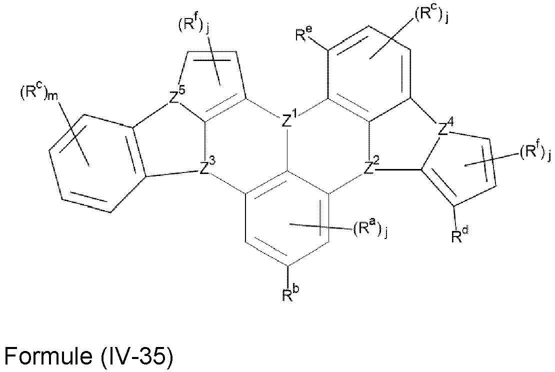 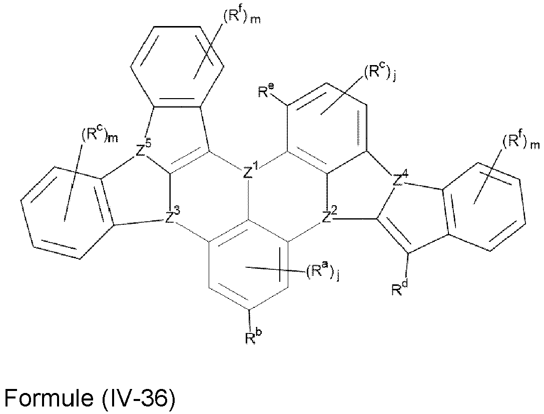 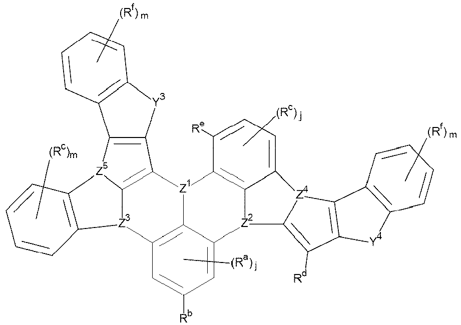 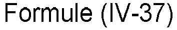 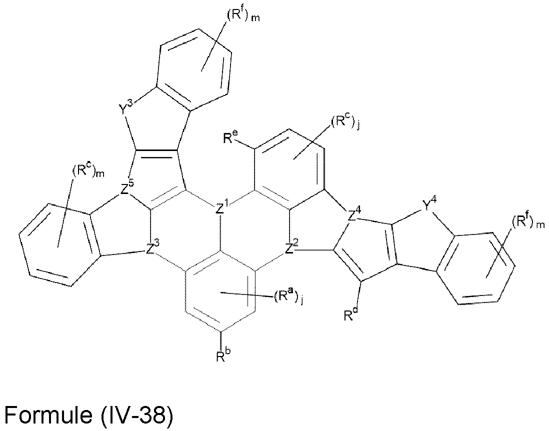 dans laquelle les symboles Z¹, Z², Z³, R^{a}, R^{b} Z⁴, Z⁵, R^{c}, R^{d}, R^{e} et R^{f} ont les significations données dans la revendication 1, les symboles Y¹, Y², Y³ et Y⁴ ont les significations données dans la revendication 2 et les autres symboles ont la signification suivante :
m est 0, 1, 2, 3 ou 4 ;
n est 0, 1, 2 ou 3 ;
j est 0, 1 ou 2 ;
k est 0 ou 1.

4. Composé selon une ou plusieurs des revendications 1 à 3, **caractérisé en ce qu'**au moins deux radicaux R, R^{a}, R^{b}, R^{c}, R^{d}, R^{e}, R^{f} forment un cycle avec les autres groupes auxquels les deux radicaux R, R^{a}, R^{b}, R^{c}, R^{d}, R^{e}, R^{f} forment un cycle condensé, dans lequel les deux radicaux R, R^{a}, R^{b}, R^{c}, R^{d}, R^{e}, R^{f} forment au moins une structure de formules (RA-1) à (RA-12) 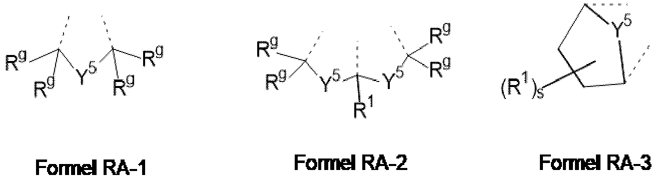 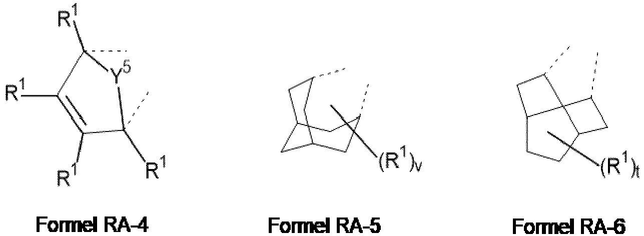 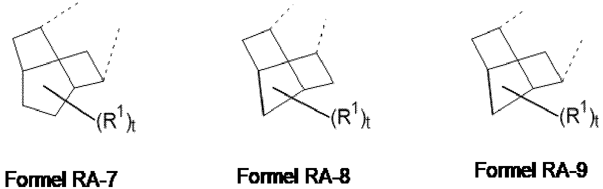 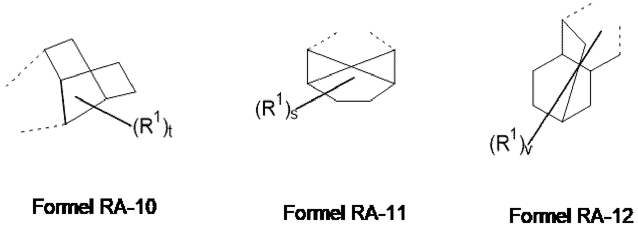 dans laquelle R¹ a la signification donnée précédemment, les liaisons en pointillés représentent les sites de liaison aux atomes des groupes auxquels se lient les deux radicaux R, R^{a}, R^{b}, R^{c}, R^{d}, R^{e}, R^{f}, et les autres symboles ont la signification suivante :
Y⁵ est à chaque occurrence identique ou différente C(R¹)₂, (R¹)₂C-C(R¹)₂, (R¹)C=C(R¹), NR¹, NAr', O ou S ;
R^{g} est à chaque occurrence identique ou différente F, un groupe alkyle, alcoxy ou thioalcoxy à chaîne droite de 1 à 40 atomes de carbone ou un groupe alcényle ou alcynyle de 2 à 40 atomes de carbone ou un groupe alkyle, alcoxy ou thioalcoxy ramifié ou cyclique de 1 à 40 atomes de carbone, alcoxy ou thioalcoxy ayant de 3 à 20 atomes de carbone, dans lequel le groupe alkyle, alcoxy, thioalcoxy, alcényle ou alcynyle peut être substitué dans chaque cas par un ou plusieurs radicaux R², dans lequel un ou plusieurs groupes CH₂ non adjacents peuvent être remplacés par R²C=CR², C=C, Si(R²)₂, C=O, C=S, C=Se, C=NR², -C(=O)O-, -C(=O)NR²-, NR², P(=O)(R¹), -O-, -S-, SO ou SO₂, ou un système cyclique aromatique ou hétéroaromatique comportant 5 à 60 atomes cycliques aromatiques, dont chacun peut être substitué par un ou plusieurs radicaux R², ou un groupe aryloxy ou hétéroaryloxy ayant de 5 à 60 atomes dans le cycle aromatique, qui peut être substitué par un ou plusieurs radicaux R²; deux radicaux R^{g} peuvent également former un système cyclique entre eux ou un radical R^{g} avec un radical R¹ ou avec un autre groupe ;
s est égal à 0, 1, 2, 3, 4, 5 ou 6 ;
t est 0, 1, 2, 3, 4, 5, 6, 7 ou 8 ;
v est 0, 1, 2, 3, 4, 5, 6, 7, 8 ou 9.

5. Composé selon une ou plusieurs des revendications 1 à 4, en ce qu'au moins deux radicaux R, R^{a}, R^{b}, R^{c}, R^{d}, R^{e}, R^{f} forment avec les autres groupes auxquels les deux radicaux R, R^{a}, R^{b}, R^{c}, R^{d}, R^{e}, R^{f} forment un cycle condensé dans lequel les deux radicaux R, R^{a}, R^{b}, R^{c}, R^{d}, R^{e}, R^{f} forment des structures de formule (RB), 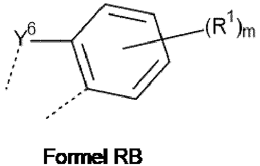 dans laquelle R¹ a la signification donnée dans la revendication 1, les liaisons en pointillés représentent les sites de liaison auxquels se lient les deux radicaux R, R^{a}, R^{b}, R^{c}, R^{d}, R^{e}, R^{f}, l'indice m est 0, 1, 2, 3 ou 4 et Y⁶ est C(R¹)₂, NR¹, NAr', BR¹, BAr¹, O ou S.

6. Composé selon une ou plusieurs des revendications 1 à 5, comprenant au moins une structure de formules (V-1) à (V-10), dans lequel lesdits composés comprennent au moins un cycle condensé, 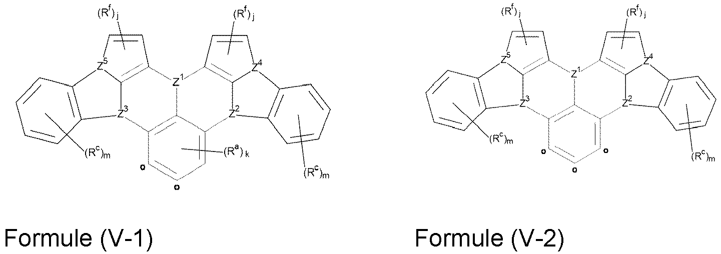 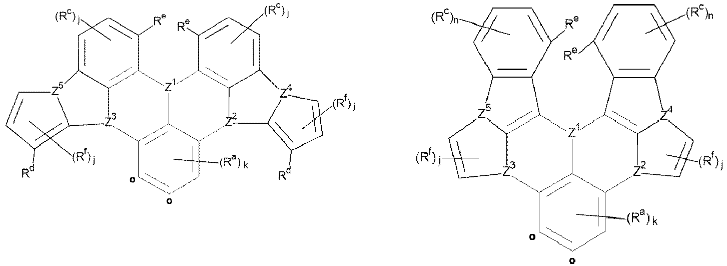 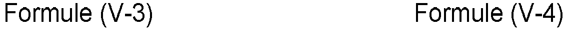 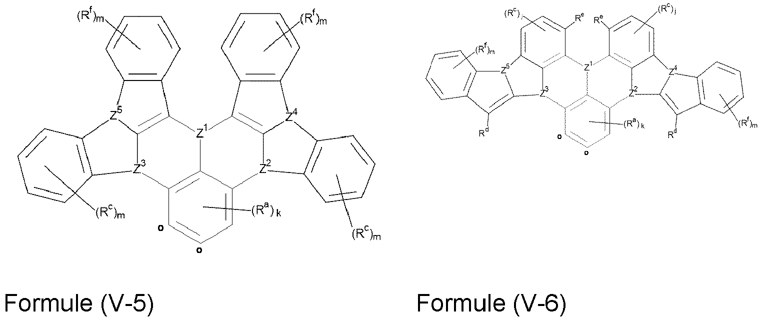 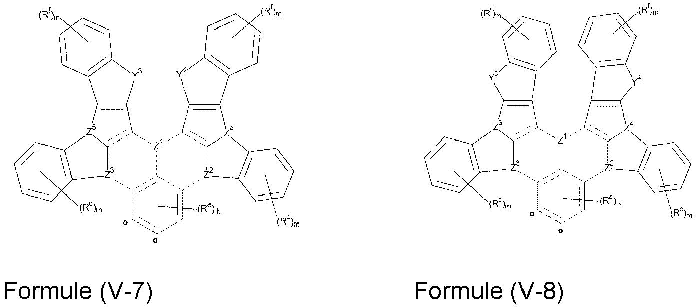 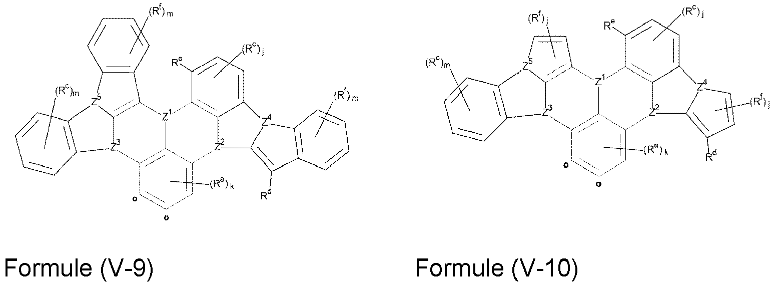 dans laquelle les symboles Z¹, Z², Z³, R^{a}, R^{b}, Z⁴, Z⁵, R^{c}, R^{d}, R^{e} et R^{f} ont les significations données dans la revendication 1, les symboles Y³ et Y⁴ ont les significations données dans la revendication 2, le symbole o représente les sites d'attachement et les autres symboles ont la signification suivante :
m est 0, 1, 2, 3 ou 4 ;
n est 0, 1, 2 ou 3 ;
j est 0, 1 ou 2.

7. Composé selon une ou plusieurs des revendications 1 à 6, comprenant au moins une structure de formules (VI-1) à (VI-9), dans lequel les composés ont au moins un cycle condensé. 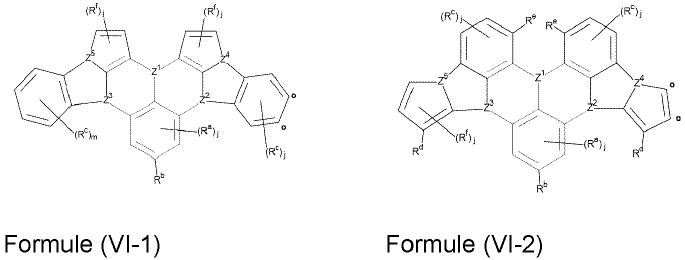 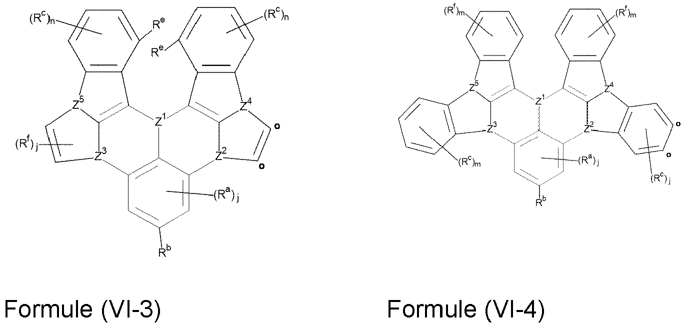 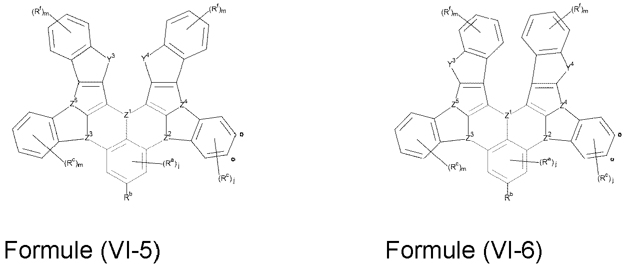 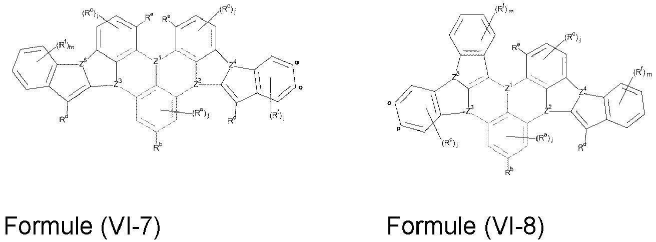 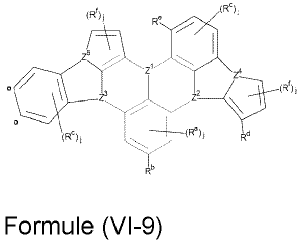 dans laquelle les symboles Z¹, Z², Z³, R^{a}, R^{b}, Z⁴, Z⁵, R^{c}, R^{d}, R^{e} et R^{f} ont les significations données dans la revendication 1, les symboles , les symboles Y³ et Y⁴ ont les significations données dans la revendication 2, le symbole o représente les sites d'attachement et les autres symboles ont la signification suivante :
m est 0, 1, 2, 3 ou 4 ;
n est 0, 1, 2 ou 3 ;
j est 0, 1 ou 2.

8. Composé selon une ou plusieurs des revendications 1 à 7, comprenant au moins une structure de formules (VII-1) à (VII-9), lesdits composés comprenant au moins un noyau condensé 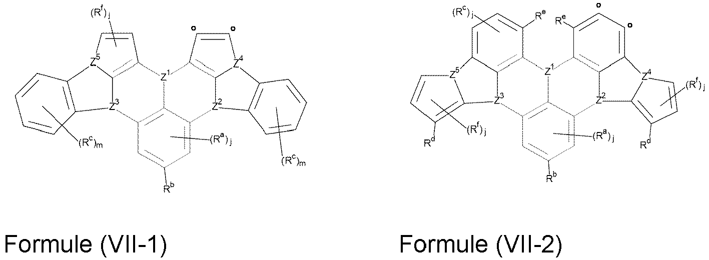 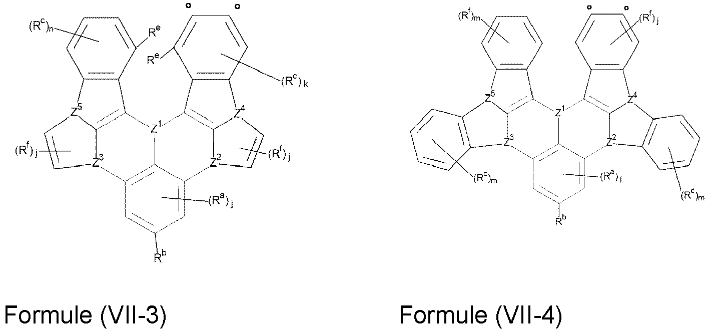 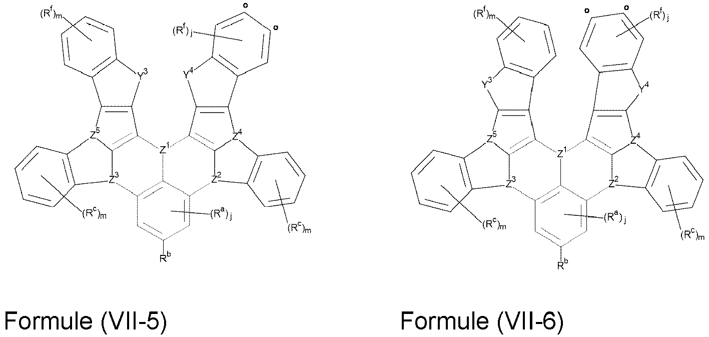 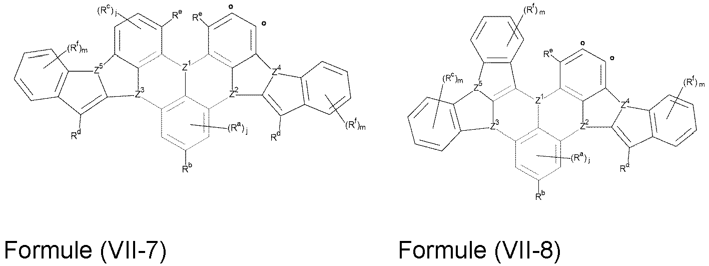 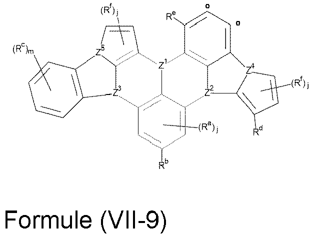 dans laquelle les symboles Z¹, Z², Z³, R^{a}, R^{b}, Z⁴, Z⁵, R^{c}, R^{d}, R^{e} et R^{f} ont les significations données dans la revendication 1, les symboles Y³ et Y⁴ ont les significations données dans la revendication 2, le symbole o représente les sites d'attachement et les autres symboles ont la signification suivante :
m est 0, 1, 2, 3 ou 4 ;
n est 0, 1, 2 ou 3 ;
j est 0, 1 ou 2.

9. Oligomère, polymère ou dendrimère contenant un ou plusieurs composés selon l'une quelconque des revendications 1 à 8, dans lequel, au lieu d'un atome d'hydrogène ou d'un substituant, une ou plusieurs liaisons des composés sont présentes avec le polymère, l'oligomère ou le dendrimère.

10. Formulation contenant au moins un composé selon l'une ou plusieurs des revendications 1 à 8 ou un oligomère, polymère ou dendrimère selon la revendication 9 et au moins un autre composé, dans laquelle l'autre composé est de préférence choisi parmi un ou plusieurs solvants.

11. Composition contenant au moins un composé selon une ou plusieurs des revendications 1 à 8 ou un oligomère, polymère ou dendrimère selon la revendication 9 et au moins un autre composé choisi dans le groupe constitué par les émetteurs fluorescents, les émetteurs phosphorescents, les émetteurs présentant un TADF, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous.

12. Procédé de préparation d'un composé selon une ou plusieurs des revendications 1 à 8, **caractérisé en ce qu'**on synthétise un squelette comportant au moins l'un des groupes Z², Z³ ou un précurseur de l'un des groupes Z², Z³ et on introduit le groupe Z¹ par une réaction de métallation, une réaction de substitution aromatique nucléophile ou une réaction de couplage.

13. Utilisation dans un dispositif électronique d'un composé selon une ou plusieurs des revendications 1 à 8, ou d'un oligomère, polymère ou dendrimère selon la revendication 9.

14. Dispositif électronique contenant au moins un composé selon l'une ou plusieurs des revendications 1 à 8 ou un oligomère, polymère ou dendrimère selon la revendication 9.
